# NEUE EUROPÄISCHE PATENTSCHRIFT

(11) **EP 0 353 640 B2**
(45) Veröffentlichungstag und Bekanntmachung des Hinweises auf die Entscheidung über den Einspruch: **15.10.2003**
(45) Hinweis auf die Patenterteilung: 12.04.1995
(21) Anmeldenummer: 89113916.4
(22) Anmeldetag: 28.07.1989
(51) Int. Cl.: C07D 239/52, A01N 43/54, C07D 403/12, C07D 413/12, C07D 409/12, C07D 401/12, C07D 405/12, C07D 491/04, C07D 249/08, C07D 473/26, C07D 239/70, C07D 239/28, C07D 251/16, C07D 251/22, C07D 251/24, C07D 239/26, C07D 475/00, A01N 43/66, A01N 43/90

(54) **Heterocyclische N-Acylsulfonamide, Verfahren zu ihrer Herstellung, sie enthaltende Mittel und ihre Verwendung als Herbizide oder Wachstumsregulatoren**
Heterocyclic N-acylsulphonamides, process for their preparation, agents containing them and their use as herbicides and plant growth regulators
N-acylsulfonamides hétérocycliques, procédé pour leur préparation, agents les contenant et leur utilisation comme herbicides régulateurs de croissance des plantes

(30) Priorität: 02.08.1988 DE 3826230
(43) Veröffentlichungstag der Anmeldung: 07.02.1990
(73) Patentinhaber: Bayer CropScience GmbH, 65929 Frankfurt/Main (DE)
(72) Erfinder: Ort, Oswald, Dr., D-6233 Kelkheim (Taunus) (DE); Willms, Lotha, Dr., D-5416 Hillscheid (DE); Bauer, Klaus, Dr., D-5450 Hanau (DE); Bieringer, Hermann, Dr., D-6239 Eppstein/Taunus (DE); Schulz, Arno, Dr., D-6234 Hattersheim am Main (DE)

(56) Entgegenhaltungen:
- EP-A- 0 255 047
- DE-A- 1 816 378
- Brown D.J, The Pyrimidines, Suppl. 1, 1970, Wiley Interscience
- J Chem Soc, 1966, pp. 2031-8
- Chem Ber, 1969, 101, pp. 2426-34
- Hermann et al, 1981, Leibigs Ann Chem
- Yamanaka et al, 1958, Chem Pharm Bull
- Kober, 1961, J Org Chem
- Geerts et al, 1976, Org Meganetic Resonanz
- Sekiya et al, 1982, Eur J Med Chem Ther
- Sekiya et al, 1983, Chem Pharm BUII
- Chemistry and Biology of Pteridines, 1954
- Hara et al, 1982, J Heterocycl Chem
- Daly and Christensen, 1965, J Am Chem Soc

## Beschreibung

Es ist bereits bekannt, daß bestimmte sulfonylierte bi- oder tricyclische heteroaromatische Carbonsäureamide herbizide und wachstumsregulatorische Eigenschaften besitzen (EP-A-244 166). Sulfonylierte monocyclische Pyridincarbonsäureamide werden als Fungizide und Mikrobiozide in der Landwirtschaft beschrieben (Chem. Abstr. 108 (19): 167298p; Chem. Abstr. 108 (15): 131590p). Eine Herbizid-Wirkung dieser Verbindungen ist nicht bekannt geworden.

Es wurde gefunden, daß heterocyclische N-Acylsulfonamide vorteilhafte herbizide und wachstumsregulatorische Eigenschaften besitzen.

Gegenstand der vorliegenden Erfindung sind daher Verbindungen der Formel I oder deren Salze 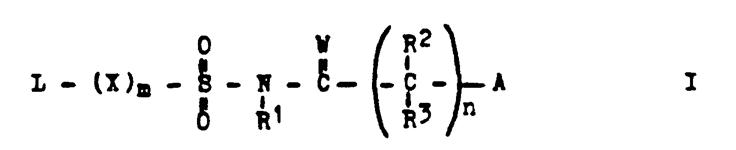 worin
- R¹: Wasserstoff, (C₁-C₄)Alkyl, (C₂-C₆)-Alkenyl oder (C₂-C₆)-Alkinyl;
- R²,R³: unabhängig voneinander Wasserstoff, (C₁-C₃)-Alkyl oder Phenyl;
- W: O, S, NR⁴ oder NOR⁴,
- R⁴: Wasserstoff, (C₁-C₃)-Alkyl, (C₁-C₃)-Haloalkyl oder Phenyl;
- X: CHR², O, NR⁴ oder NOR⁴,
- L: einen hetero- oder isocyclischen Rest der Formeln (L1) - (L5), 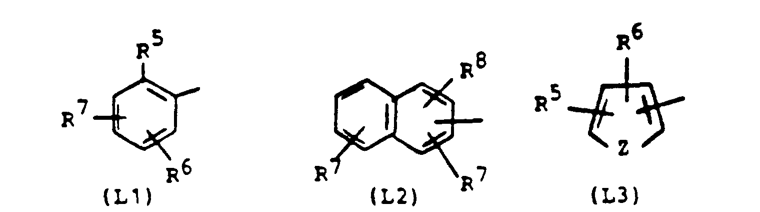 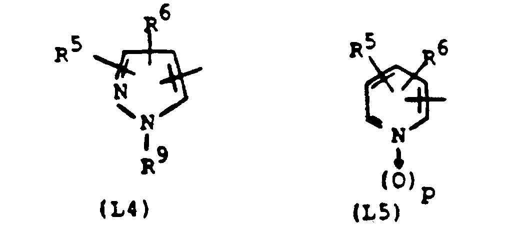
- A: einen heterocyclischen Rest der Formeln (A1) - (A8) 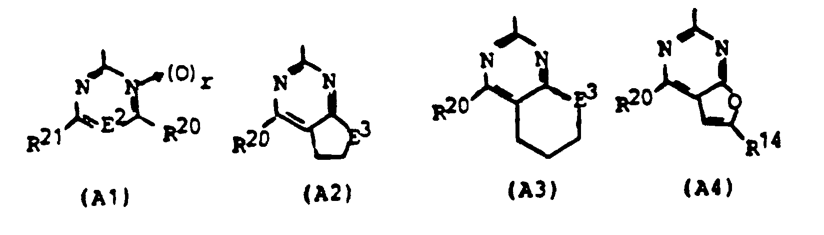 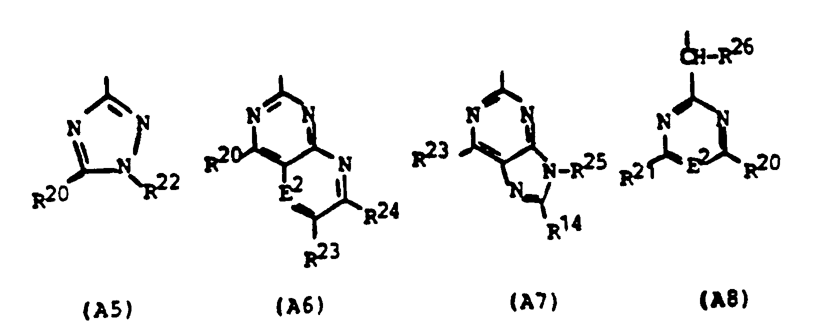
- Z: O oder S(O)_{q} ,
- E¹: O oder S(O)_{b} ,
- E²: CH oder N ,
- E³: O oder CH₂ ,
- a;m;n;p;r;s: unabhängig voneinander 0 oder 1,
- b;q: unabhängig voneinander 0, 1 oder 2,
- R⁵: Wasserstoff, Halogen, NO₂, CN, (C₁-C₄)-Alkyl, das unsubstituiert oder ein- oder mehrfach durch F, Cl, Br, oder einfach durch CN, OCH₃ oder SCH₃ substituiert ist, (C₂-C₄)-Alkenyl, das unsubstituiert oder ein- oder mehrfach durch F, Cl, Br oder einfach durch OCH₃ substituiert ist; (C₂-C₄)-Alkinyl, das unsubstituiert oder ein- oder mehrfach durch F, Cl, Br oder einfach durch OCH₃ oder Si(CH₃)₃ substituiert ist; (C₃-C₆)-Cycloalkyl, das unsubstituiert oder ein- oder mehrfach durch F, Cl oder CH₃ substituiert ist; -C(O)R¹⁰, -OCH₂CH₂OR¹⁰, -OH, -C(R¹⁰)(OR¹¹)(OR¹²); -CO₂R¹³, -C(O)NR¹⁴R¹⁵, -N₃, -SO₂NR¹⁴R¹⁵, -SO₃R¹⁶, -OSO₂R¹⁷, Phenyl, das unsubstituiert oder ein- oder mehrfach durch F, Cl, Br, CH₃ oder OCH₃ substituiert ist; -E¹R¹⁸ oder -(CH₂)ₛG,
- R⁶: Wasserstoff, Halogen; CN; NO₂; (C₁-C₄)-Alkyl, das unsubstituiert oder ein- oder mehrfach durch Halogen oder ein- bis zweifach durch -CO₂R¹³, -SO₂NR¹⁴R¹⁵, -NR¹⁴R¹⁵,(C₁-C₂)-Alkoxy, -E¹R¹⁹, (C₁-C₂)-Haloalkoxy, (C₁-C₂)-Alkylthio, (C₁-C₂)-Haloalkylthio, CN, OH oder SH substituiert ist; -CO₂R¹³, -SO₂NR¹⁴R¹⁵, -NR¹⁴R¹⁵ oder -E¹R¹⁹;
- R⁷: unabhängig voneinander Wasserstoff, (C₁-C₄)-Alkyl, (C₁-C₄)-Haloalkyl, (C₂-C₄)-Alkenyl, Phenyl oder Phenyl, das ein- oder mehrfach durch Halogen oder -E¹R¹⁹ substituiert ist; -E¹R¹⁹ oder Halogen,
- R⁸: Wasserstoff, (C₁-C₄)-Alkyl, (C₁-C₄)-Haloalkyl, Halogen, -CO₂R¹³, -SO₂NR¹⁴R¹⁵, -OSO₂R¹⁷, -S(O)_{b}R¹⁸, CN oder NO₂,
- R⁹: Wasserstoff, (C₁-C₄)-Alkyl, das unsubstituiert oder ein- oder mehrfach durch Halogen oder einfach durch Phenyl substituiert ist; (C₂-C₄)-Alkenyl; Phenyl, oder Phenyl, das ein- oder mehrfach durch Halogen, (C₁-C₄)-Alkyl, NO₂, CN oder (C₁-C₄)-Alkoxy substituiert ist,
- R¹⁰: Wasserstoff, (C₁-C₄)-Alkyl, (C₁-C₄)-Alkyl, das ein- oder mehrfach durch F, Cl, Br oder OCH₃ substituiert ist; (C₃-C₆)-Cycloalkyl, das unsubstituiert oder durch F, Cl, Br oder CH₃ ein- oder mehrfach substituiert ist; (C₂-C₄)-Alkenyl oder (C₂-C₄)-Alkinyl;
- R¹¹,R¹²: unabhängig voneinander (C₁-C₄)-Alkyl oder R¹¹ und R¹² zusammen -CH₂CH₂-, -CH₂OCH₂- oder -CH₂C(CH₃)₂CH₂- ;
- R¹³: Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist; (C₃-C₄)-Alkinyl, das unsubstituiert oder durch CH₃ oder Si(CH₃)₃ substituiert ist; (C₃-C₆)-Cycloalkyl, (C₃-C₆)-Cycloalkyl-(C₁-C₄)alkyl, (C₁-C₄)-Alkoxy oder Si(CH₃)₃,
- R¹⁴: Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
- R¹⁵: Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂- oder -(CH₂)₂O(CH₂)₂- ,
- R¹⁶: (C₁-C₄)-Alkyl oder (C₁-C₄)-Haloalkyl,
- R¹⁷: (C₁-C₄)-Alkyl oder NR¹⁴R¹⁵,
- R¹⁸: (C₁C₄)-Alkyl, (C₁-C₄)-Haloalkyl, (C₂-C₄)-Alkoxyalkyl, (C₂-C₄)-Alkenyl, (C₃-C₄)-Alkinyl, Phenyl oder Phenyl, das ein- oder mehrfach durch Halogen, (C₁-C₃)-Alkyl, (C₁-C₃)-Alkoxy oder (C₁-C₃)-Haloalkyl substituiert ist;
- R¹⁹: (C₁-C₄)-Alkyl oder (C₁-C₄)Alkyl, das ein- oder mehrfach durch F, Cl oder einfach durch OR¹⁶ substituiert ist,
- R²⁰,R²¹: unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können; -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy;
- R²²: (C₁-C₄)-Alkyl oder (C₁-C₄)-Haloalkyl,
- R²³,R²⁴: unabhängig voneinander Wasserstoff, (C₁-C₄)-Alkyl, (C₂-C₄)-Alkenyl oder (C₂-C₄)-Alkinyl;
- R²⁵: Wasserstoff, (C₁-C₄)-Alkyl oder (C₁-C₄)-Haloalkyl,
- R²⁶: Wasserstoff oder (C₁-C₃)-Alkyl,
- G: einen heterocyclischen Rest (G 1) - (G 25), 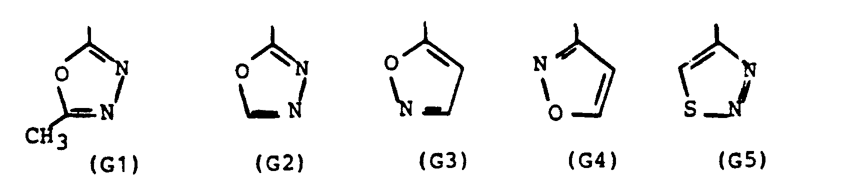 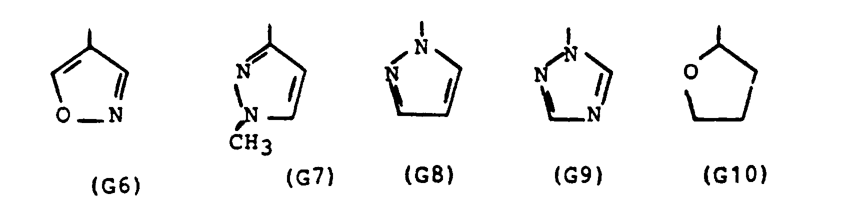 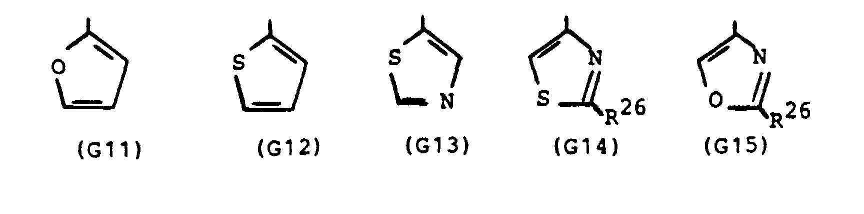 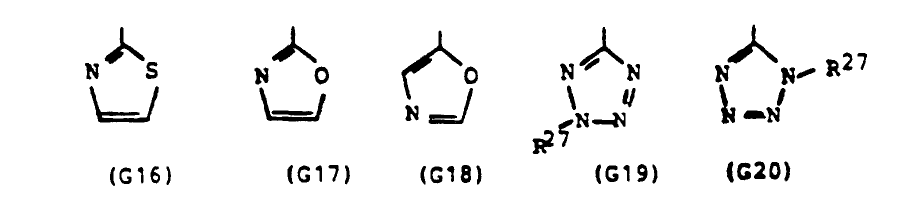 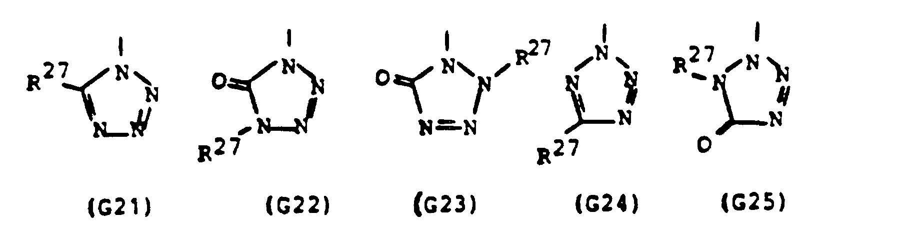
- R²⁷: Wasserstoff, (C₁-C₃)-Alkyl oder (C₂-C₄)-Alkenyl;
bedeuten.

Die Verbindungen der Formel I können ein oder mehrere Chiralitätszentren enthalten und liegen dann als Diastereomeren- oder Enantiomerengemische vor. Die Erfindung umfaßt sowohl die reinen Enantiomeren oder Diastereomeren als auch deren Gemische.

Die Verbindungen der Formel I können im Falle R¹ = H Salze bilden, bei denen der Wasserstoff der -SO₂-NH-Gruppe durch ein für die Landwirtschaft geeignetes Kation ersetzt wird. Diese Salze sind im allgemeinen Metall-, insbesondere Alkali-, Erdalkali-, gegebenenfalls alkylierte Ammonium- oder organische Aminsalze. Sie werden vorzugsweise in inerten Lösungsmitteln wie z. B. Wasser, Methanol oder Aceton bei Temperaturen von 0 - 100°C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

In den obengenannten Definitionen bedeutet der Ausdruck "Alkyl", alleinstehend oder in zusammengesetzten Worten wie "Alkylthio", "Haloalkyl", "Alkylamino" oder "Bisalkylamino" jeweils geradkettiges oder verzweigtes Alkyl.

Ebenso bedeutet Alkenyl gerad- oder verzweigtkettiges Alkenyl, z.B. 1-Propenyl, 2-Propenyl, 3-Propenyl. Alkinyl bedeutet gerad- oder verzweigtkettiges Alkinyl z.B. Ethinyl, 1-Propinyl, 2-Propinyl oder die verschiedenen Butinyl-Isomere. Alkylsulfonyl bedeutet beispielsweise Methylsulfonyl, Ethylsulfonyl oder die verschiedenen Propylsulfonyl-Isomere.

Der Ausdruck "Halogen" allein oder in zusammengesetzten Wörtern wie "Haloalkyl" bedeutet Fluor, Chlor, Brom oder Jod. Weiterhin bedeutet in zusammengesetzten Wörtern wie "Haloalkyl", daß besagtes Alkyl teilweise oder vollständig halogeniert ist. Beispiel für Haloalkyl sind CHF₂, CH₂CH₂F, CF₂CF₃ und CH₂CHFCl.

Bevorzugt von den Verbindungen der allgemeinen Formel I sind solche, worin
- A: einen Rest der Formeln (A1), (A2), (A3) oder (A4), insbesondere (A1);
- L: einen Rest der Formeln (L1), (L3), (L4) oder (L5),
- X: CH₂, CH(CH₃), 0, NH, NCH₃, NC₂H₅, NOCH₃, insbesondere CH₂, 0 oder NH,
- W: Sauerstoff,
- R¹: Wasserstoff,
- R²,R³: unabhängig voneinander Wasserstoff oder (C₁-C₃)Alkyl, insbesondere Wasserstoff,
- R⁵: Halogen, NO₂, CN, (C₁-C₃)-Alkyl, das unsubstituiert oder durch F, Cl, Br, CN, OCH₃ oder SCH₃ substituiert ist; (C₃)-Alkenyl, das unsubstituiert oder durch F, Cl oder Br substituiert ist; (C₃)-Alkinyl, (C₃)-Cycloalkyl, das unsubstituiert oder durch F, Cl oder CH₃ substituiert ist, -C-(O)R¹⁰, -OCH₂CH₂OR¹⁰, OH, -C(R¹⁰)(OR¹¹) (OR¹²), -CO₂R¹³, -C(O)NR¹⁴R¹⁵, N_{3,} -SO₂NR¹⁴R¹⁵, -OSO₂R¹⁷, -E¹R¹⁸ oder -(CH₂)ₛG;
- R⁶: Wasserstoff, Halogen, CN, NO₂, CH₃, CF₃, -E¹R¹⁹ oder (C₁-C₂)-Alkoxy-(C₁-C₂)alkyl, (C₁-C₂)-Haloalkoxy, (C₁-C₂)-Alkylthio, (C₁-C₂)-Haloalkylthio, CN, -CO₂R¹³ oder -SO₂NR¹⁴R¹⁵,
- R⁷: Wasserstoff,
- R¹⁰: (C₁-C₃)-Alkyl, Cyclopropyl oder (C₃)-Alkenyl,
- R¹¹,R¹²: (C₁-C₂)-Alkyl oder R¹¹, R¹² zusammen -CH₂CH₂-,
- R¹³: (C₁-C₃)-Alkyl, (C₃)-Alkenyl, -CH₂CH₂F, -CH₂CH₂CI, -CH₂CH₂OCH₃ oder Cyclopropylmethyl;
- R¹⁴: Wasserstoff oder CH₃ oder R¹⁴ und R¹⁵ zusammen -(CH₂)₄-, -(CH₂)₅- oder -(CH₂)₂O(CH₂)₂- ,
- R¹⁵: CH₃, CH₂CH₃, OCH₃ oder R¹⁴ und R¹⁵ zusammen -(CH₂)₄-, -(CH₂)₅- oder -(CH₂)₂O(CH₂)₂- ,
- R¹⁸: (C₁-C₃)-Alkyl, (C₁-C₃)-Haloalkyl, (C₂-C₃)-Alkoxyalkyl, Allyl, Propargyl, (C₂-C₃)-Haloalkenyl,
- R¹⁹: (C₁-C₂)-Alkyl, das unsubstituiert oder durch F, Cl oder OCH₃ substituiert ist,
- R²⁰,R²¹: unabhängig voneinander Wasserstoff, (C₁-C₂)-Alkyl, (C₁-C₂)-Alkoxy, -CH₂OCH₃, Cl, F, Br, J, -CH₂OCH₂CH₃, -NHCH₃, -N(OCH₃)CH₃, -N(CH₃)₂, -CF₃, -SCH₃, -CH(OCH₃)₂, -OCH₂CH=CH₂; -OCH₂C≡CH, -OCH₂CH₂OCH₃, -CH₂SCH₃,-OCHF₂, -SCHF₂, Cyclopropyl, -C≡CH oder -C≡C-CH₃,
- m: 0 oder 1,
- n: 0 oder 1,
- s: null und
- E¹: O oder S bedeuten.

Für R²⁰ sind besonders bevorzugt die Reste CH₃, OCH₃, CH₂CH₃, OCH₂CH₃, OCHF₂, OCH₂OCH₃, CH₂OCH₃, NHCH₃, CK(OCH₃)₂, Cl oder Cyclopropyl, und für R²¹ die Reste CH₃, OCH₃, OCH₂CH₃ oder OCHF₂.

Gegenstand der vorliegenden Erfindung sind auch Verfahren zur Herstellung der Verbindungen der Formel I, dadurch gekennzeichnet, daß man
a) zur Herstellung von Verbindungen mit W = O
   a₁) eine Verbindung der Formel II mit 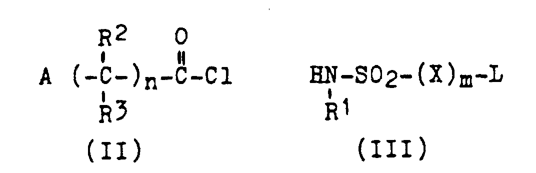 einer Verbindung der Formel III in Gegenwart einer Base umsetzt oder
   a₂) eine Verbindung der Formel (IIa) 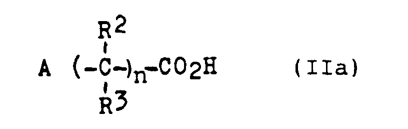 in Gegenwart von aktivierenden Reagentien wie 2-Chlor-1-methylpyridiniumchlorid(IVa), Dicyclohexylcarbodiimid (IVb) oder 1,1-Carbonyldiimidazol (IVc) und gegebenenfalls in Gegenwart einer Base mit einer Verbindung der Formel III umsetzt oder
   a₃) zur Herstellung von Verbindungen mit W = O und R¹ = H eine Verbindung der Formel (V), 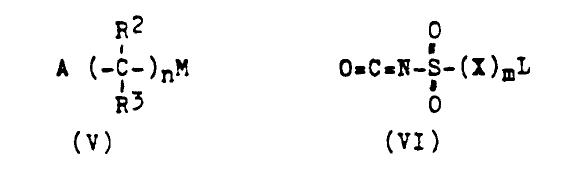 worin M Wasserstoff oder Lithium bedeutet, mit einer Verbindung der Formel (VI) umsetzt, oder
b) zur Herstellung von Verbindungen mit W = S eine unter a) erhaltene Verbindung der Formel I mit der Verbindung der Formel VII umsetzt, 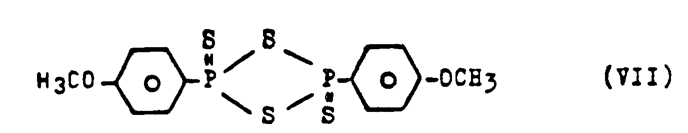
c) zur Herstellung von Verbindungen mit W= NR⁴ eine Verbindung der Formel VIII 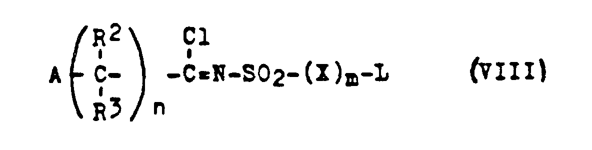 mit einem Amin der Formel H₂N-R⁴ umsetzt,
d) zur Herstellung von Verbindungen mit W= NOR⁴
   d₁) eine Verbindung der Formel IX mit 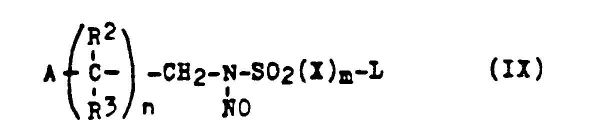 einem Alkali- oder Erdalkalihydroxid oder Ammoniumhydroxid umsetzt oder
   d₂) eine Verbindung der Formel VIII in Gegenwart einer Base mit einem Hydroxylamin der Formel H₂N-OR⁴ umsetzt, oder
e) zur Herstellung von Verbindungen der Formel I mit R¹ ≠ H eine Verbindung der Formel I mit R¹ = H in Gegenwart einer Base mit einem Alkylhalogenid der Formel R^{1'}-X¹, worin R^{1'} die Bedeutung von R¹ außer Wasserstoff besitzt und X¹ Chlor, Brom oder Jod bedeutet, umsetzt und die erhaltenen Verbindungen der Formel I gegebenenfalls in ihr Salz überführt.

Das beim Verfahren a₁) eingesetzte Säurechlorid der Formel II läßt sich in bekannter Weise aus der entsprechenden Carbonsäure der Formel (IIa) 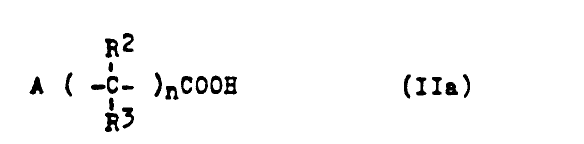 oder deren Salz mit anorganischen Säurechloriden wie Thionylchlorid oder mit Oxalylchlorid in Gegenwart geeigneter Basen, insbesondere organischer Stickstoffbasen, wie Pyridin, 2,6-Lutidin oder Triethylamin und/oder Katalysatoren wie N,N-4-Dimethylaminopyridin oder Dimethylformamid herstellen und wird zweckmäßigerweise direkt ohne Zwischenisolierung in Gegenwart der genannten Base mit der Komponente III umgesetzt. Überschüssiges Thionylchlorid oder Oxalylchlorid wird vor der Reaktion des Säurechlorids II mit dem Sulfonamid III abdestilliert.

Die Umsetzungen gemäß Verfahrensvariante a₂) sind an sich bekannt. Sie werden vorzugsweise in einem inerten aprotischen Solvens, wie Dichlormethan, 1,2-Dichlorethan, Acetonitril oder Glykoldimethylether bei Temperaturen zwischen -30 ° C bis 83 ° C durchgeführt.

Als Basen werden beispielsweise organische Stickstoffbasen wie Pyridin, Triethylamin etc. verwendet. Im Falle des Carbonyldiimidazols ist kein Basenzusatz erforderlich.

Das Verfahren a₃) ist an sich bekannt; es wird bevorzugt in einem inerten, aprotischen Solvens wie Dichlormethan, Tetrahydrofuran, 1,2-Dichlorethan oder Glykoldimethylether im Temperaturbereich von -78 ° C bis 85 °C durchgeführt.

Das Verfahren gemäß Variante b) ist ebenfalls an sich bekannt; es wird vorzugsweise in einem inerten aprotischen Solvens wie Toluol oder Xylol bei Temperaturen zwischen 0°C und 145°C durchgeführt. In Fällen, in denen die Produkte im Reaktionsmedium unlöslich sind, lassen sie sich durch einfache Filtration isolieren. Sind die Reaktionsprodukte löslich, lassen sie sich nach Abdampfen des Solvens durch Kristallisation oder Chromatographie des Rückstandes erhalten.

Die Verfahrensvarianten c) und d) sind im Prinzip bekannt. Verfahren c) und d₂) wird bevorzugt in einem inerten aprotischen Solvens wie Dichlorethan oder Toluol im Temperaturbereich zwischen 0°C und 110°C durchgeführt.

Die Imidoylchloride der Formel VIII können aus einer gemäß Variante a) hergestellten Verbindung der Formel I durch Umsetzung mit Triphenylphosphin/Tetrachlormethan oder PCl₅/POCl₃ erhalten werden. Das Verfahren wird vorzugsweise in CCl₄ oder POCl₃ bei Temperaturen zwischen 0°C und 105°C durchgeführt, vgl. Houben-Weyl, Bd. E5/1, S. 628-632 (1985); Bd.5/3 S. 916-922 und Bd.8 S. 346, 673-76.

Das an sich bekannte Verfahren e) wird bevorzugt in einem inerten, aprotischen Solvens wie Dichlormethan, Tetrahydrofuran, 1,2-Dichlorethan oder Glykoldimethylether im Temperaturintervall von -78° C bis 85°C durchgeführt.

Die den Säurechloriden der Formel (II) entsprechenden Carbonsäuren der Formel (IIa) können nach verschiedenen Methoden hergestellt werden. Möglich ist z.B. die alkalische Verseifung der entsprechenden Carbonsäureester (J. March "Advanced Organic Chemistry", 3rd edition, John Wiley & Sons, NY 1985, Seite 334-338), oder die Umsetzung der Esterverbindungen mit Lithiumiodid in Pyridin oder anderen Aminen bzw. mit Trimethylsilylchlorid und Natriumiodid (vgl. J. March, "Advanced Organic Chemistry" 3rd edition, John Wiley & Sons, NY 1985, Seite 386) oder durch Umsetzung mit Jodtrimethylsilan, s. Olah, Narany, Tetrahedron 38 2225-2277 (1982) oder durch Reaktion mit Bromwasserstoffsäure.

Die Carbonsäuren (IIa) sind ferner herstellbar aus den entsprechenden Nitrilen durch Hydrolyse, s. Monatsh. Chem. 87, 526-35 (1956) oder Überführen in die Imidoether und nachfolgende Hydrolyse, s. T. Sakamoto; Chem. Pharm. Bull. 28, 3362-8 (1980).

Ferner können die Carbonsäuren (IIa) aus den entsprechenden Benzylestern durch Hydrierung in Gegenwart eines Katalysators wie Palladium hergestellt werden, s. Houben-Weyl, "Methoden der organischen Chemie", Bd. 4/1c (1980), S. 379-387.

Alternativ dazu können die Carbonsäuren der Formel lla aus den halogenierten Heterocyclen der Formel (IIb) 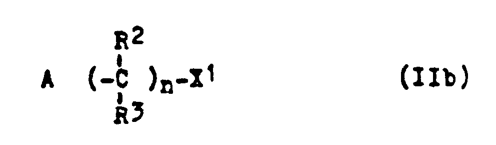 durch Reaktion mit Kohlendioxid direkt - oder indirekt über metallorganische Zwischenstufen erhalten werden, s. Volpin und Kolomnikov, Organomet. React. 5, 313-386 (1975); Sneeden, in Patai "The Chemistry of Carboxylic Acids and Esters", S. 137-173, Interscience, NY 1969; sowie Kharasch und Reinmuth, Grignard Reactions of Nonmetallic Substances", S. 913-948, Prentice-Hall, Englewood Cliffs, N.Y. 1954.

Die den Carbonsäuren IIa zugrundeliegenden Nitrile sind darstellbar aus den Verbindungen der Formel (IIc) 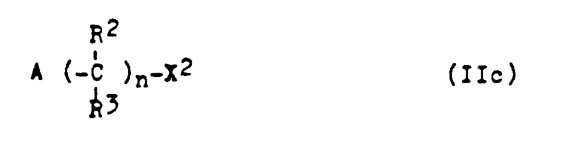 durch Umsetzung mit Alkali- oder Tetraalkylammoniumcyaniden. In Formel (IIc) bedeutet X² eine Abgangsgruppe wie Chlor, Brom, Jod, p-Tosyl, OSO₂CH₃ oder Tetraalkylammonium, s. W. Klötzer, Monatsh. Chem. 87, 526-35 (1956); H. Kobler, Liebigs Ann. Chem. 1978, 1937-45, T. Sakamoto, Chem. Pharm. Bull. 28, 3362-8 (1980); J. March, "Advanced Organic Chemistry", 3rd edition, John Wiley & Sons, NY 1985, S. 594-595; Houben-Weyl, Bd. E5/2, S. 1447-1474 (1985).

Alternativ dazu sind die Nitril-Verbindungen durch Umsetzung heterocyclischer N-Oxide der Formel A → O mit Trimethylsilylcyanid darstellbar, s. H. Yamanaka, Synthesis 1984, 681-3; und H. Vorbrüggen, Synthesis 1983, 316-9; Houben-Weyl, Bd. E5/2, S. 1444-1446 (1985).

Ferner können die Nitrile aus den Aminen der Formel A-NH₂ gemäß Sandmeyer-Reaktion d.h. durch Diazotierung und anschließender Umsetzung mit Kupfer(II)cyanid hergestellt werden.

Die halogenierten Heterocyclen der Formel (IIb) lassen sich durch Halogenierung der entsprechenden alkylierten heterocyclischen N-Oxide herstellen, s. Craig, J. Org. Chem. 1970 (35), 1721; Bauer, J. Org. Chem. 1963 (28), 1323; Hunt, J. Chem. Soc. 525-530 (1959) und Matsumura, Nippon Kagaku Zasshi, 74, 363 (1953).

Ferner können die halogenierten Heterocyclen der Formel (IIb) aus den entsprechenden Hydroxyverbindungen A(CR²R³)ₙ-OH durch Umsetzung mit halogenierenden Reagenzien, wie Thionylchlorid oder Phosphoroxichlorid erhalten werden, s. Angerstein, Ber.dtsch.Chem.Ges., 34, 3956 (1901); d'Atri, J. Med. Chem. 1984 (27), 1621-1629 oder Sakamoto, Chem. Pharm. Bull. 28, 3362-8 (1980) oder im Falle n = 0 durch Umsetzung der Amine der Formel A-NH₂ in einer Sandmeyerähnlichen Reaktion, s. Tagaki, Chem.Pharm.Bull. 11 S. 1382-8 (1963); Houben-Weyl, "Methoden der organischen Chemie", Bd. 10/3, S. 53ff, Georg Thieme Verlag, Stuttgart 1965, Bd 5/3, S. 846ff und Bd. 5/4, S. 437ff.

Die heterocyclischen Amine A-NH₂ sind bekannt oder lassen sich nach im Prinzip bekannten Verfahren herstellen, s. "The Chemistry of Heterocyclic Compounds", Bd. XVI, (1962), Interscience Publ., NY & London und Supplement I (1970) dieses Handbuches. Amino-substituierte Triazinderivate werden von Smolin und Rapaport in "The Chemistry of Heterocyclic Compounds", Bd. XIII (1959), Interscience Publ. NY & London, referiert. Die Darstellung speziell substituierter heteroyclischer Amine wird in folgenden Patenten beschrieben : US-PS 4,515,626, US-PS 4,540,782, US-PS 4,339,267, US-PS 4,487,626 und US-PS 4,421,550. Weitere Referate zur Synthese bicyclischer Pyrimidine findet man bei Braker, J.Am.Chem.Soc. 1947 (69), 3072; Mitler, Quart. J. Ind. Chem. Soc. 4, 152 (1927); Shrage; J. Org. Chem. 1951 (16), 1153; Caldwell, J. Am. Chem. Soc. 1941 (63), 2188 und Fissekis, J. Org. Chem. 1964 (29), 2670.

Die Verbindungen der Formel (II), (IIa), (IIb), (IIc) und ihre in den oben beschriebenen Verfahren verwendeten Vorstufen sind zum Teil neu und entsprechend insbesondere der Formel (II') 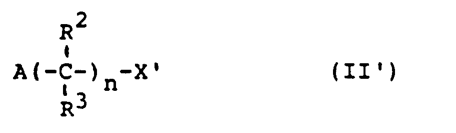 worin
- X': COCI, CN, Cl, F, Br, I, p-Tosyl, Tetraalkylammonium, N₃, CO₂R¹³, CO₂CH₂C₆H₅ oder E¹R¹⁸ bedeutet und
- A, n, R¹³, E¹ und R¹⁸: die bereits genannten Bedeutungen haben.

Die heterocyclischen Verbindungen der Formel X (X³ = CN, Cl, F, Br, I, N₃, CO₂R¹³, CO₂CH₂C₆H₅ oder E¹R¹⁸) können bevorzugt als Ausgangsprodukte zur Herstellung der obengenannten Zwischenstufen eingesetzt werden; die Verbindungen der Formel X lassen sich durch Umsetzung der Verbindungen der Formel XI mit Säurechloriden der Formel XII oder Säureanhydriden der Formel XIII herstellen. 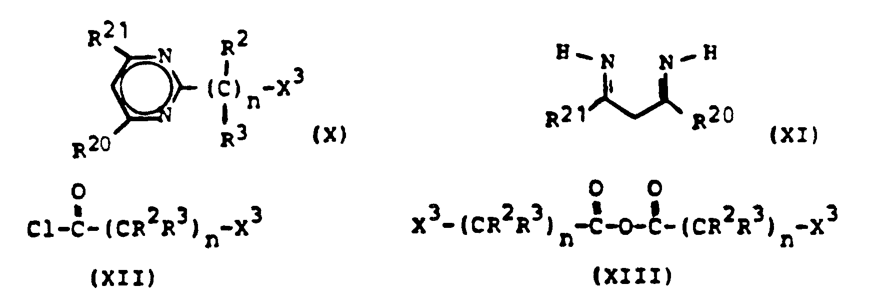 Die Reaktionen der Ketimine, Amidate oder Imidate der Formel XI werden beispielsweise in einem inerten Solvens wie Dichlormethan Toluol, Tetrahydrofuran oder 1,2-Dimethoxyethan in Gegenwart einer Base wie Triethylamin, Kaliumcarbonat oder N-Ethyldiisopropylamin im Temperaturintervall von -78°C bis 100°C durchgeführt, s. Eilingsfeld, Chem. Ber. 101, 2426-2434 (1968). Nach Eilingsfeld sind von den vier betreffenden Verbindungen jedoch nur 4,6-Diethoxypyrimidin-2-carbonsäureethylester und 2-Chlormethyl-4,6-diethoxypyrimidin als hergestellt beschrieben (s. Chem. Ber. 101, 2426).

Die als Vorstufen zur Herstellung der Carbonsäuren der Formel (IIa) eingesetzten Carbonsäureester and im Falle n = 0 auch aus den halogenierten Vorstufen A-X' durch eine Negishi-Kumada-Kreuzkupplungsreaktion durch Umsetzung mit entsprechenden zinkorganischen Verbindungen zugänglich, s. Yamanaka: Chem.Pharm. Bull. 33, 4309-4313 (1985) und Sakamoto; Synthesis 1988, 485-486.

Spezielle heterocyclische Vorstufen für die Herstellung der Verbindungen der Formel I sind in den folgenden Publikationen beschrieben:
Sakamoto; Chem. Pharm. Bull. 32, 2005-2010 (1984); (4-Methoxy-6-methylpyrimidin-2-carbonsäuremethylester).
Sakasai. Heterocycles 1979, 235-238; Mekuskiene, Zh. Vses. Khim. O-va. 1976. 21(3), 348-349; Brown, Aust. J. Chem. 1974, 27, 2251-9 (4,6-Dimethylpyrimidin-2-carbonsäuremethylester; 4,6-Dimethylpyrimidin-2-carbonsäure).
Eilingsfeld, Chem. Ber. 1968, 101, 2426-2334; (4,6-Diethoxypyrimidin-2-carbonsäureethylester; 2-Chlormethyl-4,6-diethoxypyrimidin).
Brown, Aust. J. Chem. 1977, 30, 621-627; ((4-Chloro-6-methylpyrimidin-2-yl)essigsäureethylester; (4.6-Dichloropyrimidin-2-yl)essigsäuremethylester) Yamanaka, Heterocycles 1976, 5, 255-260 und Yamanaka, Chem. Pharm. Bull. 1979, 27, 2291-2294; (4-Benzyloxy-6-methyl-2-pyrimidinylessigsäuremethylester, 4-Methoxy-6-methyl-2-pyrimidinylessigsäuremethylester, 4-Ethoxy-6-methyl-2-pyrimidinylessigsäuremethylester).

Brown, Aust. J. Chem. 1978, 31, 649-659; (4,6-Dichloropyrimidin-2-yl)-essigsäuremethylester; (4-Chloro-6-methoxypyrimidin-2-yl)-essigsäuremethylester, (4,6-Dimethoxypyrimidin-2-yl)-essigsäuremethylester; (4-Chloro-6-methylpyrimidin-2-yl)-essigsäuremethylester; nebst entsprechenden Carbonsäuren).
Yamanaka, Chem. Pharm. Bull. 1985, 33, 4309-4313, Chem. Pharm. Bull. 1981, 29, 2837-2843; Sakamoto, Chem. Pharm. Bull. 1980, 28, 3362-3368, Niitsuma, Heterocycles 1978, 10, 171-176; Kost, Khim, Geterotsikl. Soedin. 1978, 10, 1400-1405; Brown, Aust. J. Chem. 1974, 27, 2251-2259; Mamaev, Khim. Qeterotsikl. Soedin., Sb.1: Azotsoderzhashchie Geterotsikly 1967, 354-359; ((4,6-Dimethylpyrimidin-2-yl)-essigsäureethylester).

EP-A 154132; (1H-1-Methyl-1,2,4-triazol-3-carbonsäureethylester).
FR-A 2 499 081; (1H-1-Methyl-1,2,4-triazol-3-carbonsäuremethylester).
Vakhatova, Khim, Geterotsikl. Soedin, 1981, 2, 264-267: (4-Chloro-6-methoxy-s-triazin-2-essigsäureethylester).
Zaitseva, Zh. Obshch. Khim. 1964, 34, 2976-2979; Schaefer, J. Org. Chem. 1962, 27, 3608-3613; (4,6-Dimethyl-s-triazin-2-carbonsäureethylester).
Kober, J. Org. Chem. 1961, 26, 4705-4706; (4,6-Dichloro-s-triazin-2-essigsäureethylester).
4,6-disubstituierte 2-Halogen- bzw. 2-Halogenmethylpyrimidine und -triazine sind in einer großen Zahl bereits bekannt; siehe z. B. EP-A-0255047, J. Chem. Soc. (C) 1966, 2031 und Beilsteins Handbuch der Organischen Chemie.
Die Herstellung von 4,6-disubstituierten 2-Cyano- oder 2-Cyanomethylpyrimidinen und -triazinen sind in EP-A-0094260, Liebigs Ann. Chem. 1981, 33-341 und Chem. Pharm. Bull. 6 (1959) 509-515 beschrieben.
Aus J. Org. Chem. 26 (1961) 957-959 sind 4,6-Bis(methylthio)-s-triazin-2-carbonsäure und deren Methyl und Ethylester bekannt.
Eine Reihe von bicyclisch verbrückten Pyrimidinen sind bekannt aus DE-A-1816378, J. Heterocycl. Chem. 19 (1982) 1527-1529, Chem. Pharm. Bull. 31 (1983) 2254-2261, J. Med. Chem.-Chim. Ther. 17 (1982) 75-79, Org. Magn. Resonance 8 (1976) 607-610, Ciba Foundation on Chemistry and Biology of Pteridines, London, 1974, 104-123 und J. Am. Chem. Soc. 78 (1956) 225-227.

Gegenstand der Erfindung sind auch einige neue Zwischenprodukte der Formel (II'). Neue Verbindungen der Formel (II') sind insbesondere solche, worin
- X': COCI, CN, F, p-Tosyl, Tetraalkylammonium, N₃, CO₂R¹³, CO₂CH₂C₆H₅ oder E¹R¹⁸,
- A: einen heterocyclischen Rest der genannten Formel (A1), worin r = 1, oder (A2), (A3), (A4) oder (A5), wobei im Fall (A5) n = 1 ist, oder (A6), (A7) oder (A8)
bedeuten und (A1) bis (A8), E¹, R², R³, R¹³, R¹⁸ und n wie in Formel (I) definiert sind.

Neue Verbindungen der Formel (II') sind insbesondere auch solche, worin
- X': COCl, CO₂R¹³ oder CO₂CH₂C₆H₅ bedeutet,
- A: einen heterocyclischen Rest der Formel (A1), worin r = 0 und E² = CH sind,
bedeutet und (A1), R², R³, R¹³ und n wie in Formel (I) definiert sind, wobei
1) im Falle n = 0 und X' = COOH das Paar R²⁰/R²¹ nicht CH₃/CH₃ ist,
2) im Falle n = 0 und X' =CO₂CH₃ das Paar R²⁰/R²¹ nicht CH₃/CH₃ oder OCH₃/CH₃ ist,
3) im Falle n = 0 und X' =CO₂C₂H₅ das Paar R²⁰/R²¹ nicht OC₂H₅/OC₂H₅ oder Cl/Cl ist,
4) im Falle n = 1, R² = R³ = H und X' = COOH, COONa oder COOCH₃ das Paar R²⁰/R²¹ nicht H/F, H/Cl, H/Br, H/I, H/CH₃, H/OCH₃, H/SC₂H₅, Cl/SC₂H₅, SC₂H₅/SC₂H₅, Benzyloxy/CH₃, OCH₃/CH₃, OC₂H₅/CH₃, CI/CI, Cl/OCH₃, OCH₃/OCH₃ oder Cl/CH₃ ist,
5) im Falle n = 1, R² = R³ = H und X' = CO₂C₂H₅ das Paar R²⁰/R²¹ nicht Cl/CH₃ oder CH₃/CH₃ ist.

Neue Verbindungen der Formel (II') sind insbesondere auch solche, worin
- X': COCI, CO₂R¹³ oder CO₂CH₂C₆H₅ bedeutet,
- A: einen heterocyclischen Rest der Formel (A1), worin r = 0 und E² = N sind,
bedeutet und (A1), R², R³, R¹³ und n wie in Formel (I) definiert sind, wobei
1) im Falle n = 0 und X' =CO₂C₂H₅ das Paar R²⁰/R²¹ nicht CH₃/CH₃ oder CCl₃/NH₂ oder SCH₃/SCH₃ ist,
2) im Falle n = 1, R² = R³ = H und X' = CO₂C₂H₅ das Paar R²⁰/R²¹ nicht CI/OCH₃ oder CI/CI ist,
3) im Falle n = 1, R² = R³ = H und X' = CN das Paar R²⁰/R²¹ nicht NH₂/NH₂ ist.
4) im Falle n = 0 und X' = COOH das Paar R²⁰/R²¹ nicht OCH₃/OCH₃ oder SCH₃/SCH₃ ist und
5) im Falle n = 0 und X' = CO₂CH₃ das Paar R²⁰/R²¹ nicht SCH₃/SCH₃ ist.

Neue Verbindungen der Formel (II') sind insbesondere auch solche, worin
- X': COCl, CN, CO₂R¹³ oder CO₂CH₂C₆H₅ bedeutet, n = 0 ist,
- A: einen heterocyclischen Rest der Formel (A5) bedeutet und
(A5), R², R³ und R¹³ wie in Formel (I) definiert sind, wobei im Falle n = 0,
X' =CO₂CH₃ oder CO₂C₂H₅ und R²⁰ = H der Rest R²² nicht gleich CH₃ ist.

Die als Zwischenprodukte in den Verfahren a₁) und a₂) verwendeten Sulfonamide der Formel III werden im Falle m=0 aus den entsprechenden Anilinen durch Diazotierung und Austausch der Diazogruppe mit Schwefeldioxid in Gegenwart eines Katalysators wie Kupfer(I)chlorid in Salzsäure oder Essigsäure und Umsetzen des entstandenen Sulfonylchlorids mit Ammoniak erhalten, vgl. Meerwein, Chem. Ber. 1957, 90, 841-852. Die Zwischenprodukte der Formel III (mit X=0 und m=1 werden aus den entsprechenden Phenolen durch Umsetzung mit Chlorsulfonylisocyanat und anschließender Hydrolyse erhalten, vgl. Lohaus, Chem. Ber. 1972, 105, 2791-2799.

Die Verbindungen der Formel III mit X= NR⁴ und m= 1 werden aus den entsprechenden Anilinen durch Reaktion mit Amidosulfochlorid in Gegenwart einer Hilfsbase wie Triethylamin in bekannter Weise hergestellt.

Die Sulfonamide der Formel III mit X= CHR² und m= 1 werden aus den entsprechenden Benzylhalogeniden durch Reaktion mit Thioharnstoff, anschließender Oxidation mit Chlor und nachfolgender Reaktion mit Ammoniak nach üblichen Methoden erhalten. Die Sulfonamide der Formel III mit X= CHR² und m= 1 werden auch aus den entsprechenden Benzylhalogeniden nach Überführung in das Grignard-Reagenz durch Reaktion mit SO₂ und nachfolgender Reaktion des entstandenen Sulfinatsalzes mit Hydroxylamin-O-sulfonsäure in gepufferter wäßriger Lösung erhalten.

Die Herstellung der Sulfonylisocyanate der Formel VI erfolgt nach für den Fachmann bekannten Standardverfahren, s. "Newer Methods of Preparative Organic Chemistry", Bd. VI, 223-241, Academic Press, NY & London; Lohaus, Chem. Ber. 1972, 105, 2791-2799.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z. B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den Perennierenden.

Unter den spezifischen Kulturbedingungen im Reis vorkommenden Unkräuter wie z. B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindung vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoff auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrüber, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die Verbindungen der Formel (I) oder deren Kombination mit einem oder mehreren der genannten Herbizide bzw. Herbizidgruppen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wäßrige Lösungen (SC), Emulsionen, versprühbare Lösungen, Dispersionen auf Öl- oder Wasserbasis (SC), Suspoemulsionen (SC), Stäubemittel (DP), Beizmittel, Boden- bzw. Streugranulate (FG), wasserdispergierbare Granulate (WG), ULV_Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohole, Alkyl-oder Alkylphenolsulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Boden- bzw. Streugranulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoff der Formel (I), 1 bis 99,9 Gew.-%, insbesondere 5 bis 99,8 Gew.-% eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei wasserdispergierbaren Granulaten hängt der Wirkstoffgehalt zum Teil davon ab, ob die wirksame Verbindung flüssig oder fest vorliegt.

Im allgemeinen liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids u.a. variiert die erforderliche Aufwandmenge der Verbindungen der Formel (I). Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Folgende Beispiele dienen zur Erläuterung der Erfindung:

### A. Formulierungsbeispiele

a) Ein Stäubemittel wird erhalten, indem man 10 Gew.-Teile einer Verbindung der Formel (I) und 90 Gew.-Teile Talkum oder Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten, indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-Dinaphthylmethan-6,6'-disulfonsaures Natrium,
   2 Gewichtsteile Oleylmethyltaurinsaures Natrium,
   1 Gewichtsteile Polyvinylalkohol,
   17 Gewichtsteile Calciumcarbonat und
   50 Gewichtsteile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### Chemische Beispiele

### Beispiel 1

### 2-Ethoxycarbonyl-4,6-dimethoxypyrimidin

Zu einer Suspension von 25,4 g Malondiimidsäure-dimethylester dihydrochlorid in 100 ml Dichlormethan werden 76 ml N-Ethyldiisopropylamin in 35 ml Dichlormethan bei -35°C zugetropft. Nach beendeter Zugabe rührt man noch 15 min nach und tropft dann bei dieser Temperatur 14 ml Oxalsäureethylesterchlorid zu. Man läßt nun auf Raumtemperatur aufwärmen und rührt noch 2 h. Es wird auf 200 ml Eiswasser gegossen, die organische Phase abgetrennt und noch zweimal mit Wasser gewaschen. Die organische Phase wird über CaCl₂ getrocknet und eingeengt. Man erhält so 21,7 g 2-Ethoxycarbonyl-4,6-dimethoxypyrimidin vom Schmp. 54-55 ° C (aus Hexan).

### Beispiel 2

### 4,6-Dimethoxypyrimidin-2-carbonsäure

17,1 g 2-Ethoxycarbonyl-4,6-dimethoxypyrimidin werden in 300 ml Ethanol gelöst und mit 3,4 g Natriumhydroxid in 50 ml Wasser versetzt. Diese Lösung wird 4 h bei Raumtemperatur gerührt und 1 h unter Rückfluß erhitzt. Man kühlt ab und entfernt die Hauptmenge des Ethanols am Rotationsverdampfer. Der Rückstand wird in Wasser aufgenommen und mit Dichlormethan extrahiert. Die wäßrig alkalische Phase wird mit 6N Salzsäure auf einen pH 2-3 angesäuert und vom ausgefallenen Produkt abgesaugt. Man erhält so 9,6 g 4,6-Dimethoxypyrimidin-2-carbonsäure vom Schmp. 155-157°C.

### Beispiel 3

### 1-Brom-1-(4,6-dimethoxypyrimidin-2-yl)ethan

Zu einer gerührten Suspension von 101 g Malondiimidsäuredimethylester-dihydrochlorid und 266 g Kaliumcarbonat in 1500 ml Dichlormethan tropft man während 1 h bei -30 °C 58,7 ml 2-Brom-propionylbromid in 200 ml Dichlormethan. Man läßt auf Raumtemperatur aufwärmen, rührt 4 h nach und versetzt vorsichtig mit soviel Wasser bis die Salze vollständig gelöst sind (Kühlung!). Man trennt die Phasen, wäscht die organische Phase noch zweimal mit gesättigter Natriumhydrogencarbonatlösung, trocknet über Kaliumcarbonat und destilliert das Solvens ab. Nach Filtration des Rückstands über Kieselgel (Eluens:Dichlormethan) erhält man 37 g 1-Brom-1-(4,6-dimethoxypyrimidin-2-yl)ethan
¹H-NMR(CDCl₃): δ= 2,05(d, J= 7Hz; 3H); 3,98(s, 6H); 5,08(q, J= 7Hz; 1H); 5,93(s, 1H).

Auf gleiche Weise wird dargestellt 2-Chlormethyl-4,6-dimethoxypyrimidin vom Schmp. 40-42 °C.

### Beispiel 4

### 2-Cyanomethyl-4,6-dimethoxypyrimidin

16,7 g getrocknetes Natriumcyanid werden unter N₂-Schutzgas bei 90°C in 850 ml Dimethylsulfoxid gelöst. Bei dieser Temperatur werden 52,8 g 2-Chlormethyl-4,6-dimethoxypyrimidin gelöst in 280 ml Dimethylsulfoxid, während 2 h zugetropft. Man rührt noch 1 h bei 90°C nach, kühlt auf Raumtemperatur und gießt auf 300 ml Wasser. Die wäßrige Phase wird sieben mal mit Ether extrahiert, die vereinigten organischen Phasen mit ges. äqu. Kochsalzlösung gewaschen, über Magnesiumsulfat getrocknet und im Rotationsverdampfer eingeengt. Man erhält so 29,1 g Rohprodukt, aus dem nach Säulenchromatographie (SiO₂; CH₂Cl₂/CH₃OH; 98,5/1,5) 2-Cyanomethyl-4,6-dimethoxypyrimidin vom Schmp. 65-67°C erhalten werden.

### Beispiel 5

### (4,6-Dimethoxypyrimidin-2-yl)-essigsäuremethylester

In eine Lösung aus 230 ml Methylacetat und 6,4 ml Methanol leitet man 15 min bei 0°C bis 10°C einen kräftigen Strom Chlorwasserstoffgas ein. Unter weiterem Einleiten von Chlorwasserstoff tropft man nun 23,8 g 2-Cyanomethyl-4,6-dimethoxypyrimidin in 250 ml Methylacetat zu, nach Beendigung des Zutropfens stoppt man das Gaseinleiten und rührt noch 5 h bei 10°C bis 15°C nach. Es wird 200 ml abs. Diethylether hinzugegeben, auf 5°C abgekühlt und der Niederschlag unter Stickstoff abgesaugt. Der Rückstand wird mit Ether nachgewaschen und trocken gesaugt. Man erhält so 31,5 g des Imidomethylesterhydrochlorid vom Schmp. 110-112°C, die mit 250 ml Wasser versetzt und 4 h bei 40°C gerührt werden. Man kühlt auf Raumtemperatur ab und extrahiert viermal mit je 150 ml Diethylether. Die organische Phase wird über Magnesiumsulfat getrocknet und das Solvens abdestilliert. Man erhält 19,5 g (4,6-Dimethoxypyrimidin-2-yl)essigsäuremethylester vom Sdp. 110°C/0,04 mbar (Kugelrohrdestillation).

### Beispiel 6

### (4,6-Dimethoxypyrimidin-2-yl)-essigsäure

20 g (4,6-Dimethoxypyrimidin-2-yl)-essigsäuremethylester werden 2 h mit 3,9 g Natriumhydroxid in 300 ml Wasser bei 50 ° C gerührt. Man kühlt auf Raumtemperatur ab, extrahiert dreimal mit Diethylether, säuert die Wasserphase mit 2N Salzsäure auf pH 2 an und extrahiert viermal mit 150 ml Ethylacetat. Die organische Phase wird über Natriumsulfat getrocknet, das Solvens abdestilliert und der Rückstand im Vakuum getrocknet. Man erhält so 13,1 g (4,6-Dimethoxypyrimidin-2-yl)-essigsäure vom Schmp. 175-177°C.

### Beispiel 7

### 2-Ethoxyphenylsulfamat

Eine Lösung von 19,2 g 2-Ethoxyphenoxysulfonylisocyanat in 150 ml Tetrachlormethan wird bei 30°C bis 40°C tropfenweise mit 2,2 g Wasser versetzt. Man rührt bis zum ende der Kohlendioxidentwicklung nach bei Raumtemperatur, kühlt auf 0°C ab, saugt das Produkt ab und wäscht den Filterrückstand mit eiskaltem Tetrachlormethan nach. Nach Trocknen im Vakuum erhält man 16 g 2-Ethoxyphenylsulfamat vom Schmp. 62-65 ° C.

### Beispiel 8

### 1-(2-Methoxycarbonylphenyl)sulfuryldiamid

Zu 6,4 g Anthranilsäuremethylester und 4,4 g Triethylamin in 50 ml abs. Dichlormethan tropft man eine Lösung von 5 g Amidosulfochlorid in 40 ml abs. Dichlormethan zu. Man rührt 0,5 h bei 40 ° C nach, dann 1 h bei Raumtemperatur und destilliert das Solvens im Rotationsverdampfer ab. Der Rückstand wird mit 100 ml 1N Salzsäure versetzt, 0,5 h nachgerührt und der Feststoff abgesaugt. Der Filterrückstand wird gut mit Wasser nachgewaschen und im Vakuum getrocknet Man erhält so 7,9 g 1-(2-Methoxycarbonylphenyl)-sulfuryldiamid vom Schmp. 116-118°C.

### Beispiel 9

### 1-(2-Chlorphenyl)-3-[(4,6-dimethoxypyrimidin-2-yl)carbonyl]-sulfuryldiamid

Zu einer Mischung aus 2,3 g Dicyclohexylcarbodiimid, 0,12 g 4-Dimethylaminopyrimidin und 2,2 g 4,6-Dimethoxypyrimidin-2-carbonsäure in 40 ml Dichlormethan tropft man bei 0 °C-2 °C eine Lösung von 2,1 g 1-(2-Chlorphenyl)sulfuryldiamid zu. Man rührt 4 h bei dieser Temperatur nach, läßt über Nacht bei Raumtemperatur stehen und saugt vom ausgefallenen Harnstoff ab. Das Filtrat wird 10 min mit 100 ml 1N Natriumcarbonatlösung verrührt, die Phasen getrennt und die wäßrige Phase noch zweimal mit Dichlormethan extrahiert. Die sodaalkalische Phase wird auf einen pH 2-3 angesäuert, 15 min gerührt, der Niederschlag abgesaugt und im Vakuum getrocknet. Man erhält 1,3 g 1-(2-Chlorphenyl)-3-[(4,6-dimethoxypyrimidin-2-yl)carbonyl]-sulfuryldiamid vom Schmp. 127-129°C

### Beispiel 10

### 2,6-Dichlorphenyl-N-[(4,6-dimethoxypyrimidin-2-yl)carbonyl]-sulfamat

Zu einer Mischung aus 2,3 g Dicyclohexylcarbodiimid, 120 mg 4-Dimethylaminopyridin und 2,2 g 4,6-Dimethoxypyrimidin-2-carbonsäure in 80 ml abs. Dichlormethan gibt man bei 0°C bis 2°C portionsweise 2,4 g 2,6-Dichlorphenylsulfamat zu, rührt 0,5 h bei 0°C und 3 h bei Raumtemperatur nach. Man saugt ab, wäscht die organische Phase mit 100 ml 1N Salzsäure, dann mit Wasser und trocknet über Natriumsulfat. Es wird das Solvens abdestilliert und der Rückstand aus Diisopropylether umkristallisiert. Man erhält 3,2 g 2,6-Dichlorphenyl-N-[(4,6-dimethoxypyrimidin-2-yl)carbonyl]-sulfamat vom Schmp. 136 - 139 °C.

### Beispiel 11

### N-[2-(4,6-Dimethoxypyrimidin-2-yl)acetyl]-2-methoxycarbonylbenzylsulfonamid

Zu einer Lösung von 1,9 g Dicyclohexylcarbodiimid, 100 mg 4-Dimethylaminopyridin und 2,1 g 2-Methoxycarbonylbenzylsulfonamid in 40 ml abs. Dichlormethan tropft man bei 0°C bis 2°C eine Lösung von 1,9 g (4,6-Dimethoxypyrimidin-2-yl)essigsäure in 40 ml abs. Dichlormethan zu. Man rührt 1 h bei 0°C nach, läßt auf Raumtemperatur aufwärmen und rührt noch 0,5 h. Es wird abgesaugt und das Filtrat im Rotationsverdampfer eingeengt. Den Rückstand versetzt man mit 100 ml 2N Sodalösung rührt 0,5 h bei Raumtemperatur und saugt ab. Die sodaalkalische Phase wird zweimal mit Diethylether extrahiert und mit konz. Salzsäure auf pH 2-3 eingestellt. Man rührt 15 min nach, saugt ab, wäscht den Filterrückstand einmal mit Wasser, zweimal mit Ether und trocknet im Vakuum. Man erhält so 2,4 g N-[2-(4,6-Dimethoxypyrimidin-2-yl)acetyl]-2-methoxycarbonylbenzylsulfonamid vom Schmp. 98-101 °C.

### Beispiel 12

### N-[(4,6-Dimethoxypyrimidin-2-yl)carbonyl]-2-methoxycarbonylbenzolsulfonamid

Zu einer Mischung aus 2,3 g Dicyclohexylcarbodiimid, 120 mg 4-Dimethylaminopyridin und 2,2 g 4,6-Dimethoxypyrimidin-2-carbonsäure in 80 ml abs. Dichlormethan gibt man bei 0°C bis 2°C portionsweise 2,2 g 2-Methoxycarbonylbenzolsulfonamid zu. Man rührt 0,5 h bei 0°C, 2 h bei Raumtemperatur und läßt über Nacht stehen. Es wird vom ausgefallenen Harnstoff abfiltriert, das Filtrat im Vakuum eingeengt und der Rückstand 0,5 h mit 100 ml 2N Sodalösung verrührt. Die wäßrige Phase wird mit Ether extrahiert und mit konz. Salzsäure auf pH 2-3 gestellt. Man saugt das ausgefallene Produkt ab und trocknet im Vakuum. Es werden so 1,3 g N-[(4,6-Dimethoxypyrimidin-2-yl)carbonyl]-2-methoxycarbonylbenzolsulfonamid vom Schmp. 163-165 ° C (aus Ethylacetat) erhalten.

### Beispiel 13

### 4-Methoxy-6-methyl-pyrimidin-2-carbonsäureethylester

Zur gerührten Suspension von 70,2 g Methyl-3-amino-2-butenimidat-dihydrochlorid in 350 ml Dichlormethan tropft man bei -30°C 182 ml Triethylamin in 50 ml Dichlormethan zu. Man rührt 0,5 h bei dieser Temperatur nach und tropft dann 42 ml Oxalsäureethylesterchlorid zu. Man rührt 1h bei -30°C nach und läßt während 12h auf Raumtemperatur aufwärmen. Man verrührt die organische Phase mehrere Male mit Wasser, trocknet über Na₂SO₄ und engt ein. Der Rückstand wird nach Kieselgelfiltration (Eluens: CH₂Cl₂) im Vakuum destilliert. Man erhält so 51,5 g 4-Methoxy-6-methylpyrimidin-2-carbonsäureethylester vom Sdp. 105-115°C/0,05 mbar.

### Beispiel 14

### 4-Methoxy-6-methylpyrimidin-2-carbonsäure

Zur gerührten Lösung von 51,5 g 4-Methoxy-6-methylpyrimidin-2-carbonsäureethylester in 280 ml Ethanol wird eine Lösung aus 11,5 g Natriumhydroxid in 156 ml Ethanol und 78 ml Wasser zugegeben. Man läßt 12h bei Raumtemperatur nachrühren und saugt das ausgefallene Natriumsalz der Carbonsäure ab. Das Natriumsalz wird in eine Lösung aus 17,3 g Acetylchlorid in 220 ml Methanol eingetragen und vom ausgeschiedenen Natriumchlorid abgesaugt. Die methanolische Lösung wird einrotiert. Als Rückstand erhält man 30,9 g 4-Methoxy-6-methylpyrimidin-2-carbonsäure vom Schmp. 140-141°C.

### Beispiel 15

### 2-Ethoxybenzylsulfonamid

Eine Grignard-Lösung aus 30,5 g 2-Ethoxybenzylchlorid und 6,1 g Magnesiumspäne in 250 ml Diethylether wird bei -60° bis -70°C zu 50 ml Schwefeldioxid getropft. Man rührt 0,5 h bei -70°C nach, läßt auf Raumtemperatur aufwärmen und saugt das entstandene 2-Ethoxybenzyl-sulfinat-Magnesiumsalz (47 g, Schmp.: 134-139°C, Zers.) ab. Das Sulfinatsalz wird in 900 ml Wasser und 16,4 g wasserfreiem Natriumacetat gelöst. Dazu gibt man bei Raumtemperatur unter Rühren 22,7 g Hydroxylamin-0-sulfonsäure. Nach 12h Rühren wird das Produkt abgesaugt und im Vakuum getrocknet. Man erhält so 23,9 g 2-Ethoxybenzylsulfonamid vom Schmp.: 92-93°C.

### Beispiel 16

### 2-Ethoxy-N-[4-methoxy-6-methylpyrimidin-2-yl)carbonyl]-benzylsulfonamid

Zu einer Mischung aus 2,3 g Dicyclohexylcarbodiimid, 120 mg 4-Dimethylaminopyridin und 2,0 g 4-Methoxy-6-methylpyrimidin-2-carbonsäure in 80 ml abs. Dichlormethan gibt man bei 0°C bis 2°C portionsweise 2,2 g 2-Ethoxybenzylsulfonamid zu, rührt 0,5 h bei 0 ° C und 3h bei Raumtemperatur nach. Man saugt ab, rotiert ein und verrührt den Rückstand in einem Gemisch aus Aceton und 1M Natriumcarbonatlösung. Die wäßrige Lösung wird mit Ether extrahiert, mit konz. HCl auf pH 2-3 eingestellt und mit Dichlormethan extrahiert. Die Dichlormethanphase wird einrotiert und der Rückstand aus Ether/Heptan umkristallisiert. Man erhält so 2,2 g 2-Ethoxy-N-[4-methoxy-6-methylpyrimidin-2-yl)carbonyl]-benzylsulfonamid vom Schmp.: 111-113°C.

Die Verbindung der nachfolgenden Tabellen lassen sich analog zu den in Beispielen 1 bis 16 beschriebenen Verfahrensweisen herstellen.

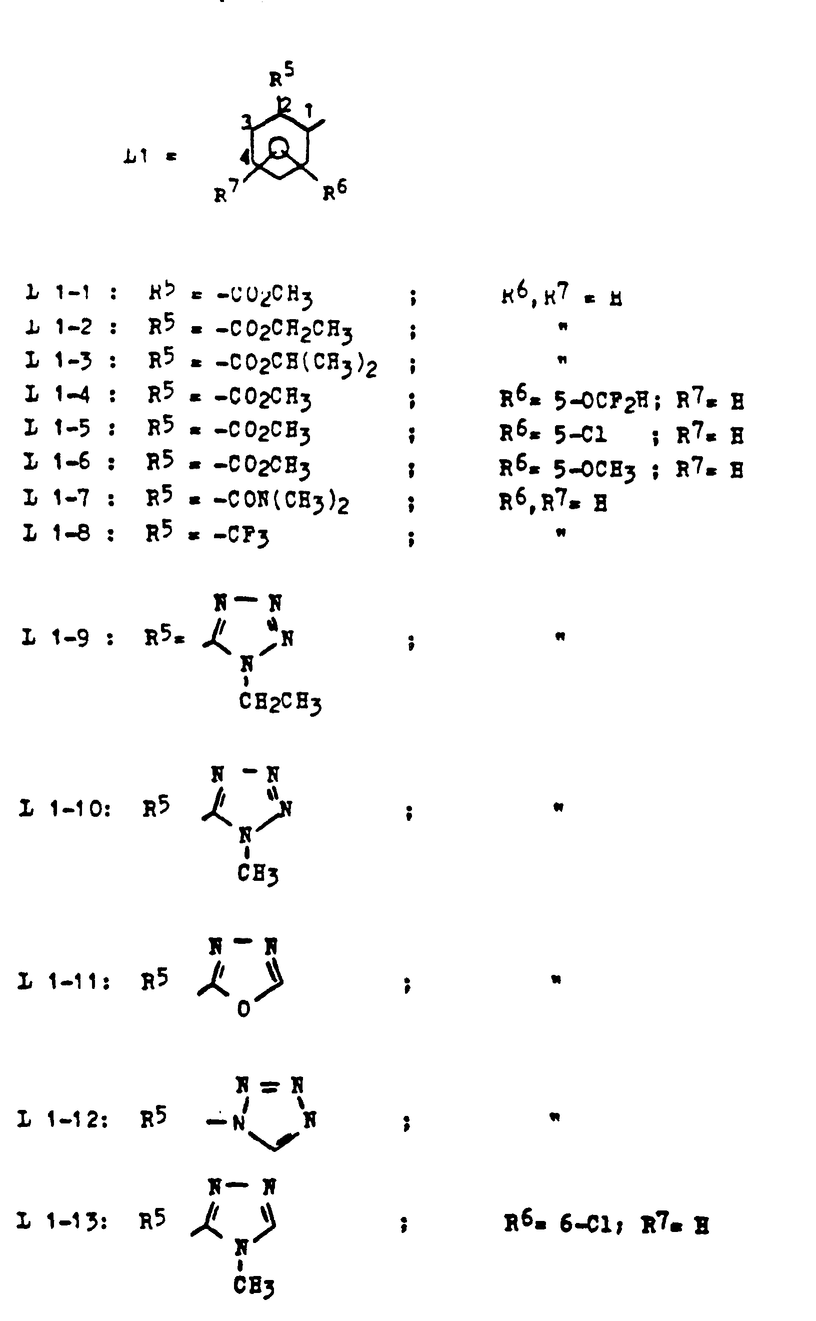

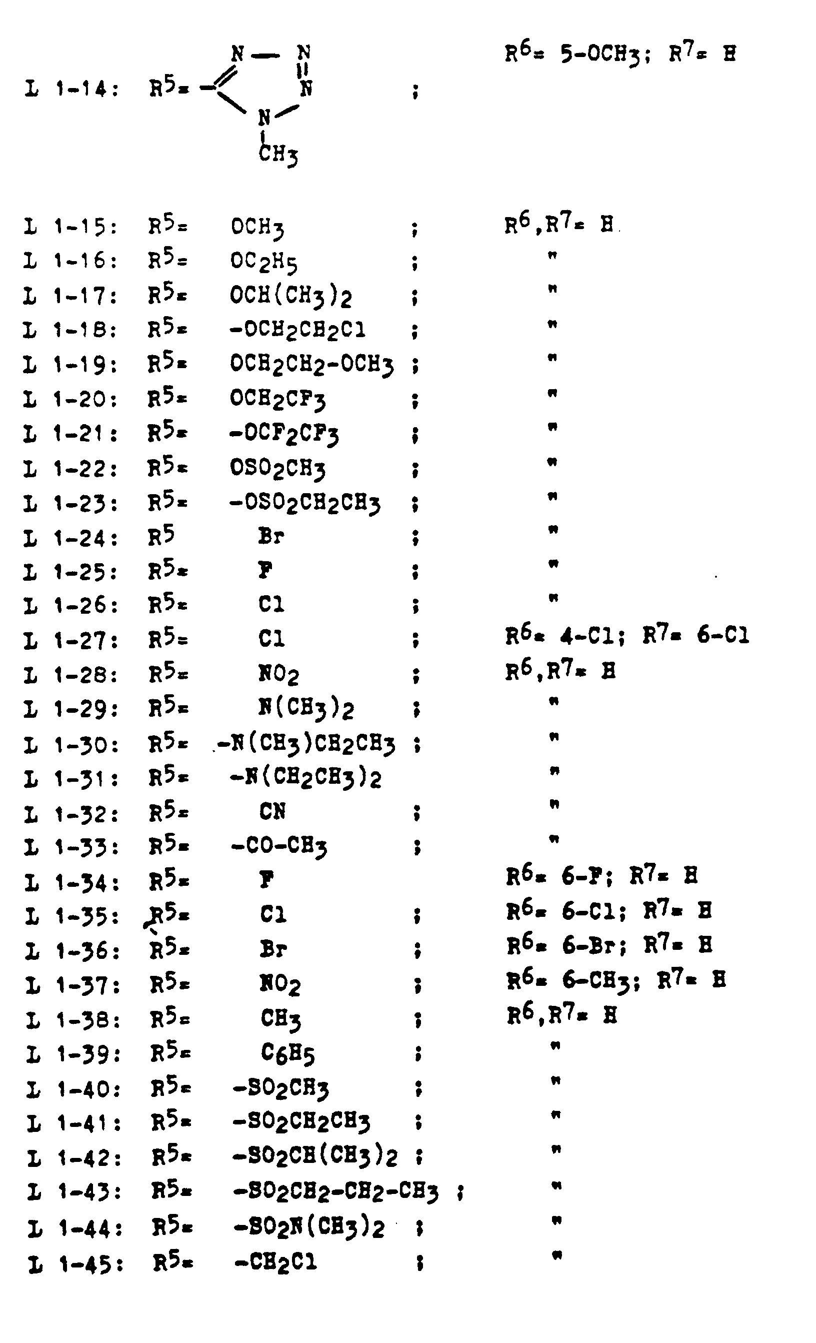

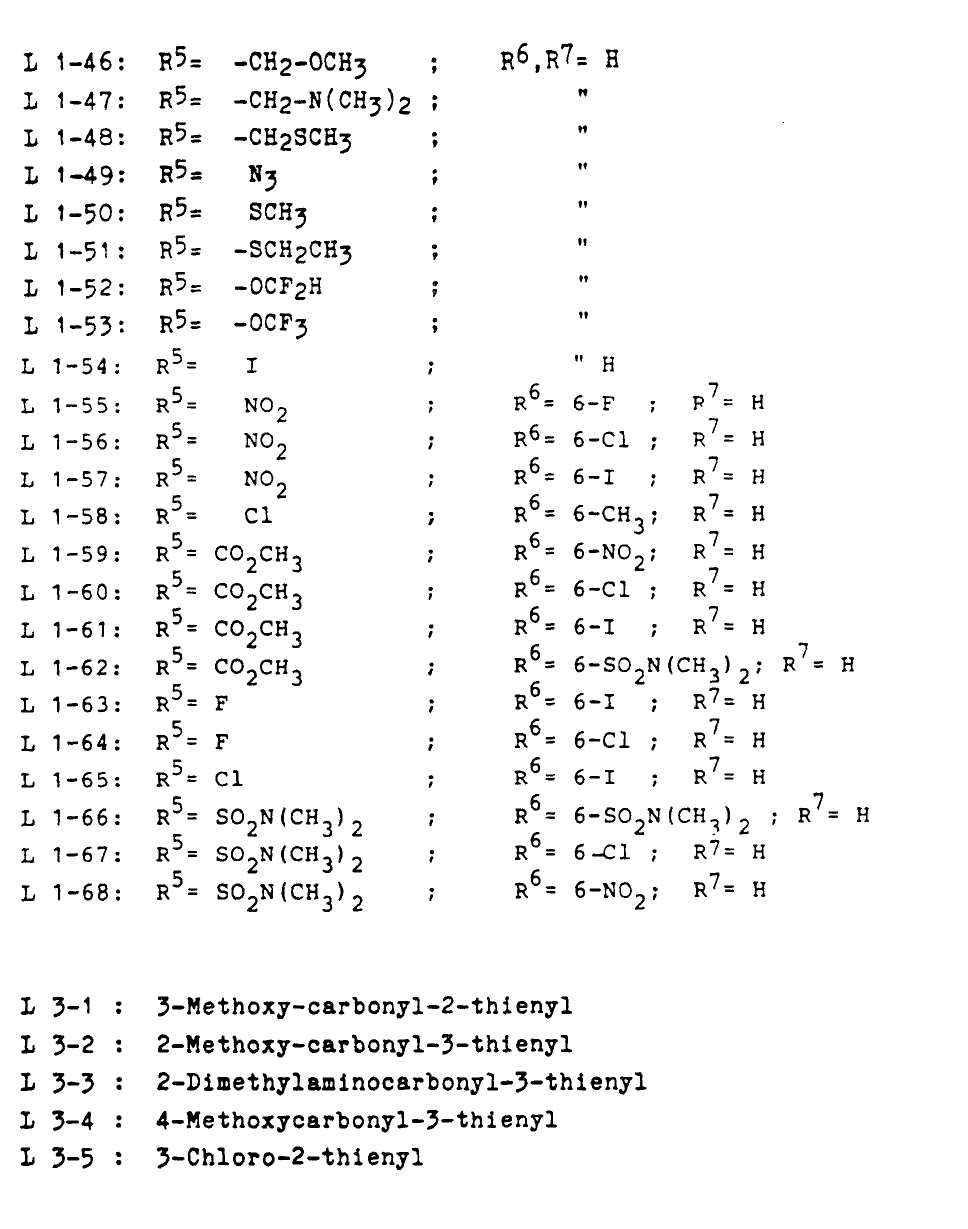

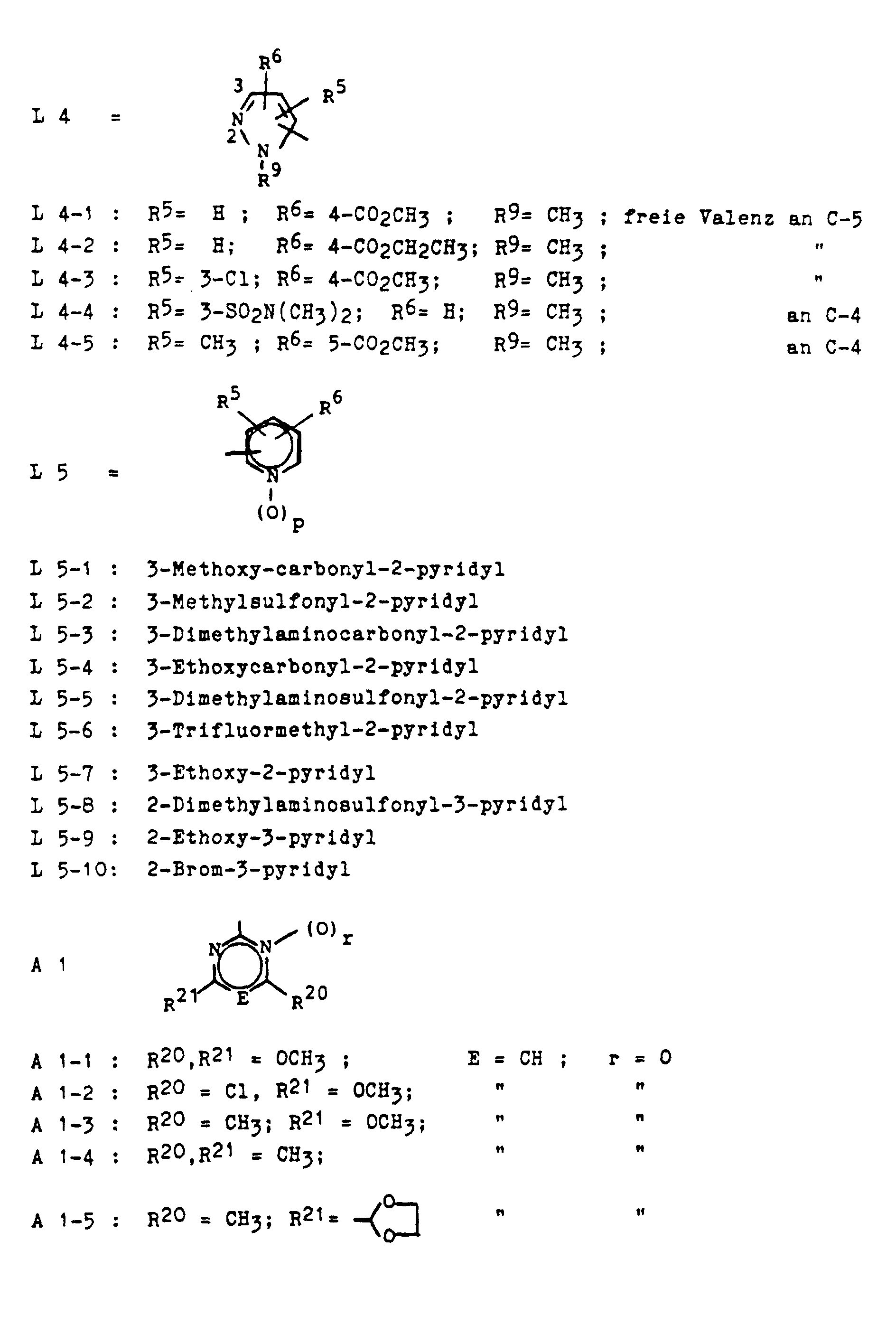

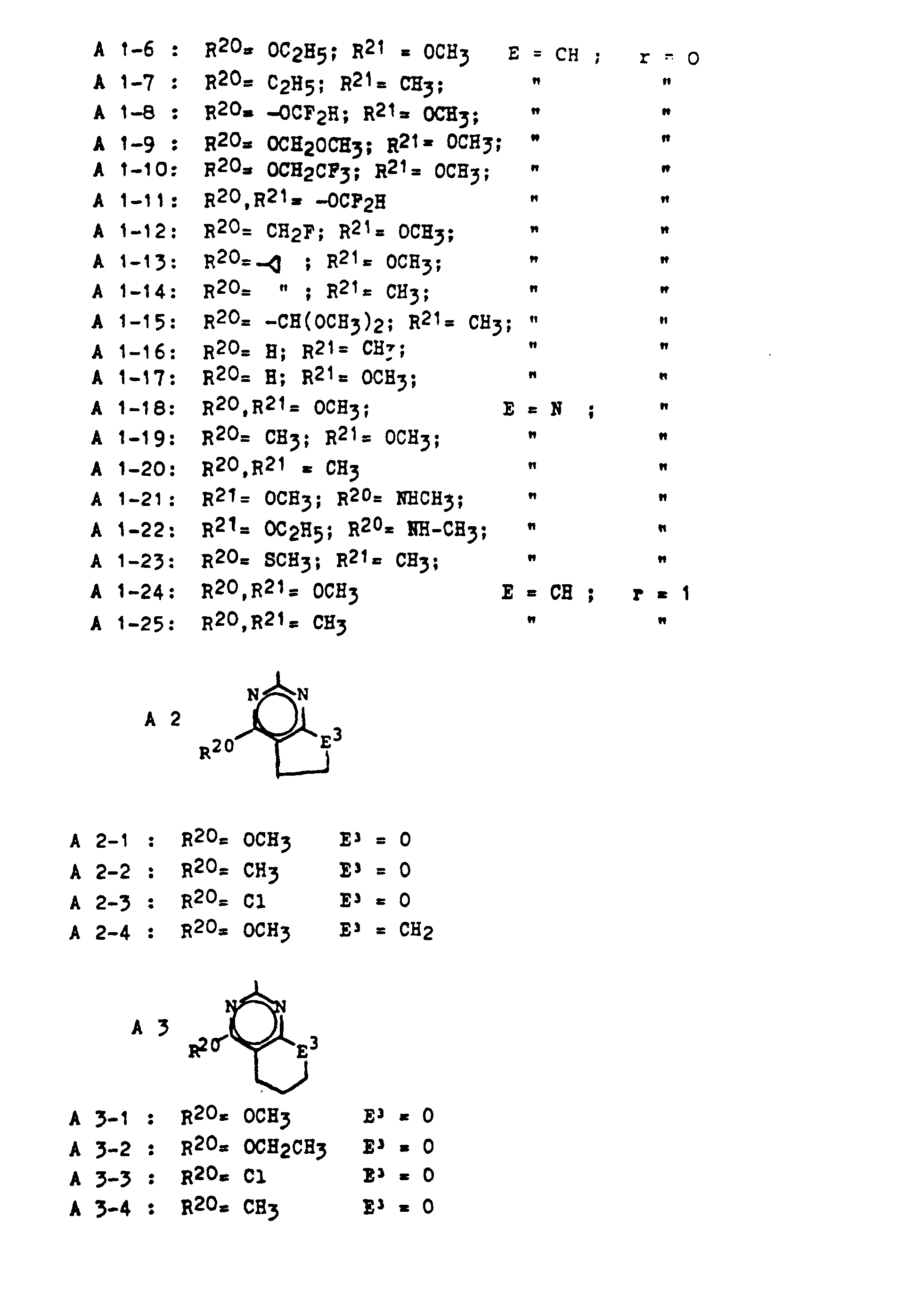

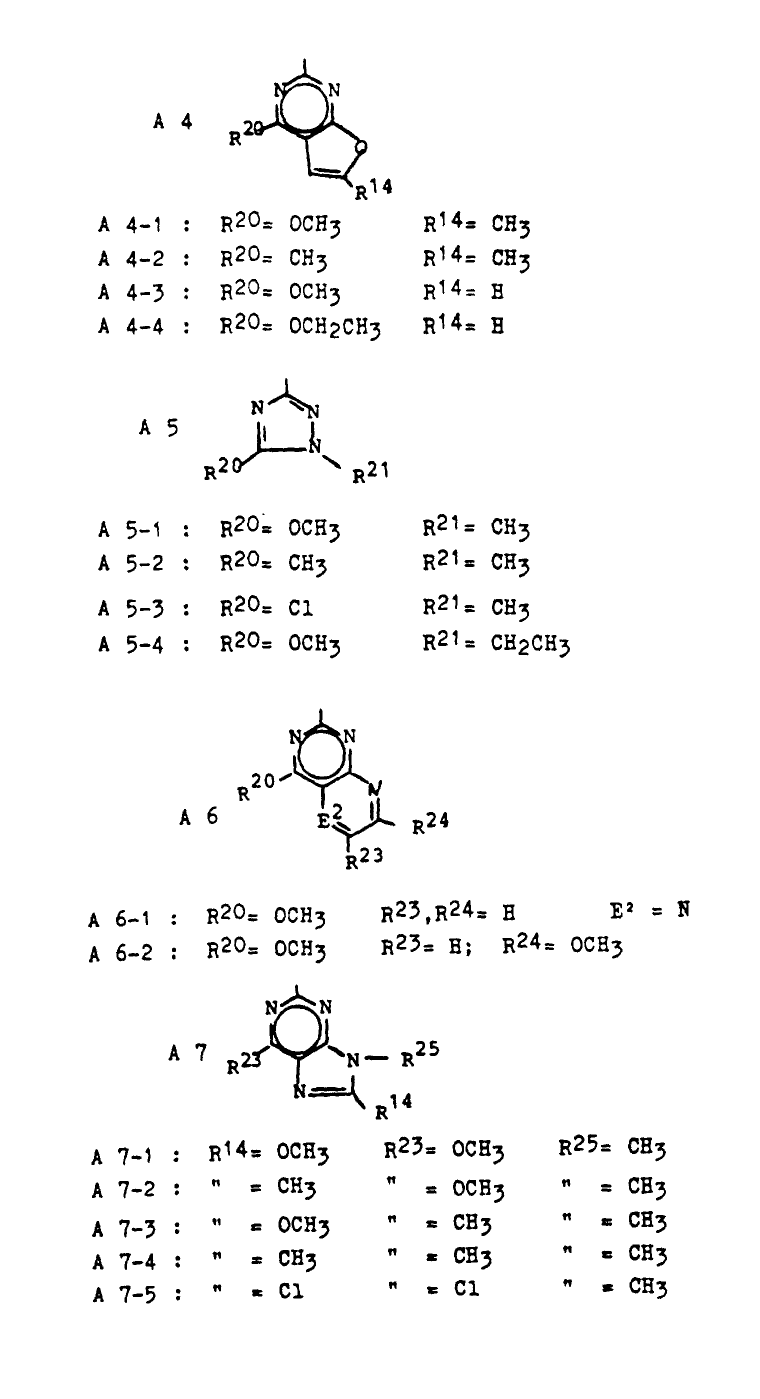

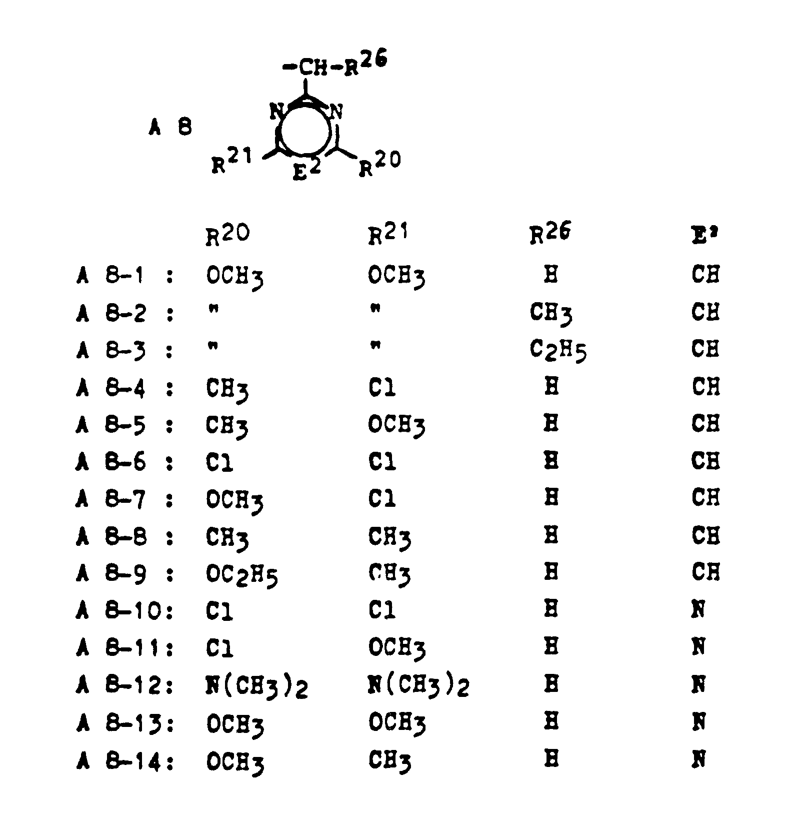

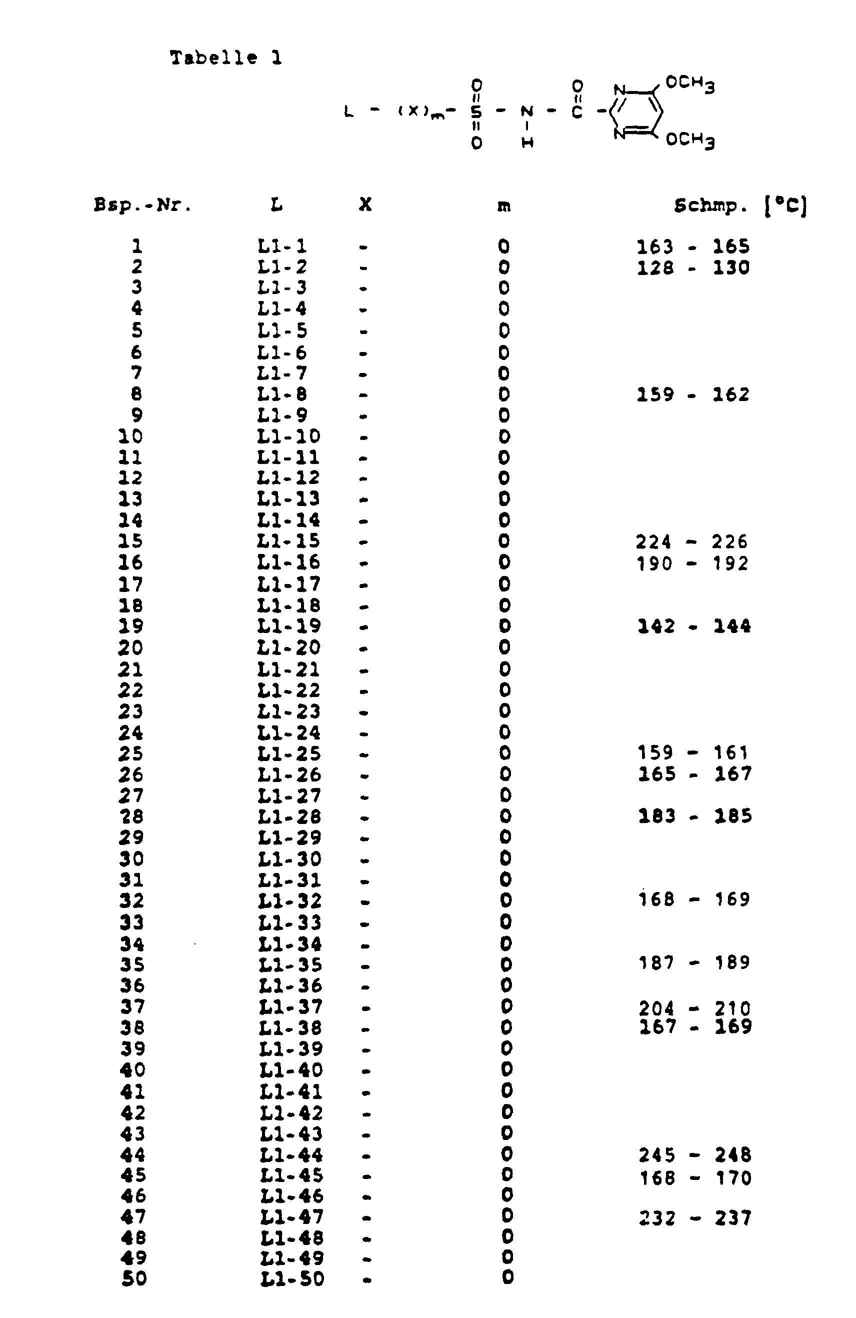

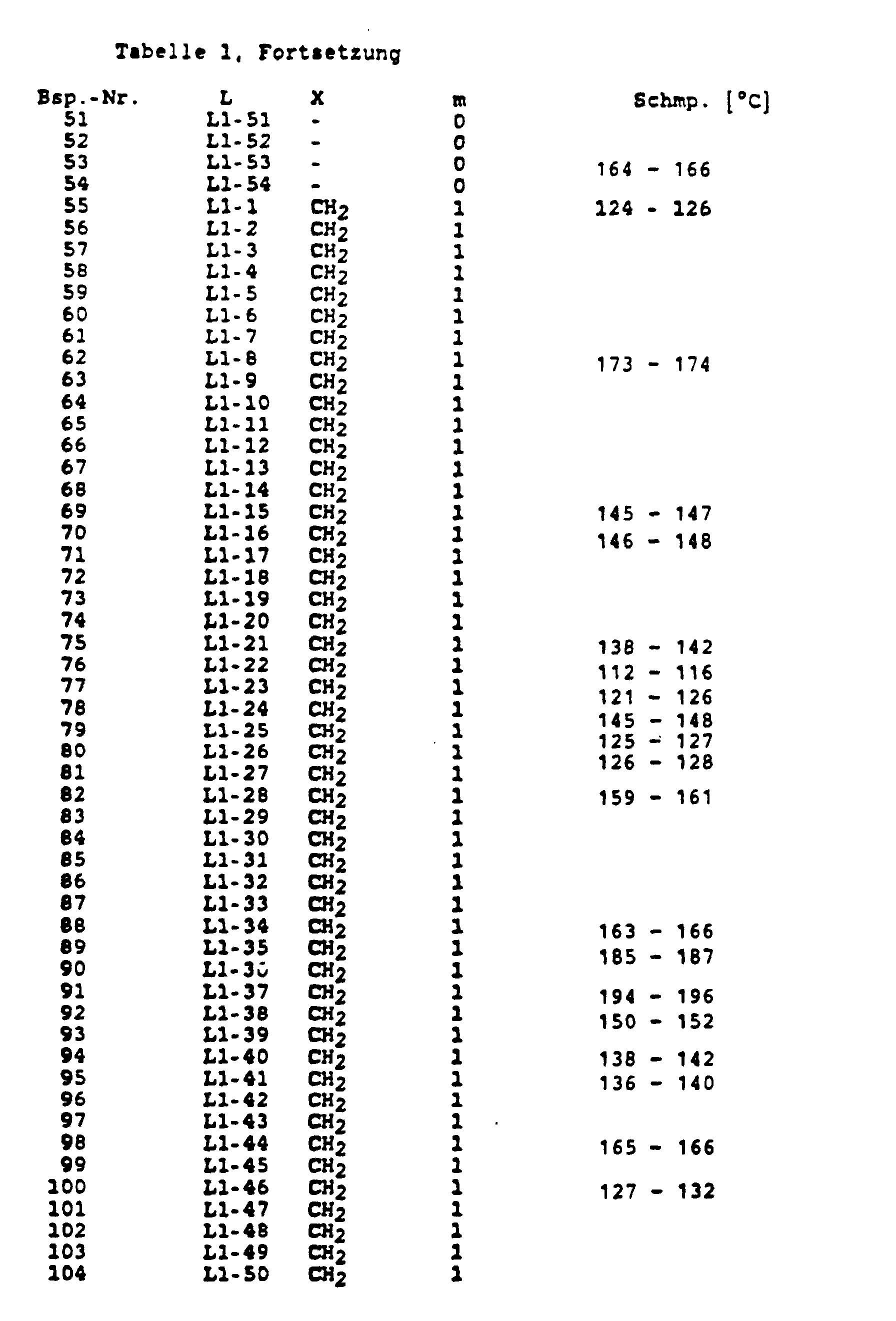

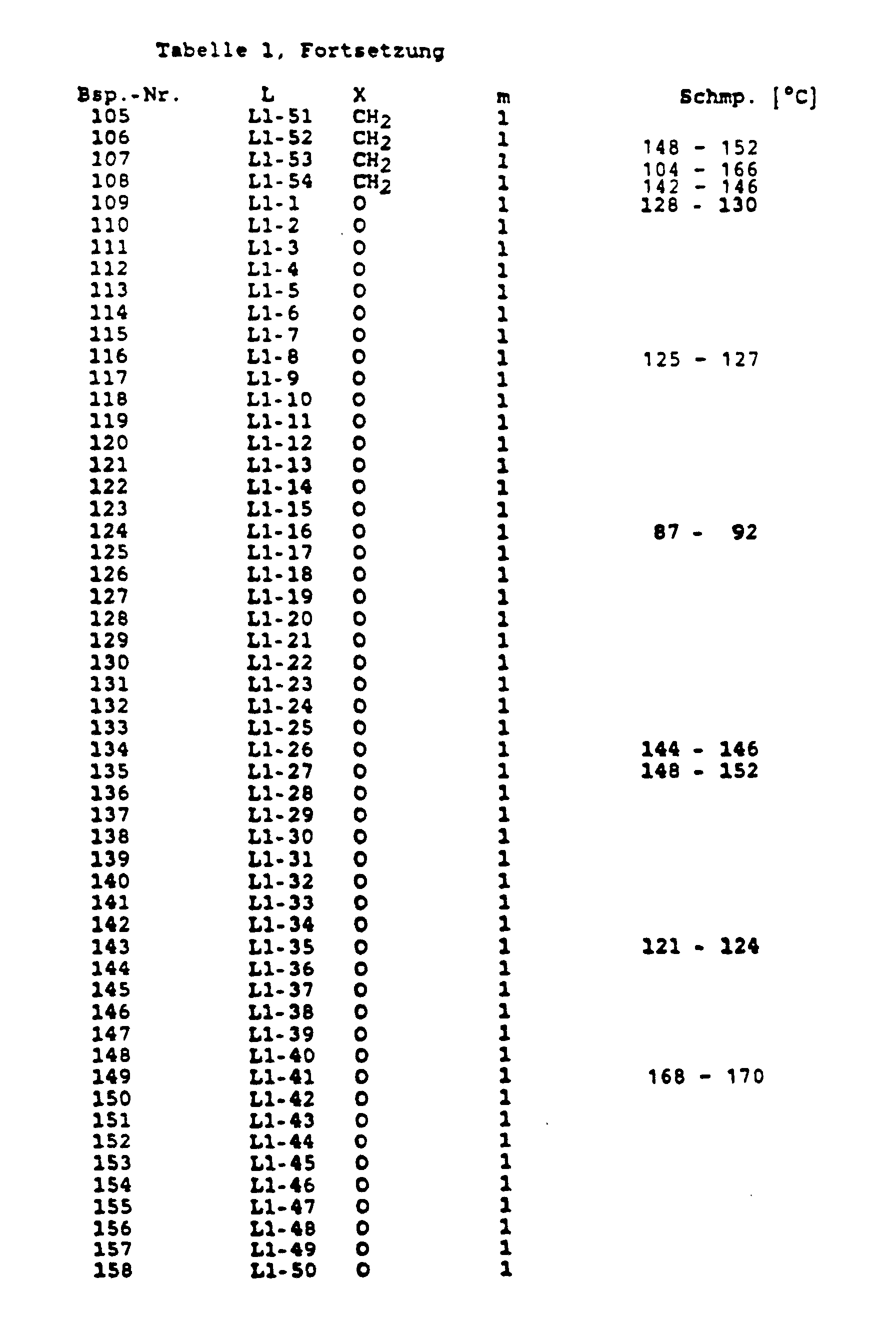

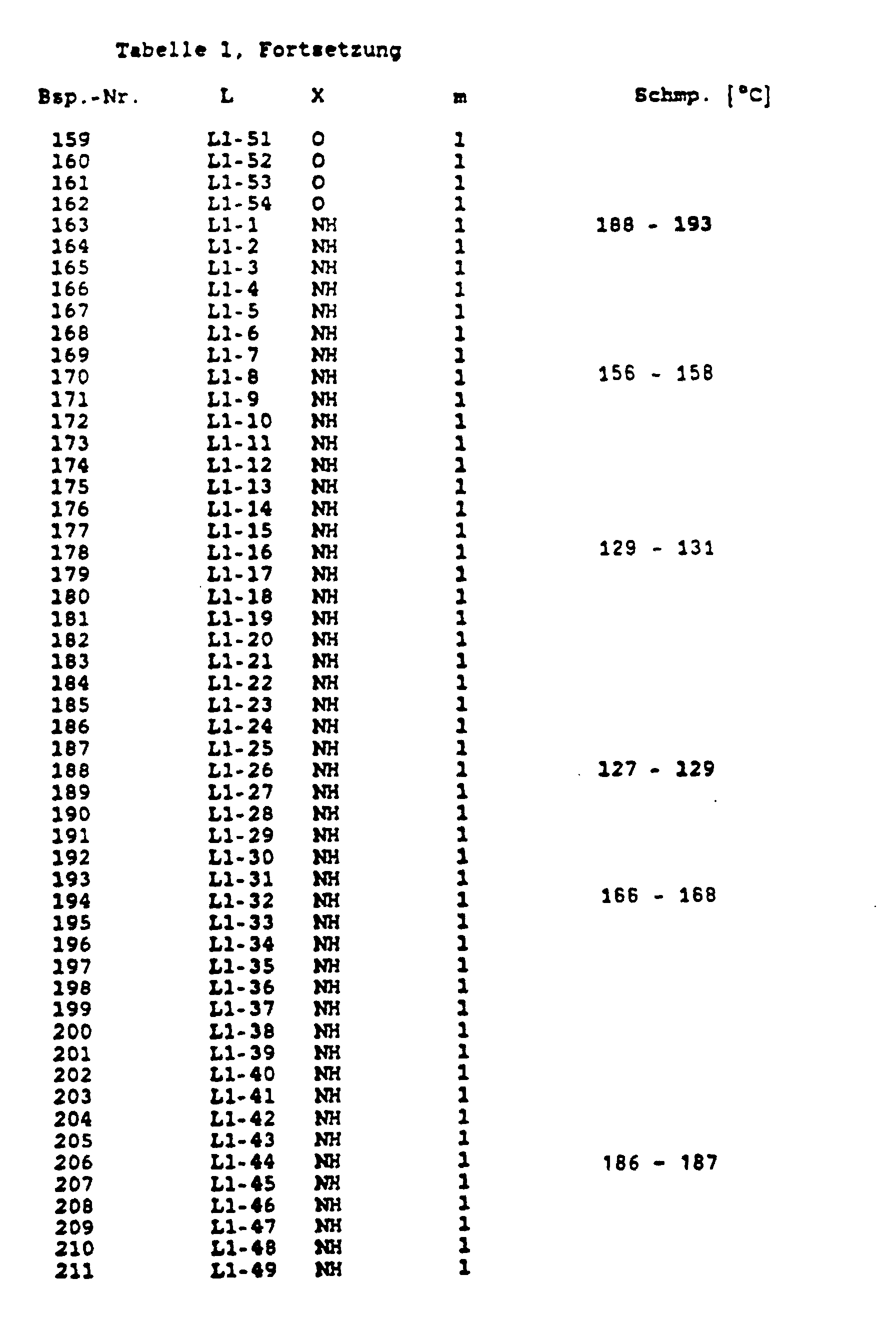

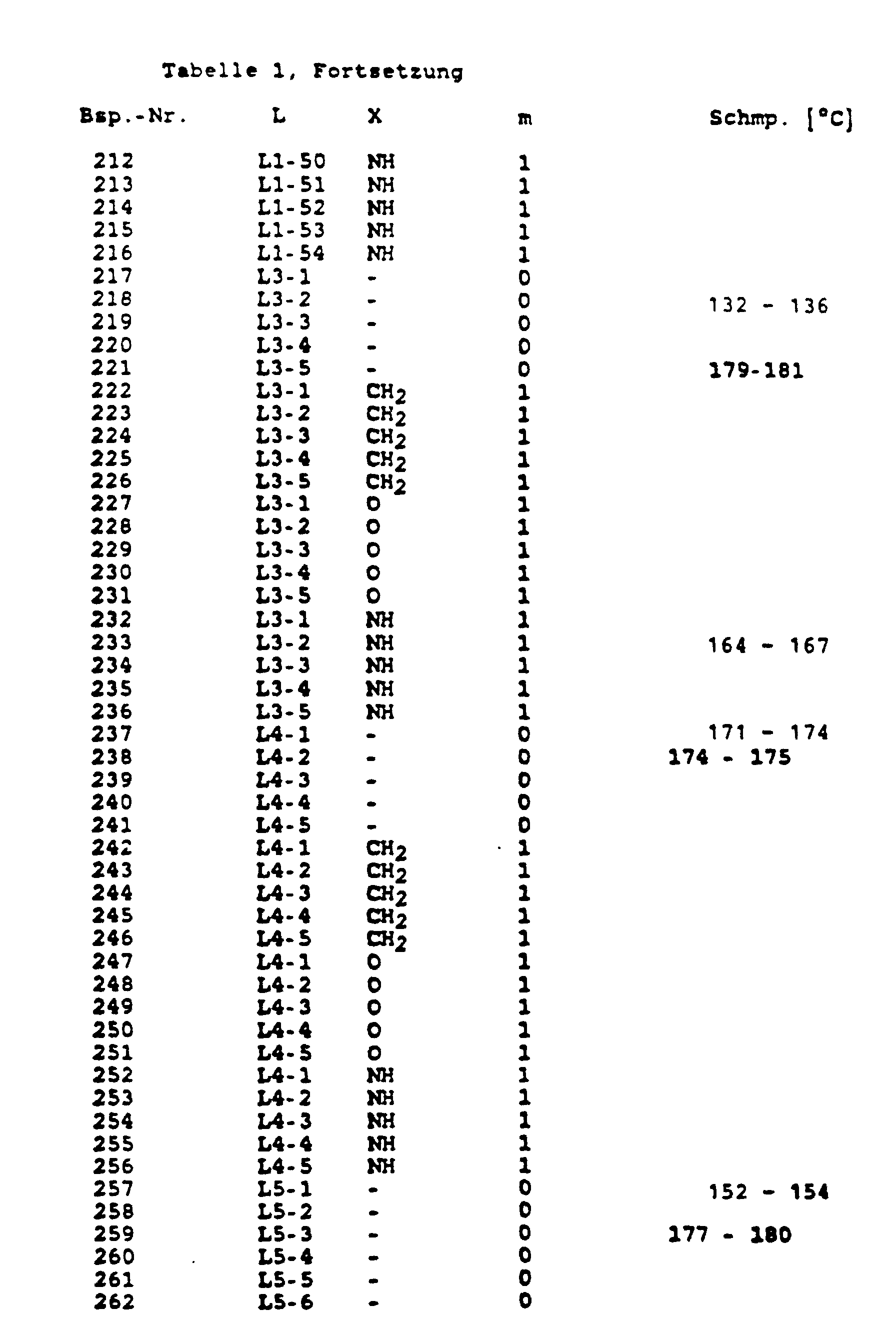

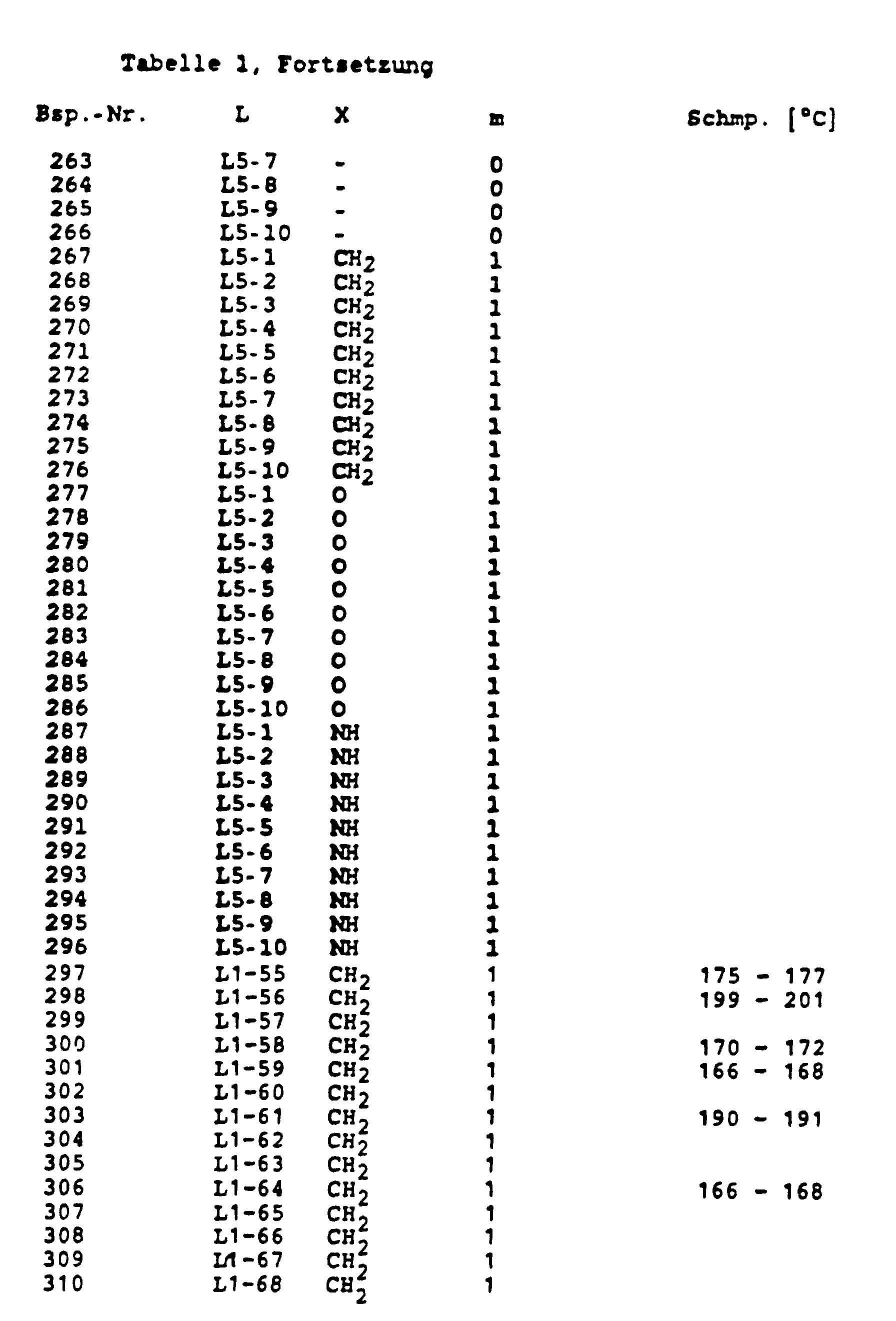

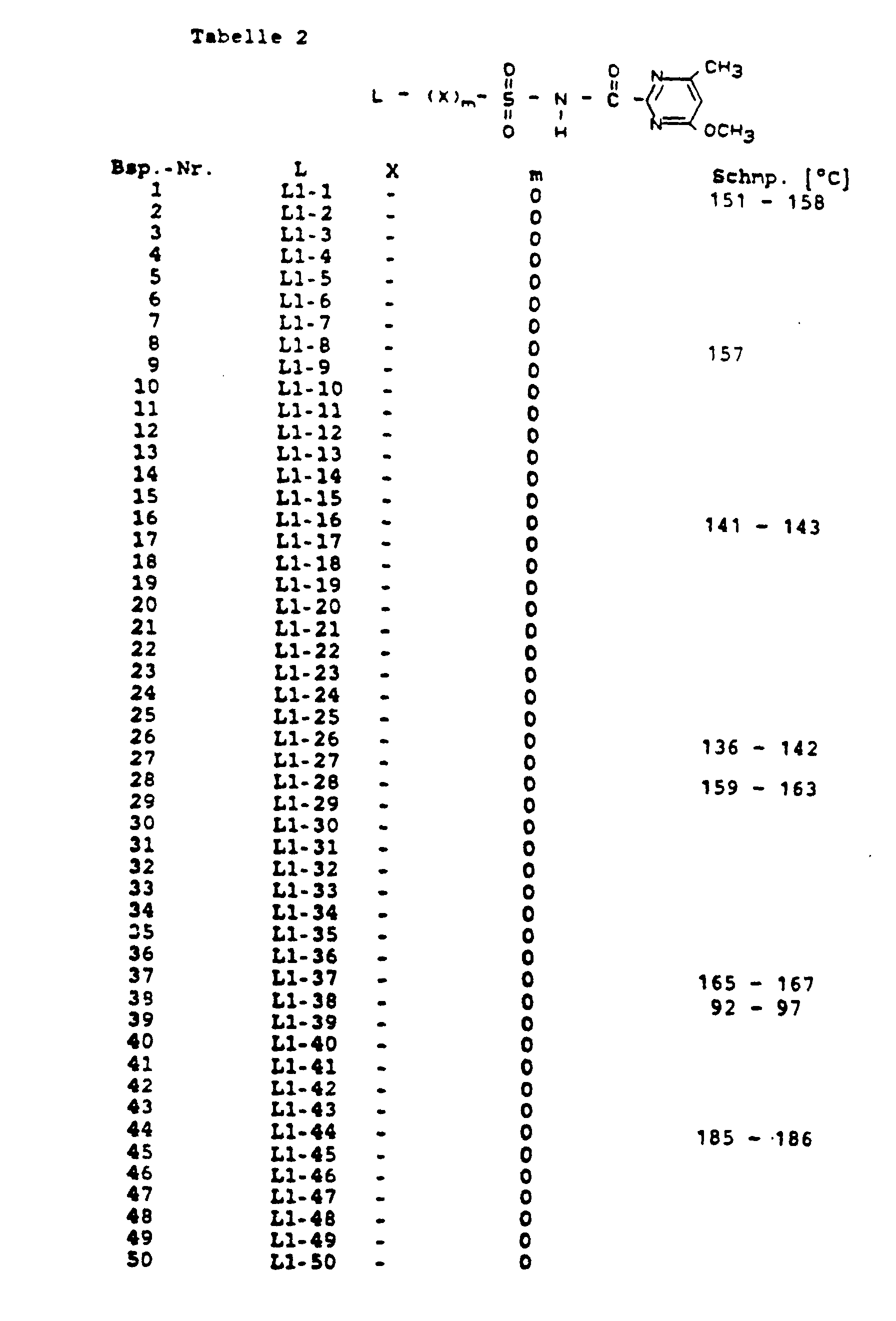

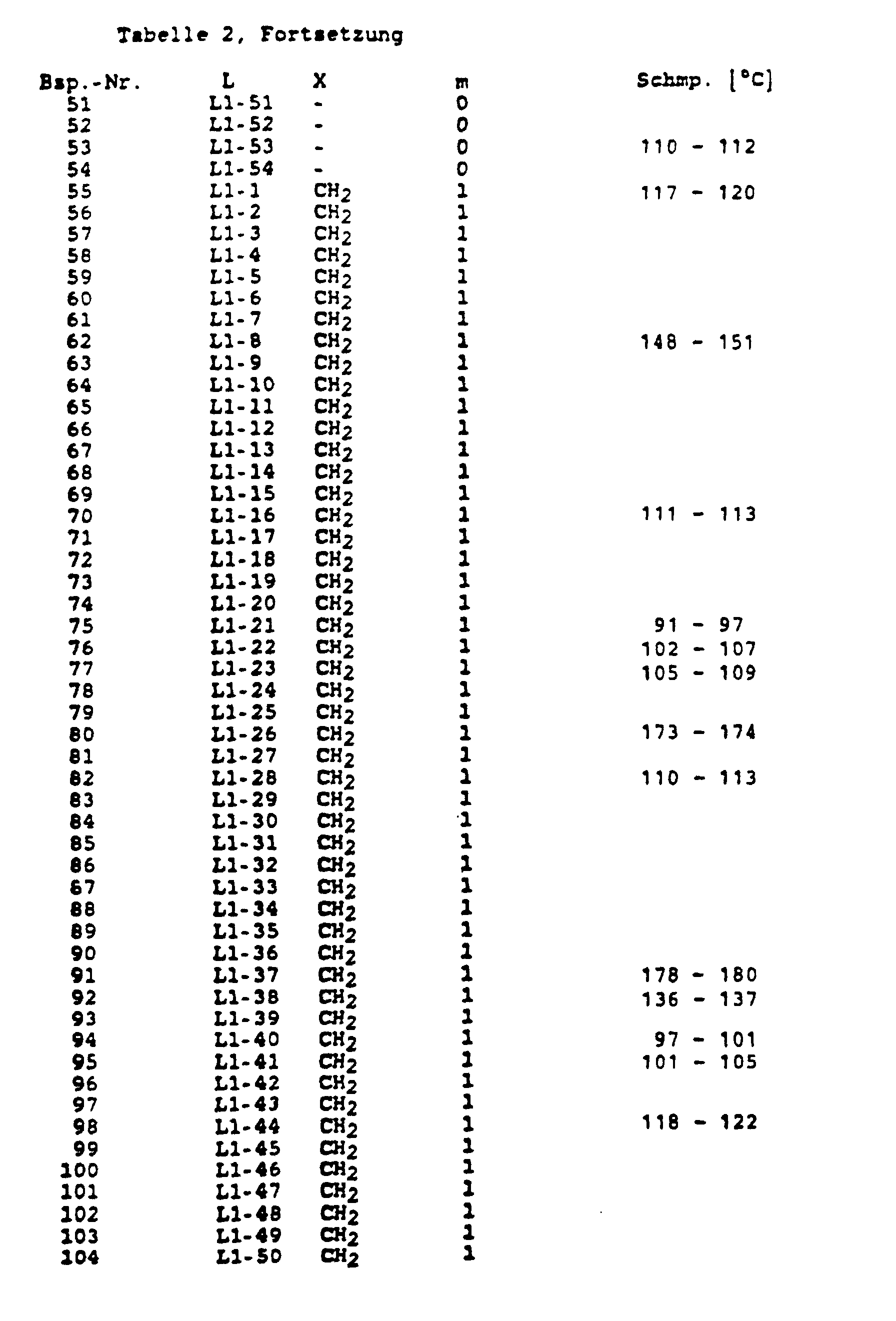

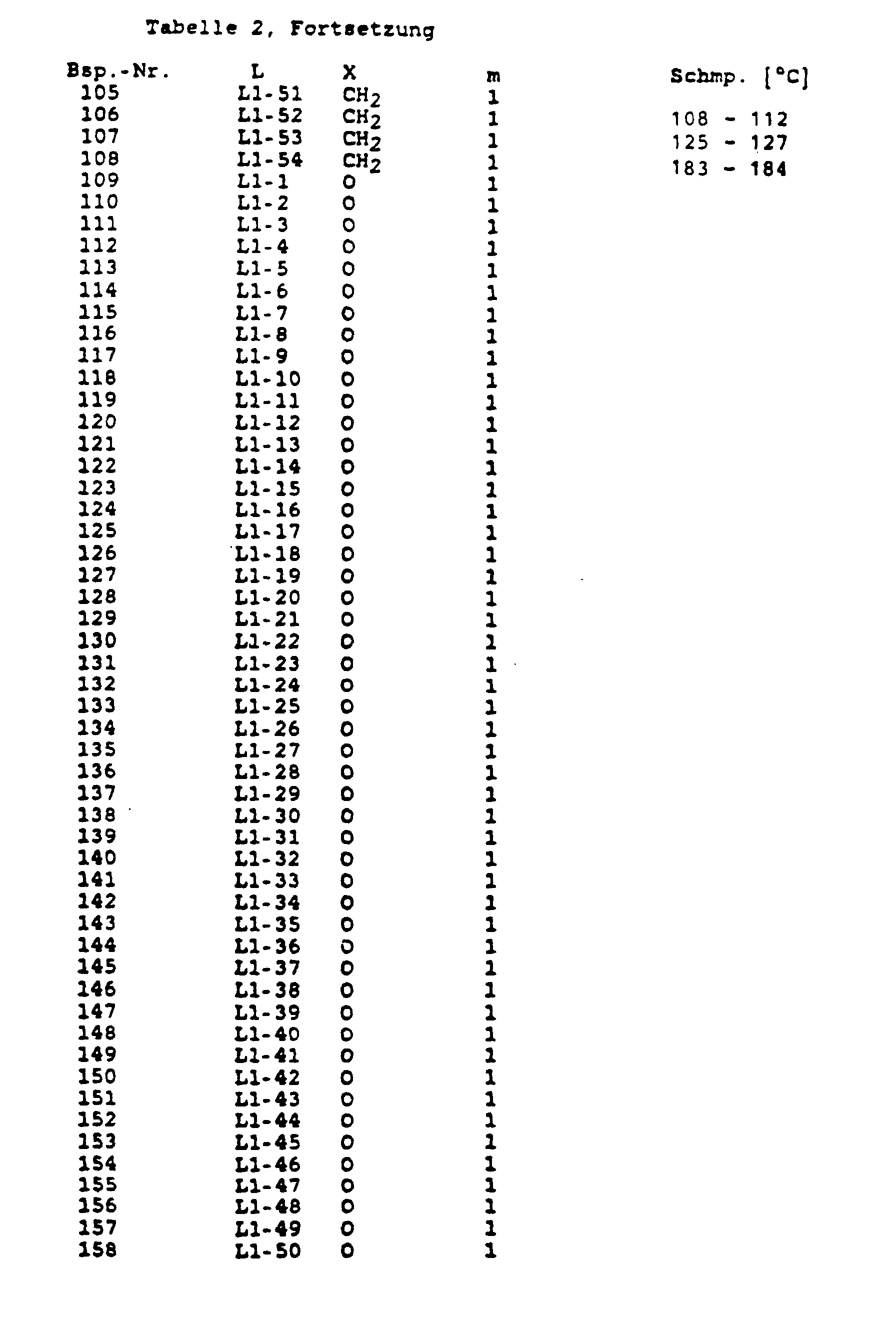

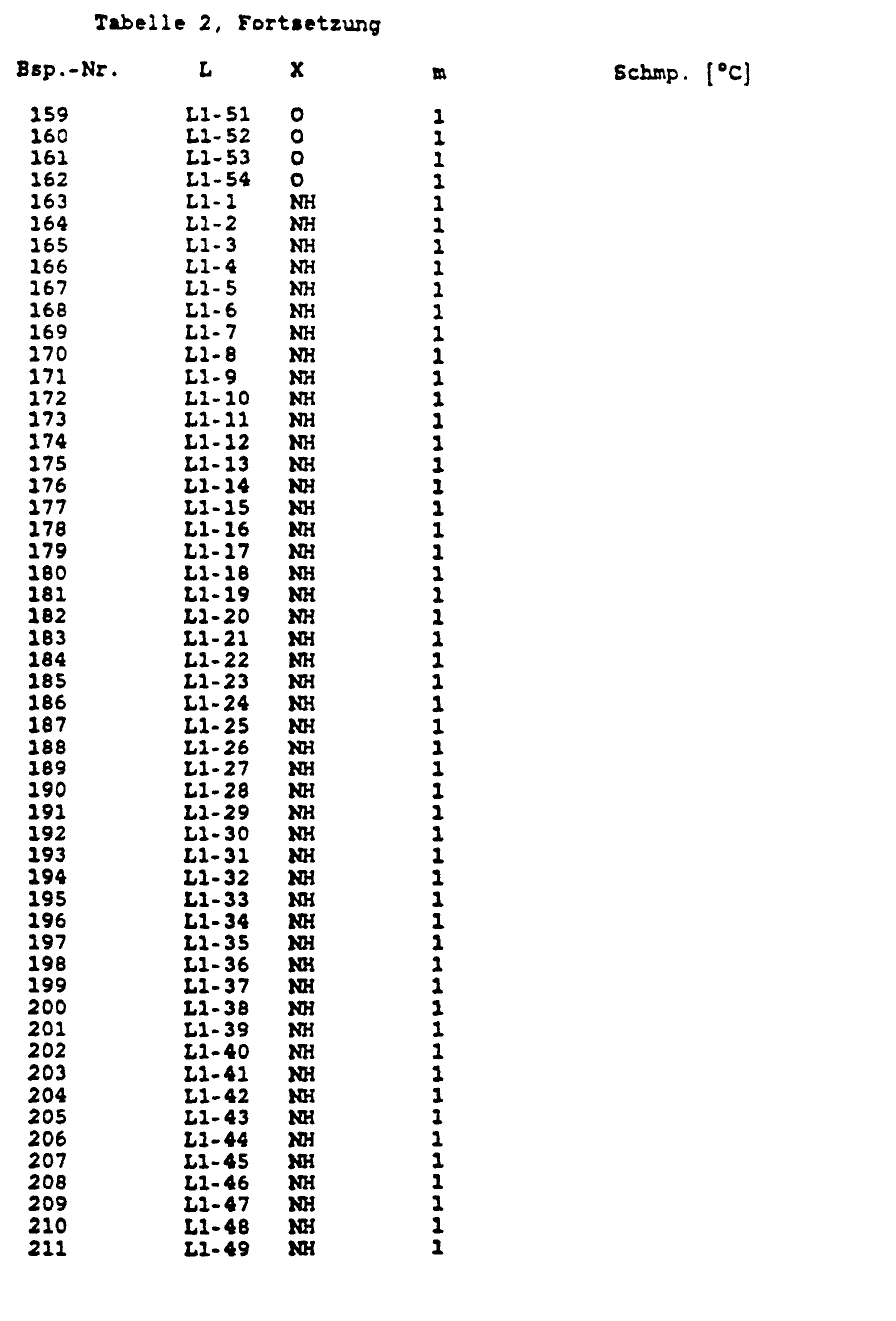

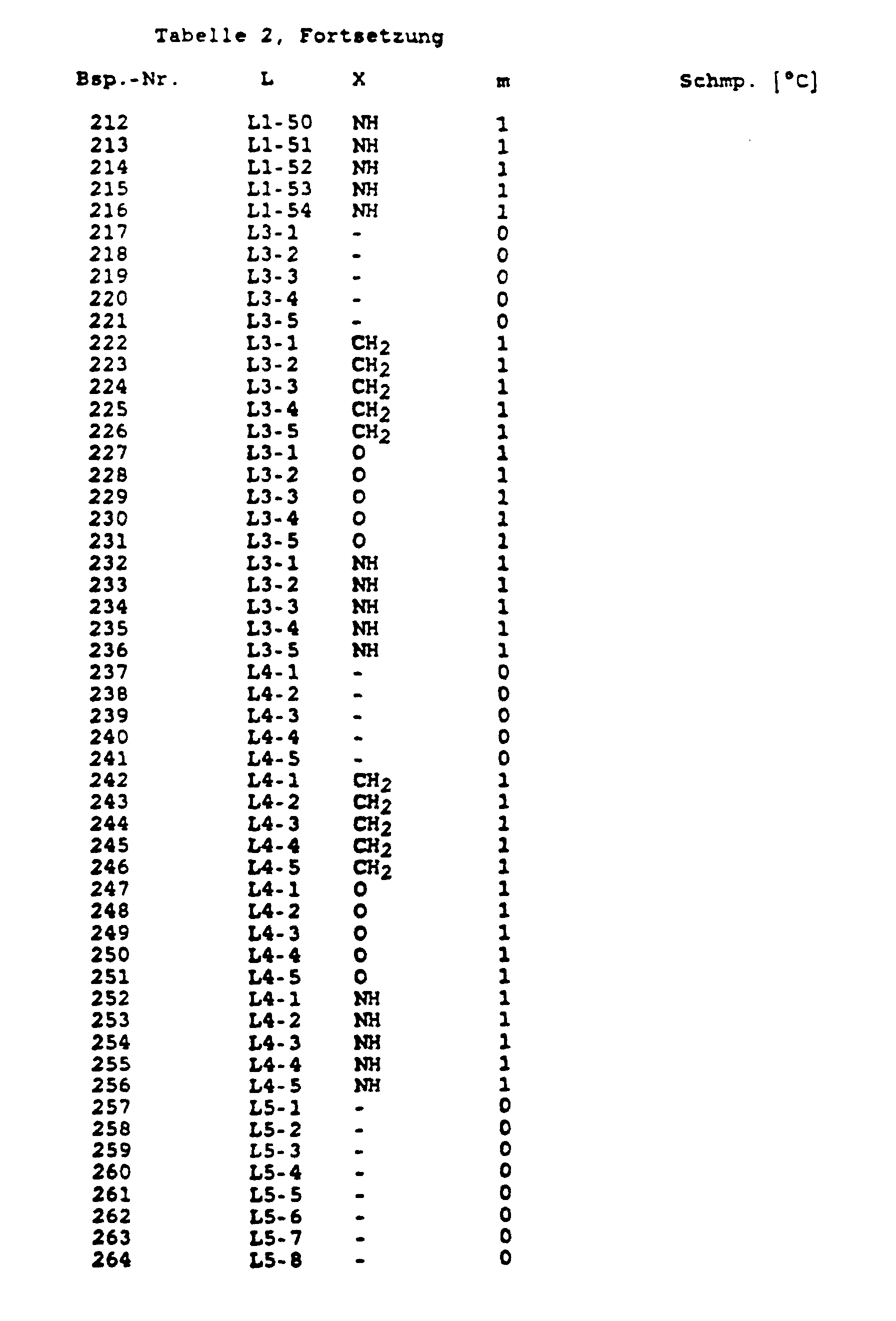

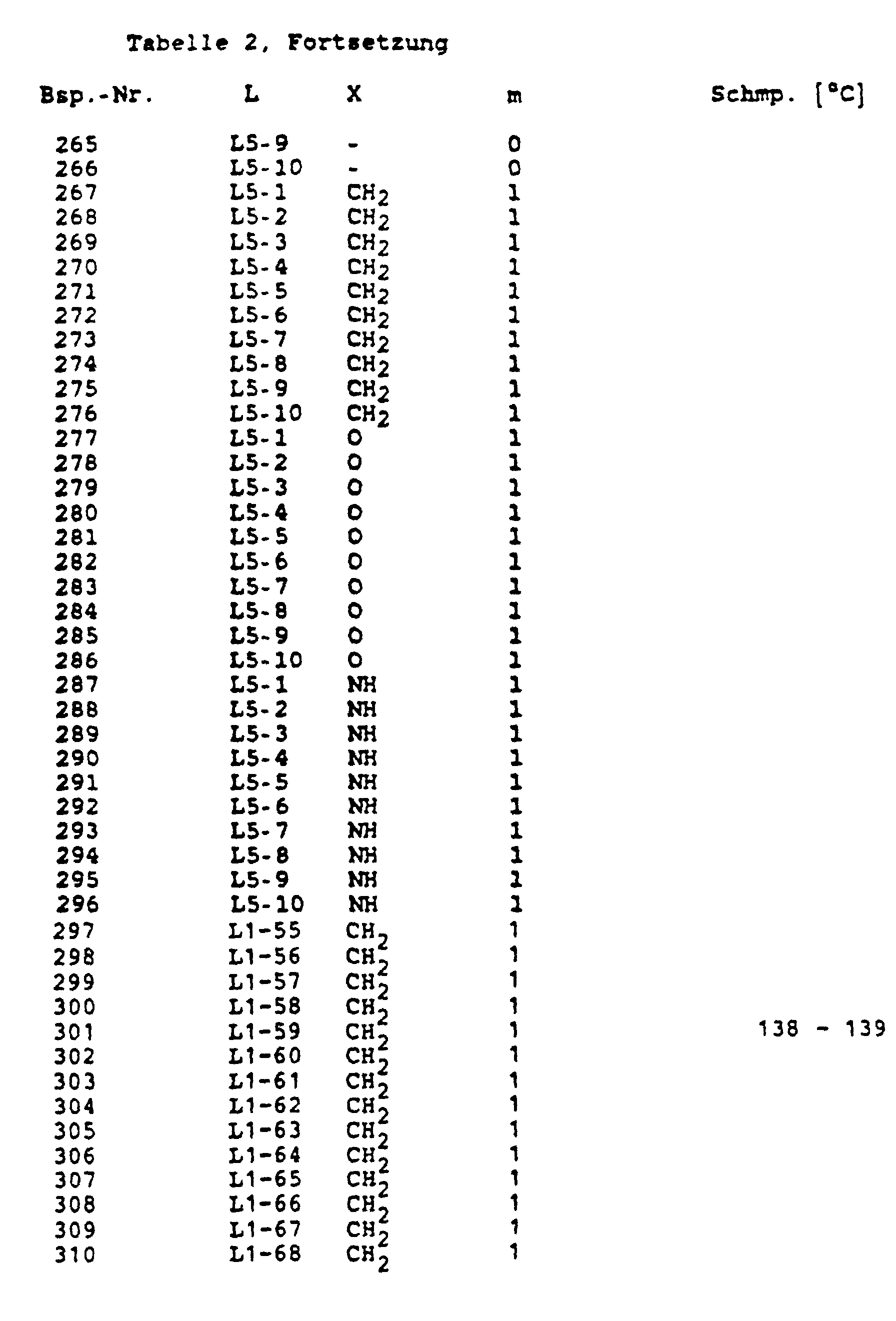

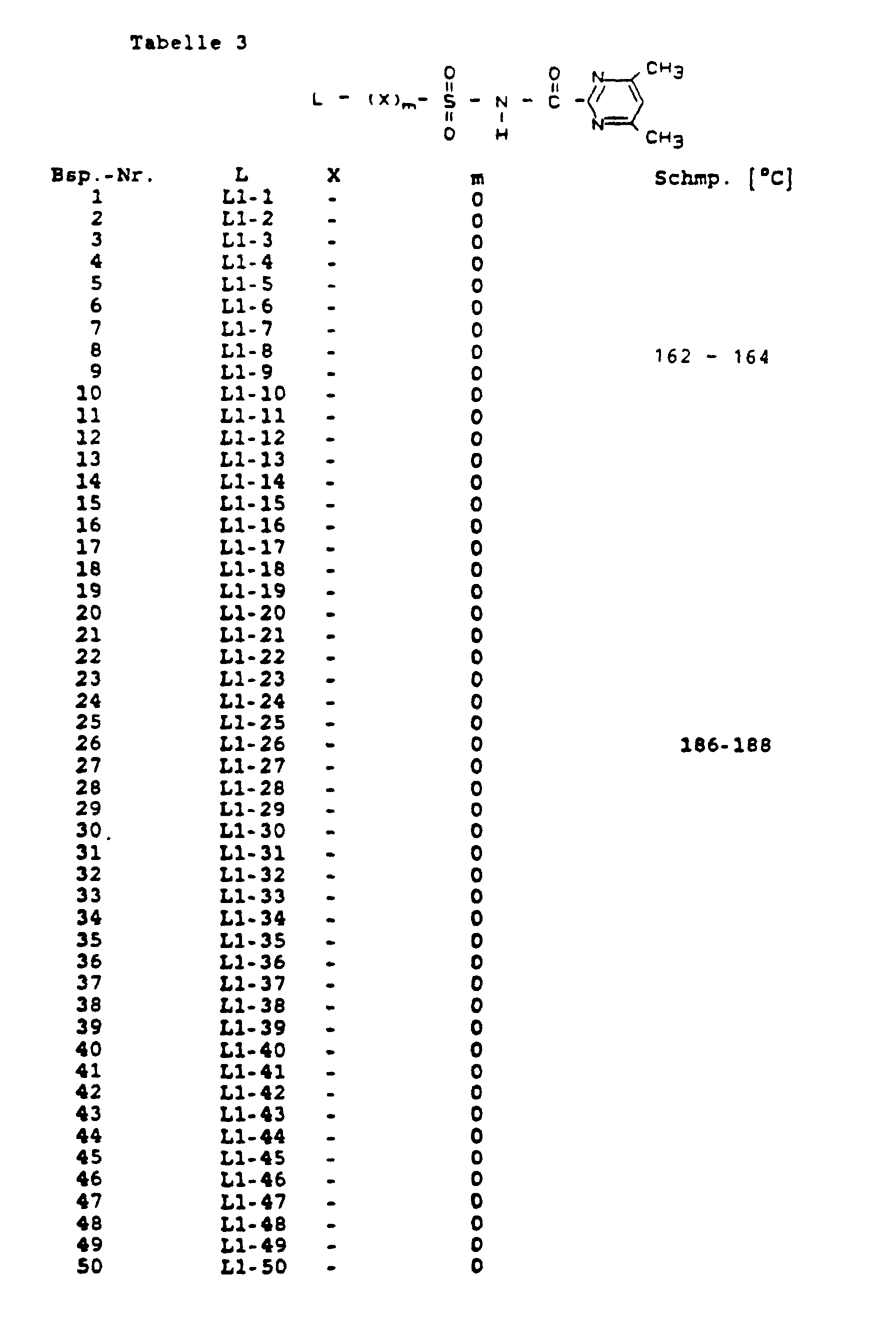

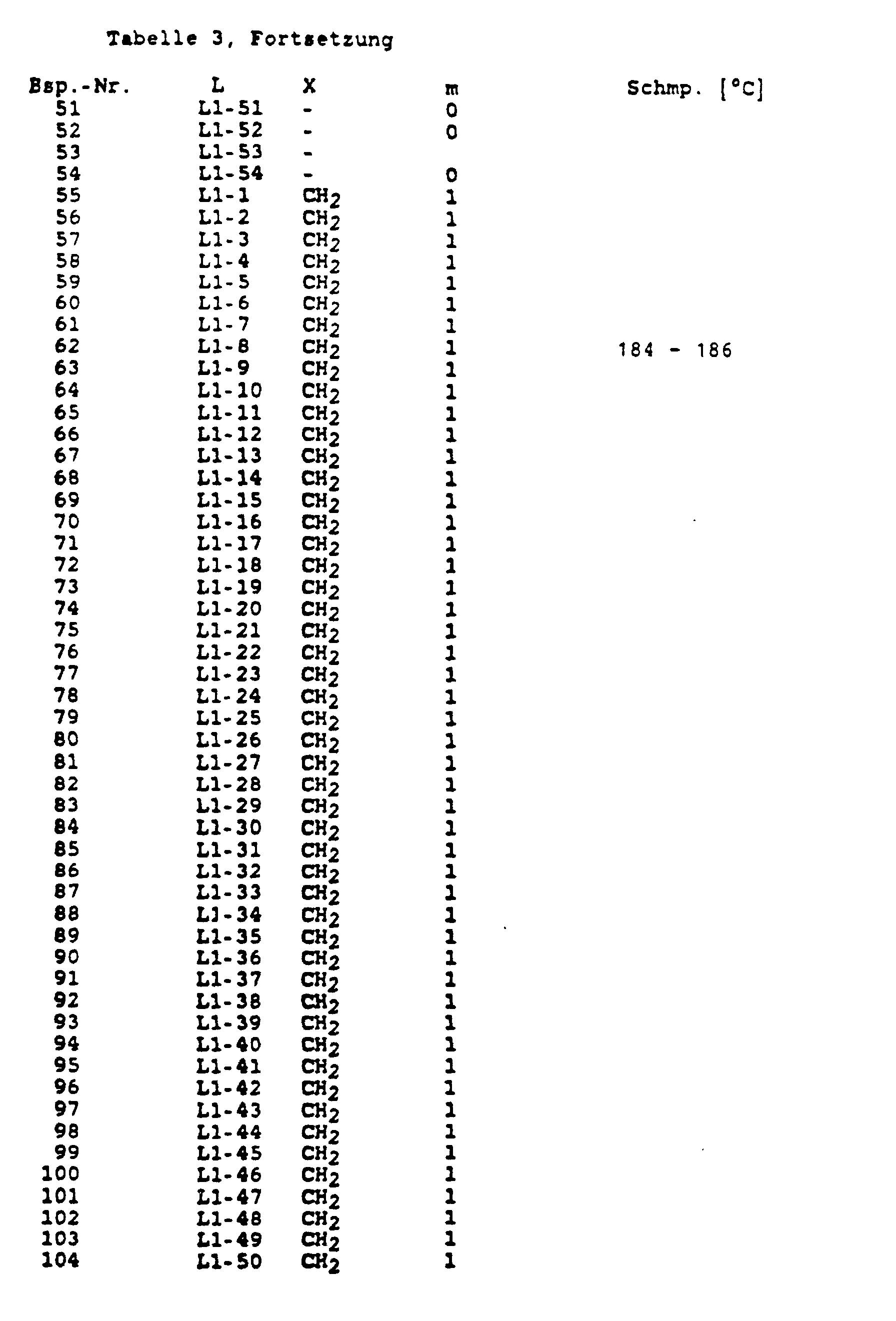

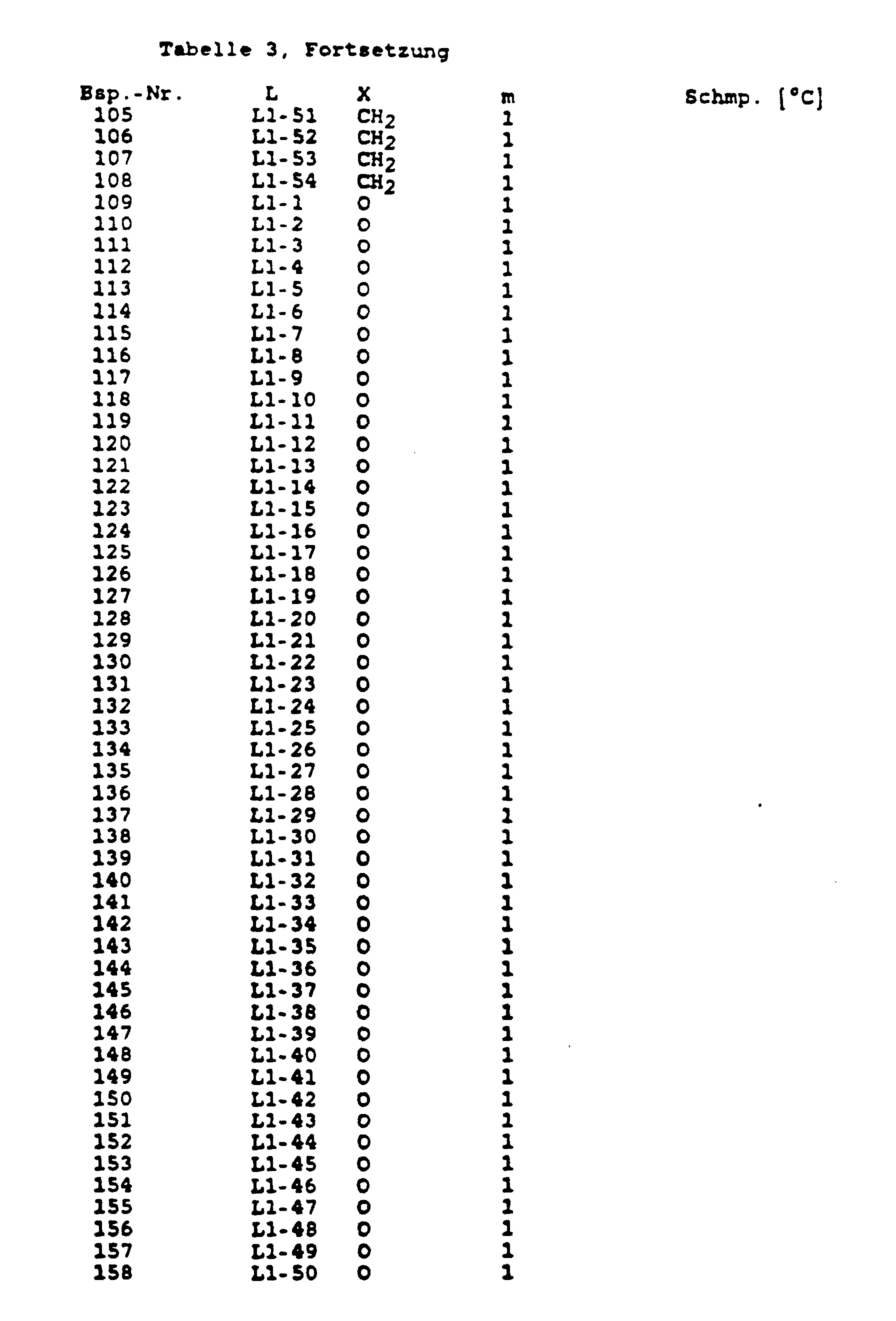

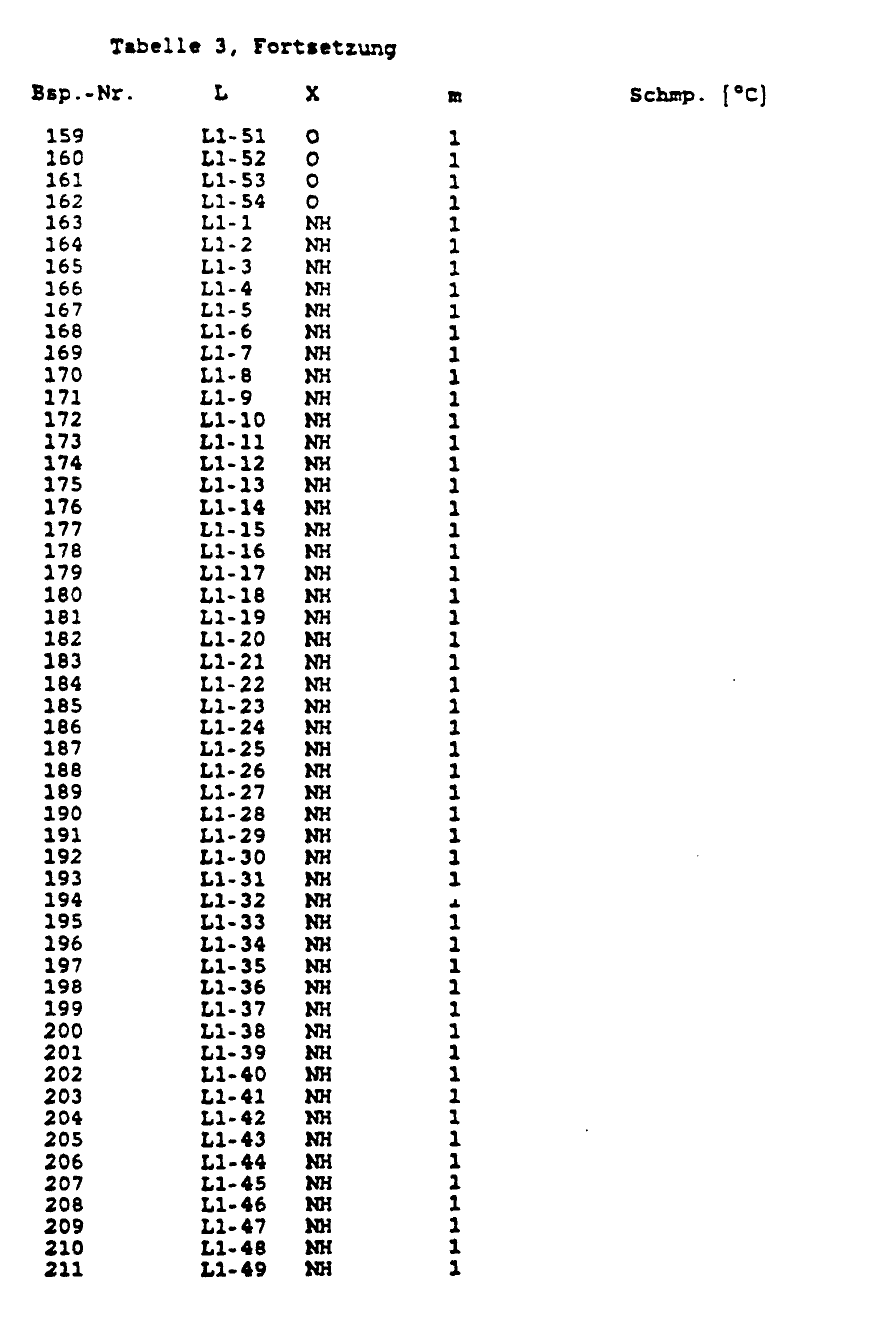

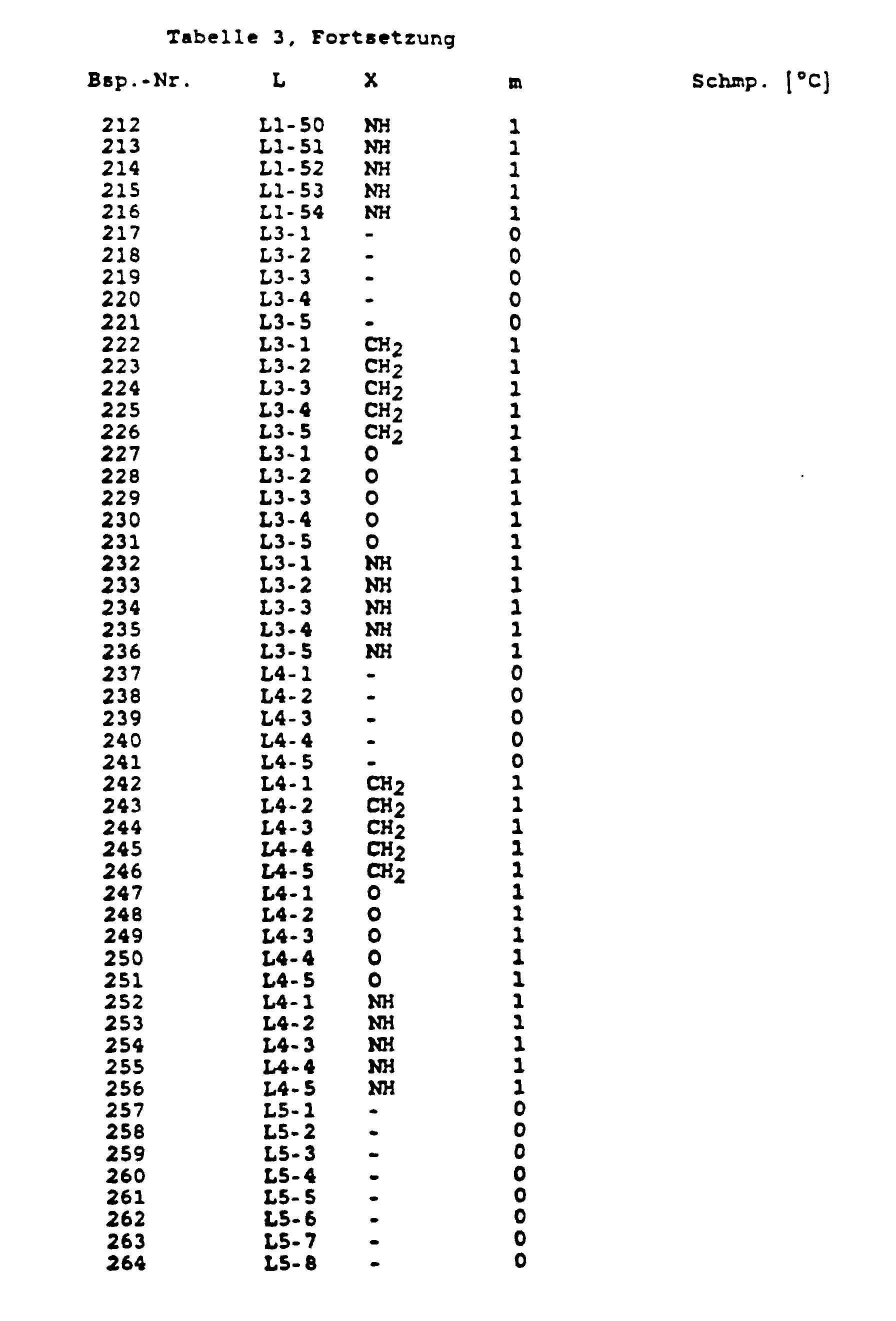

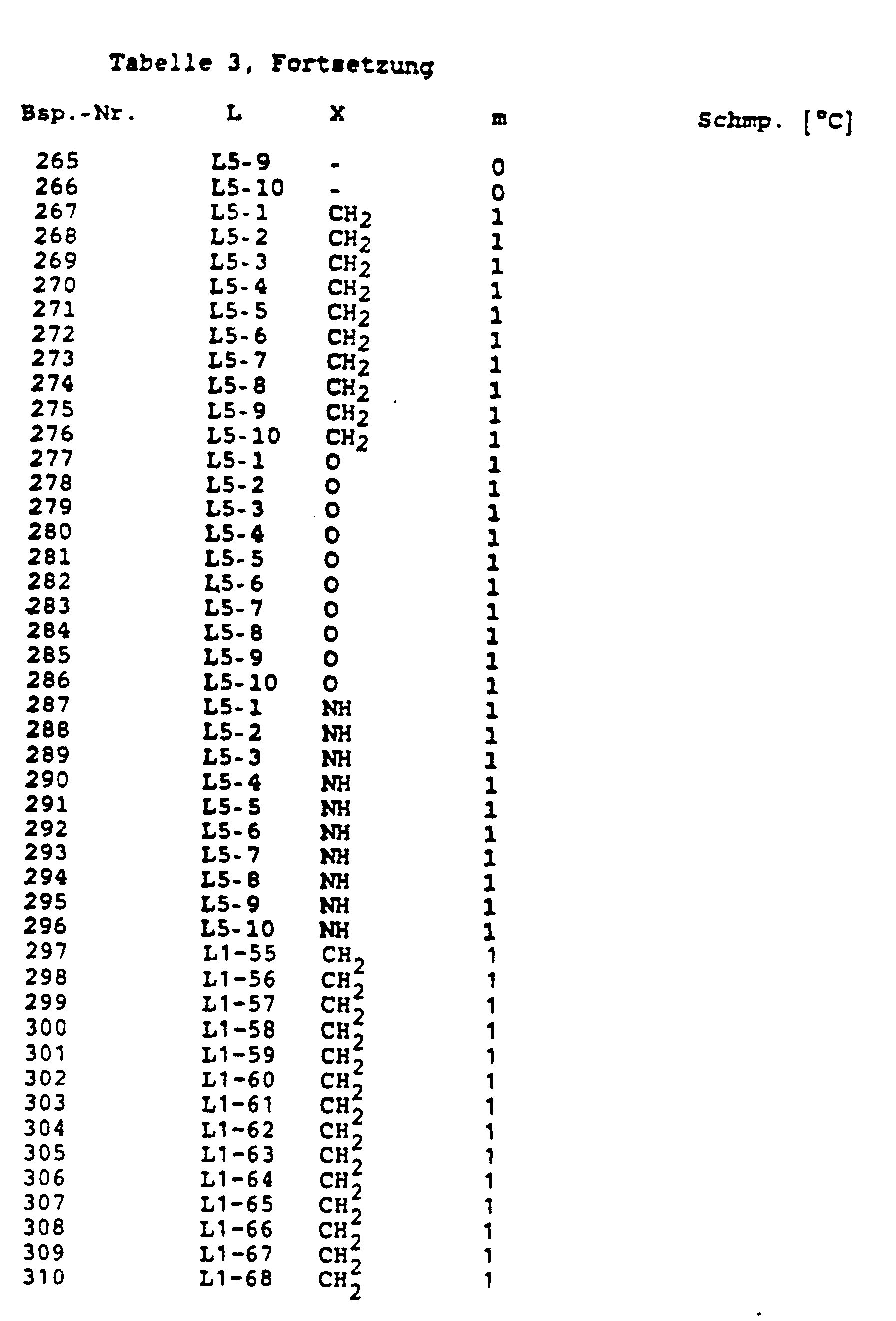

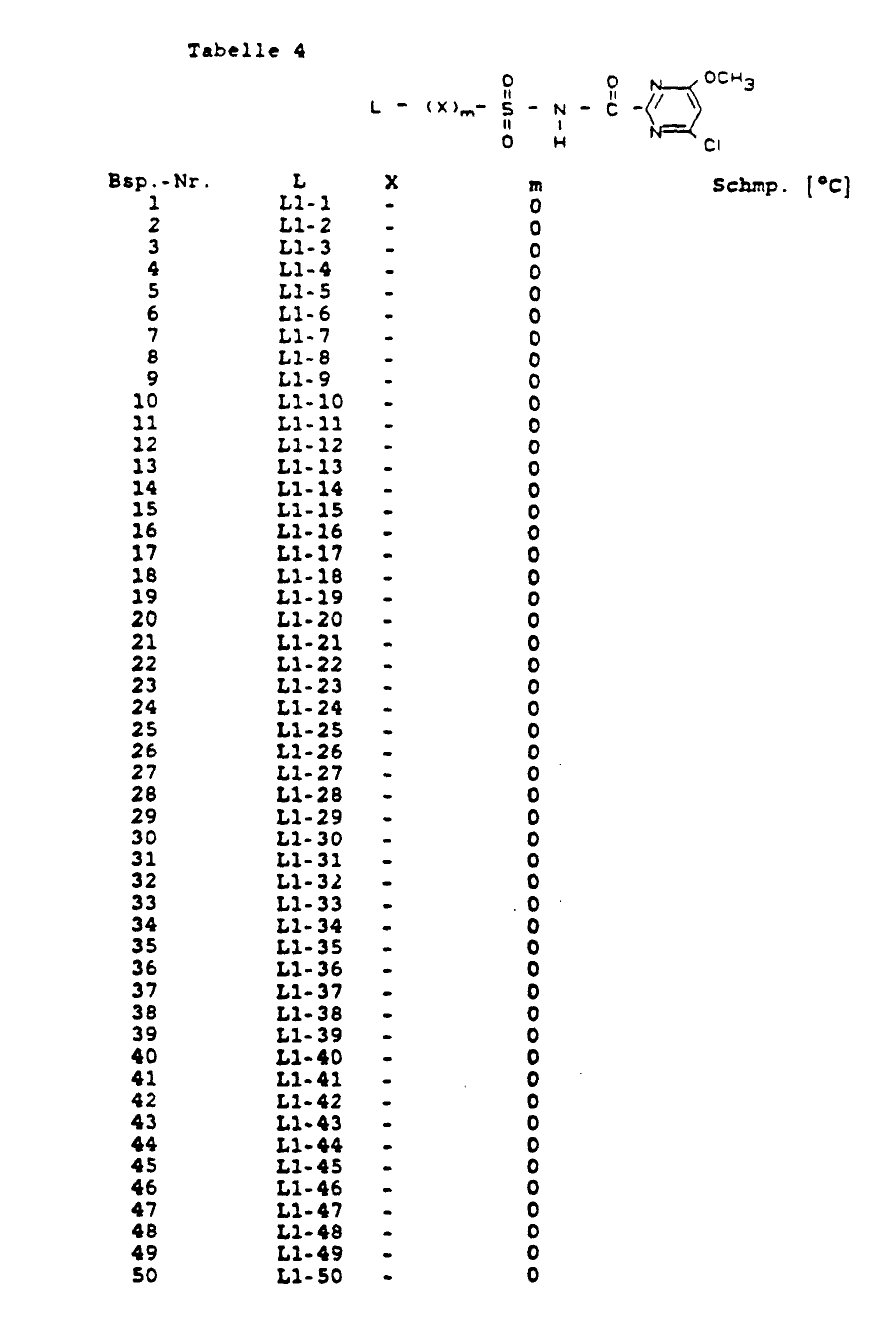

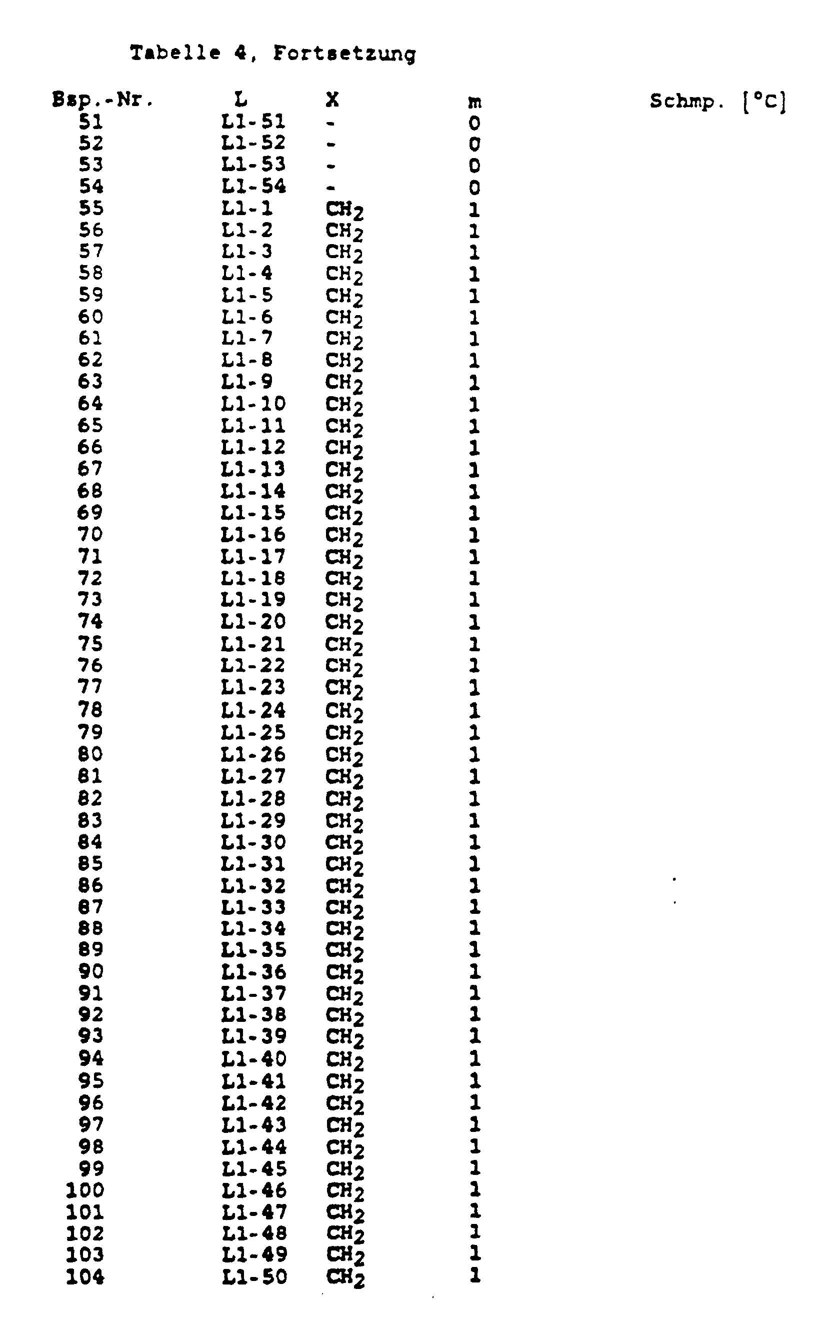

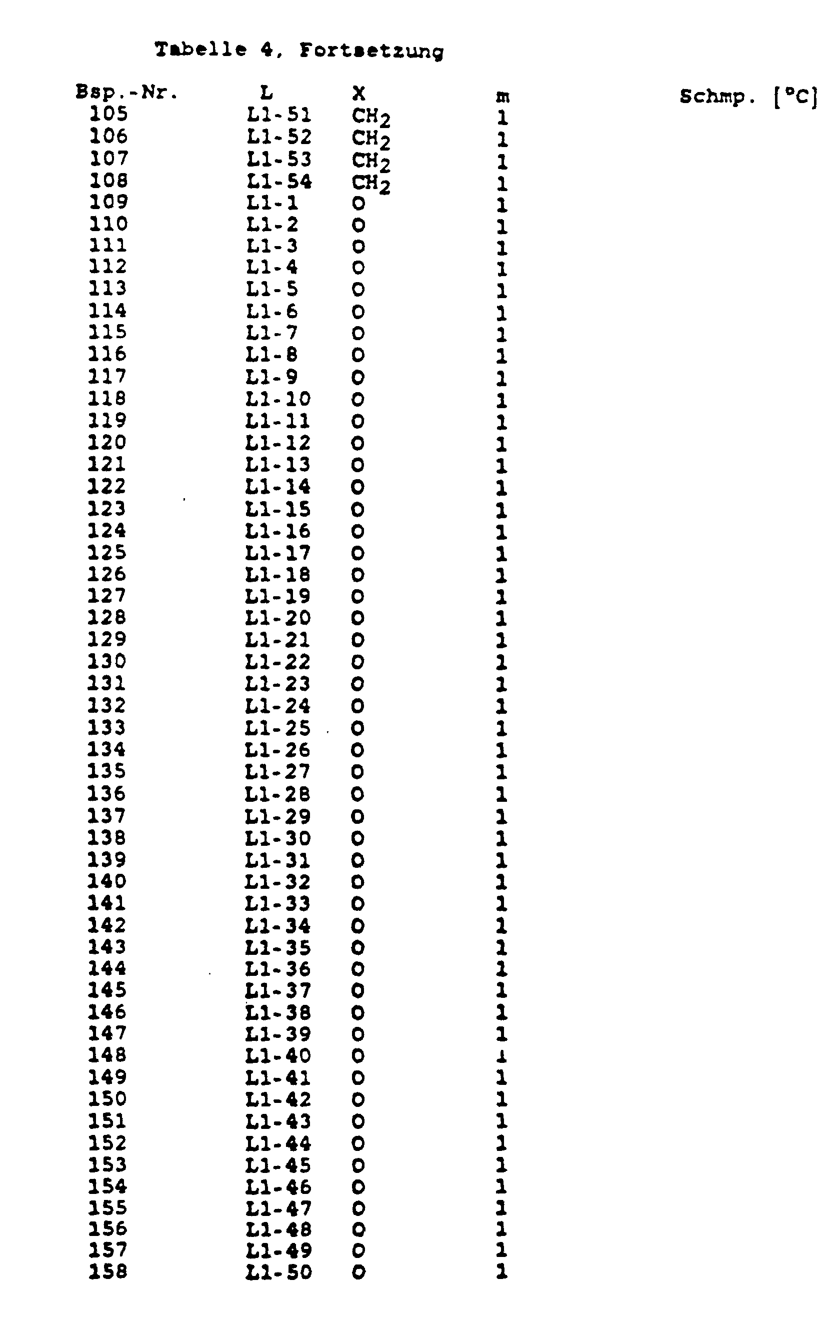

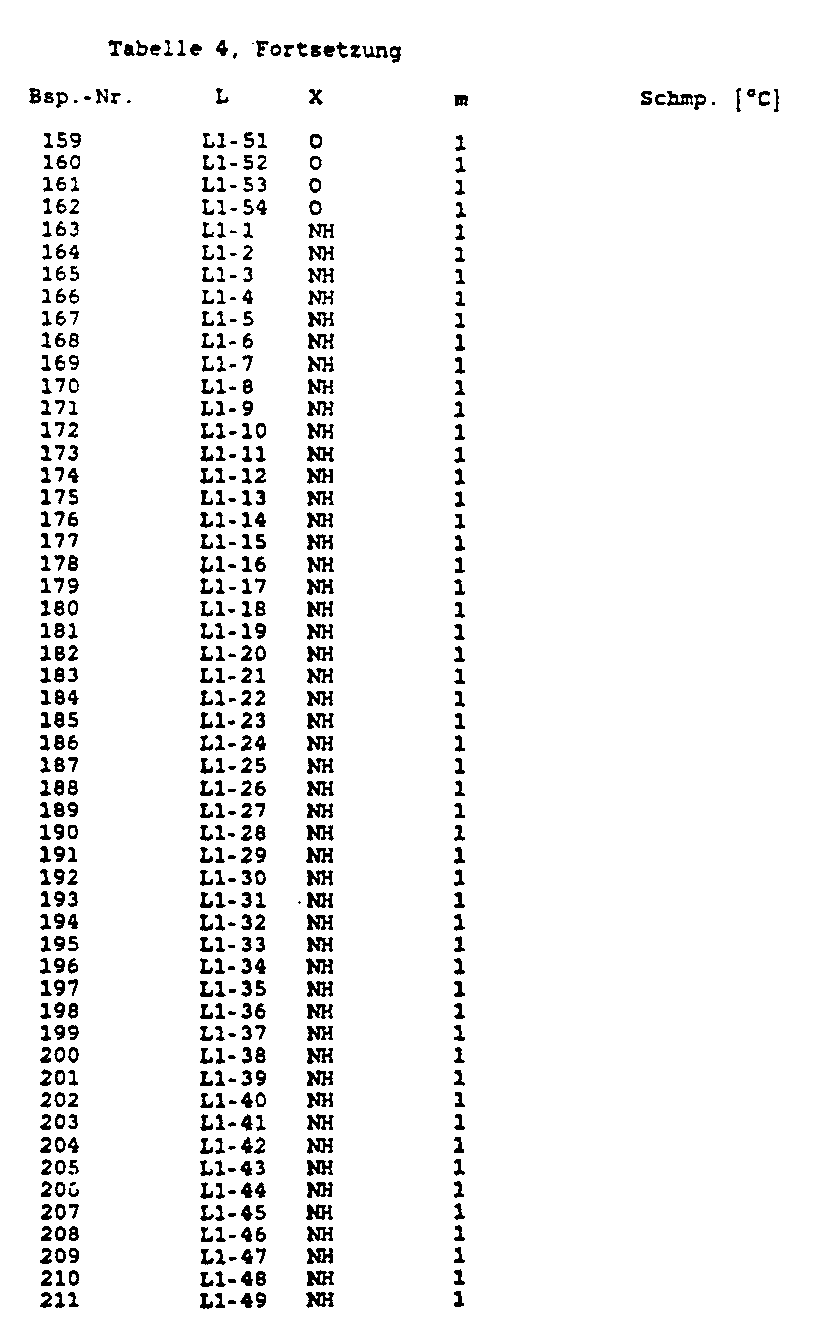

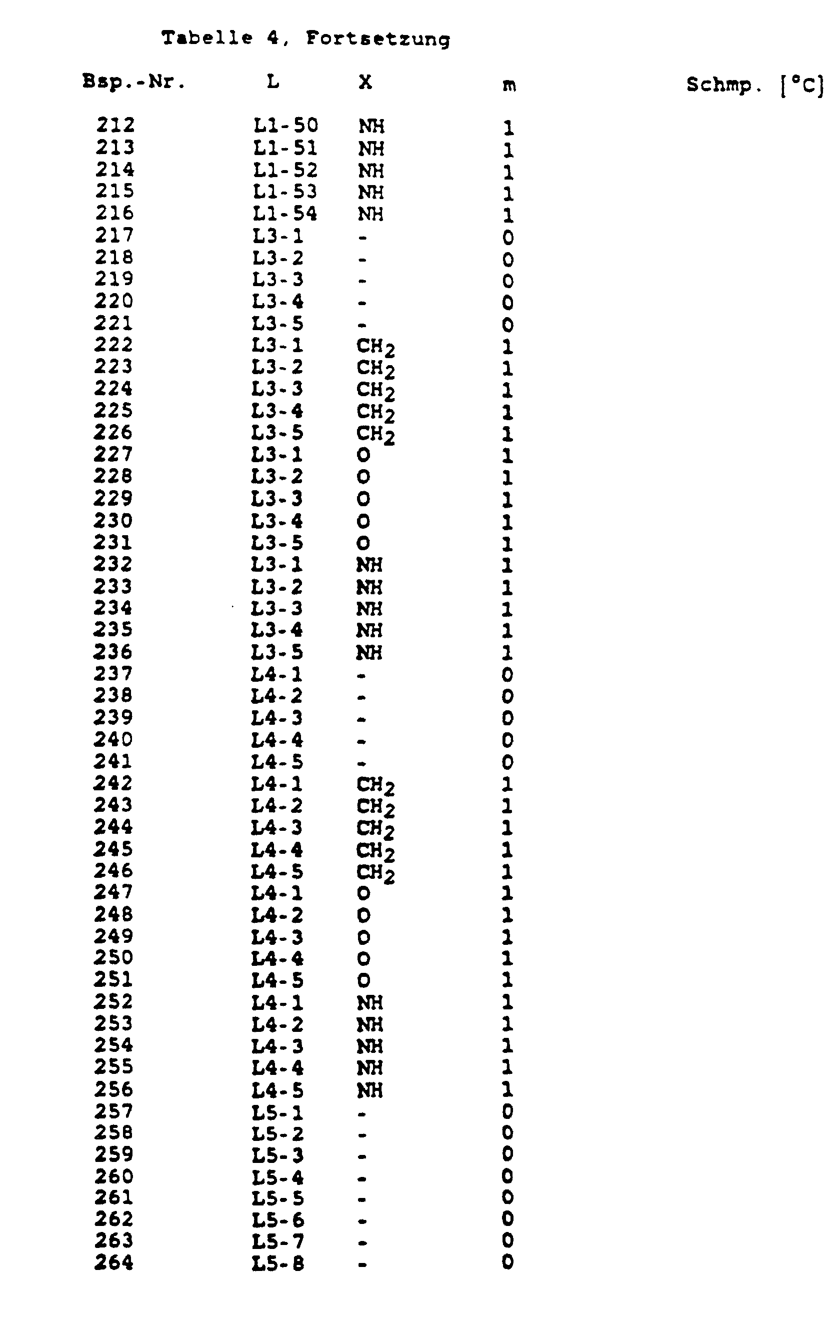

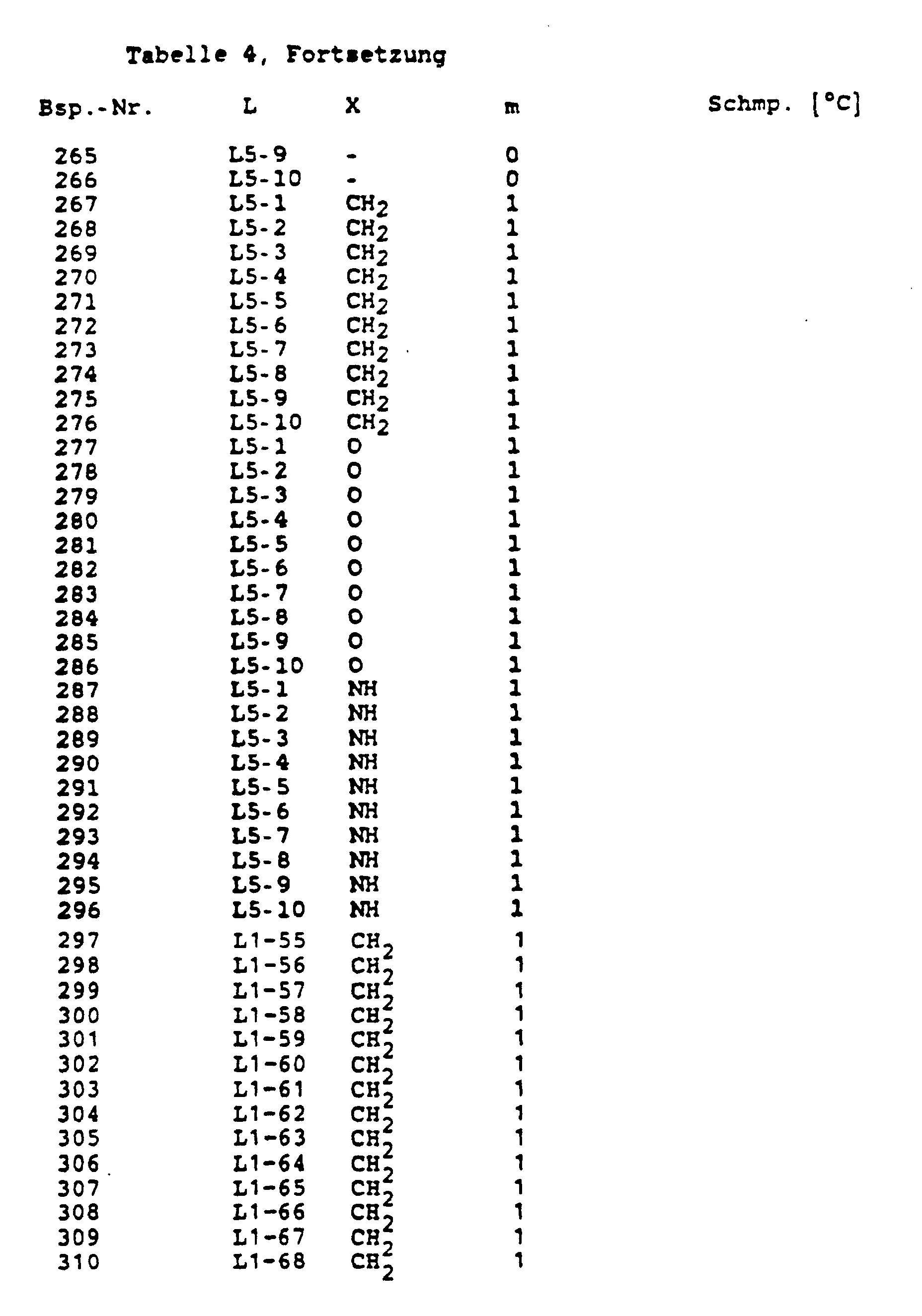

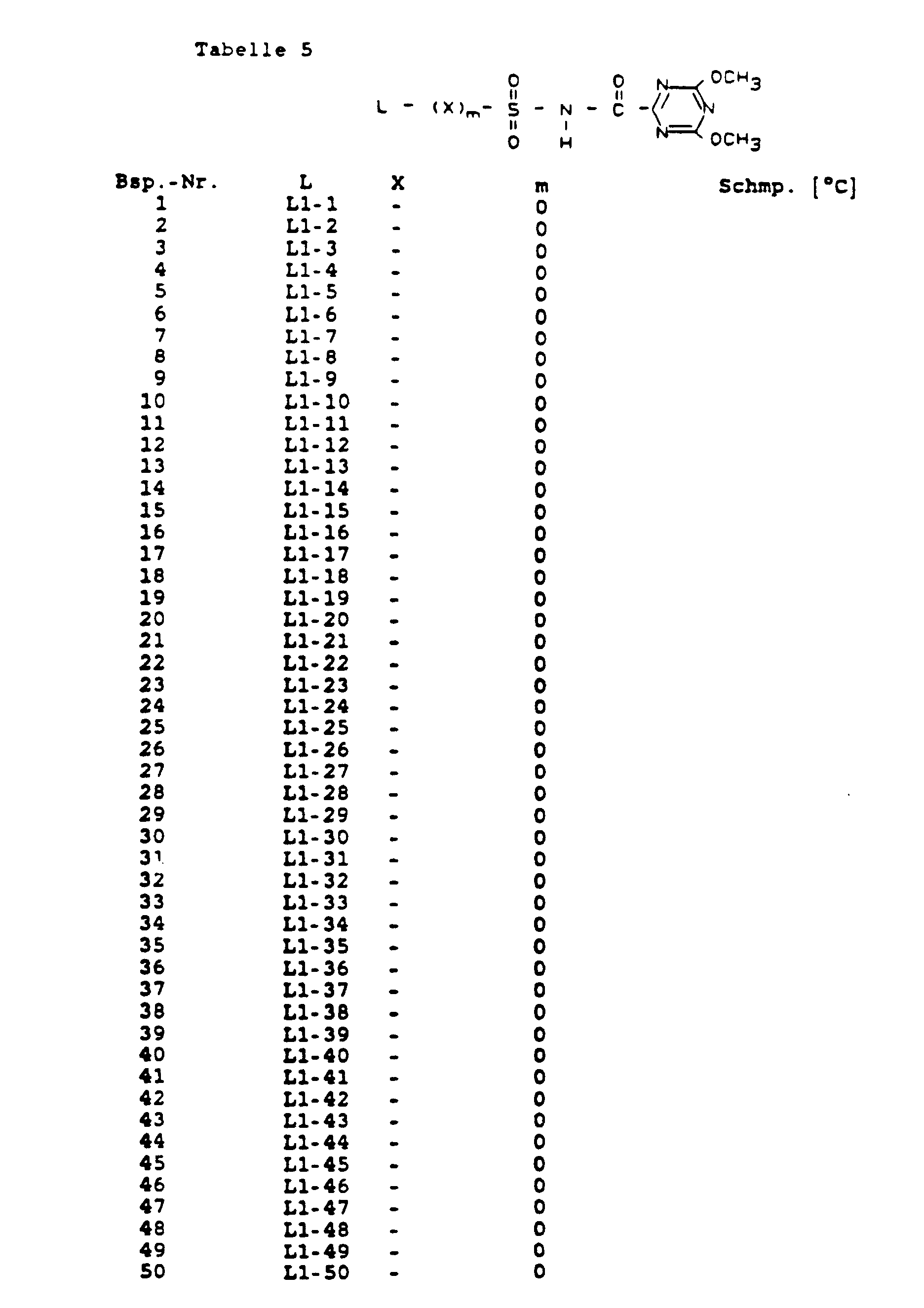

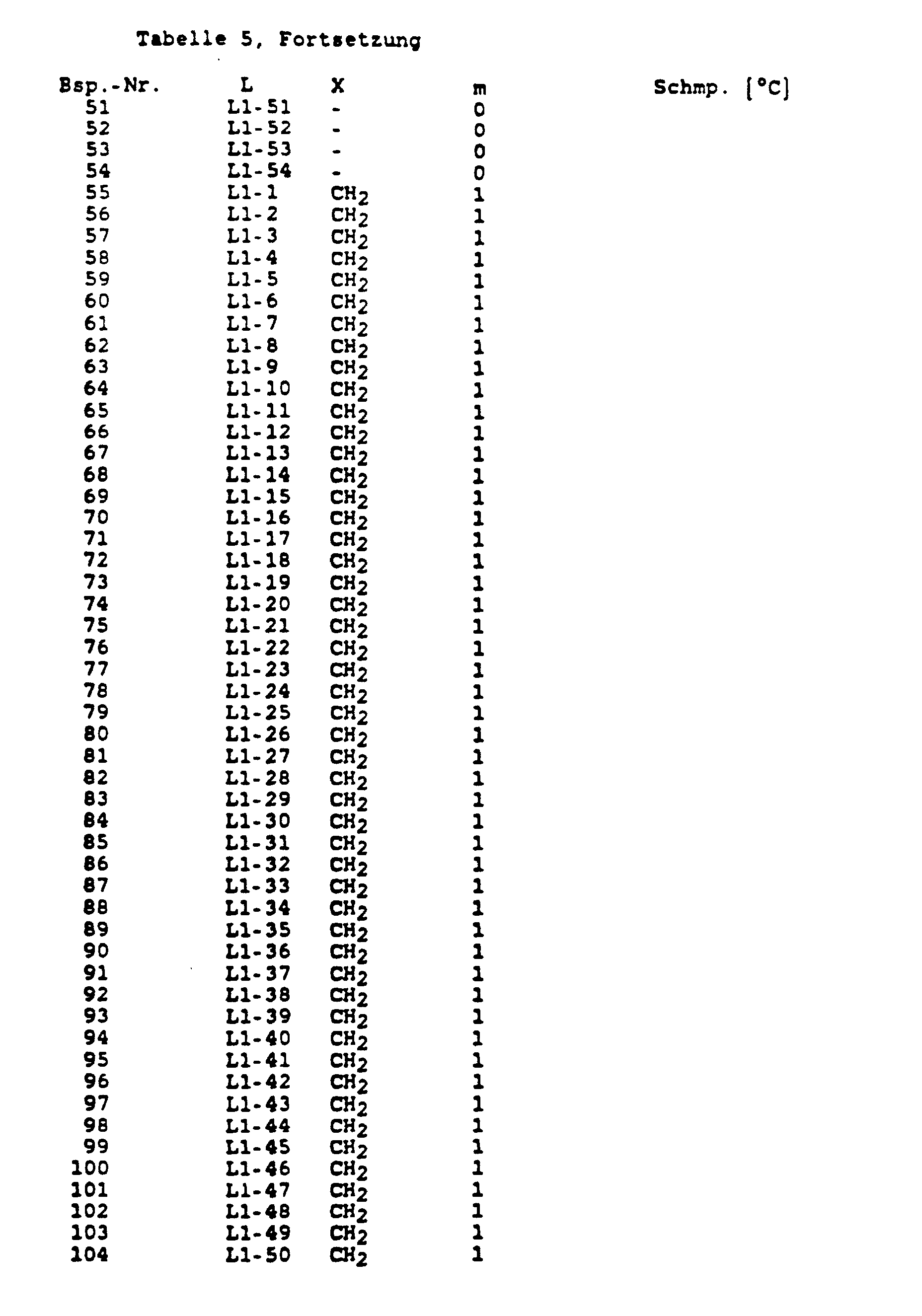

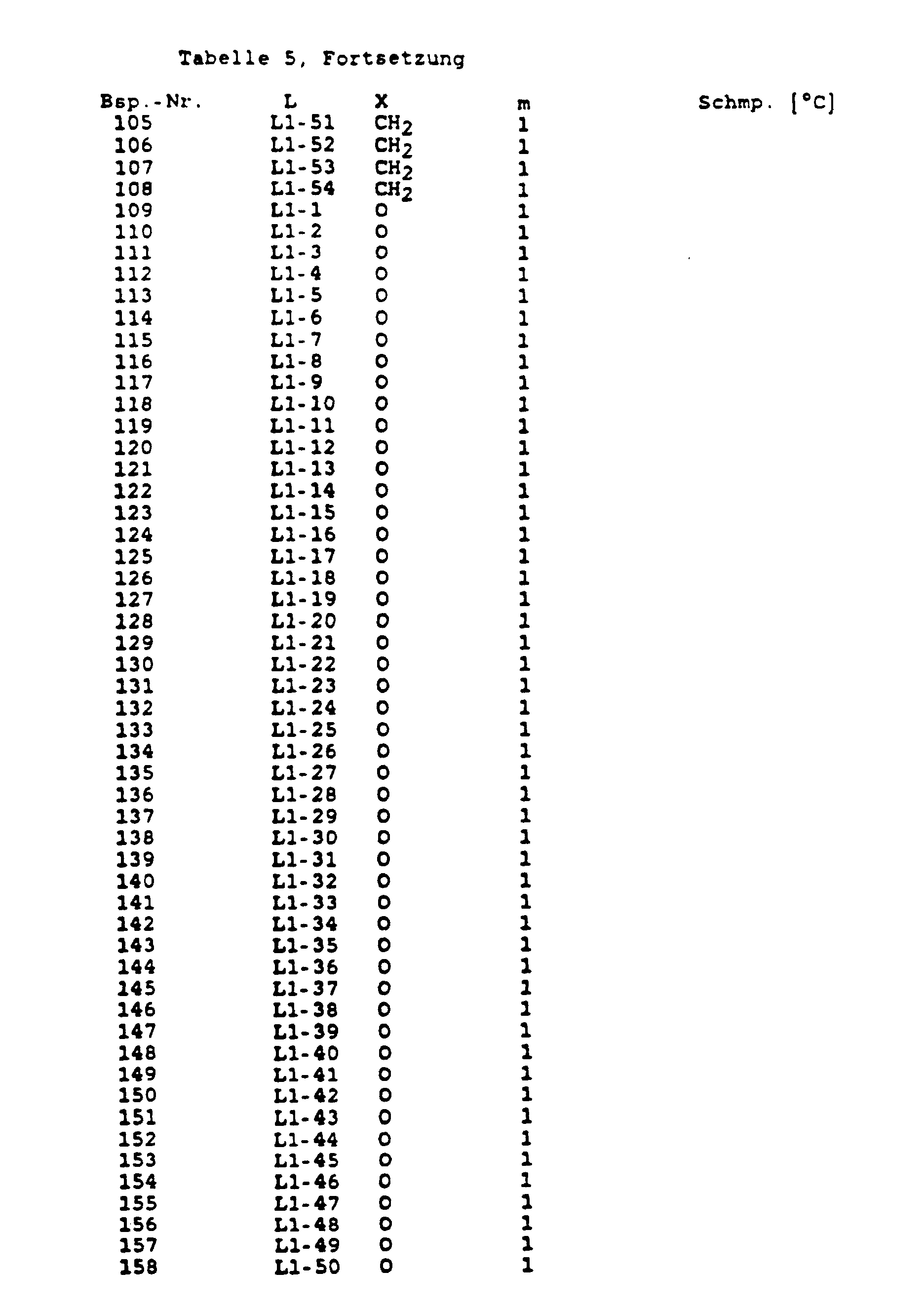

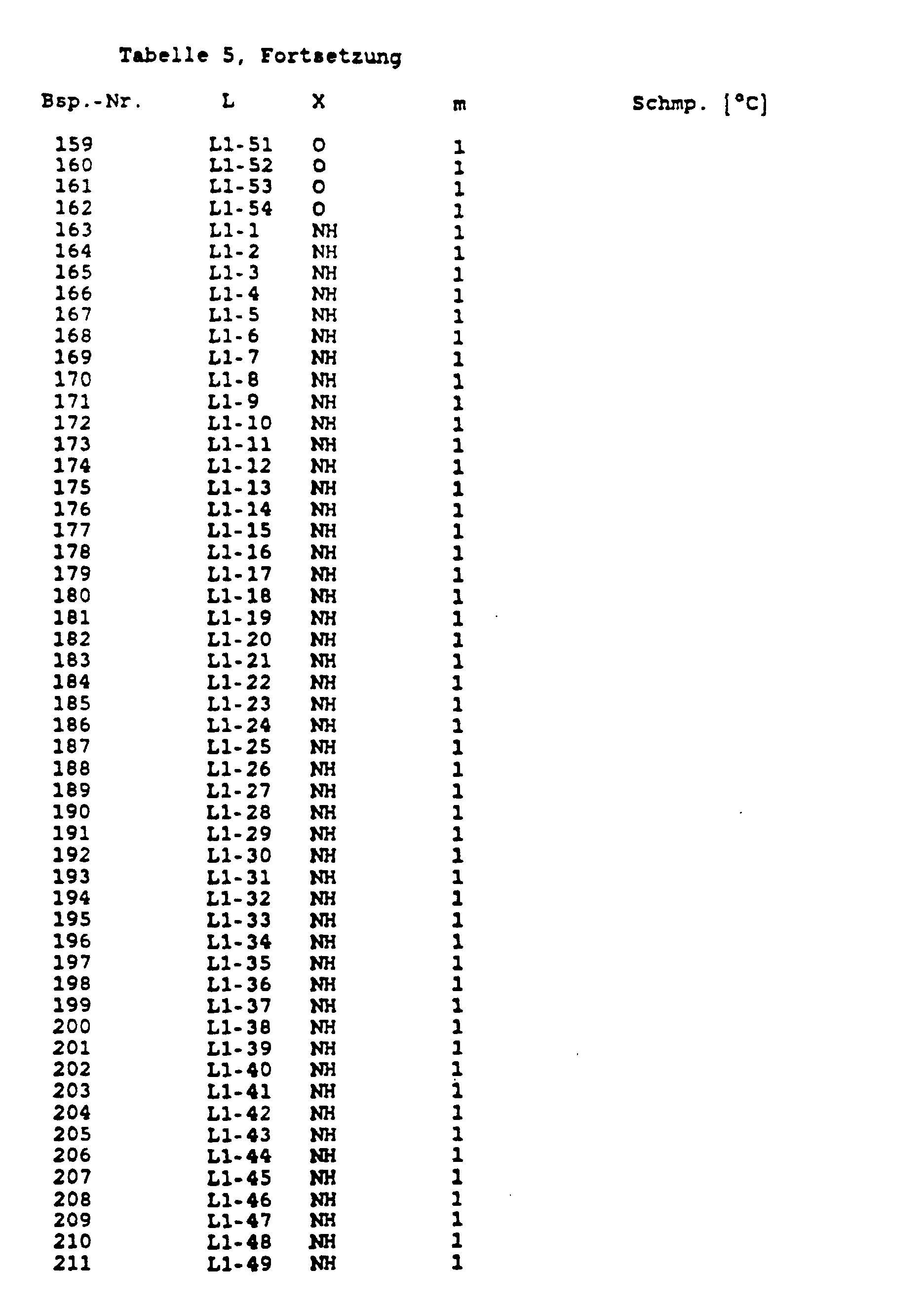

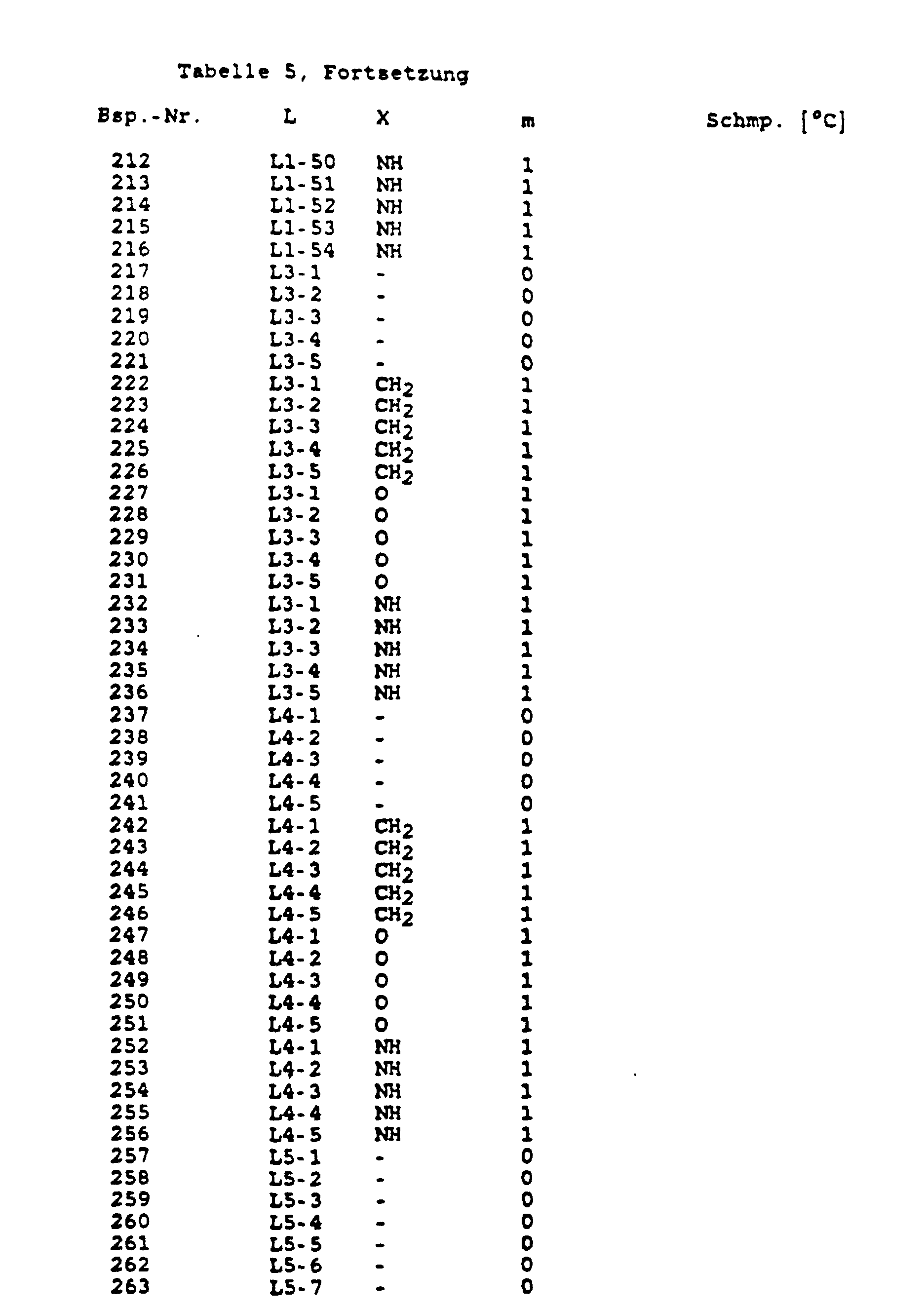

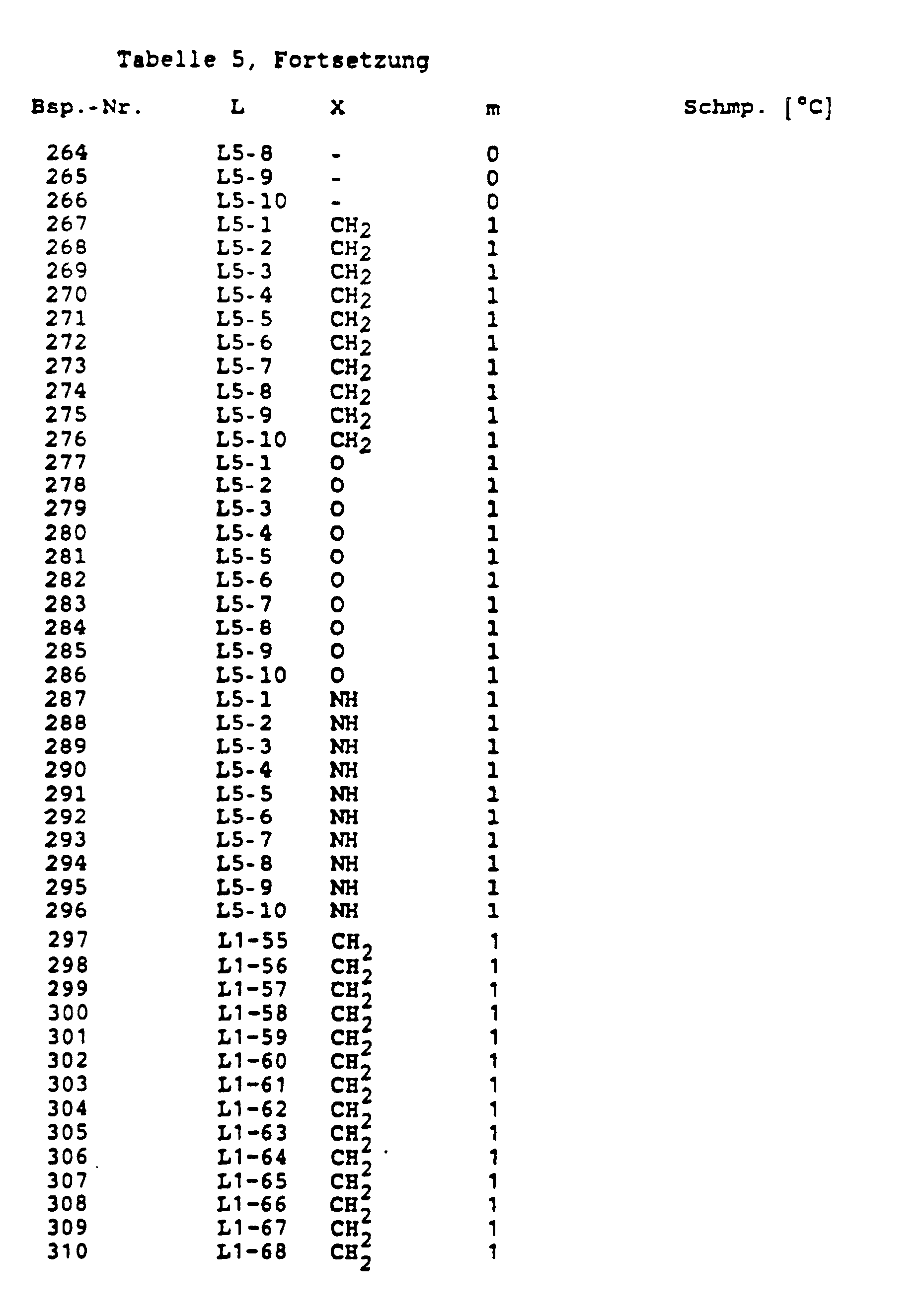

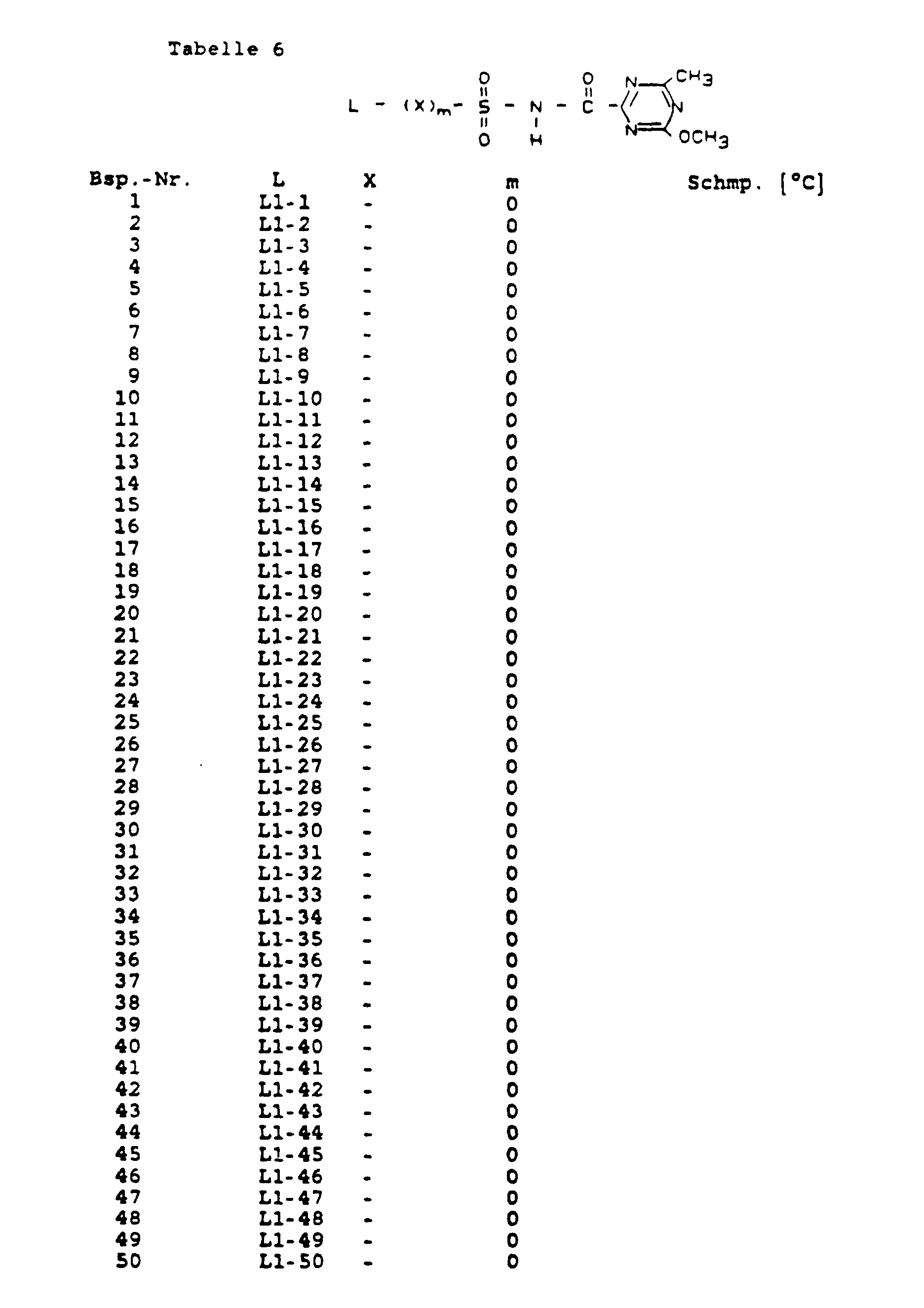

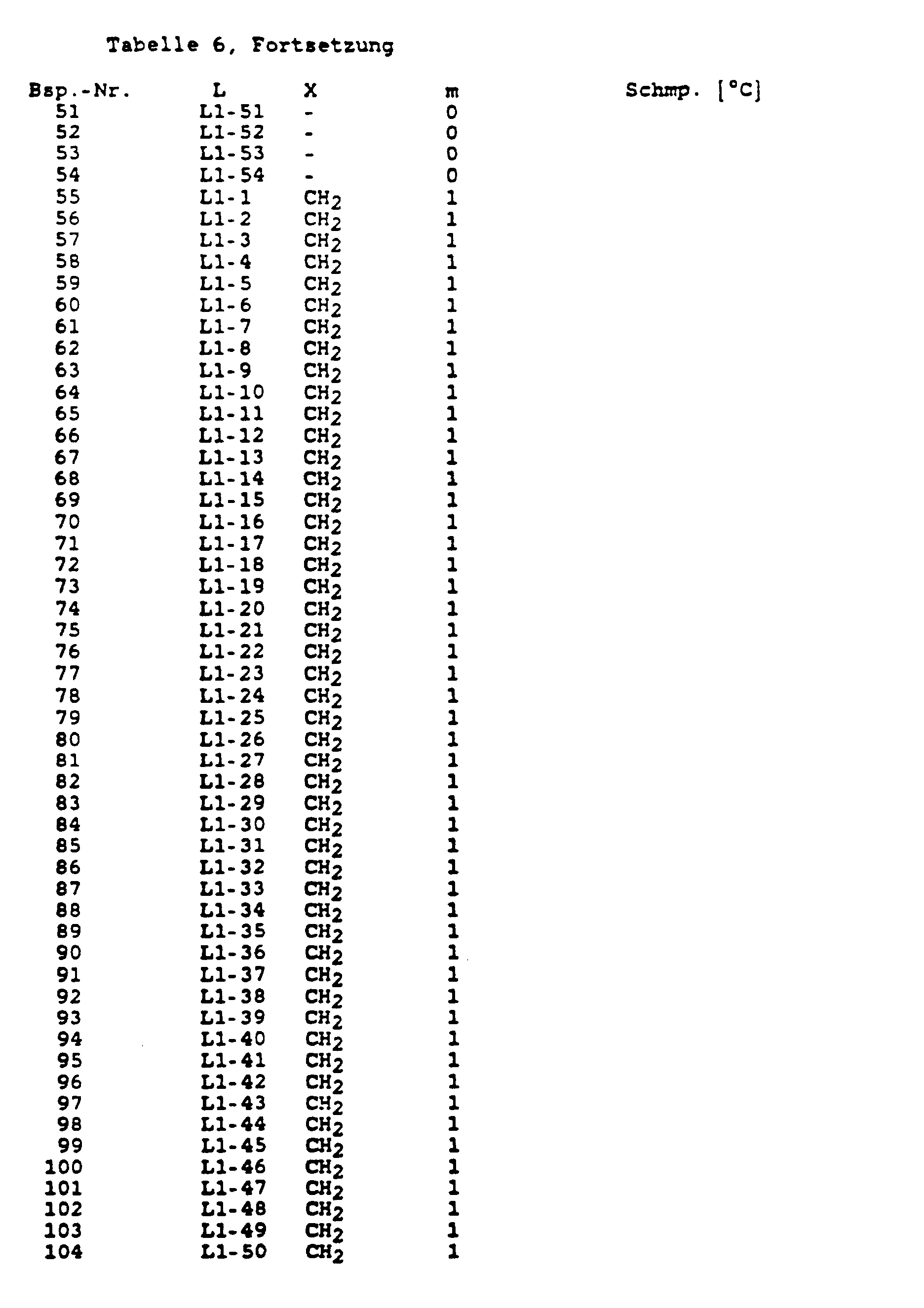

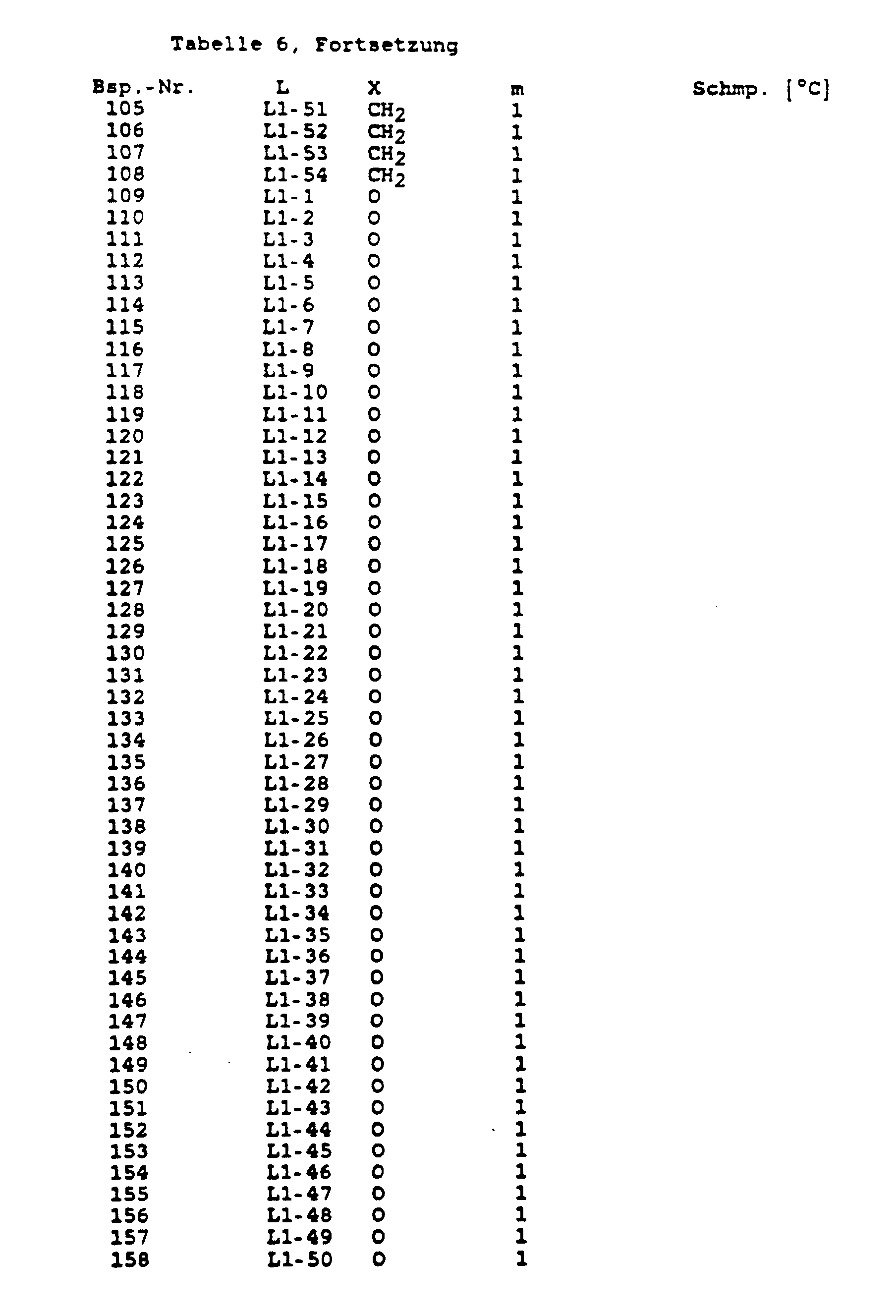

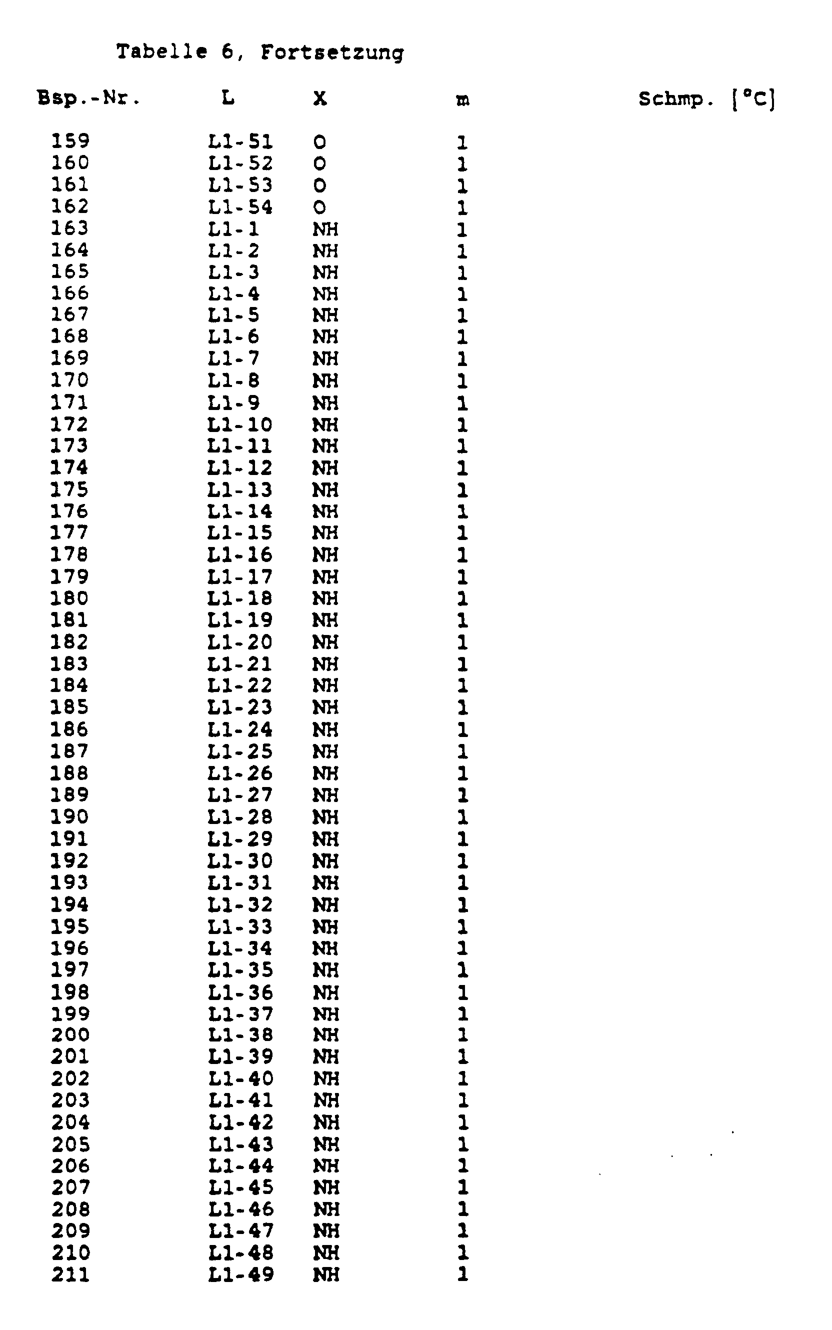

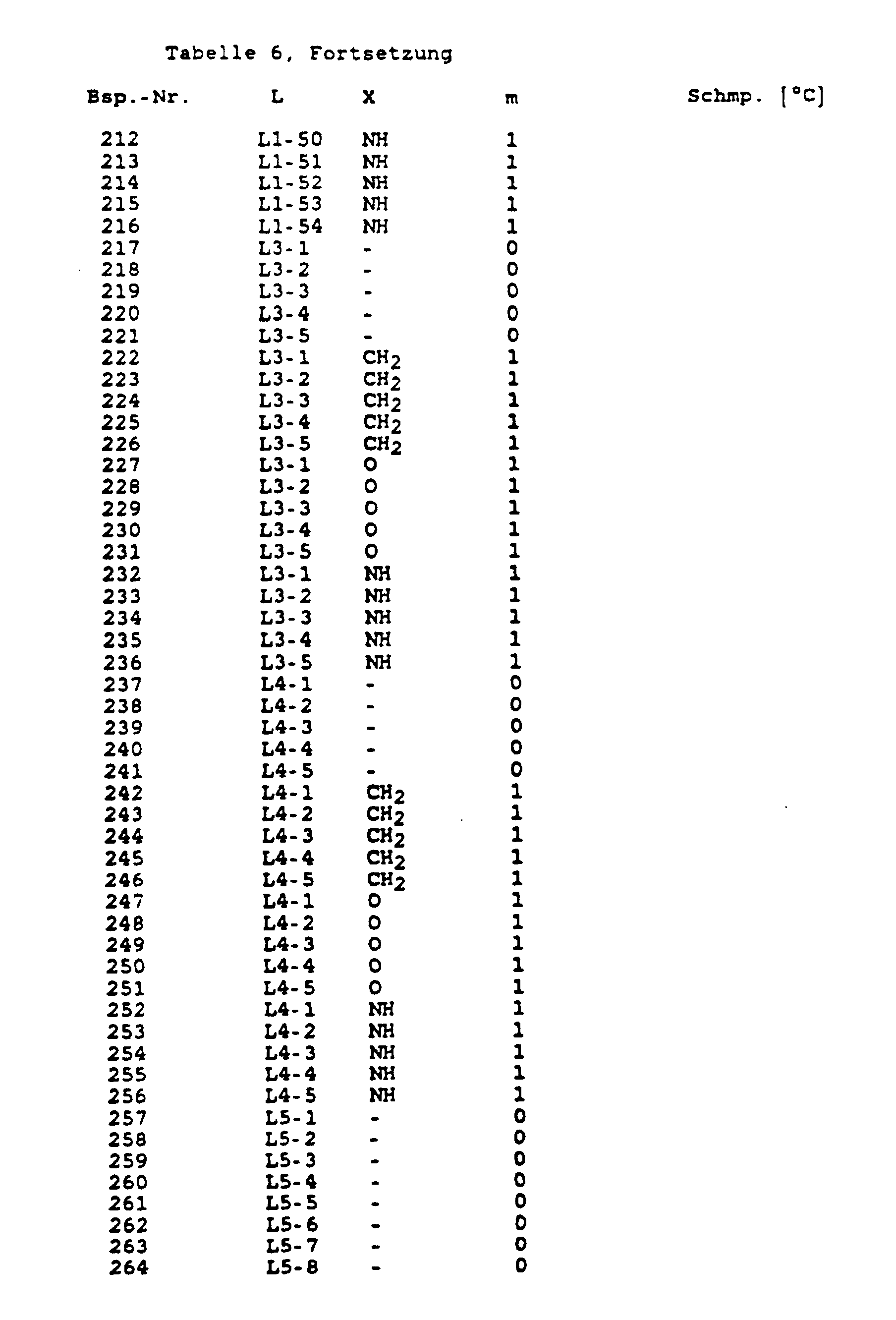

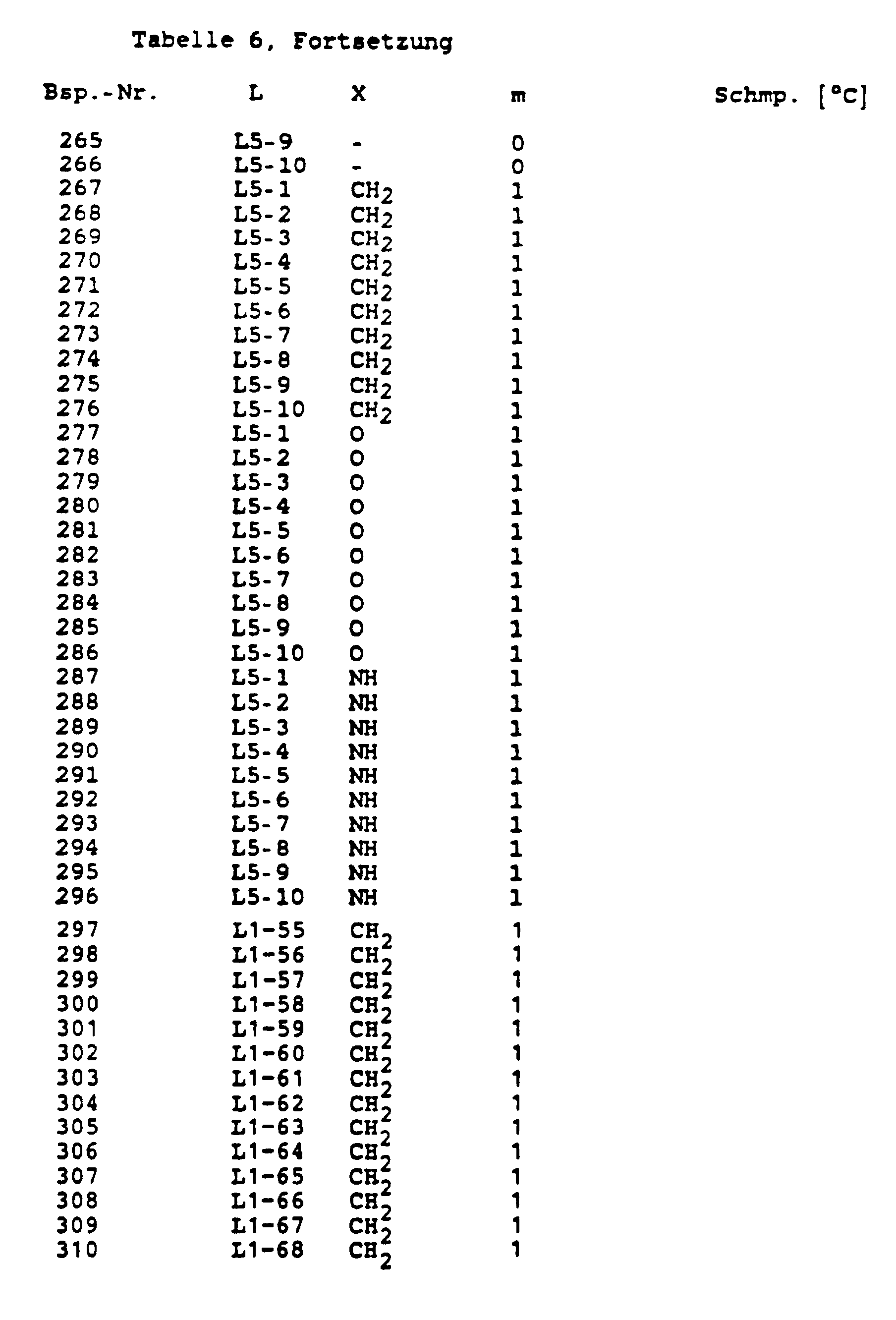

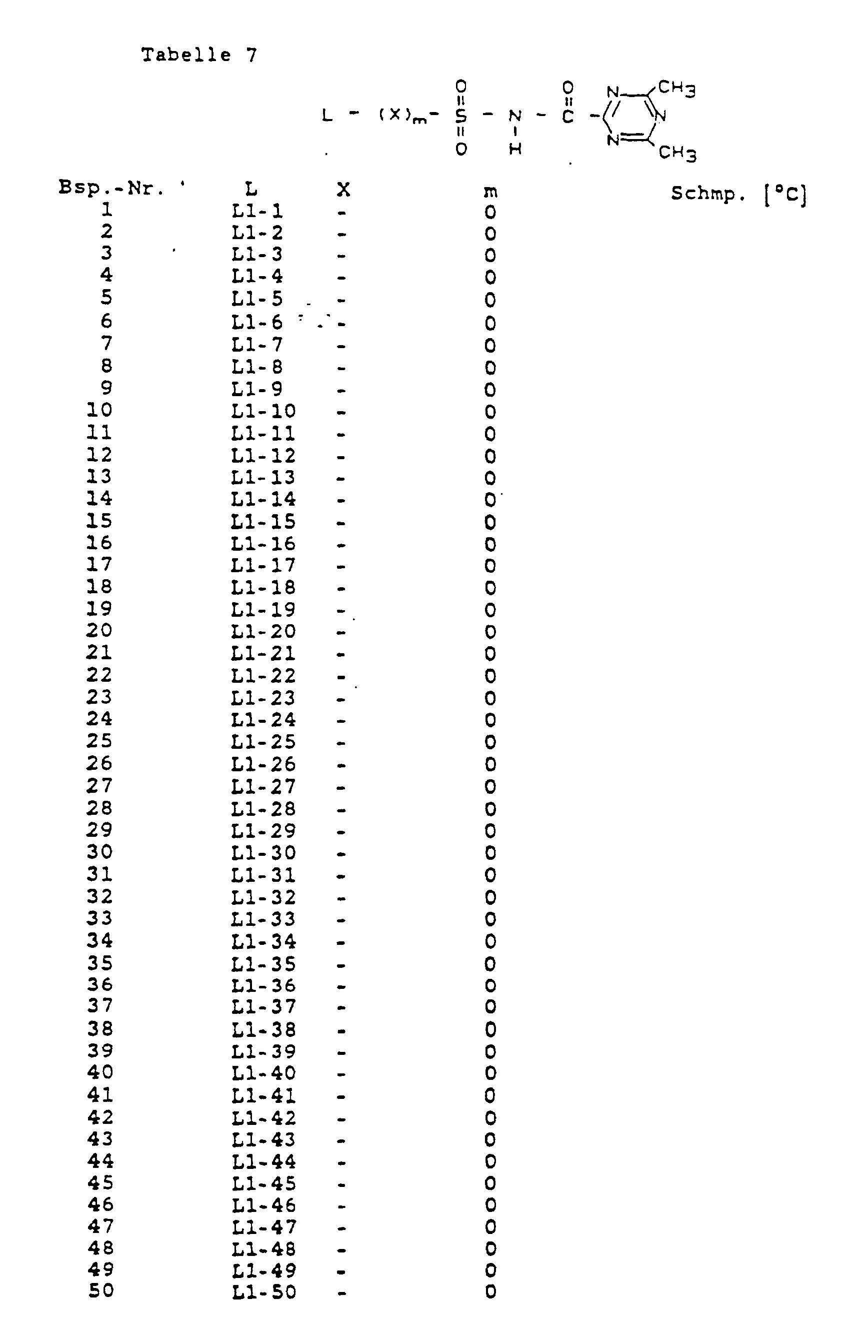

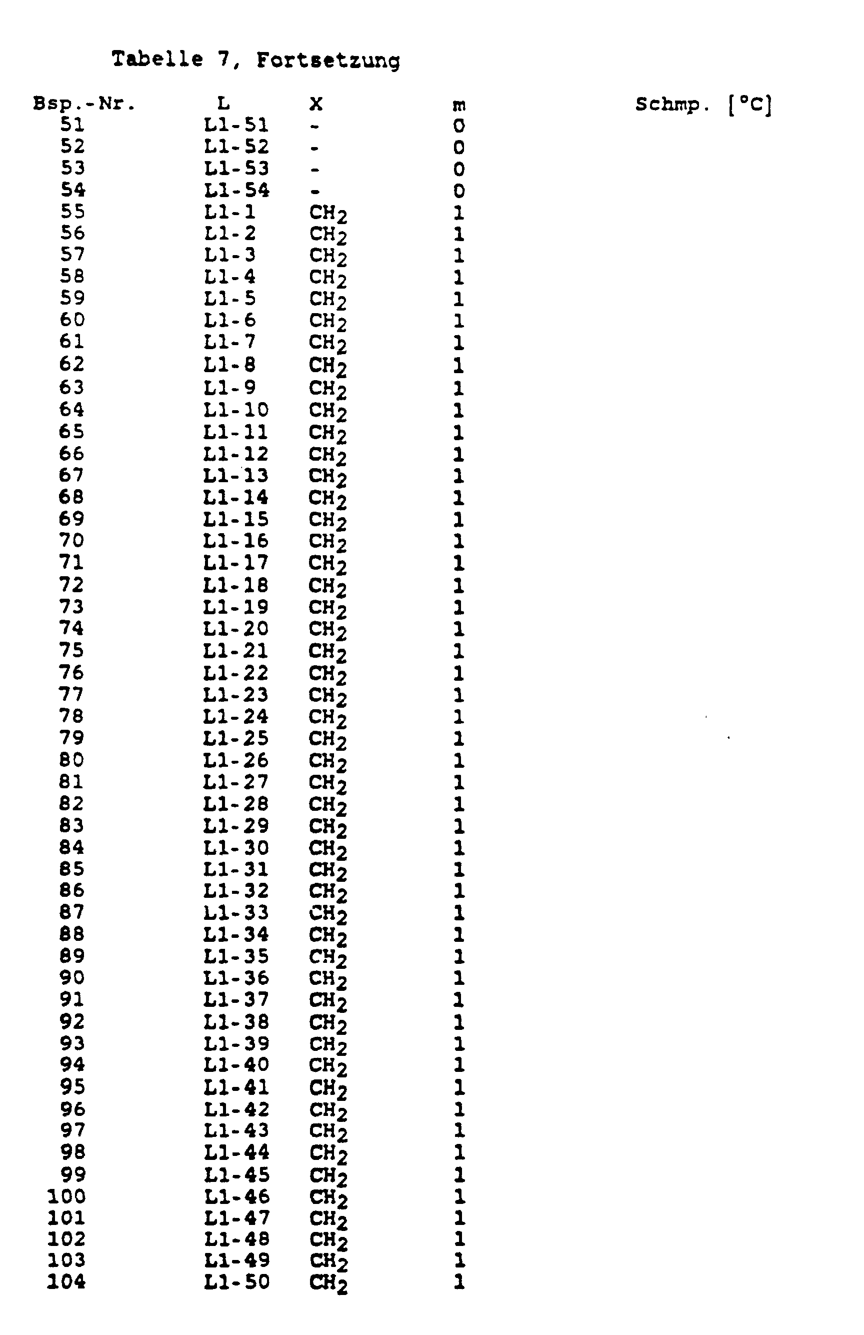

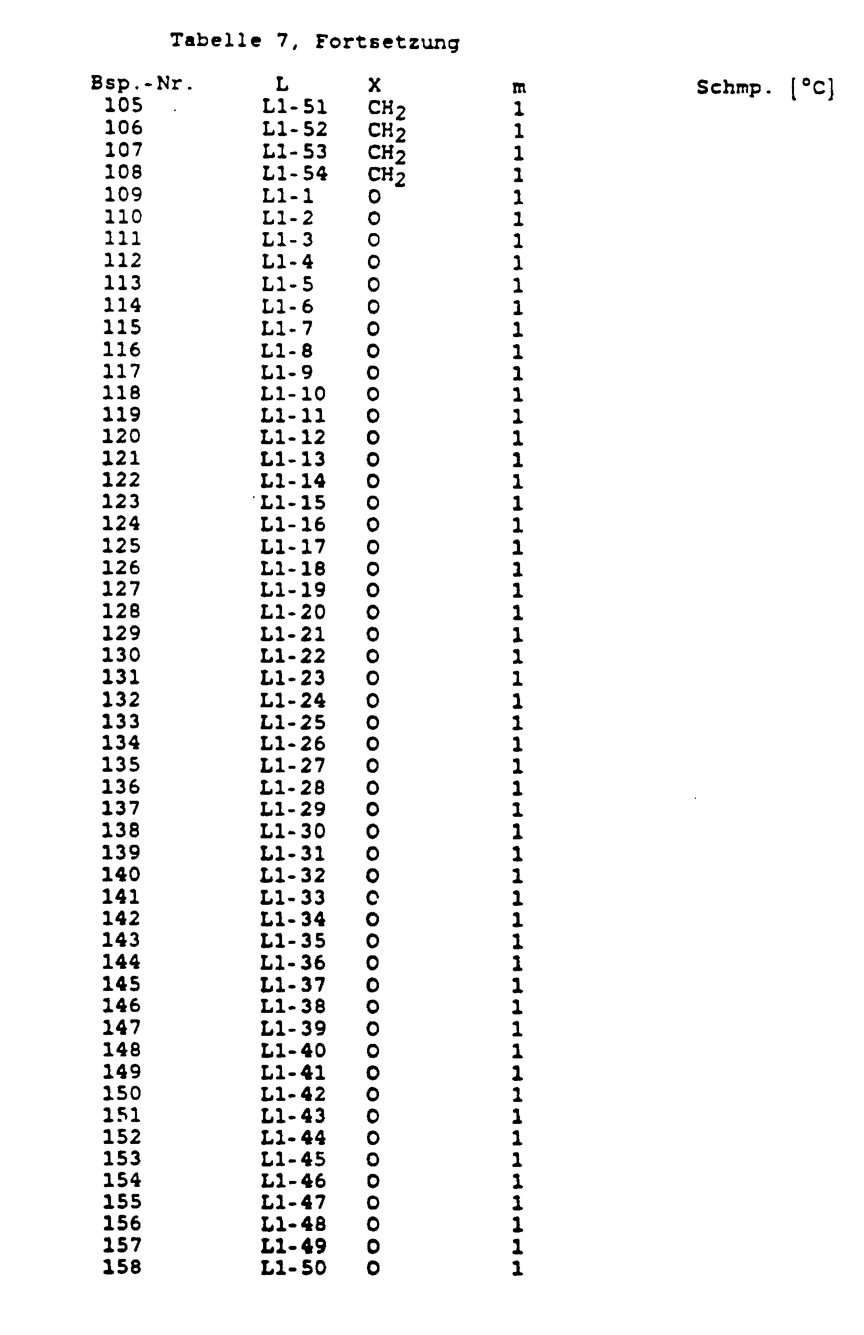

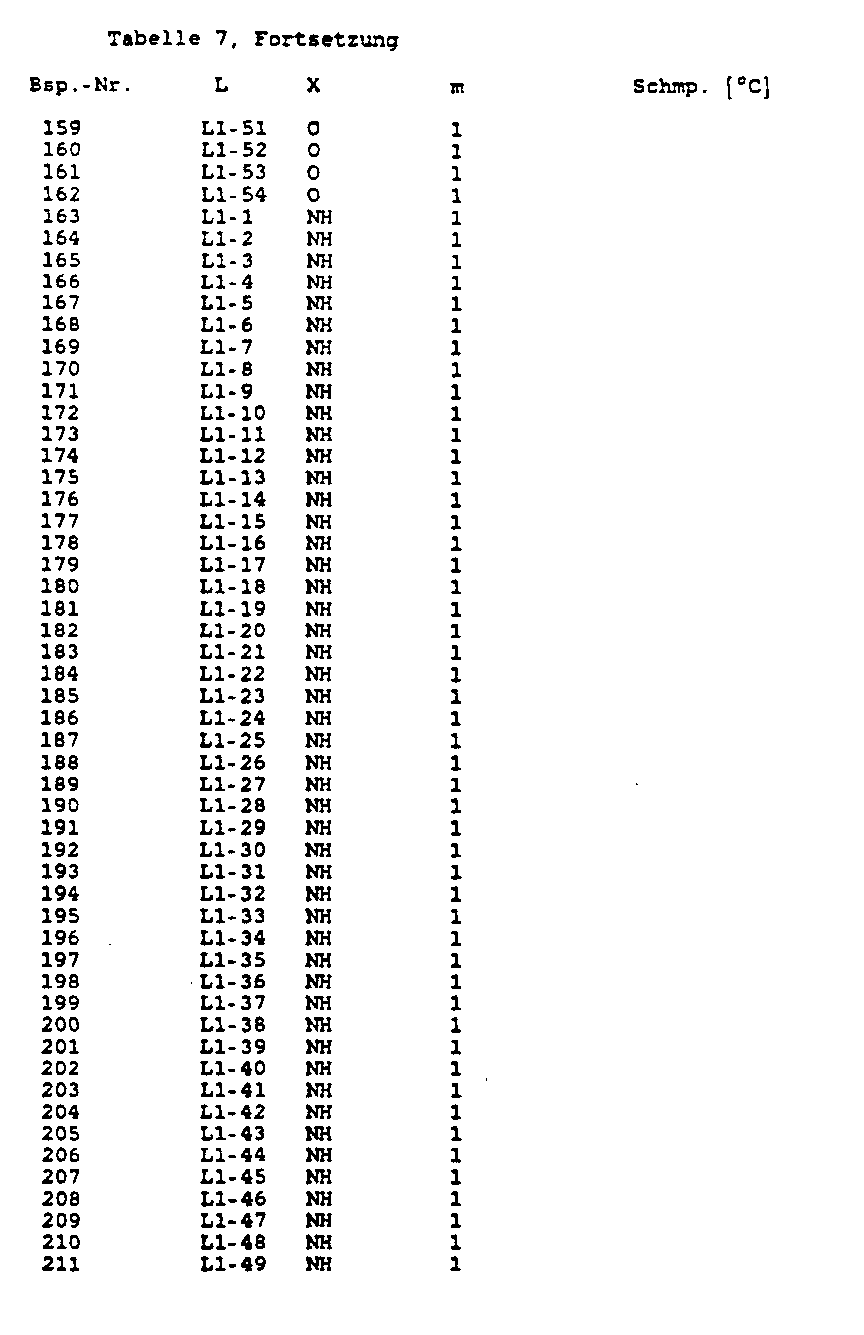

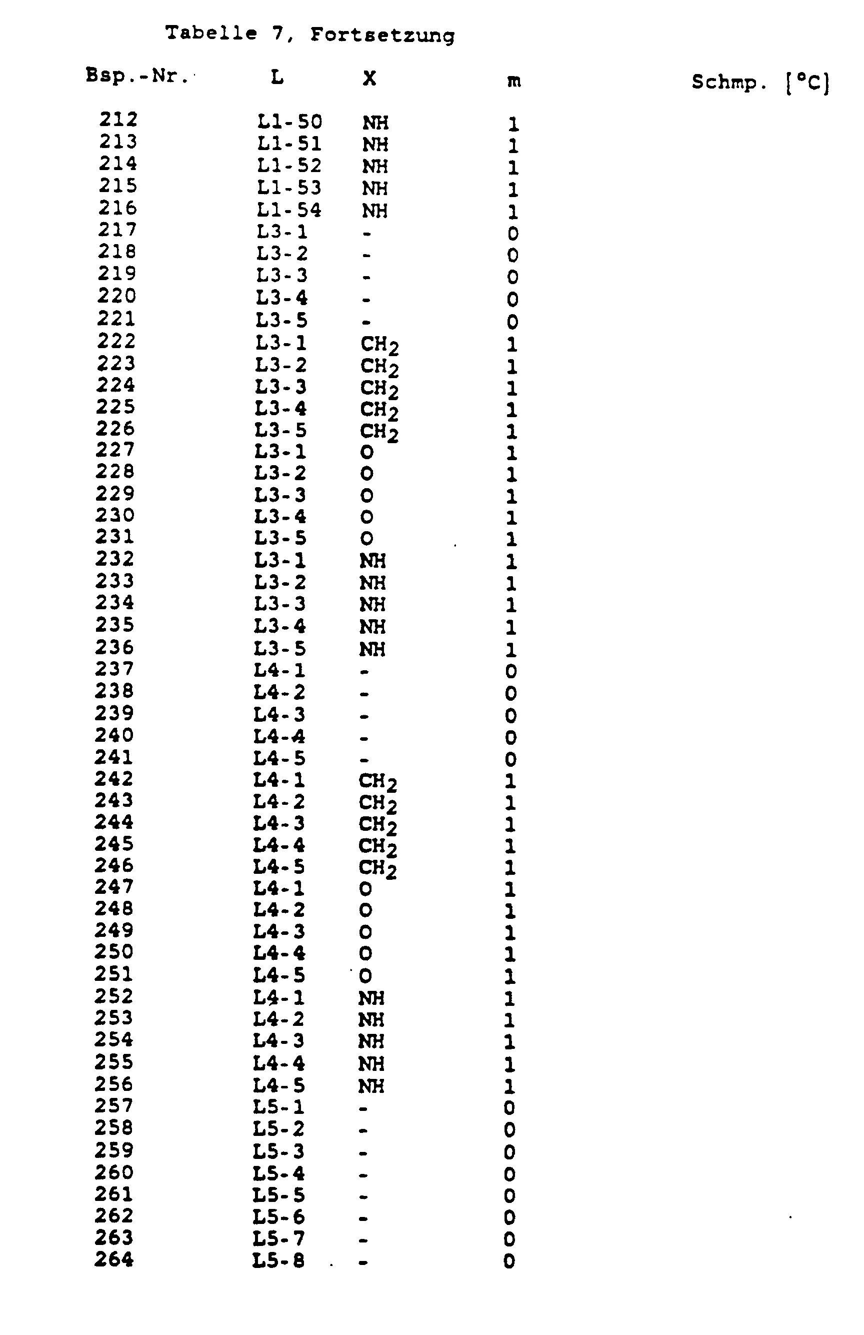

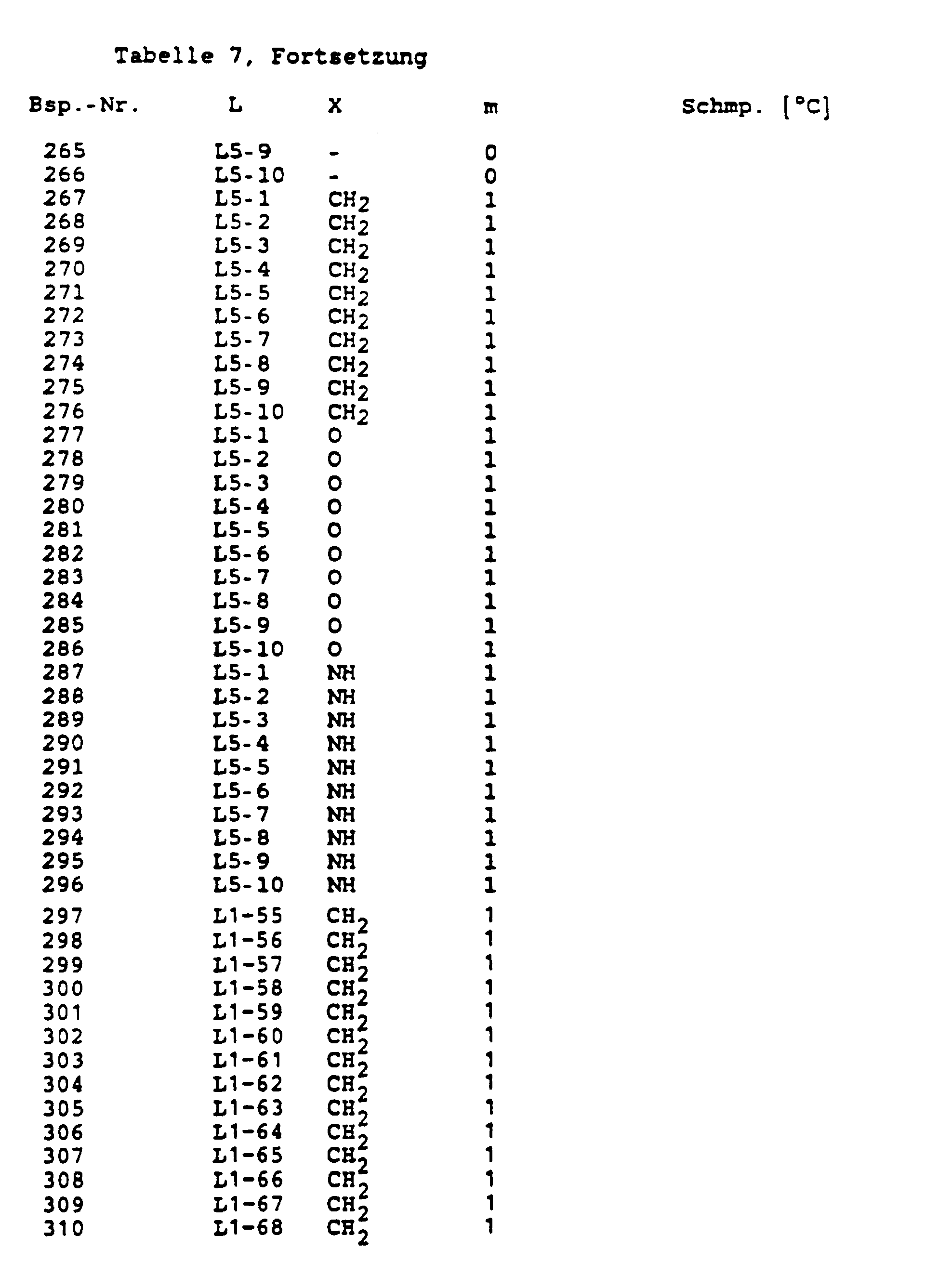

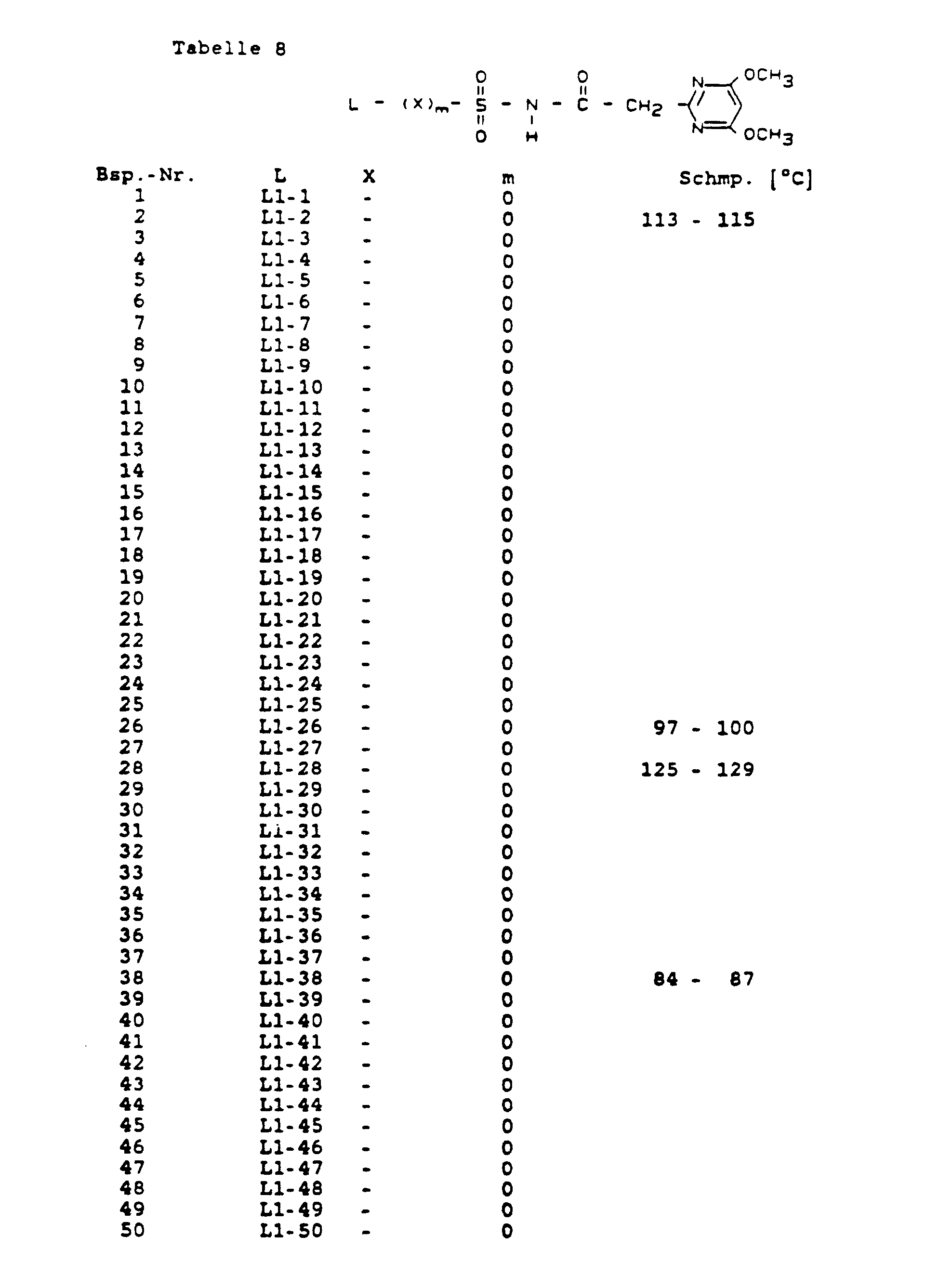

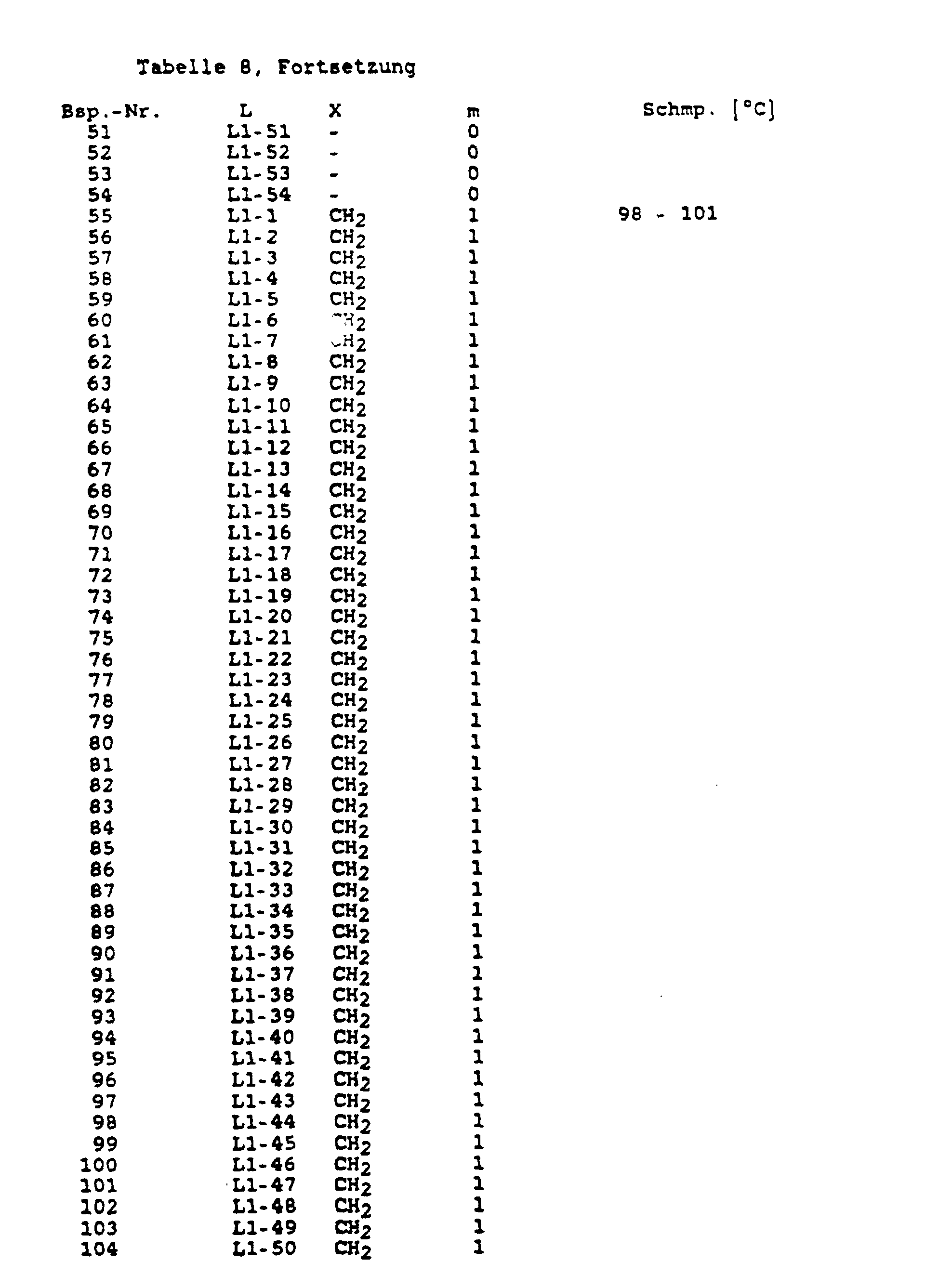

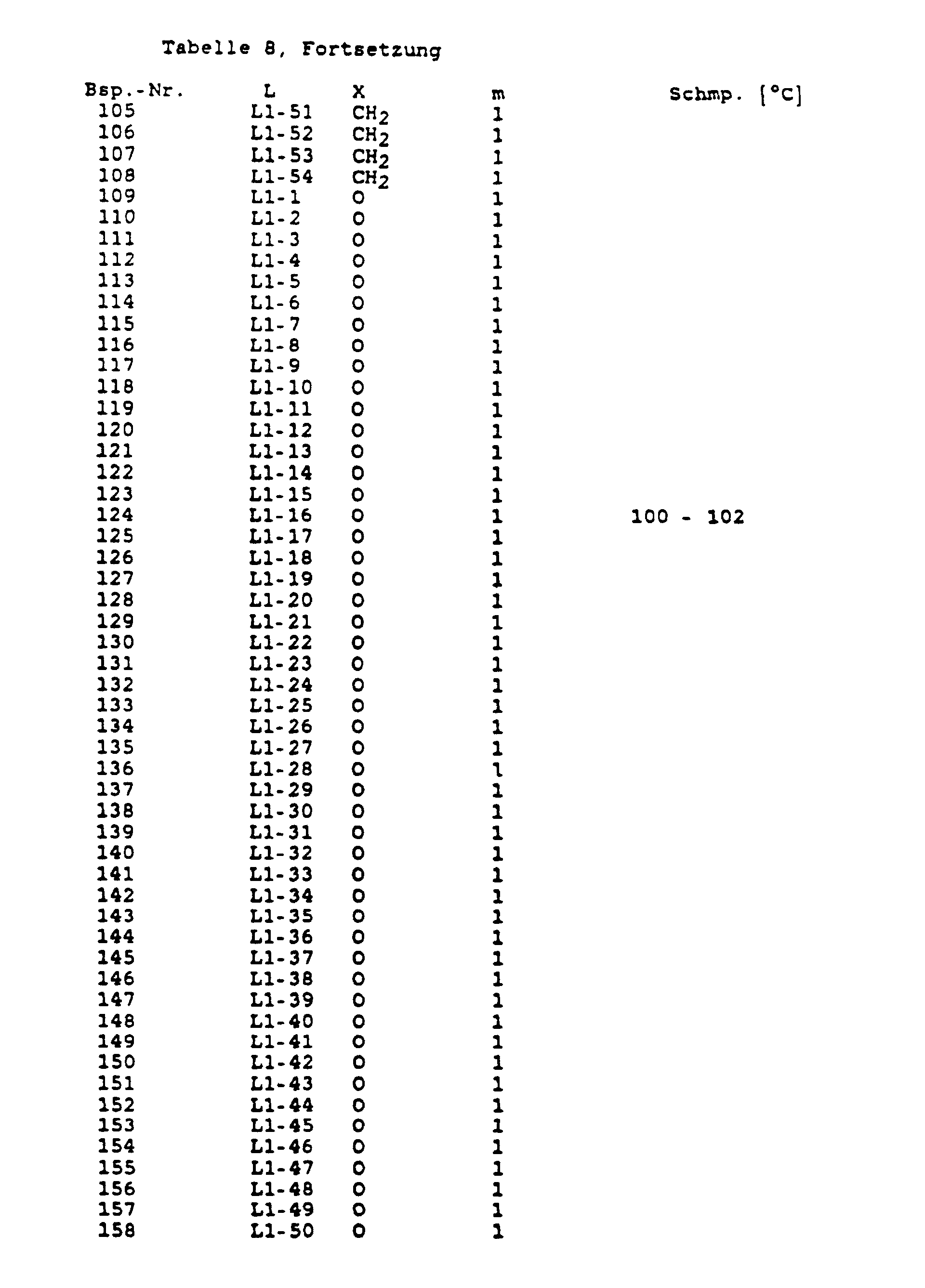

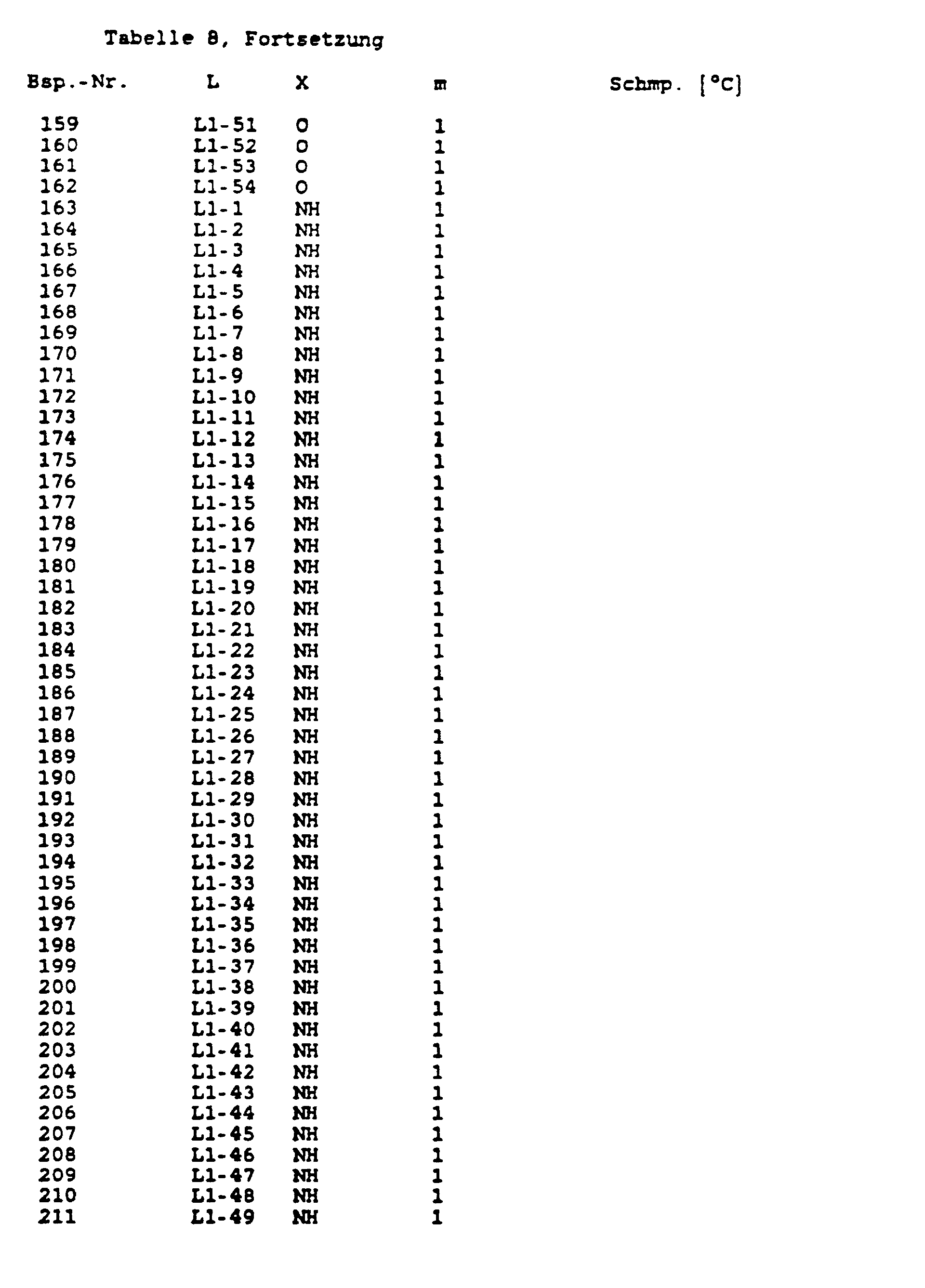

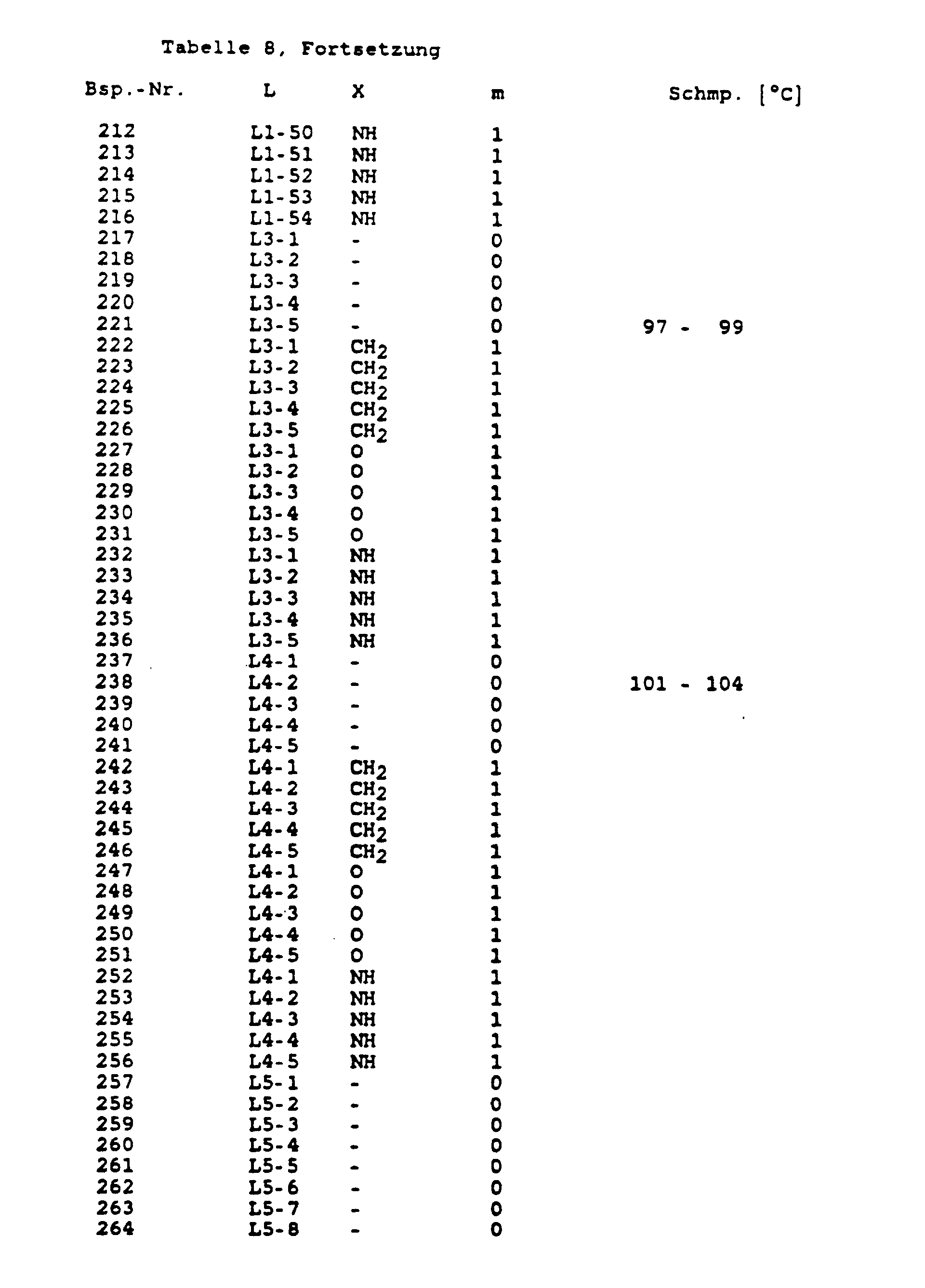

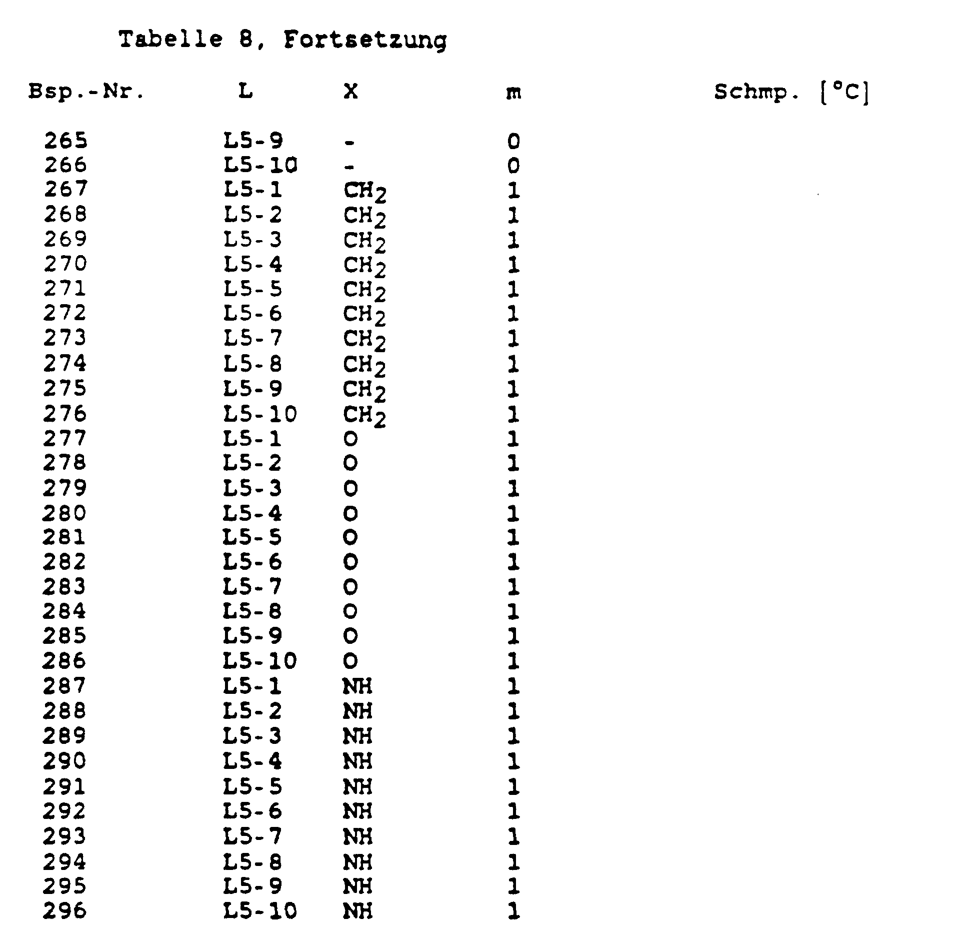

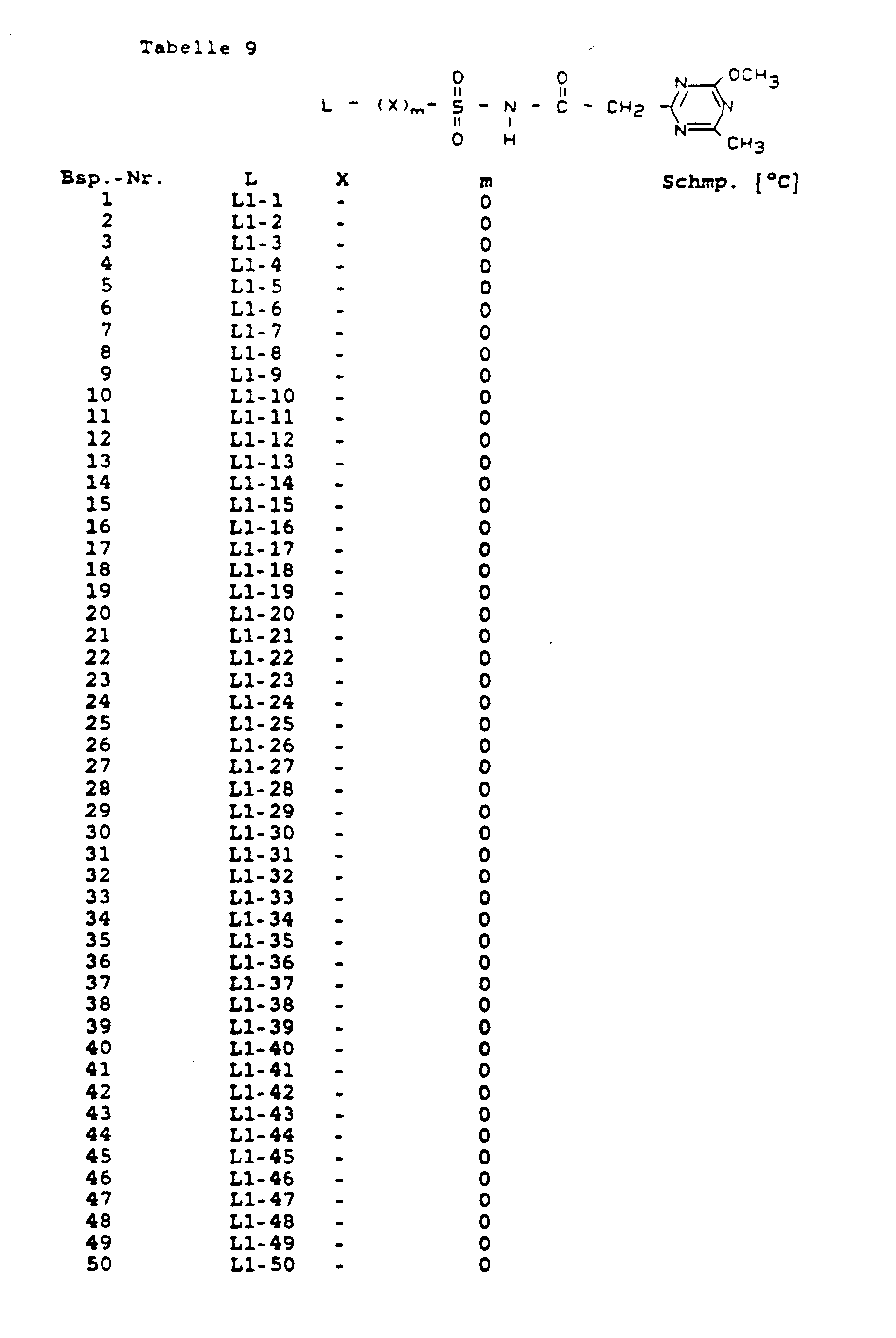

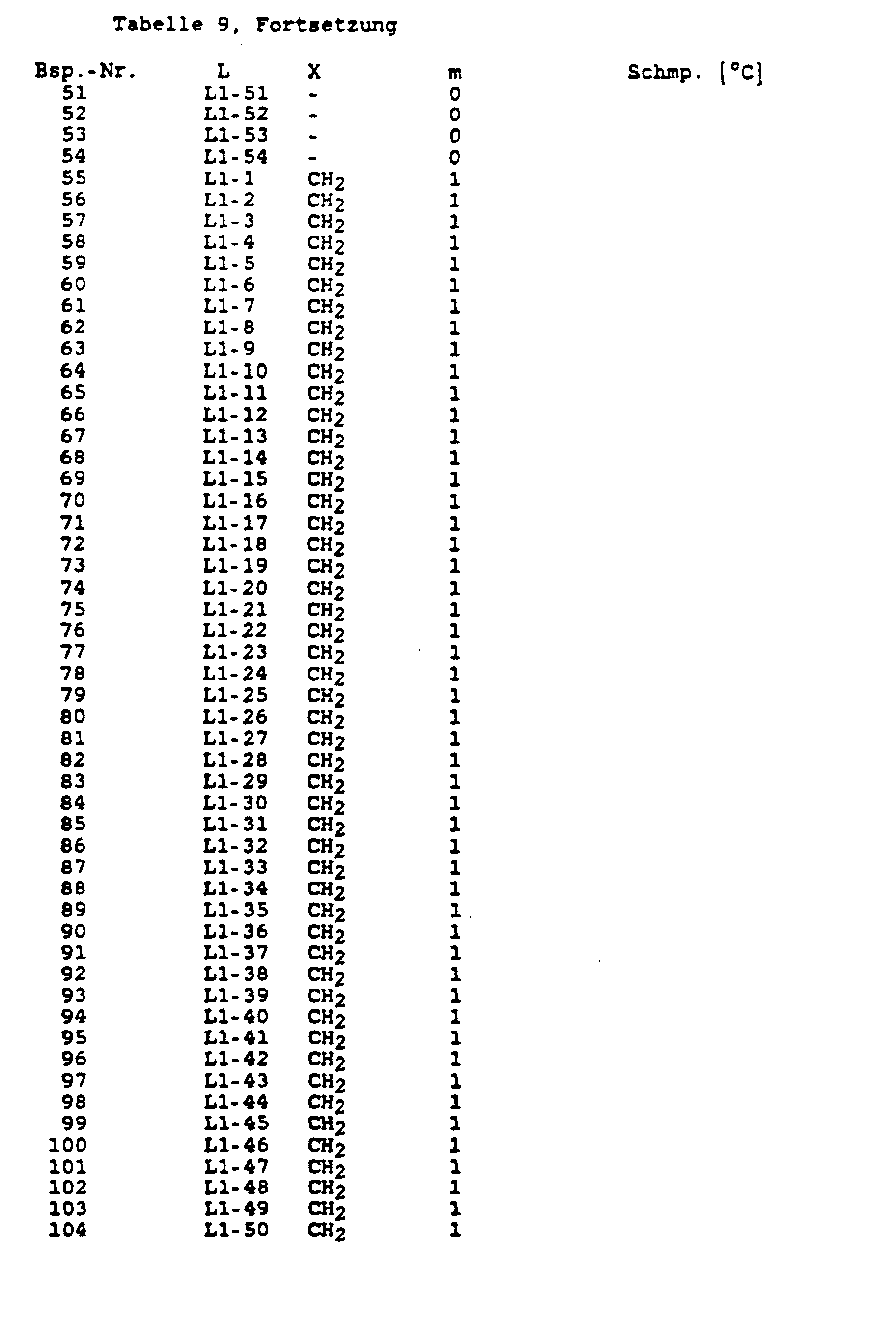

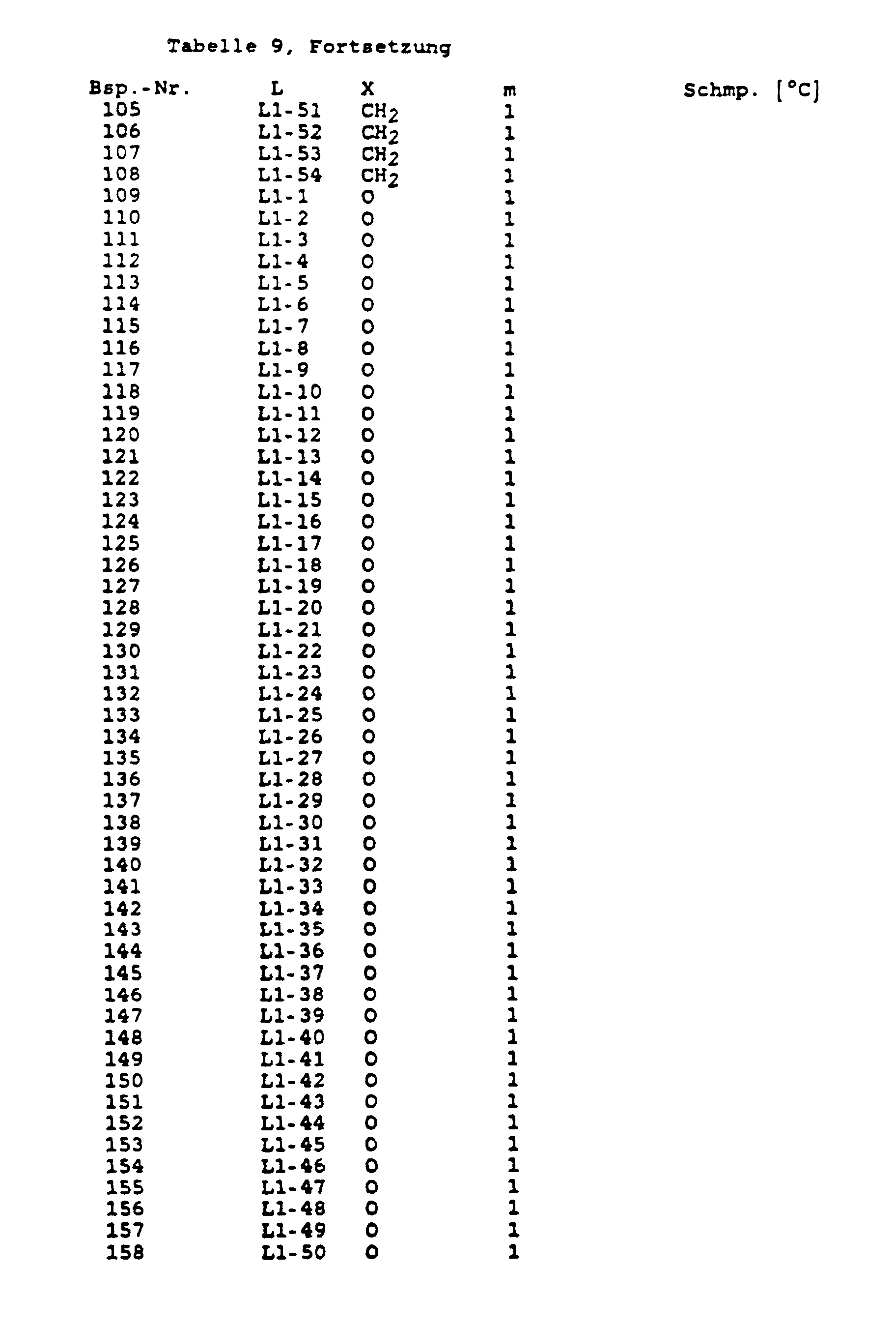

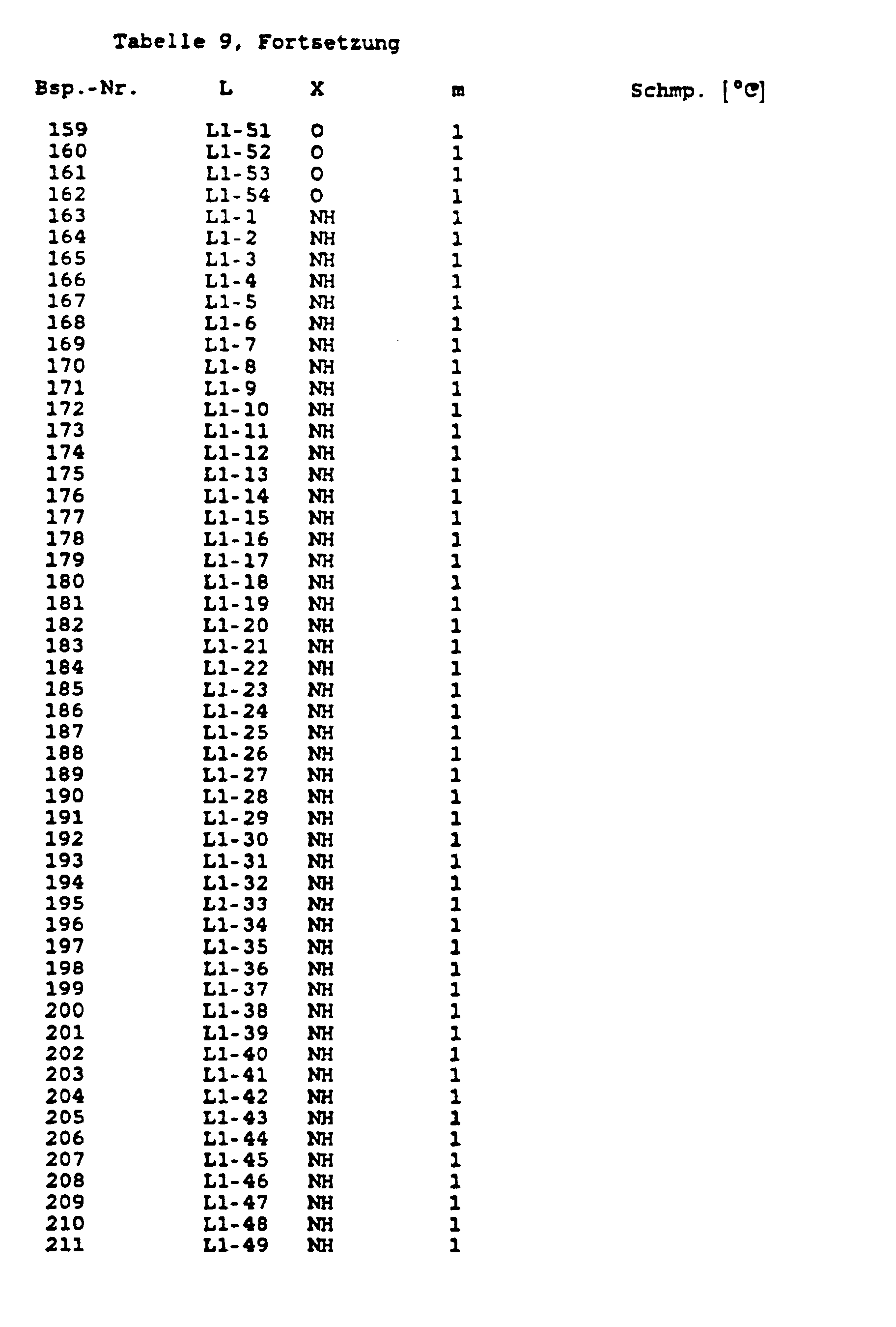

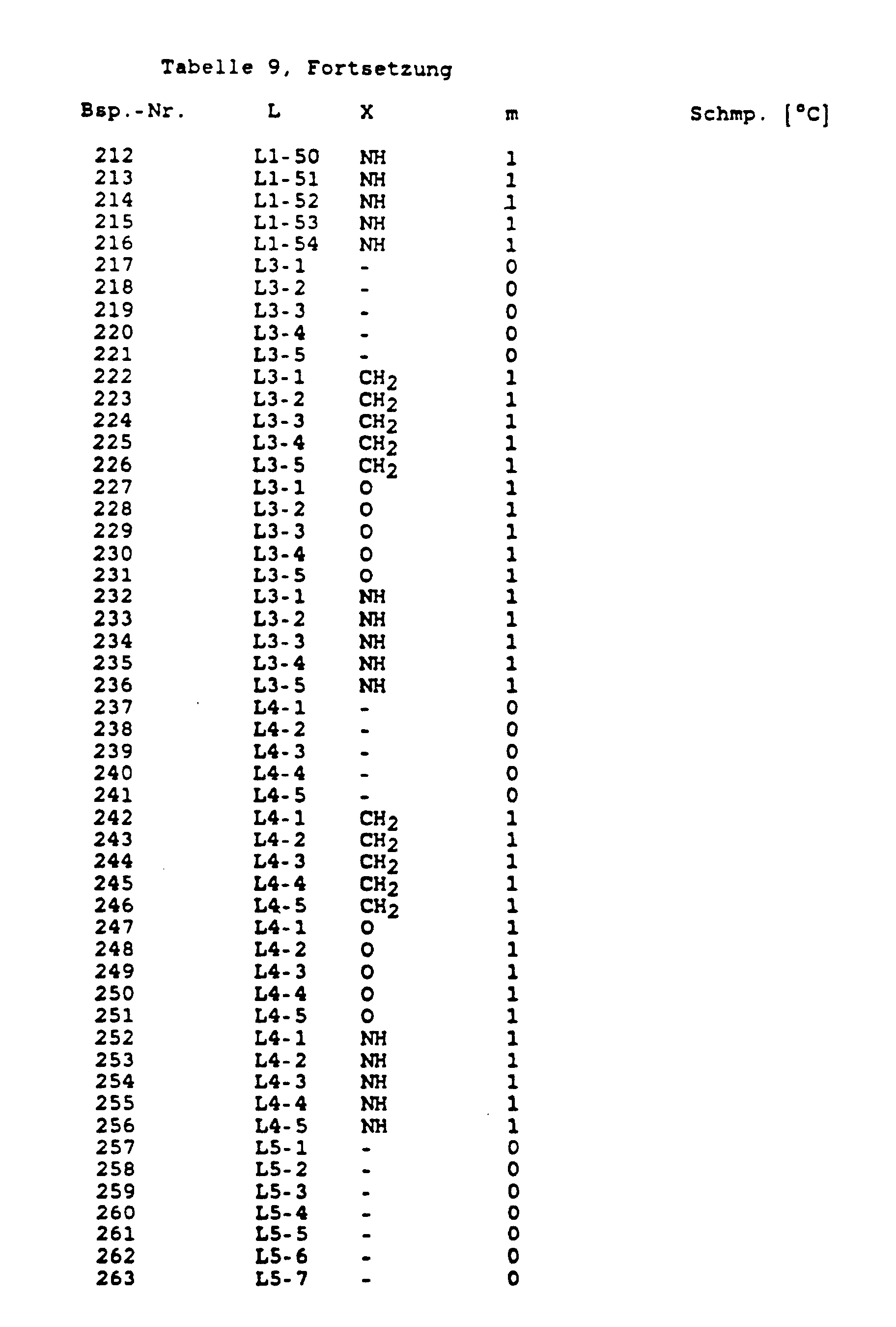

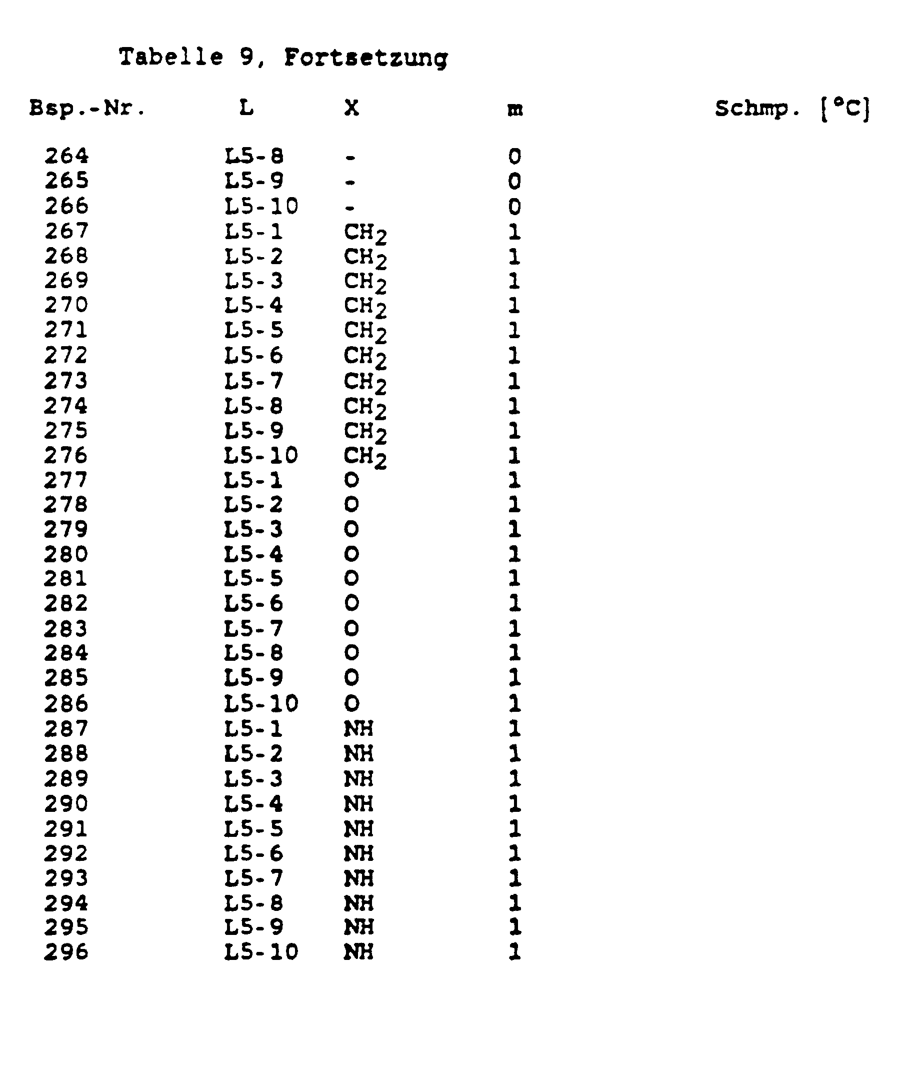

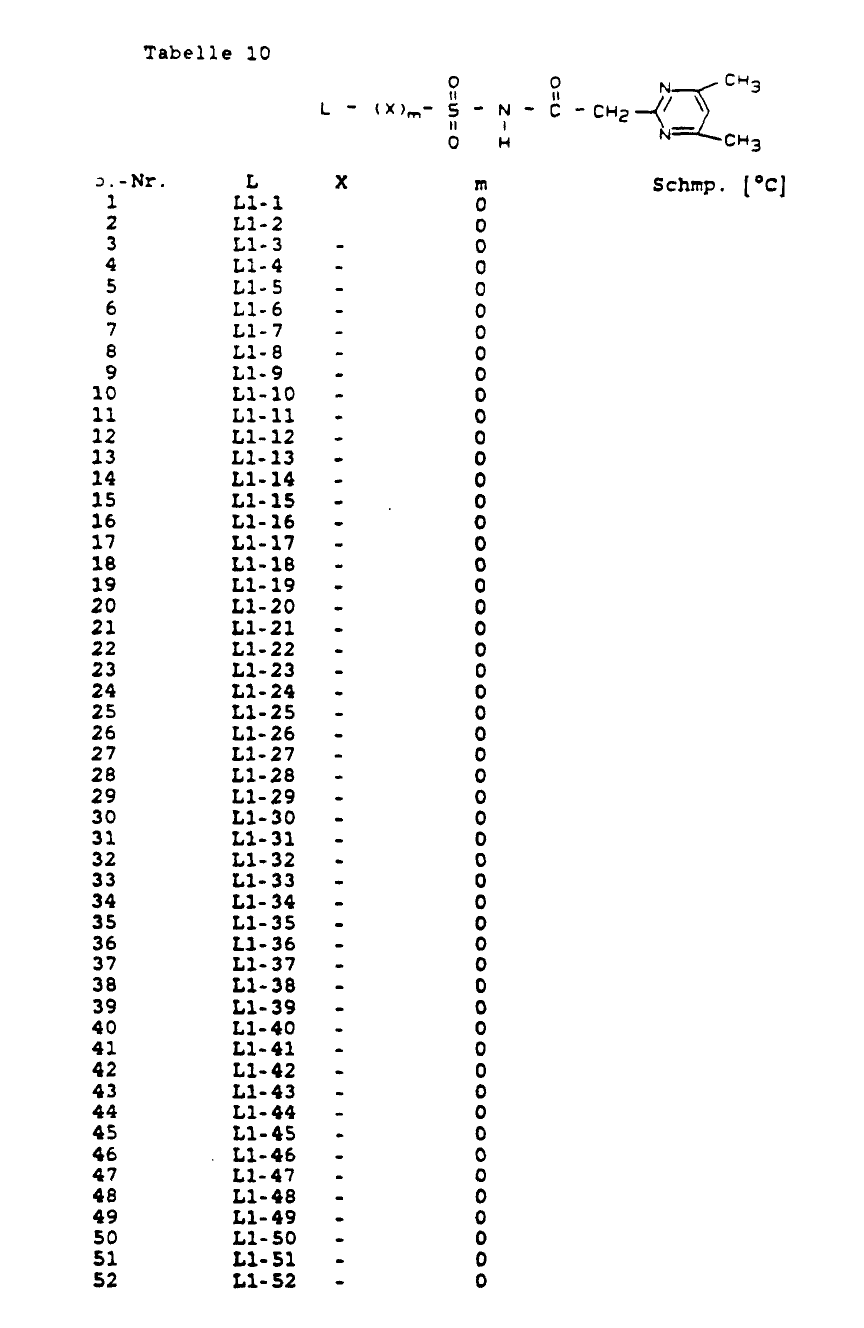

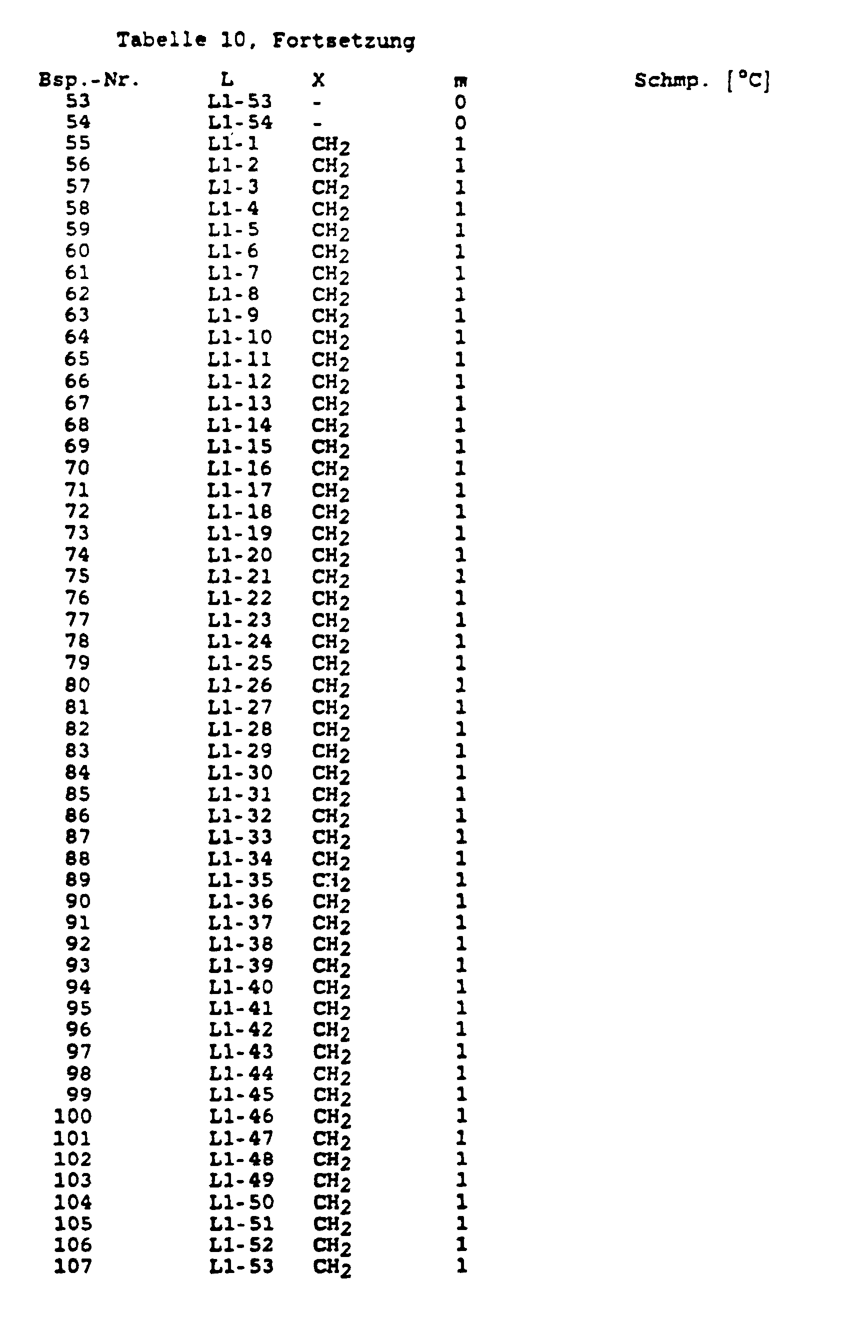

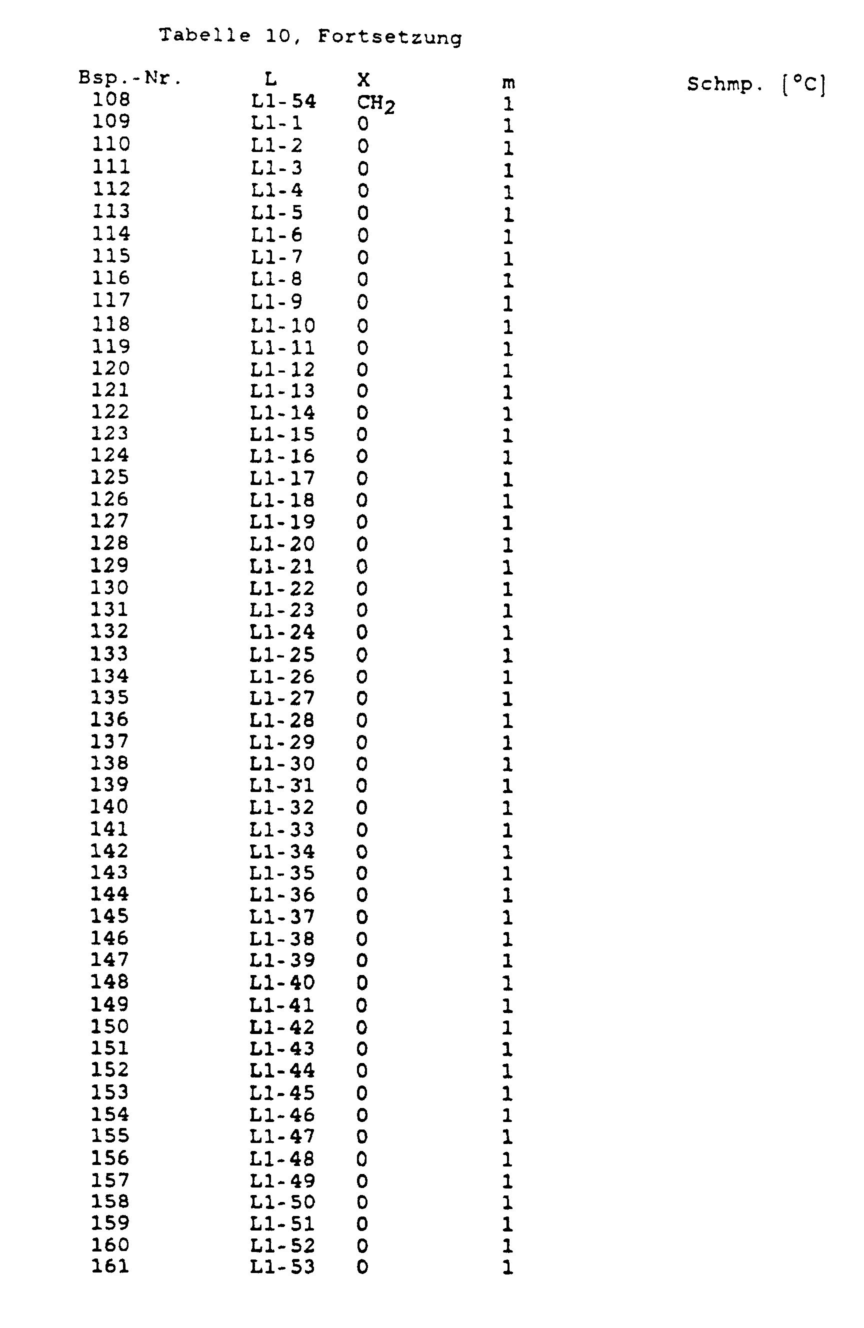

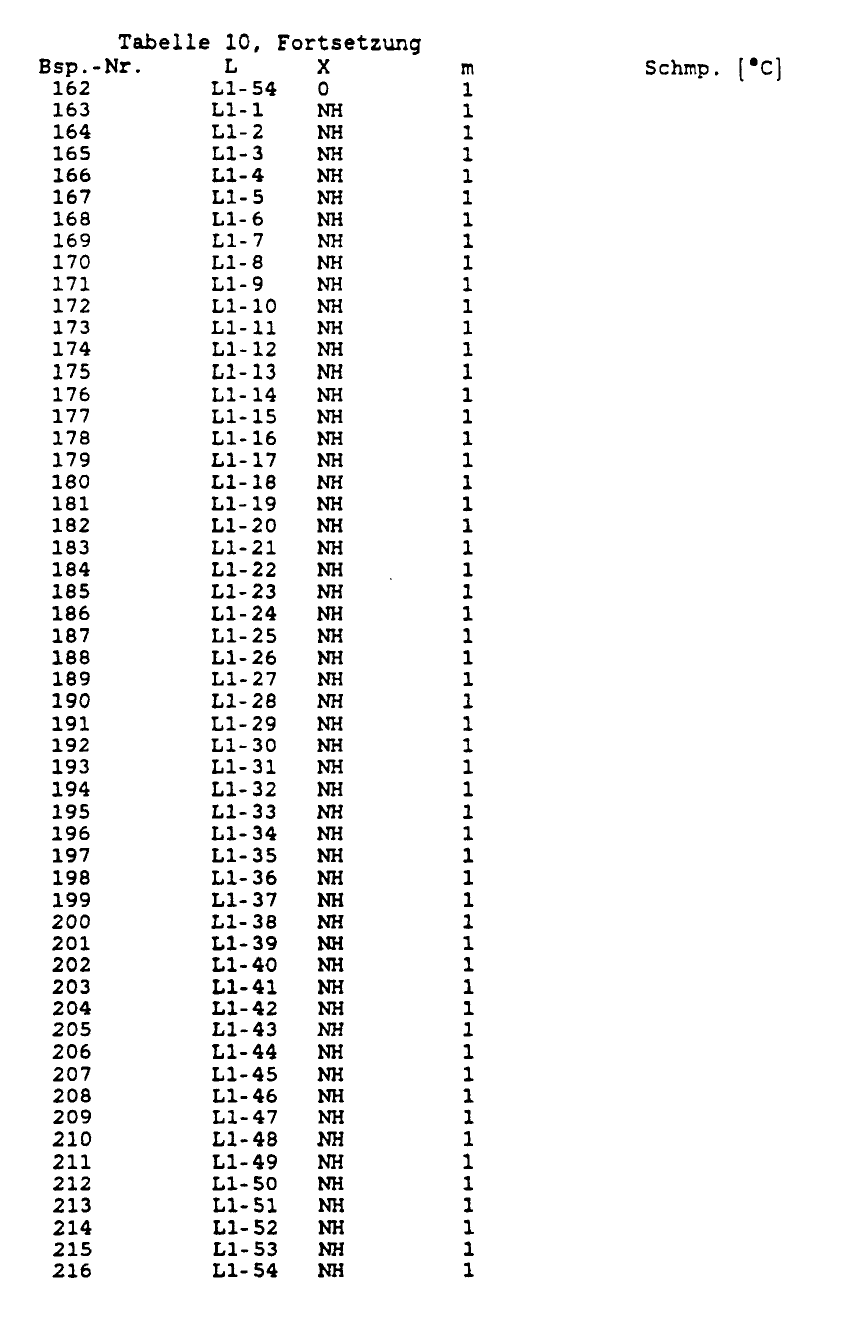

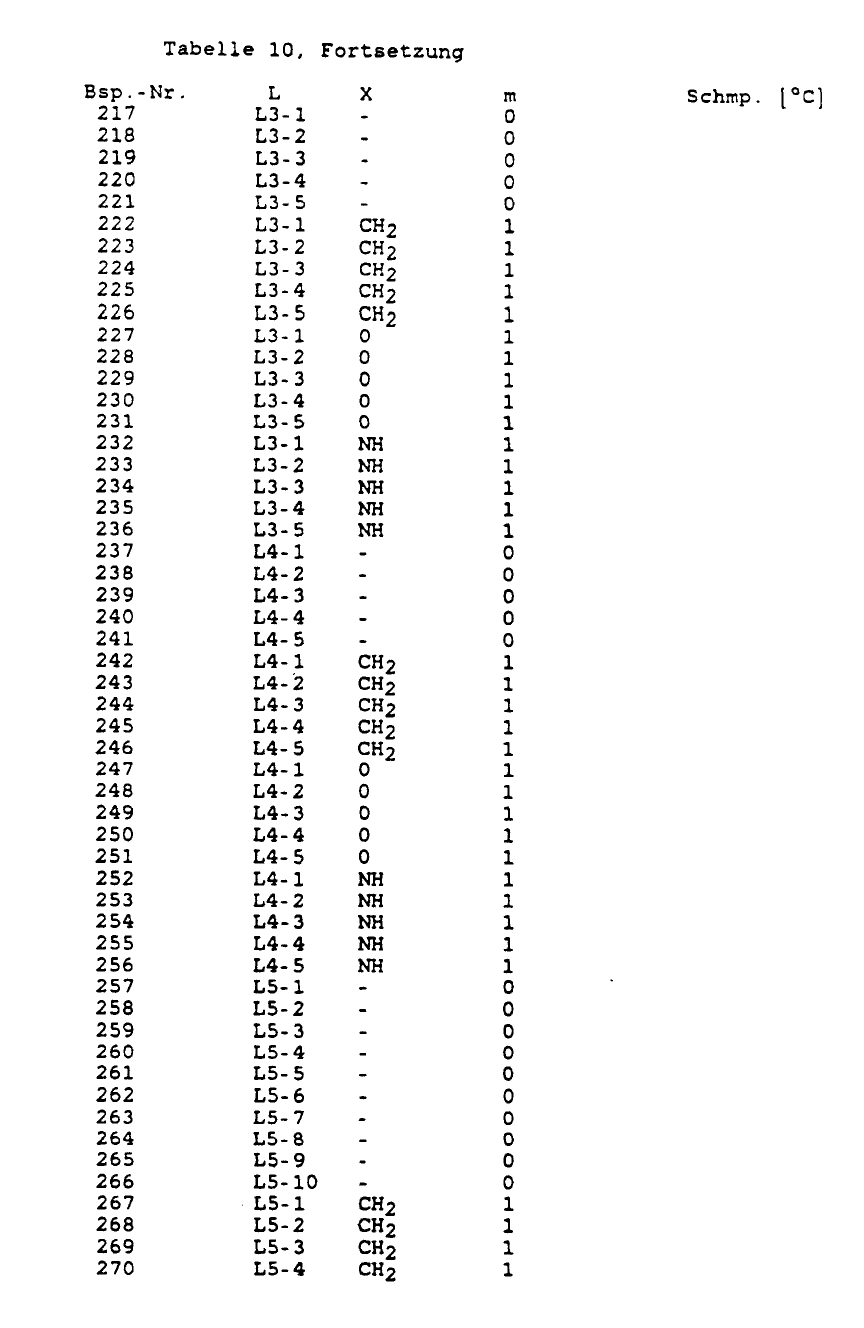

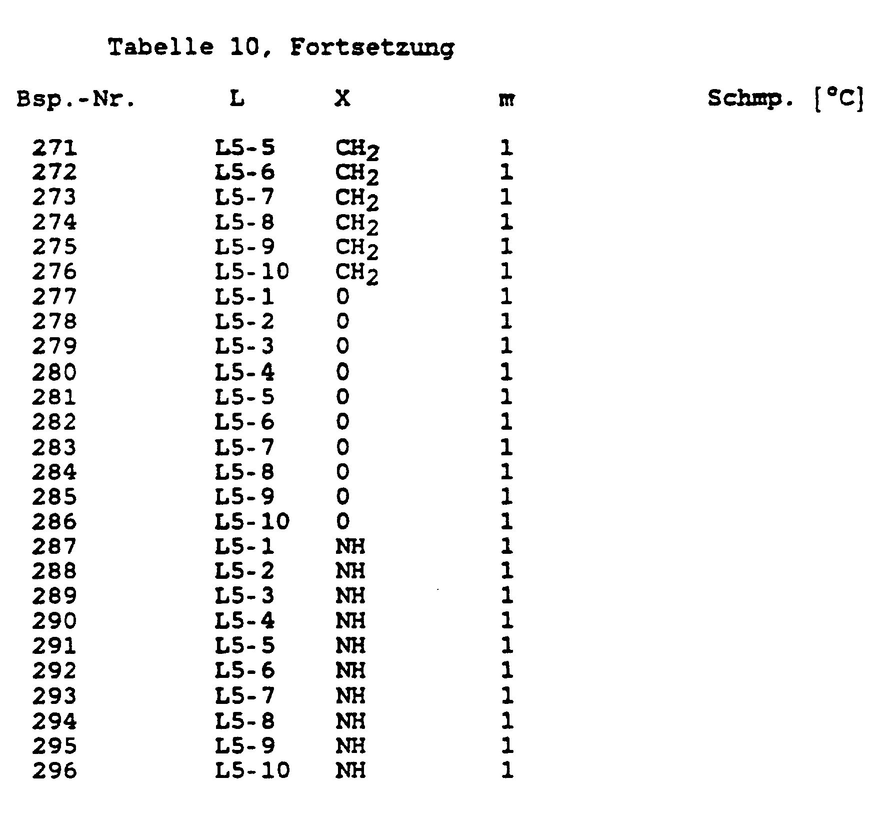

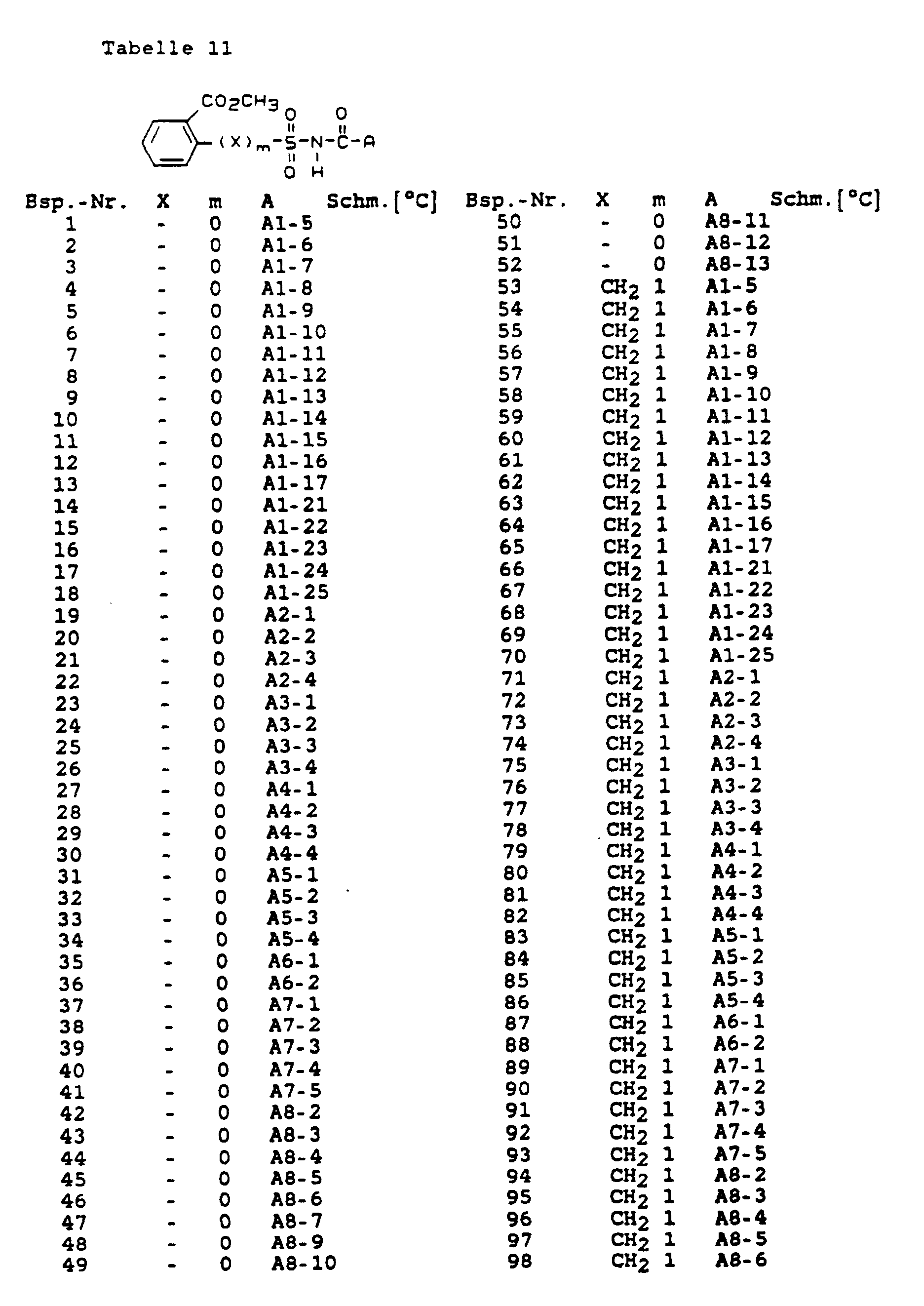

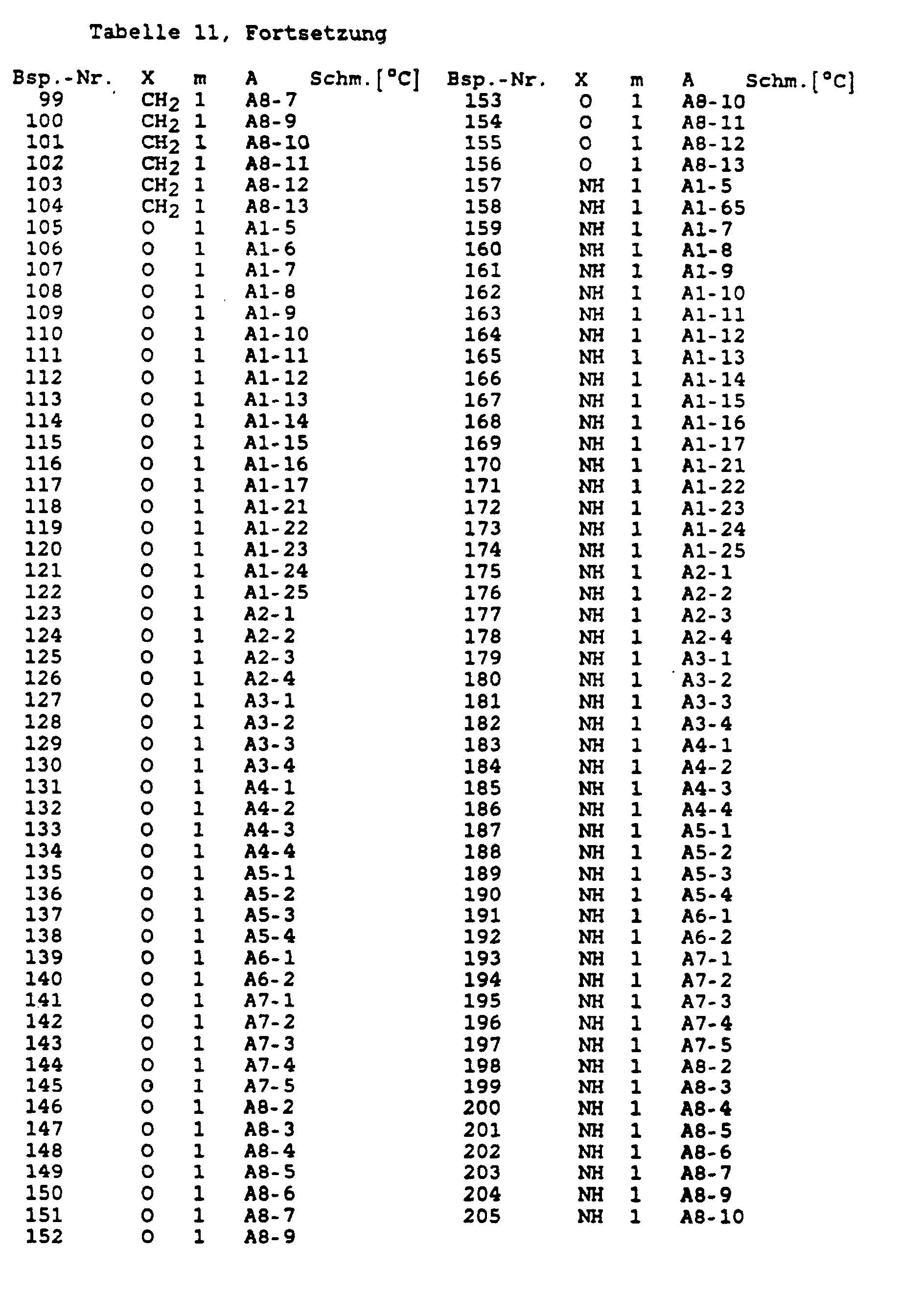

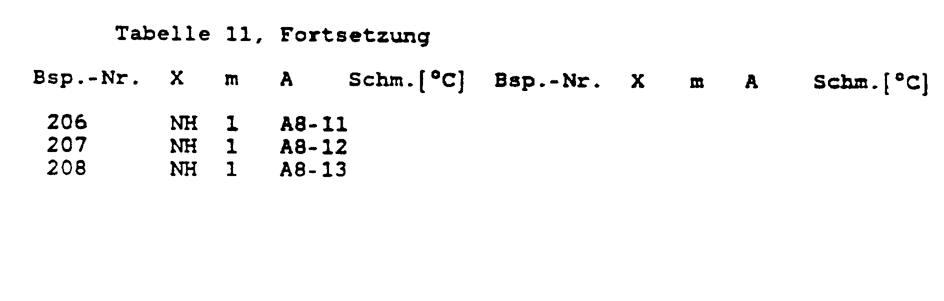

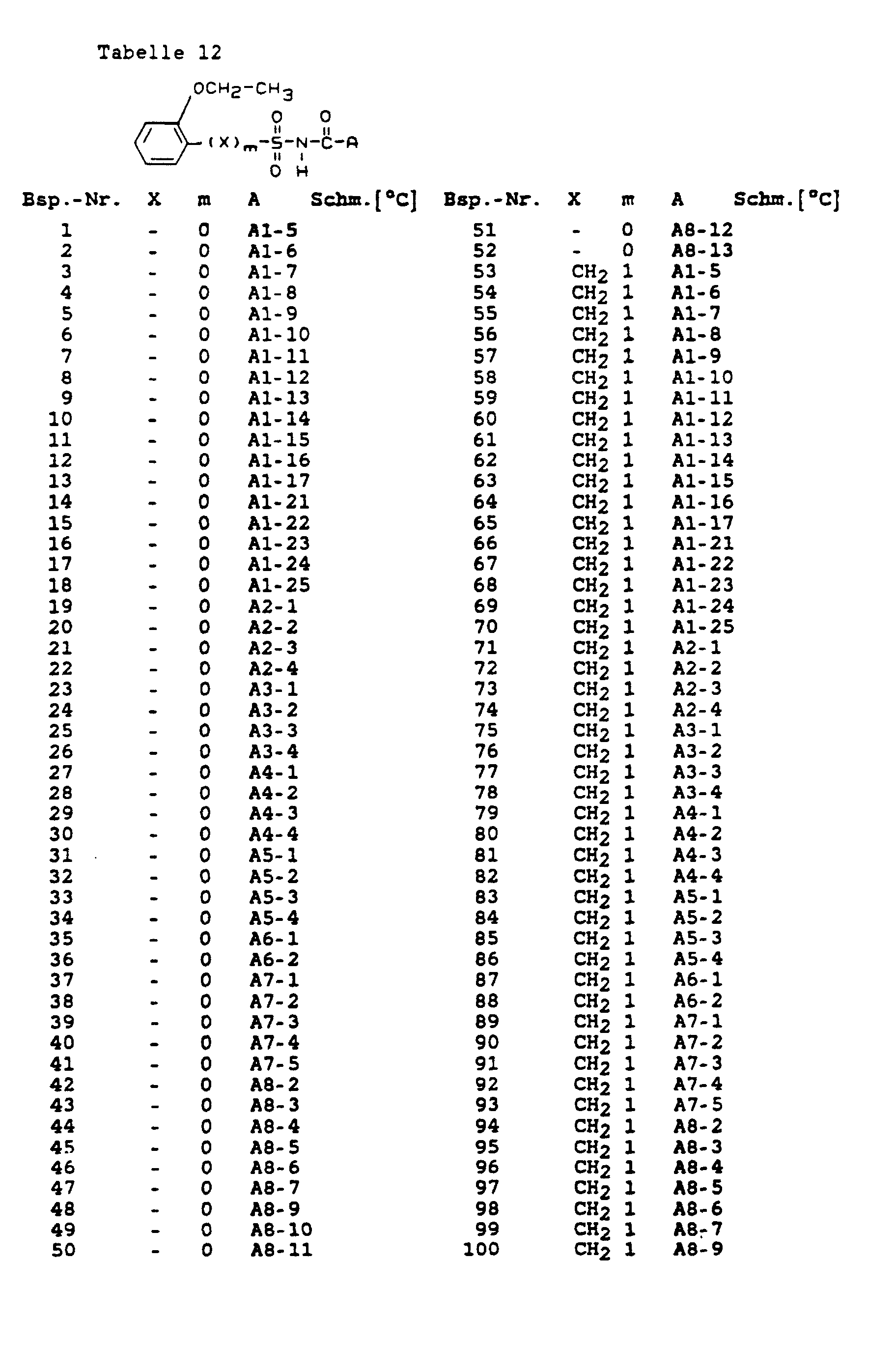

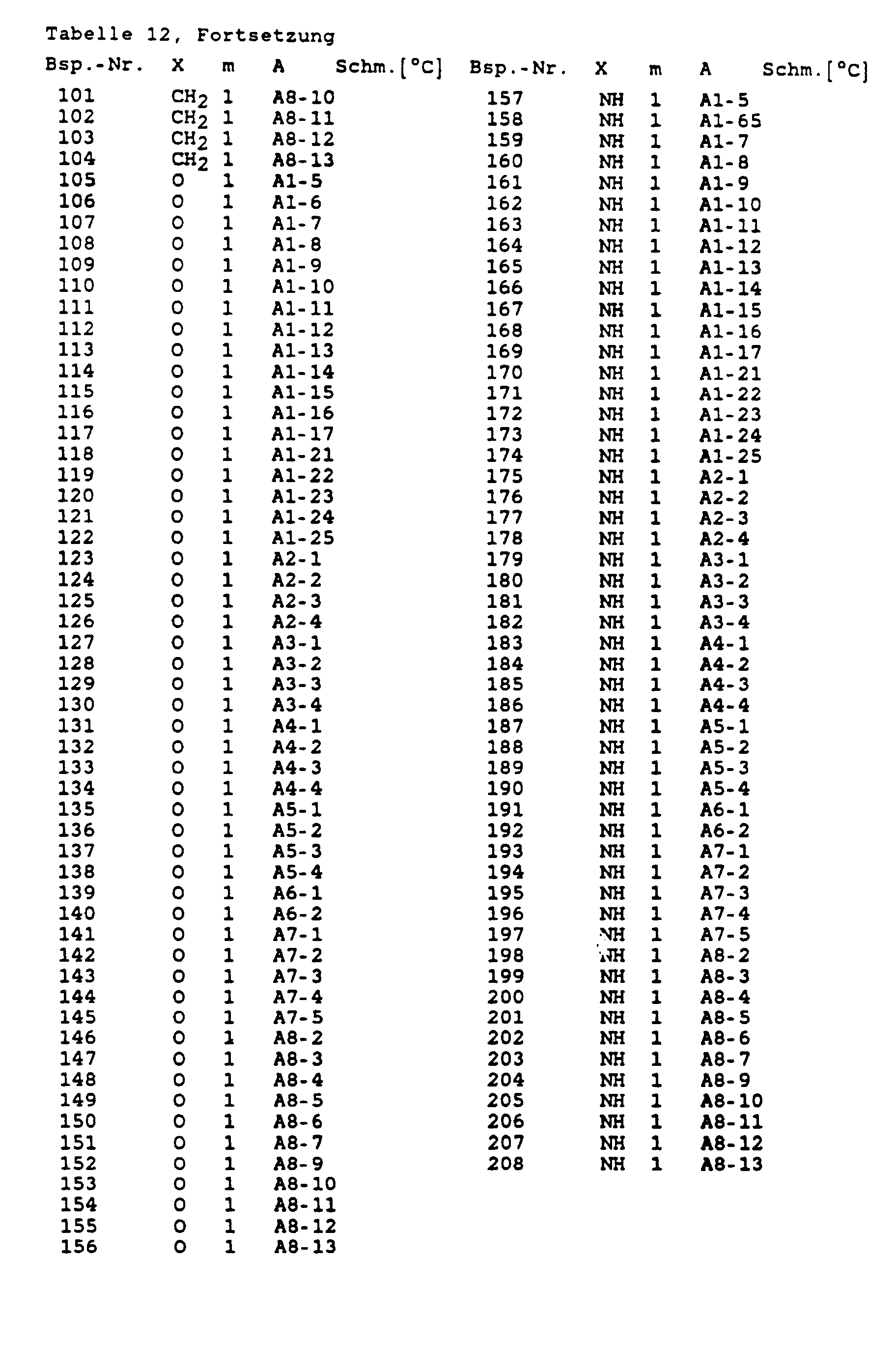

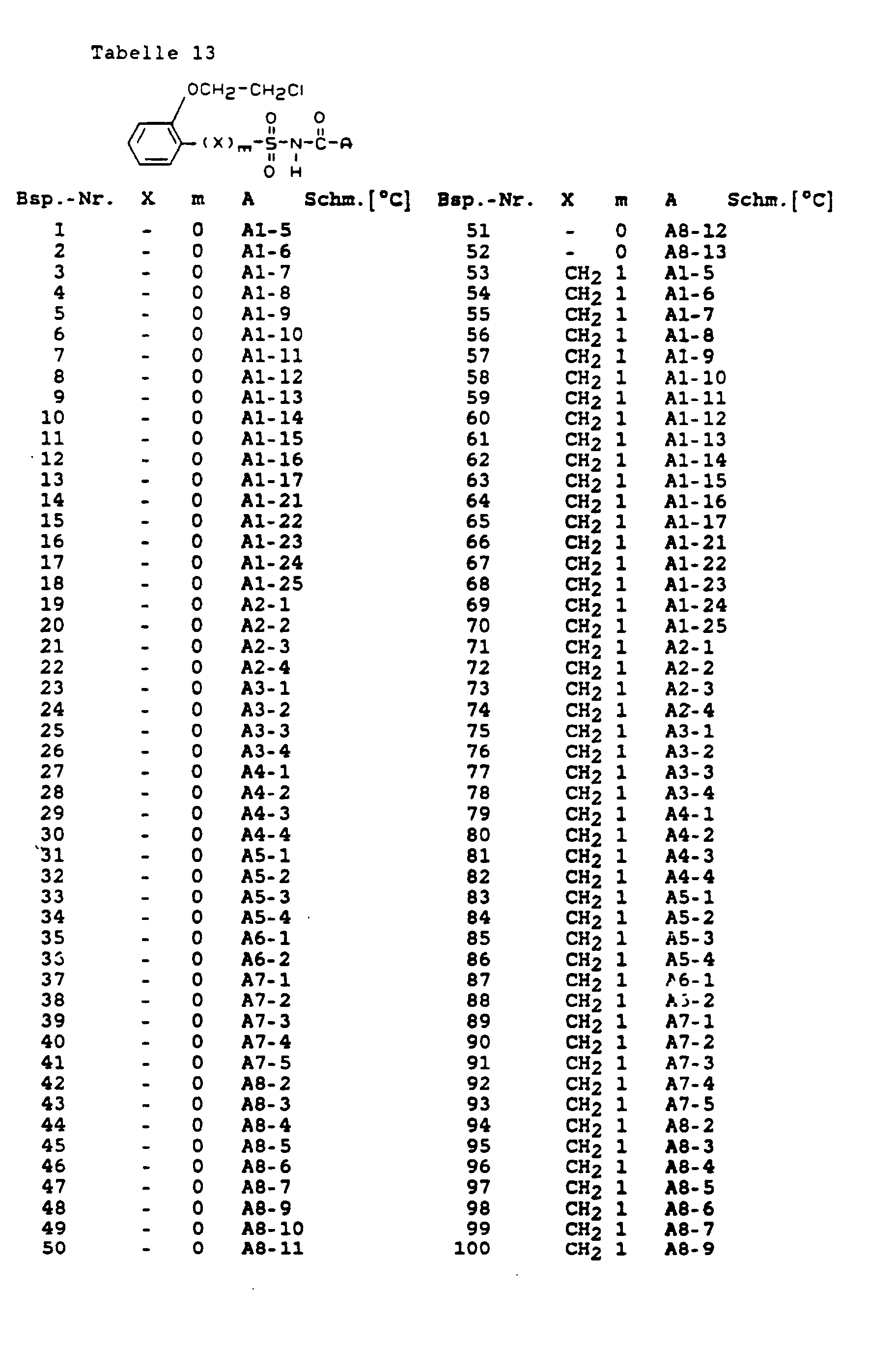

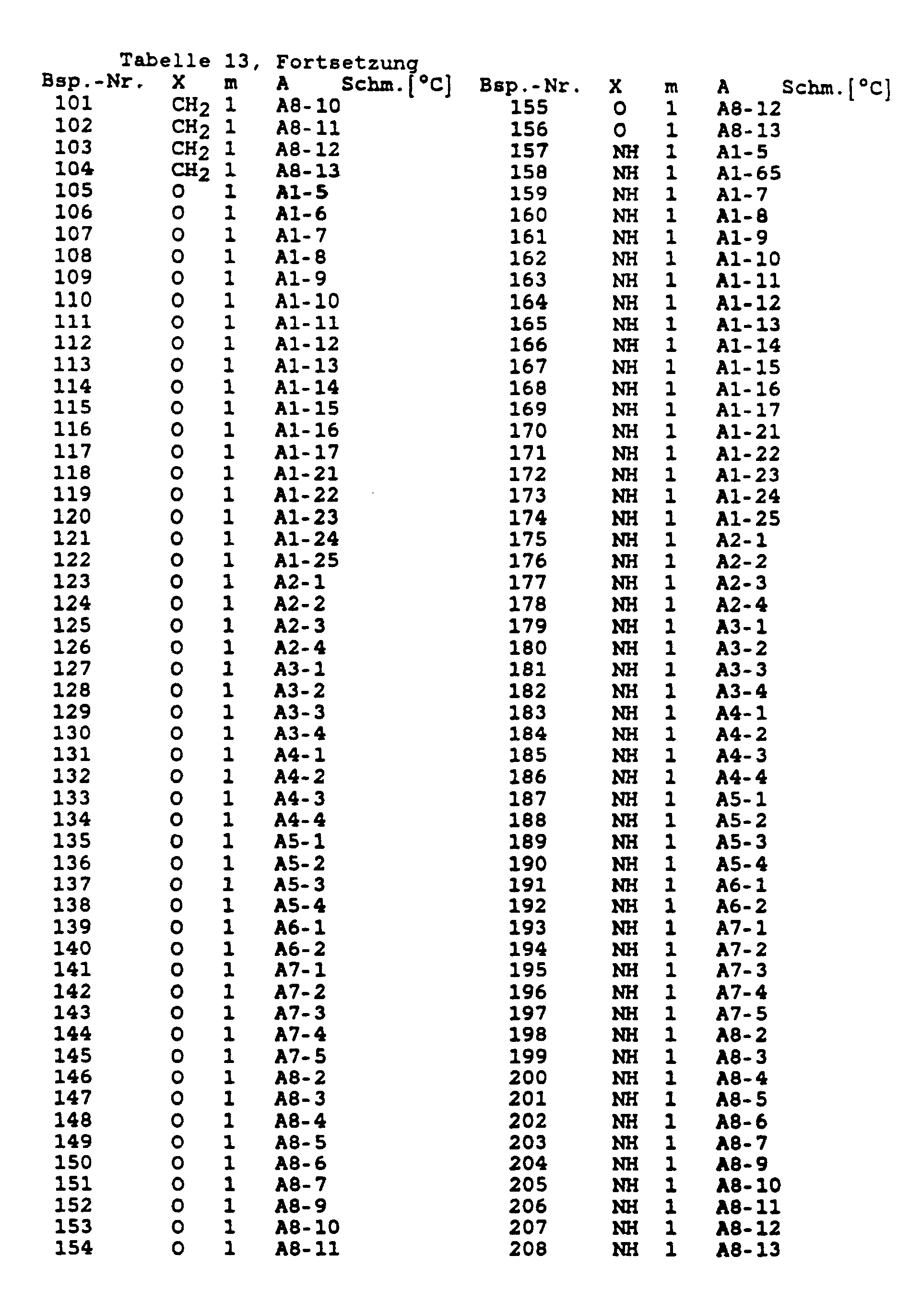

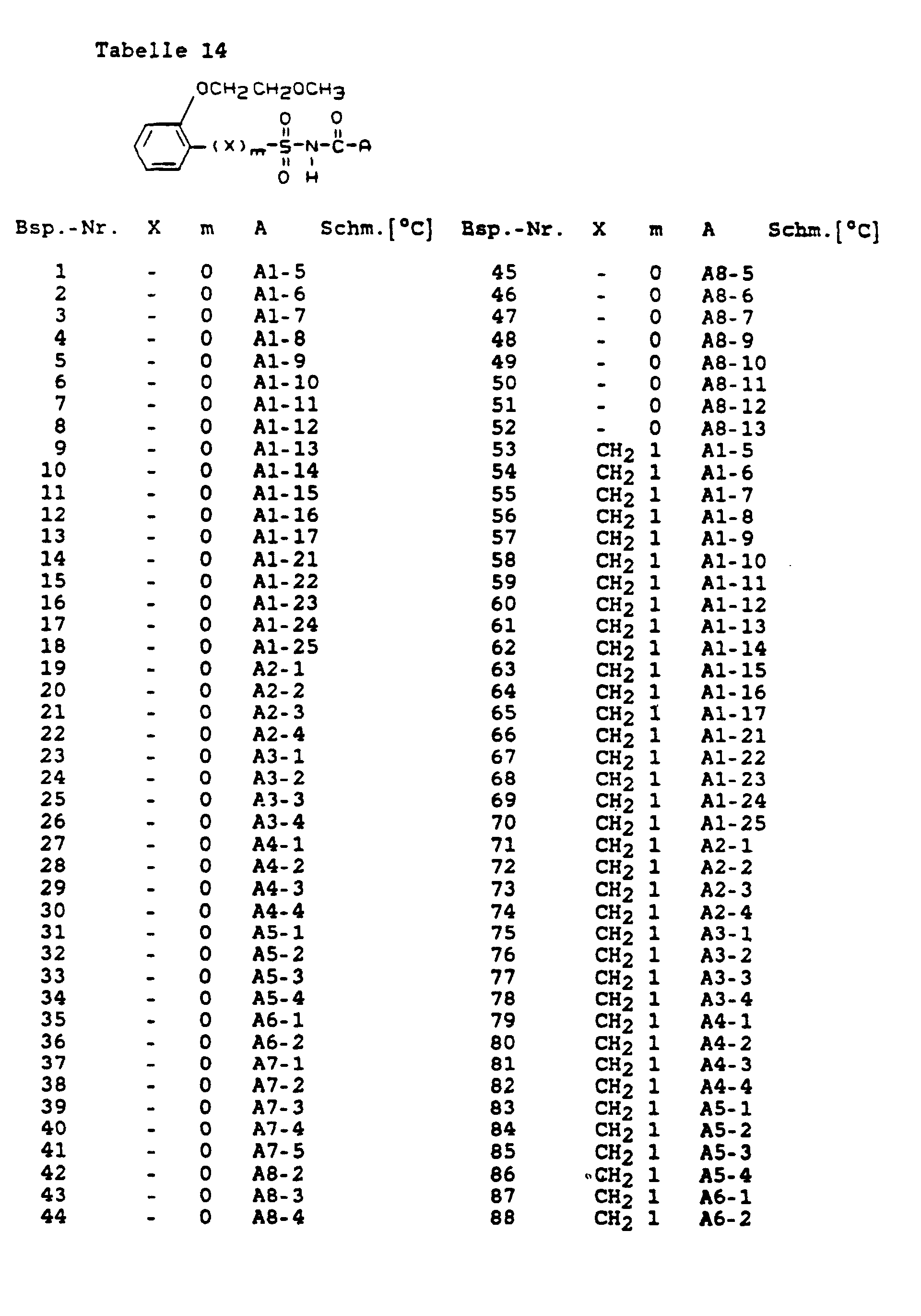

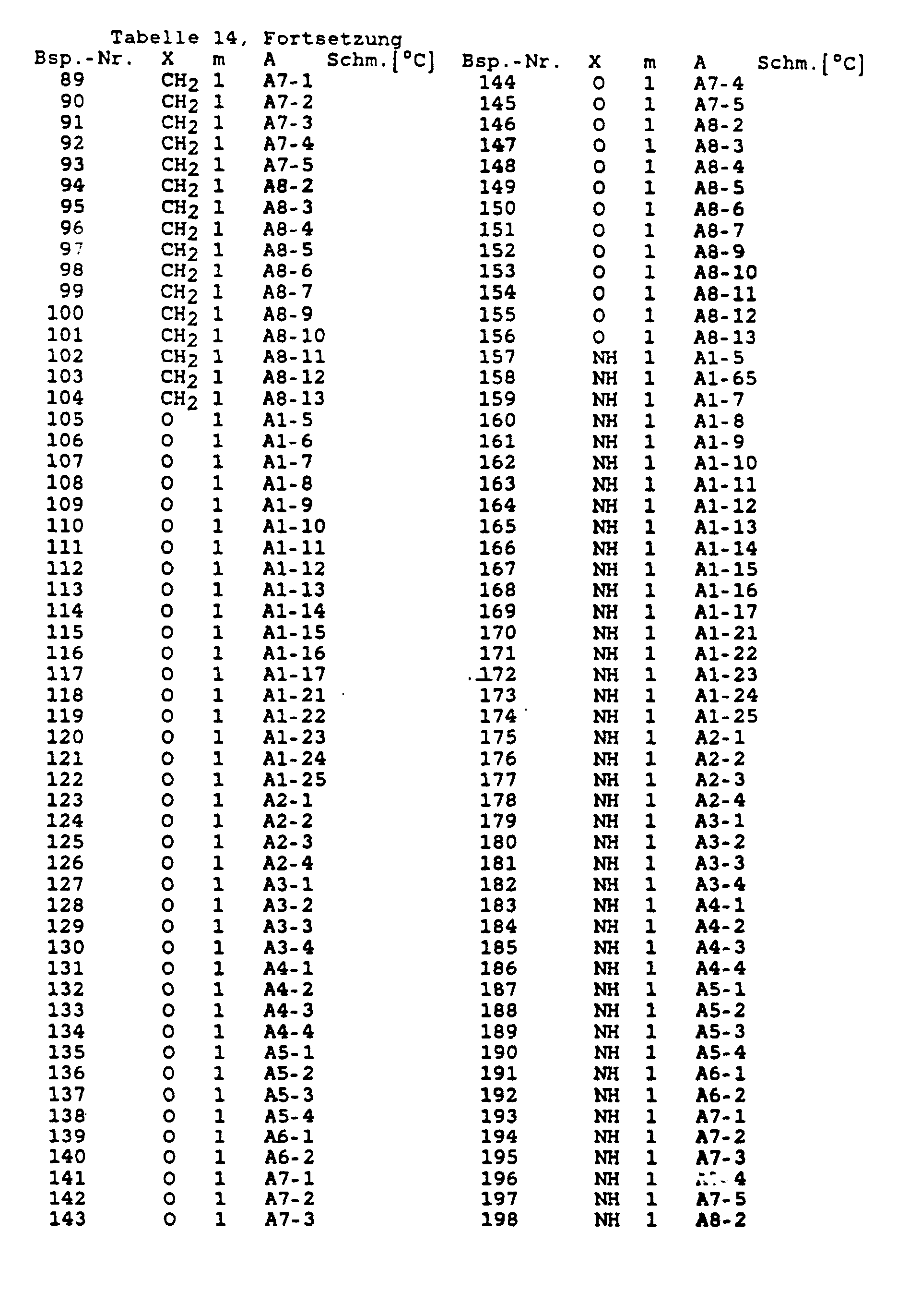

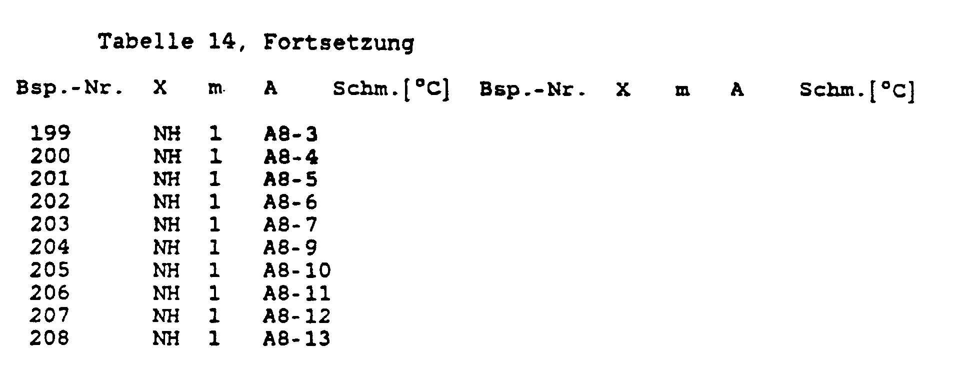

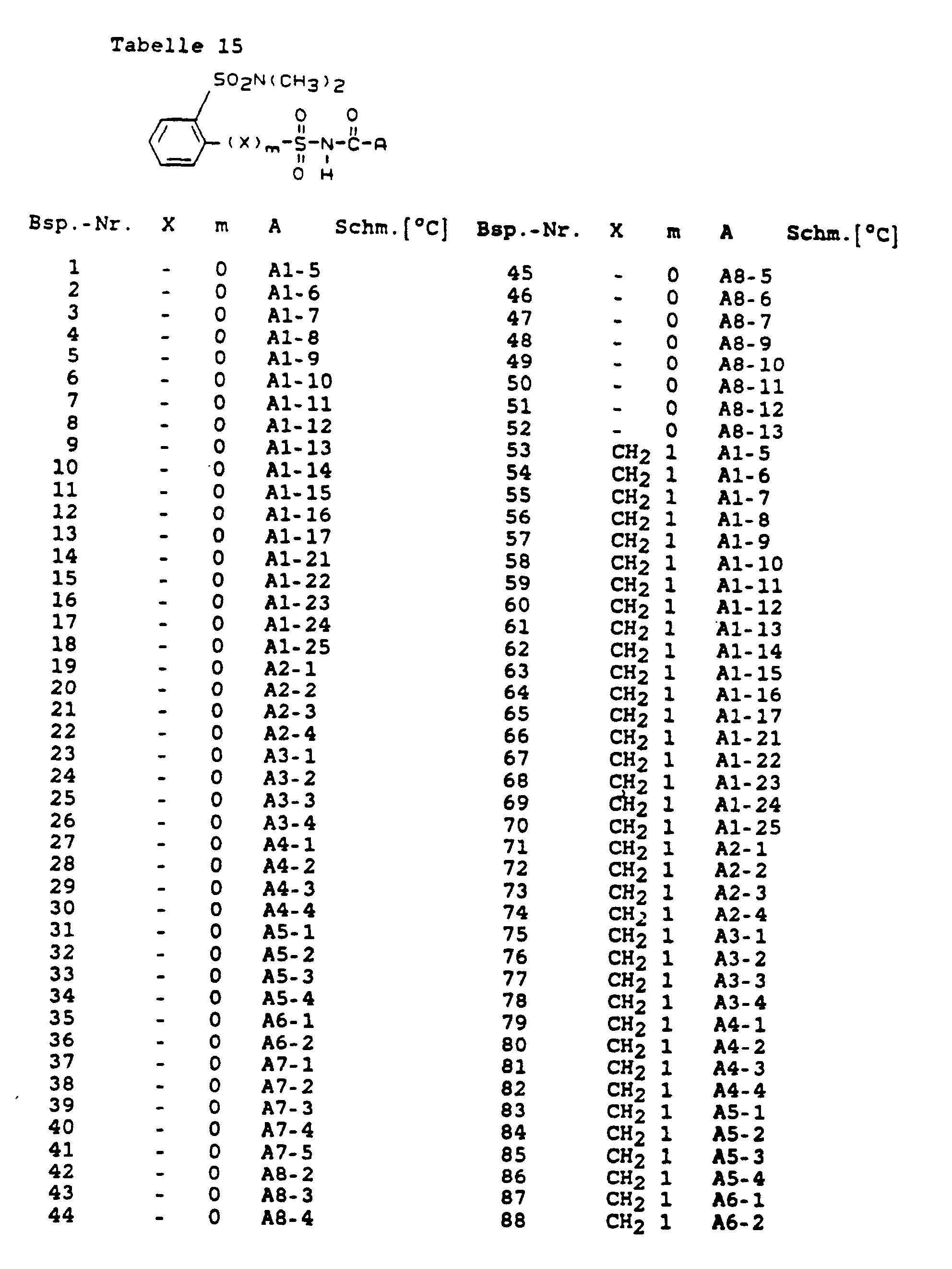

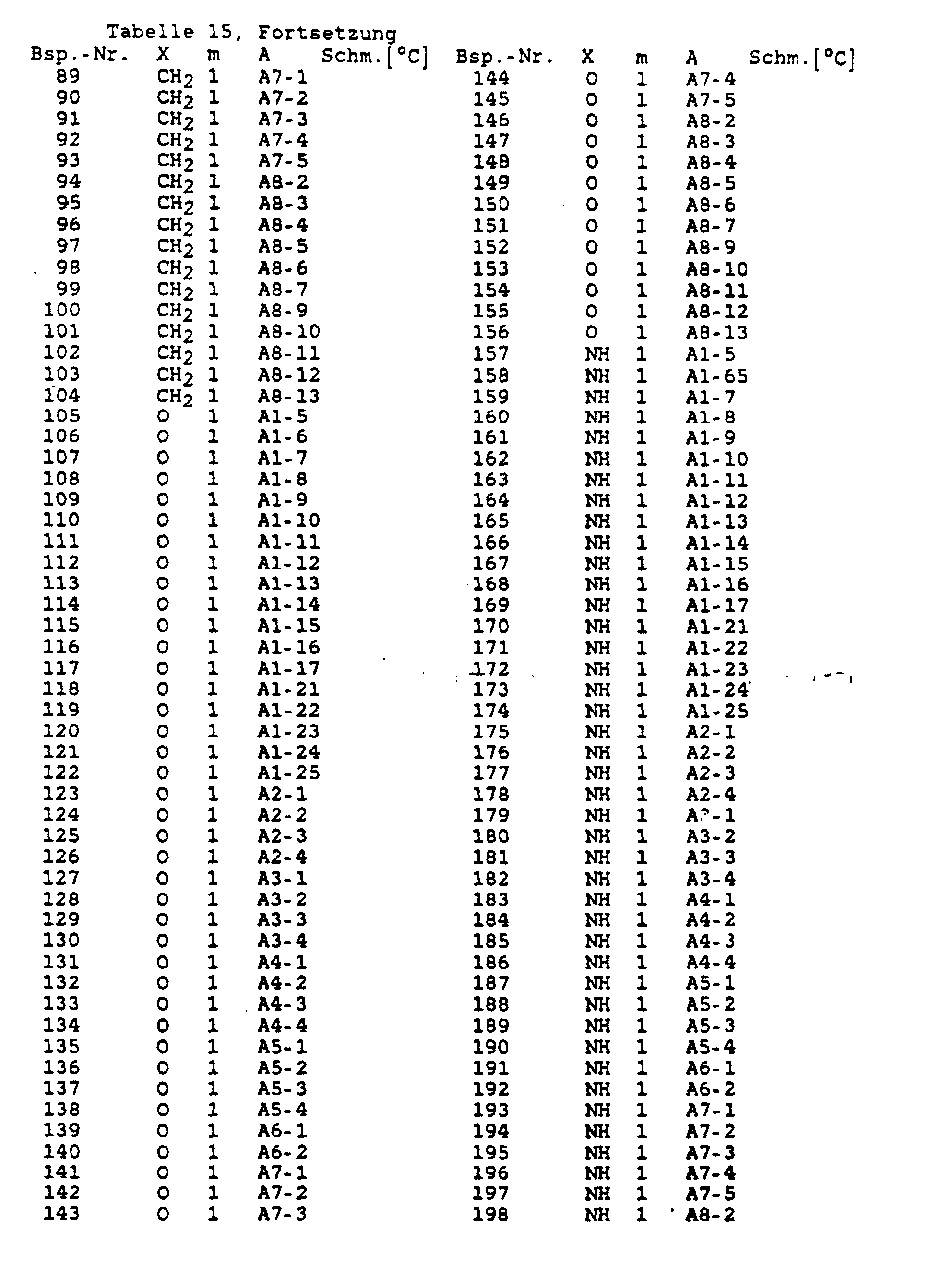

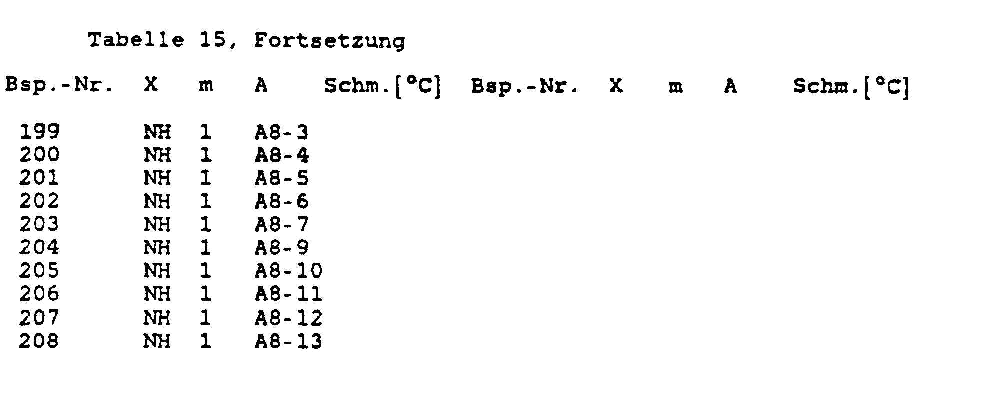

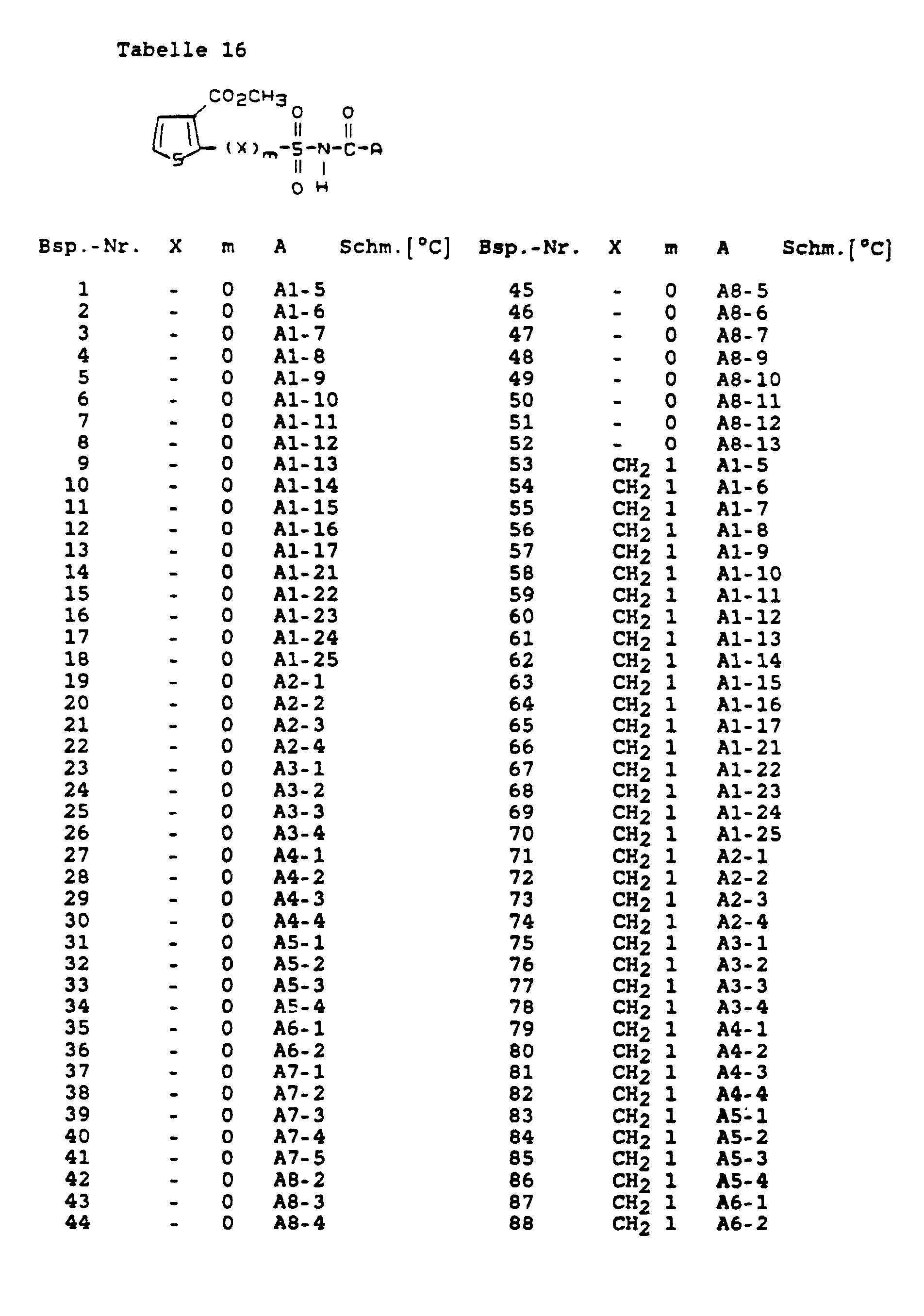

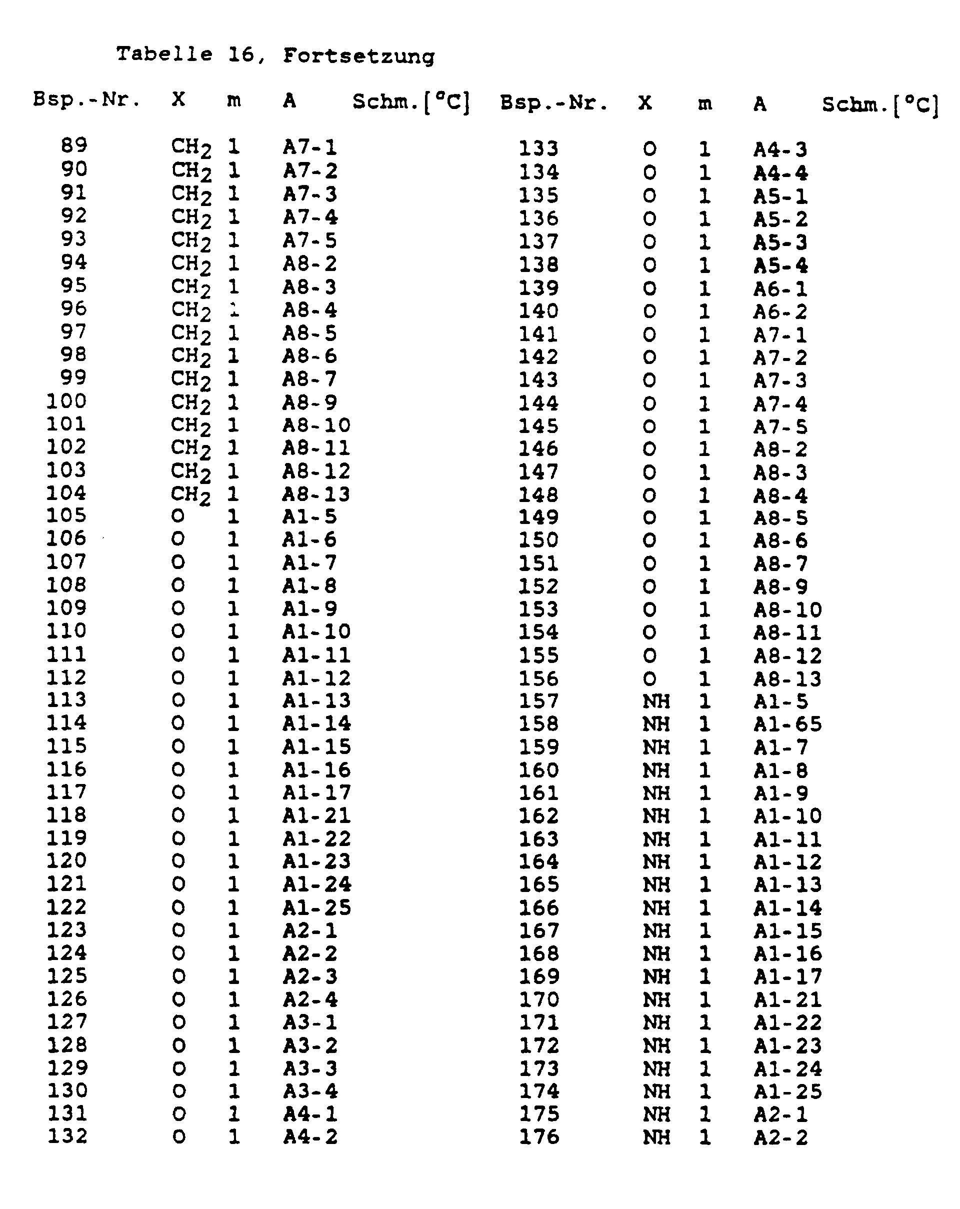

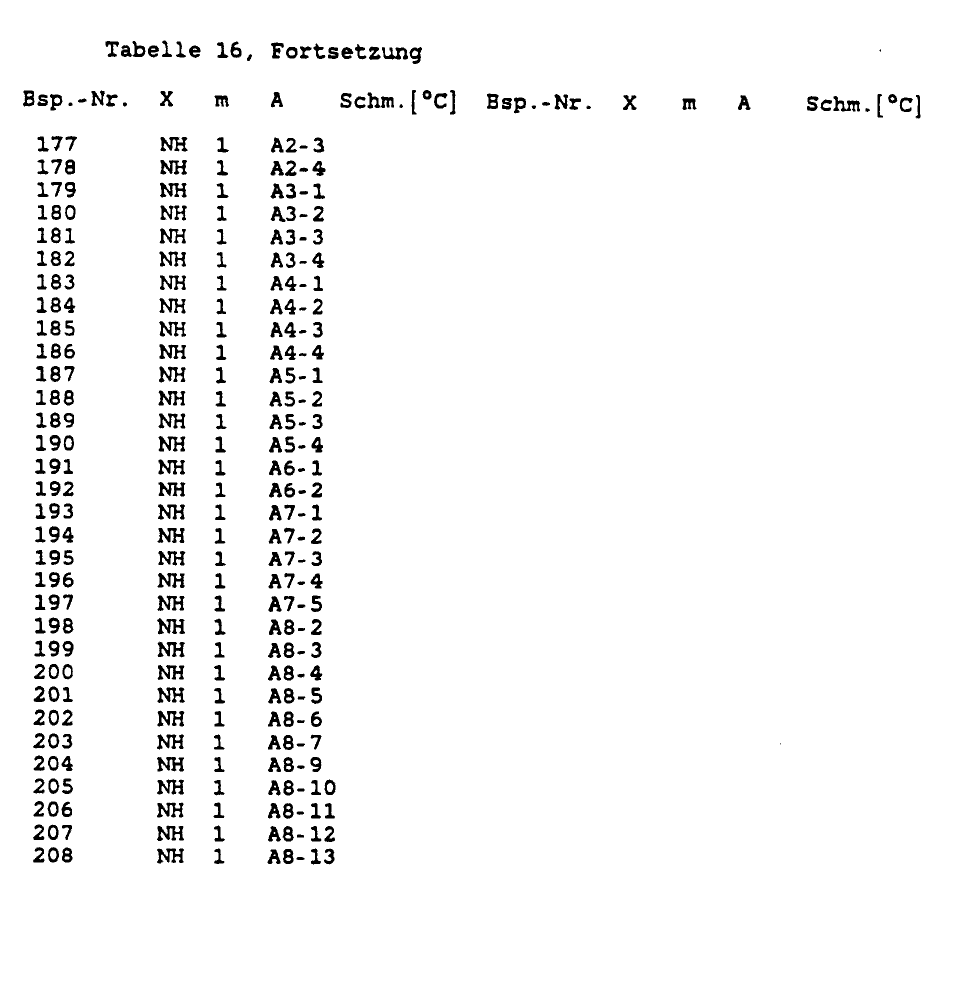

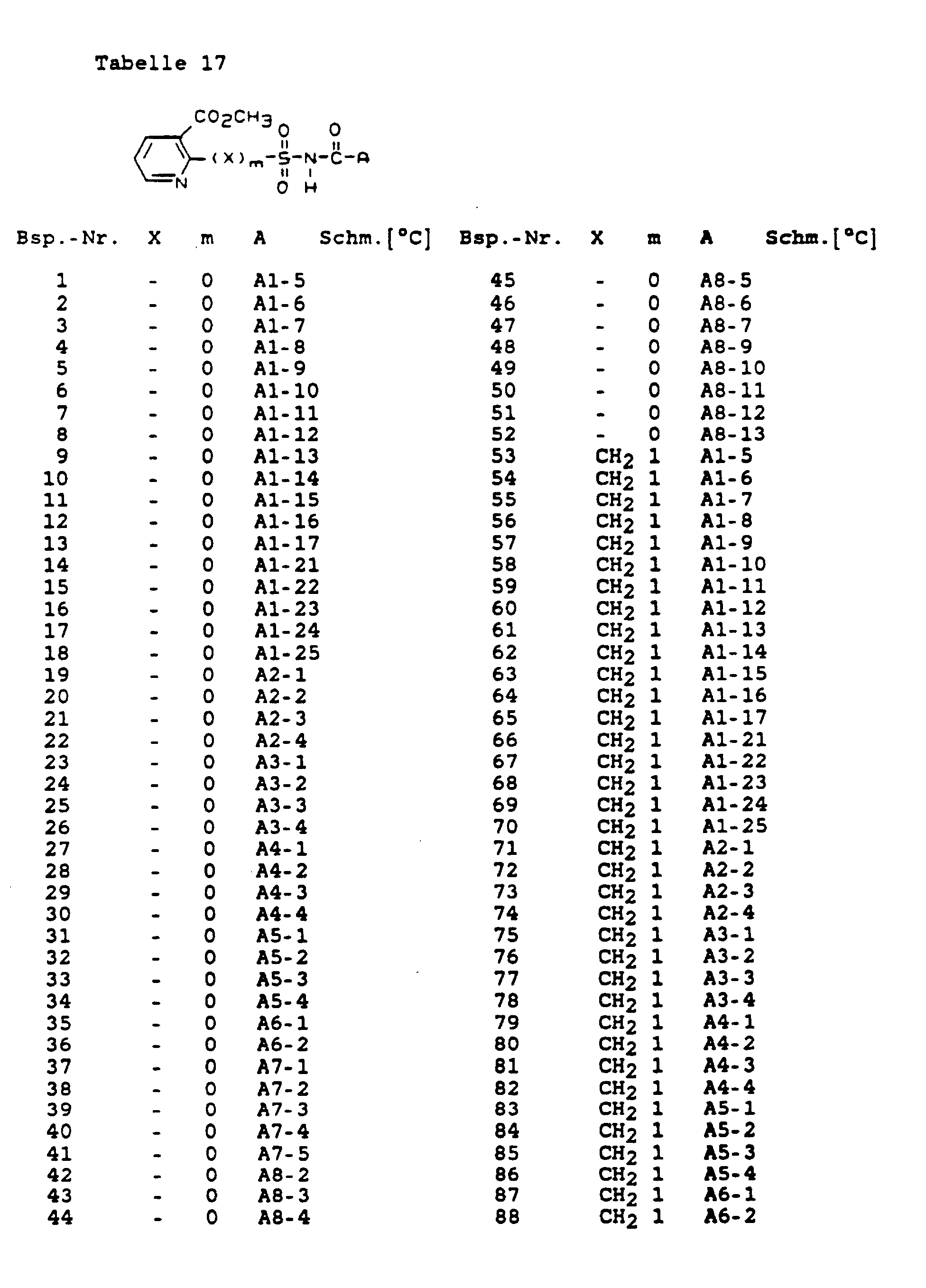

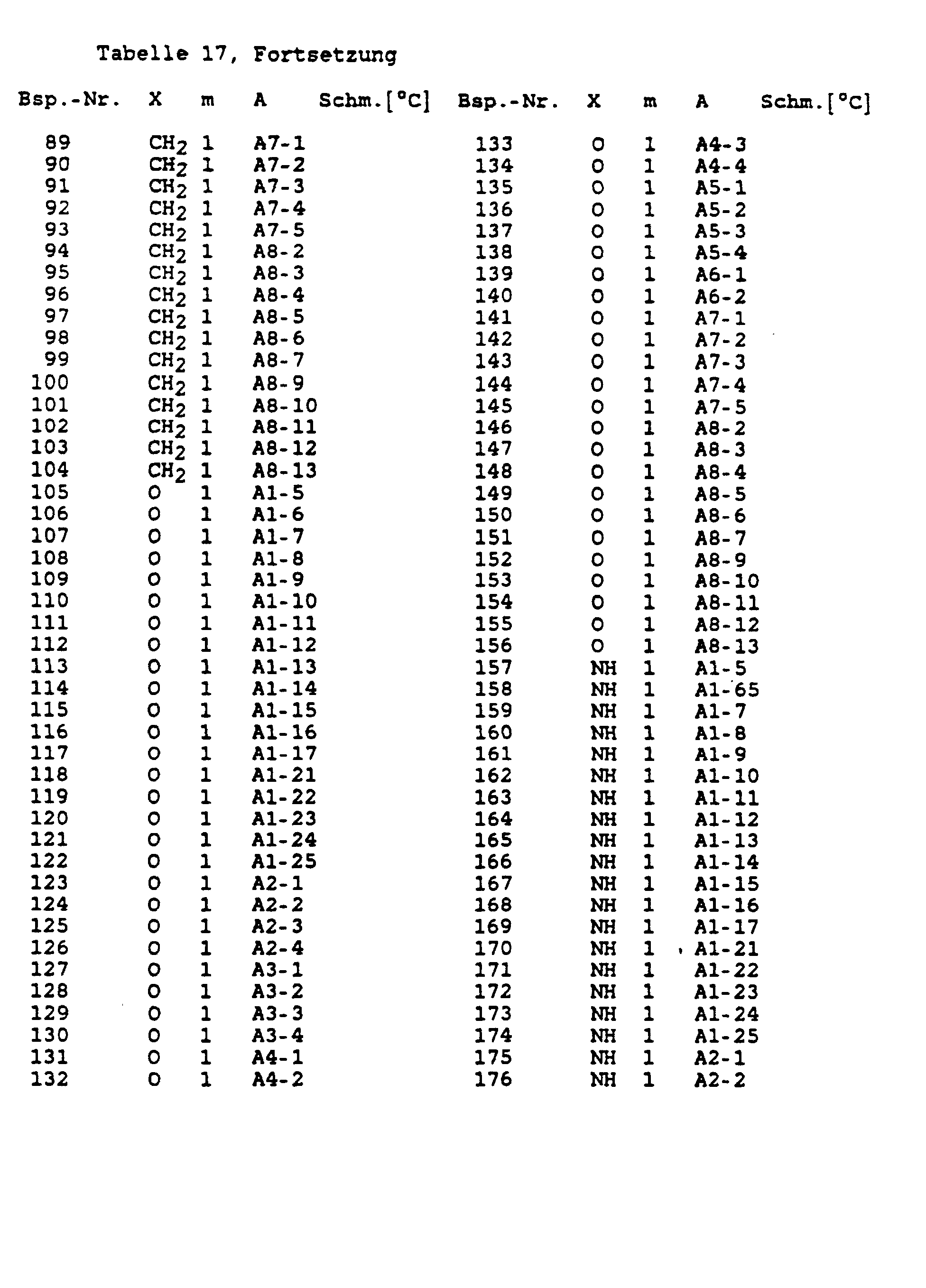

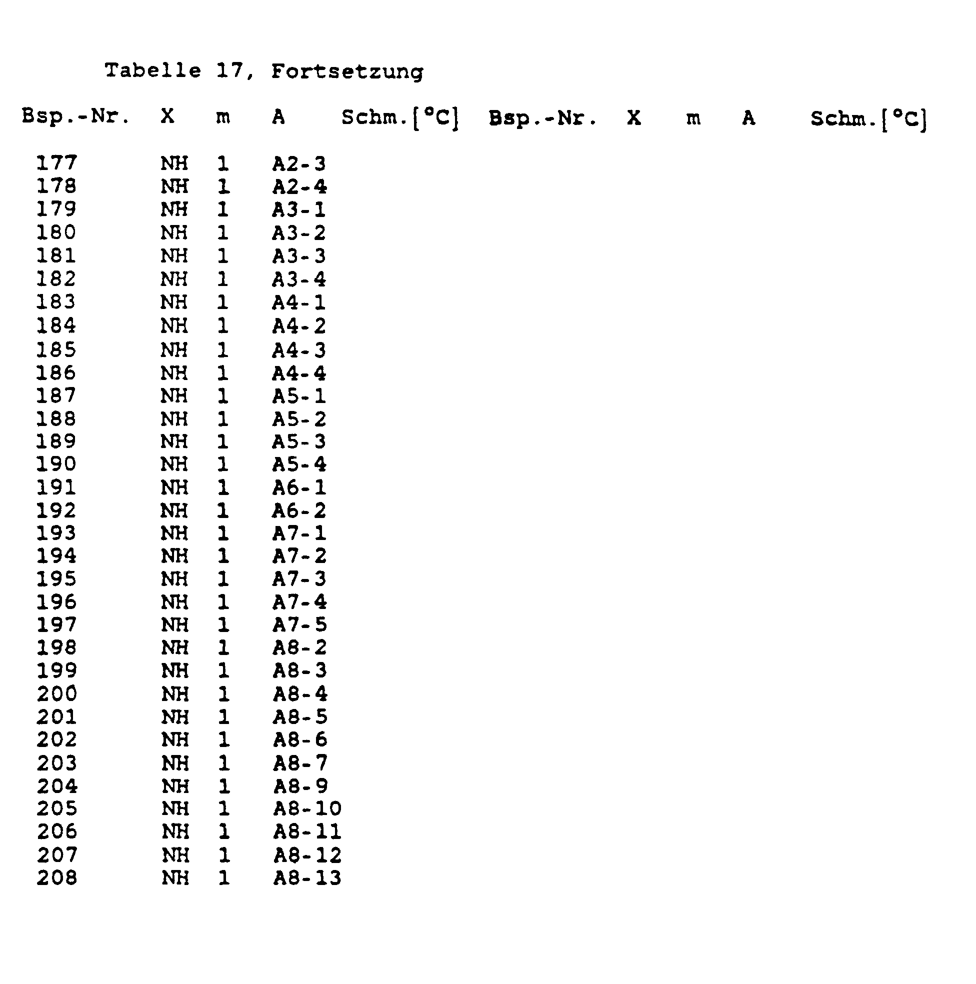

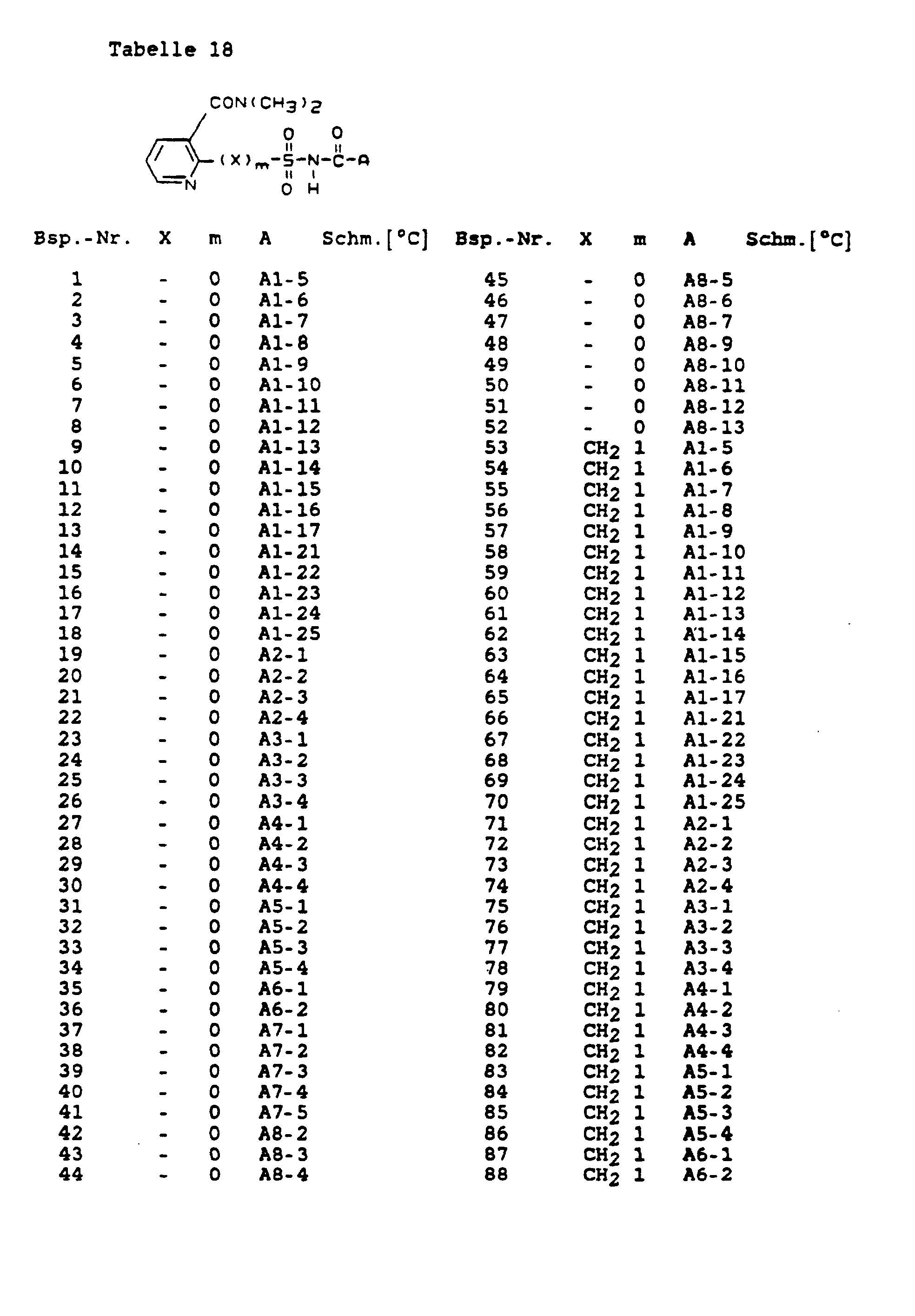

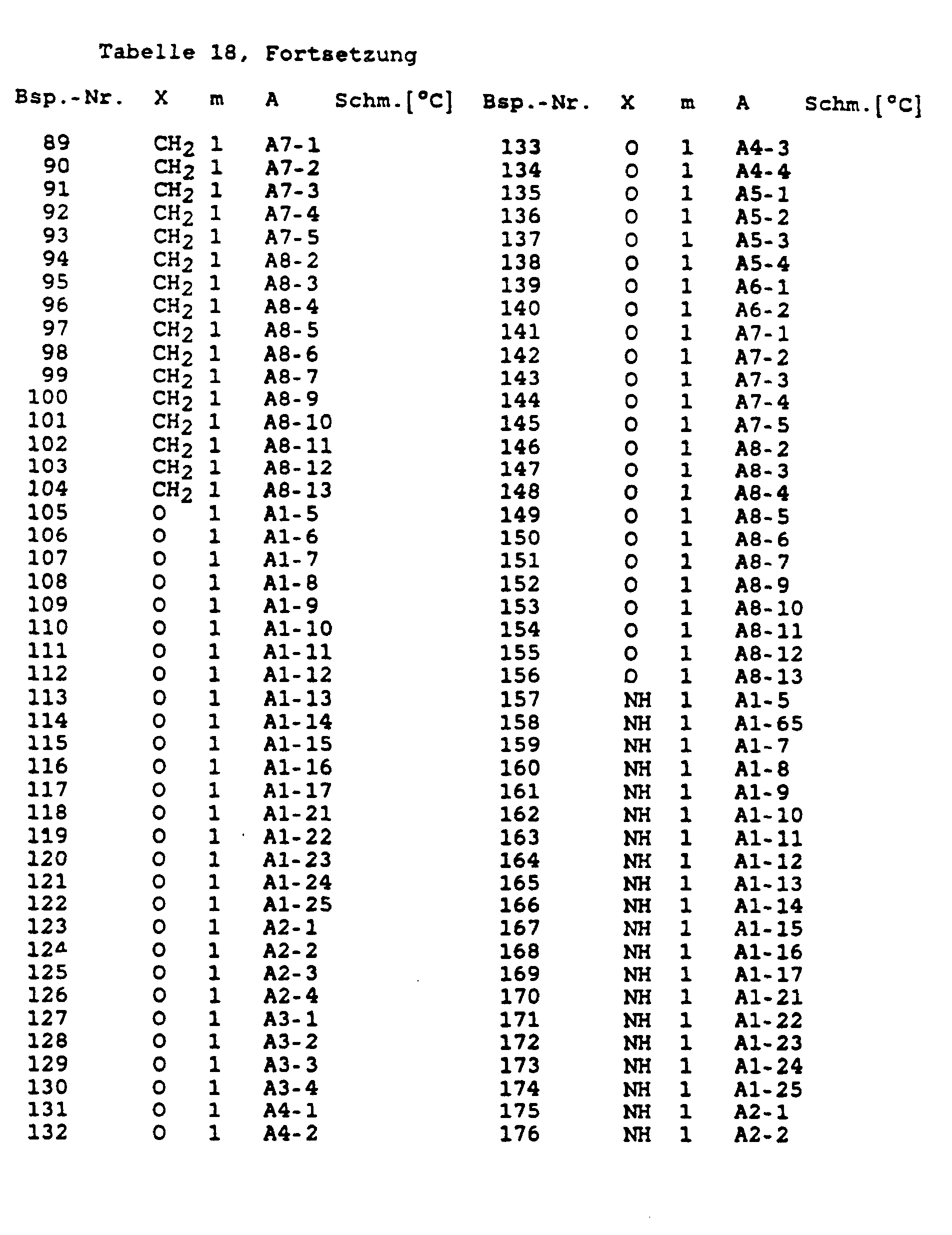

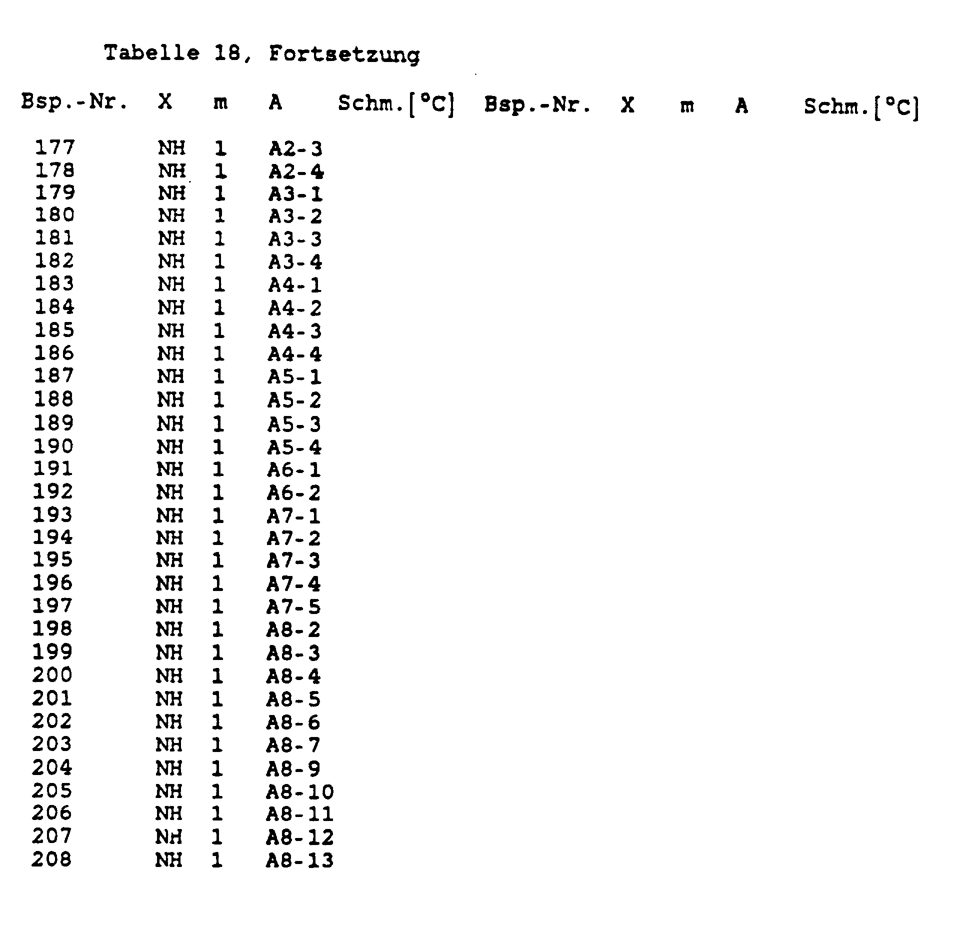

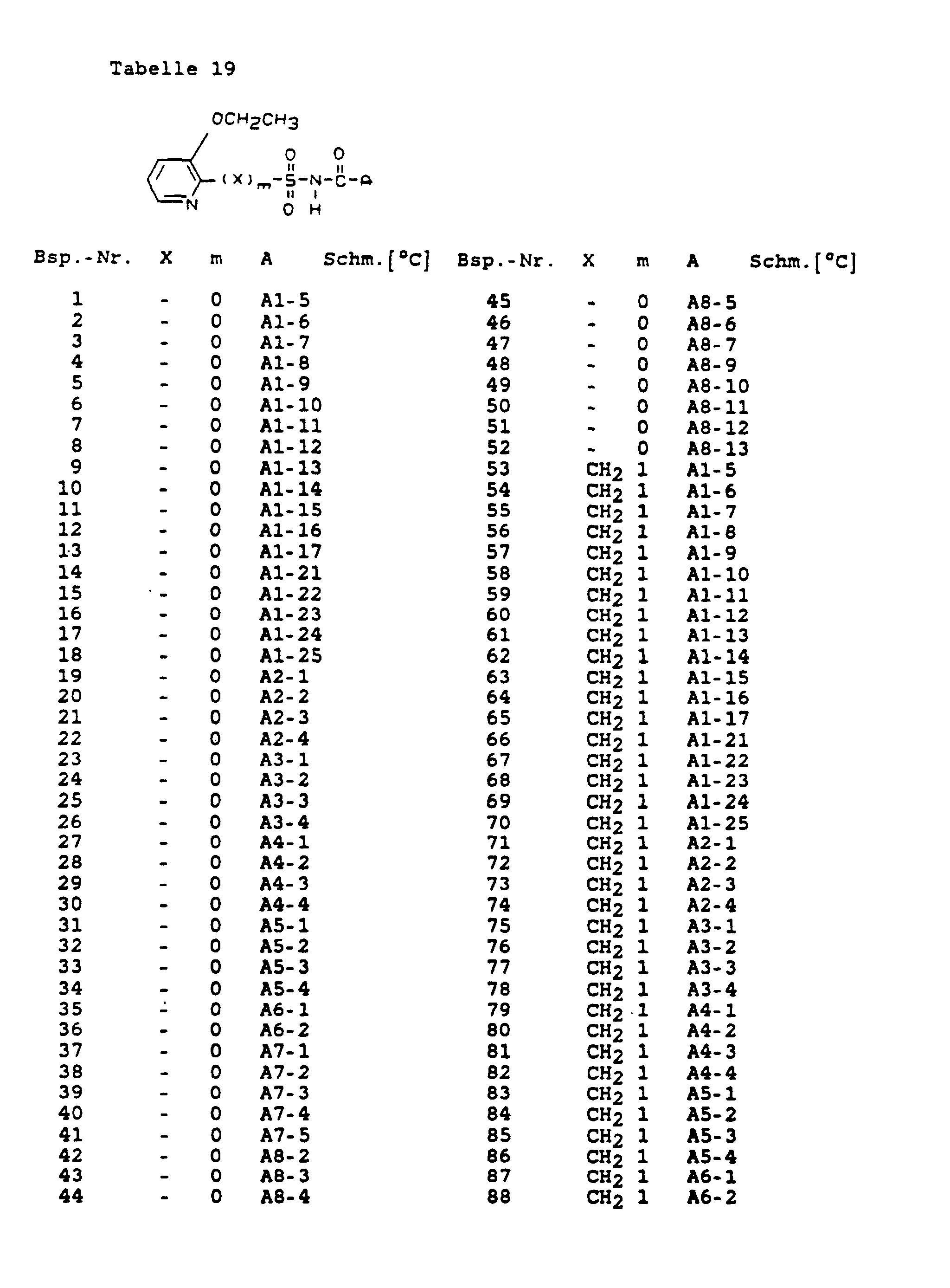

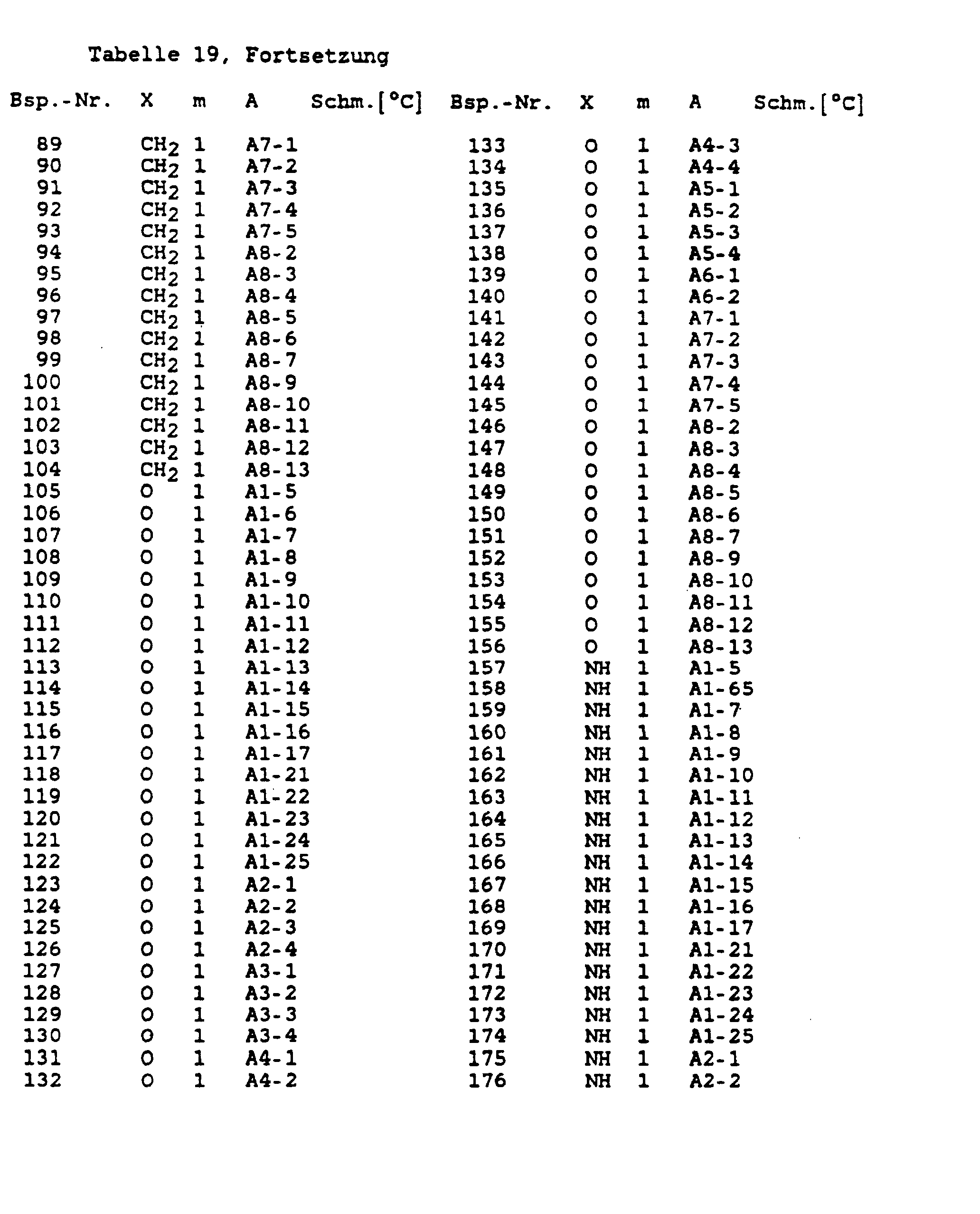

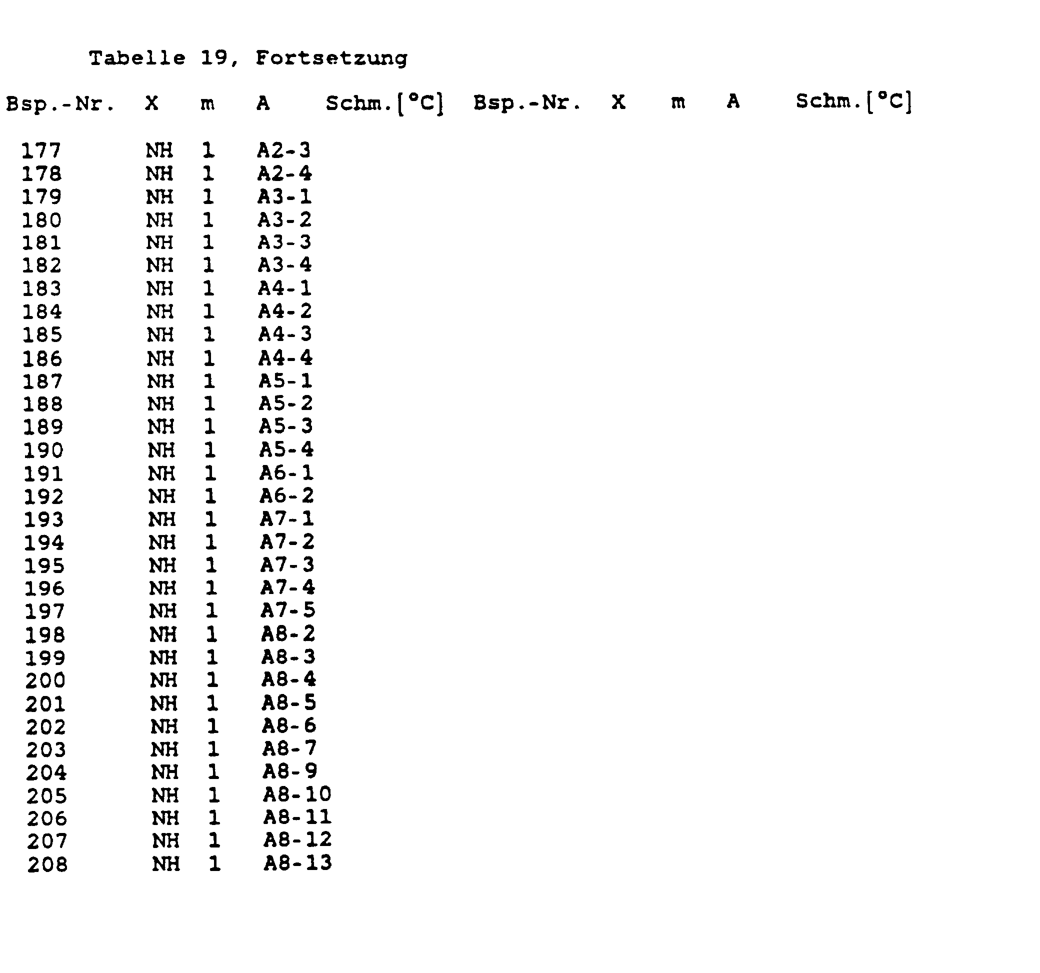

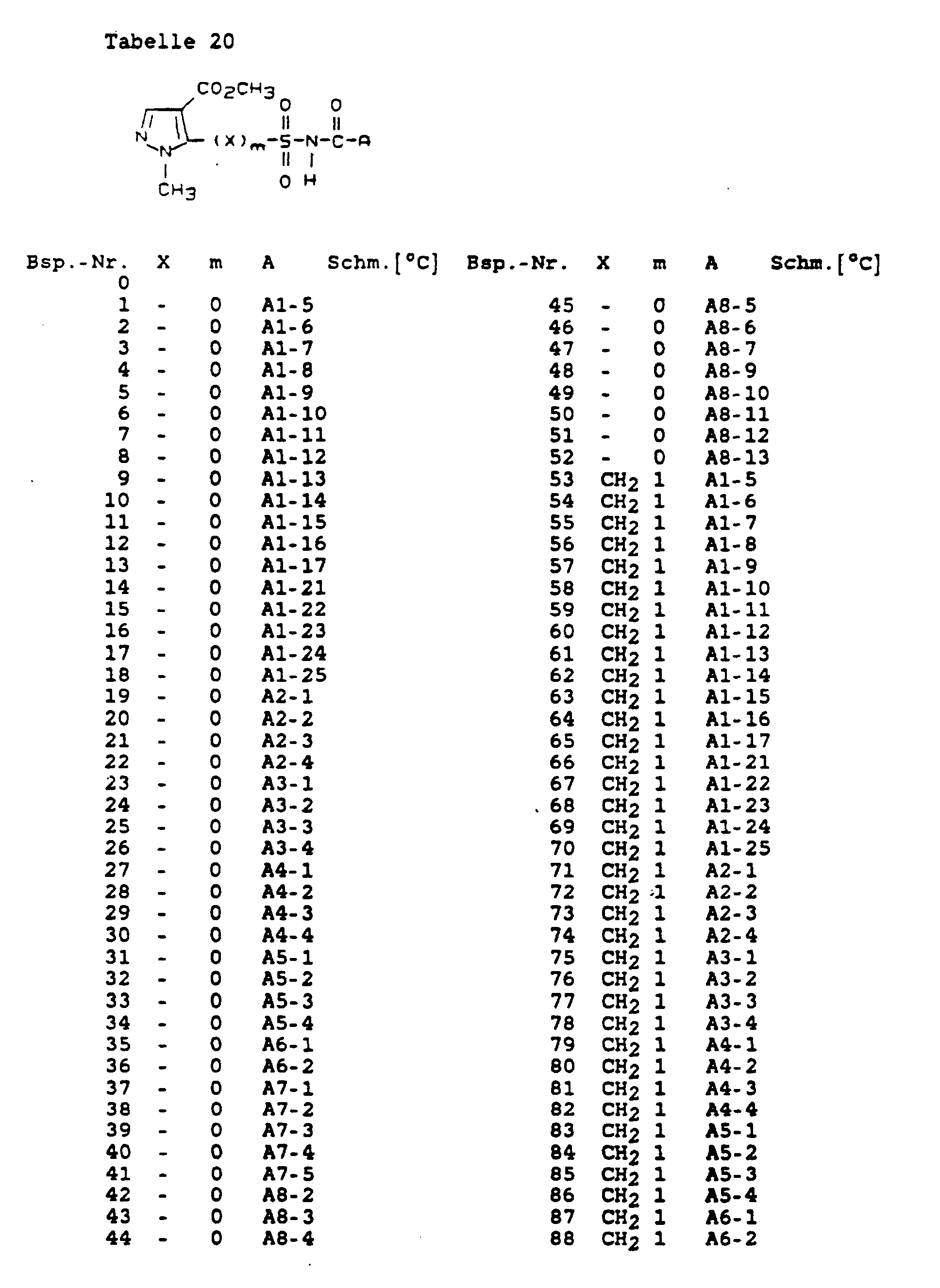

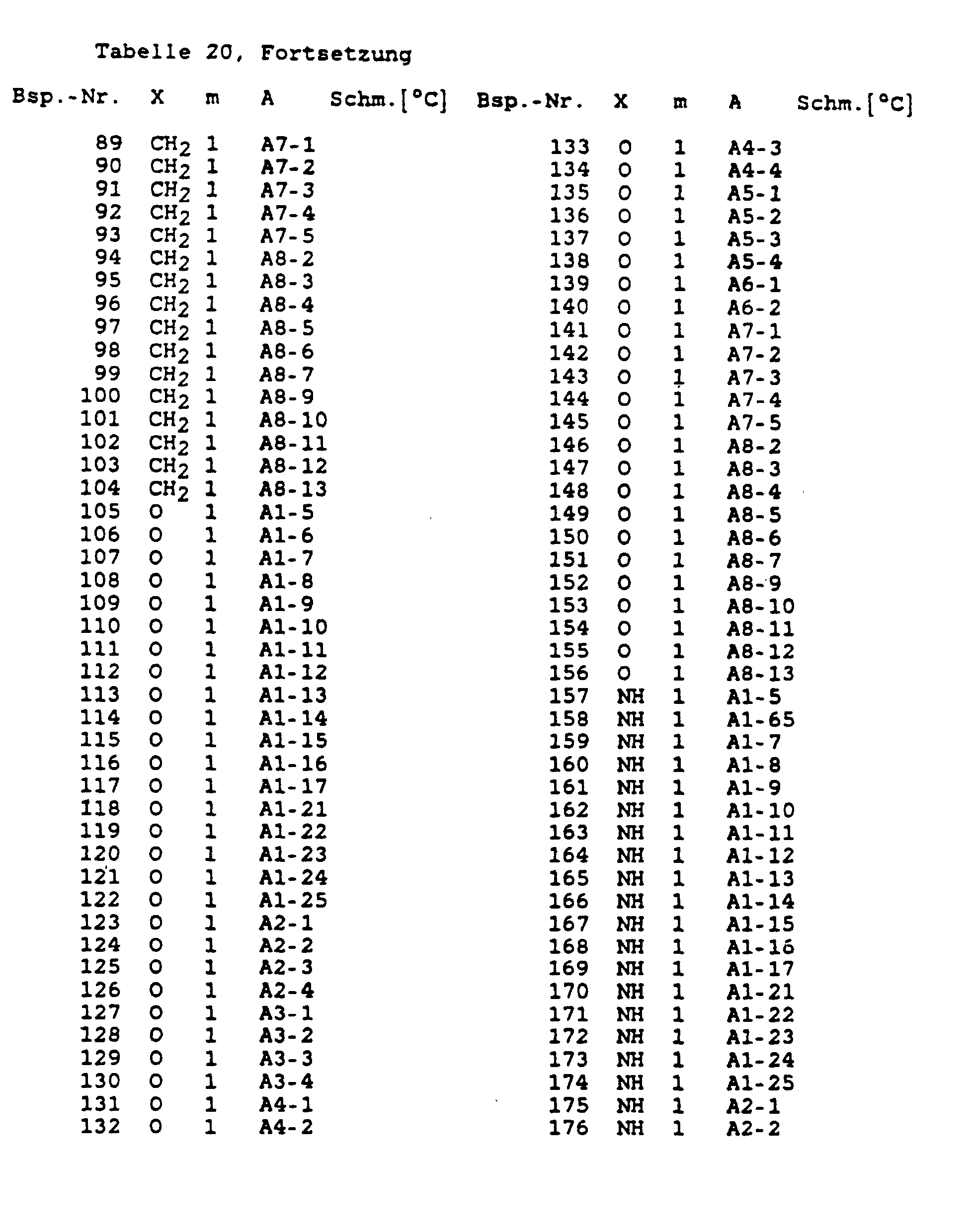

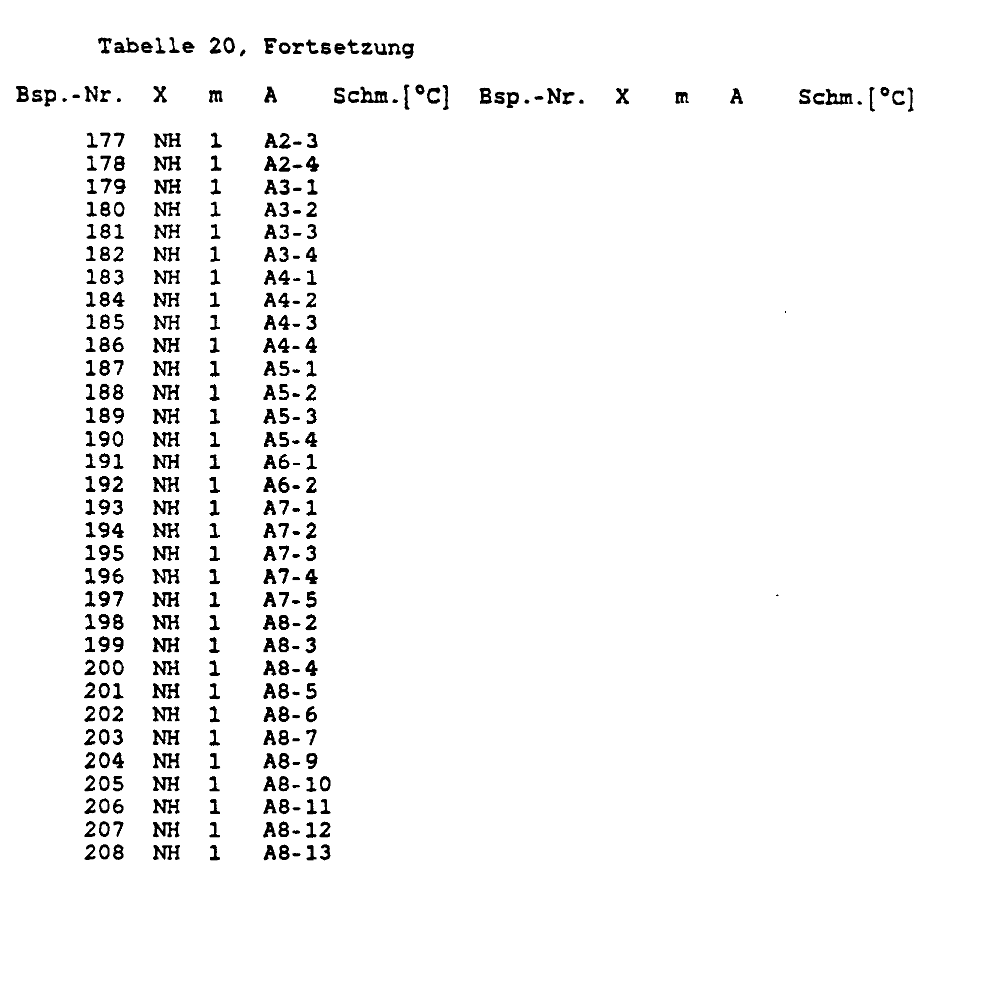

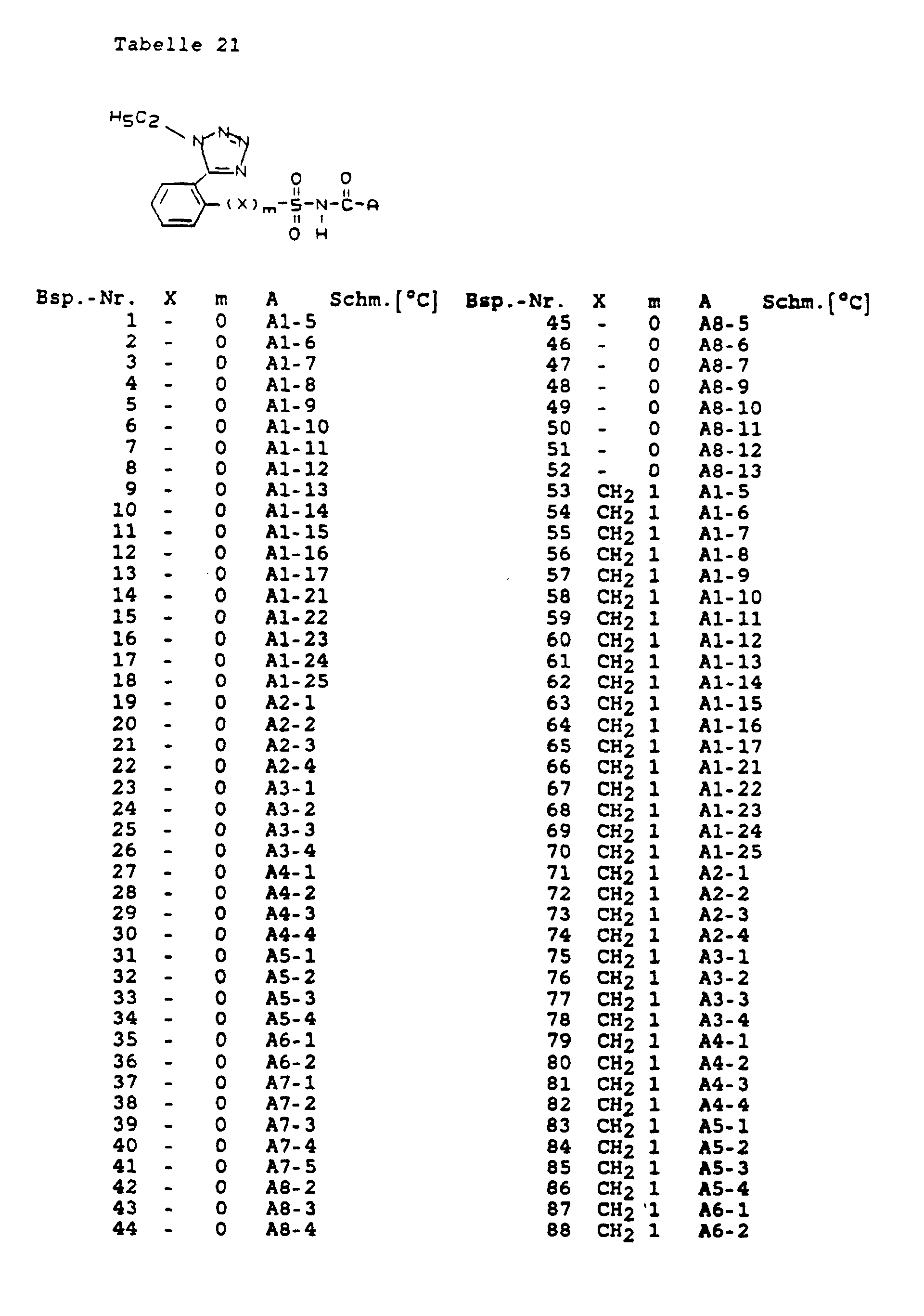

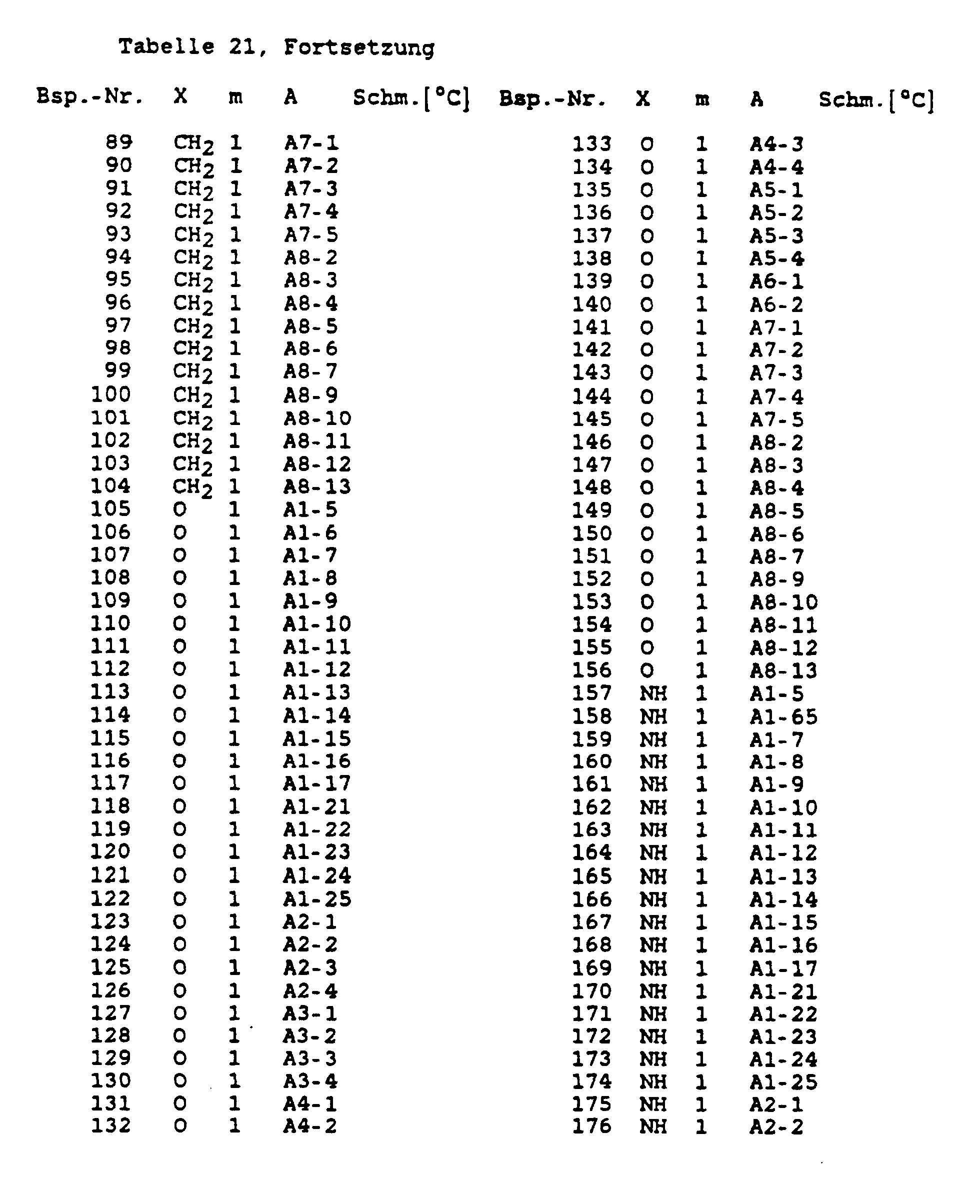

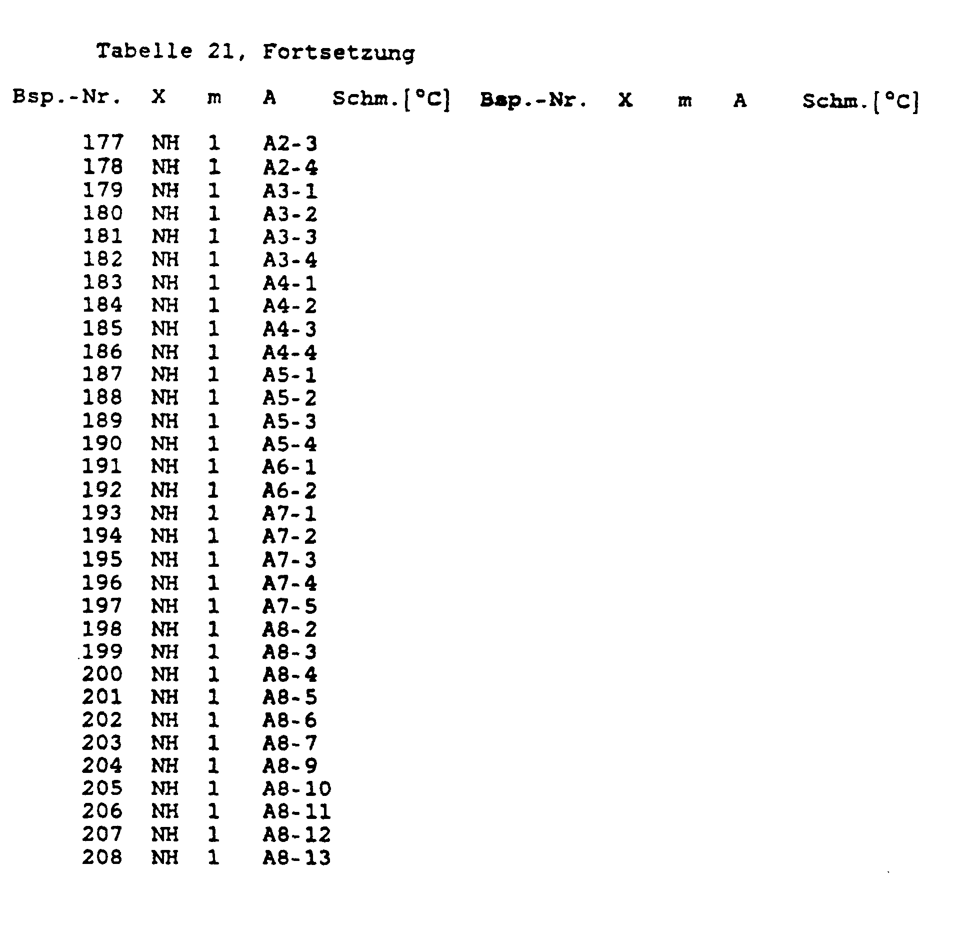

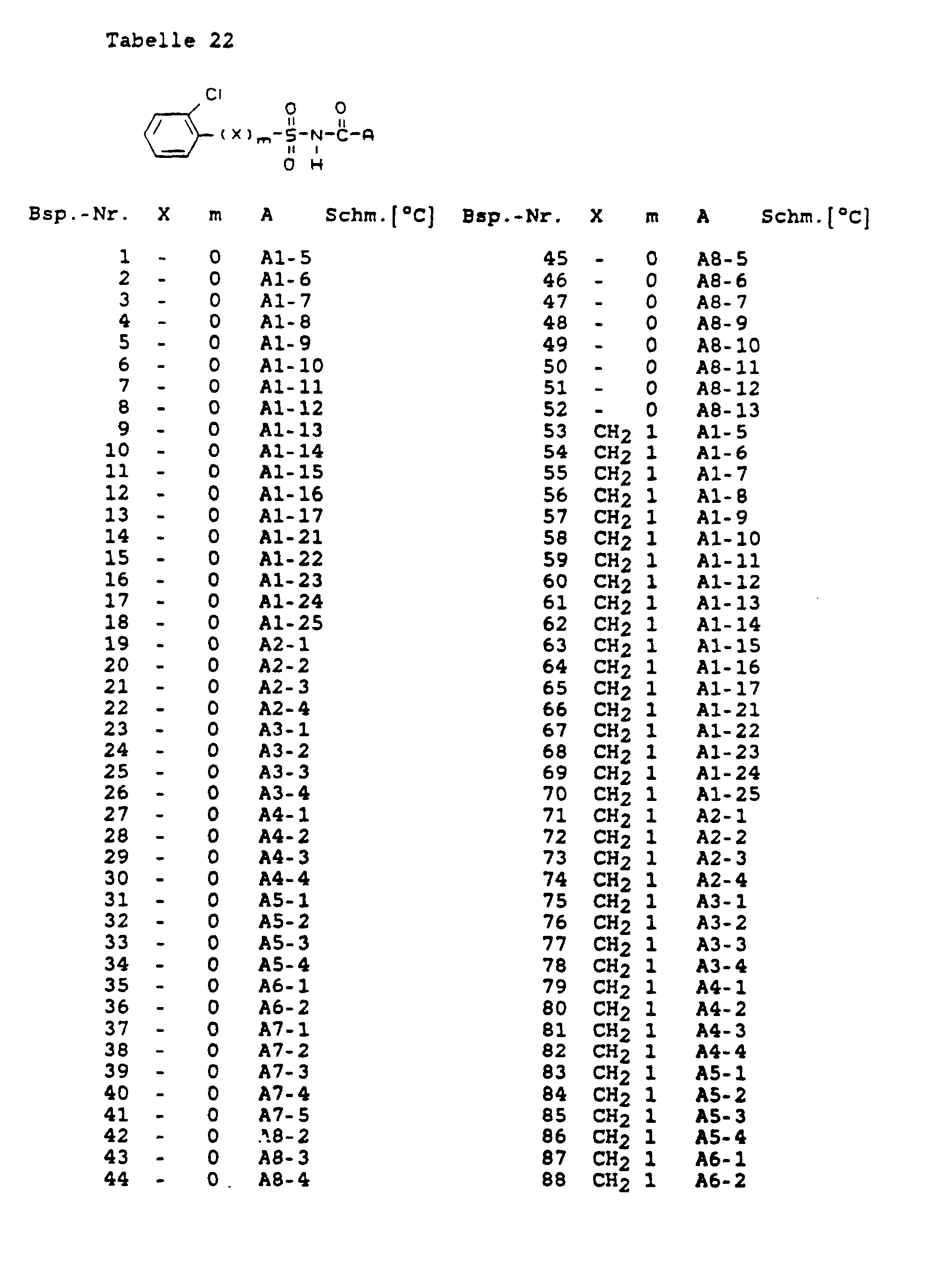

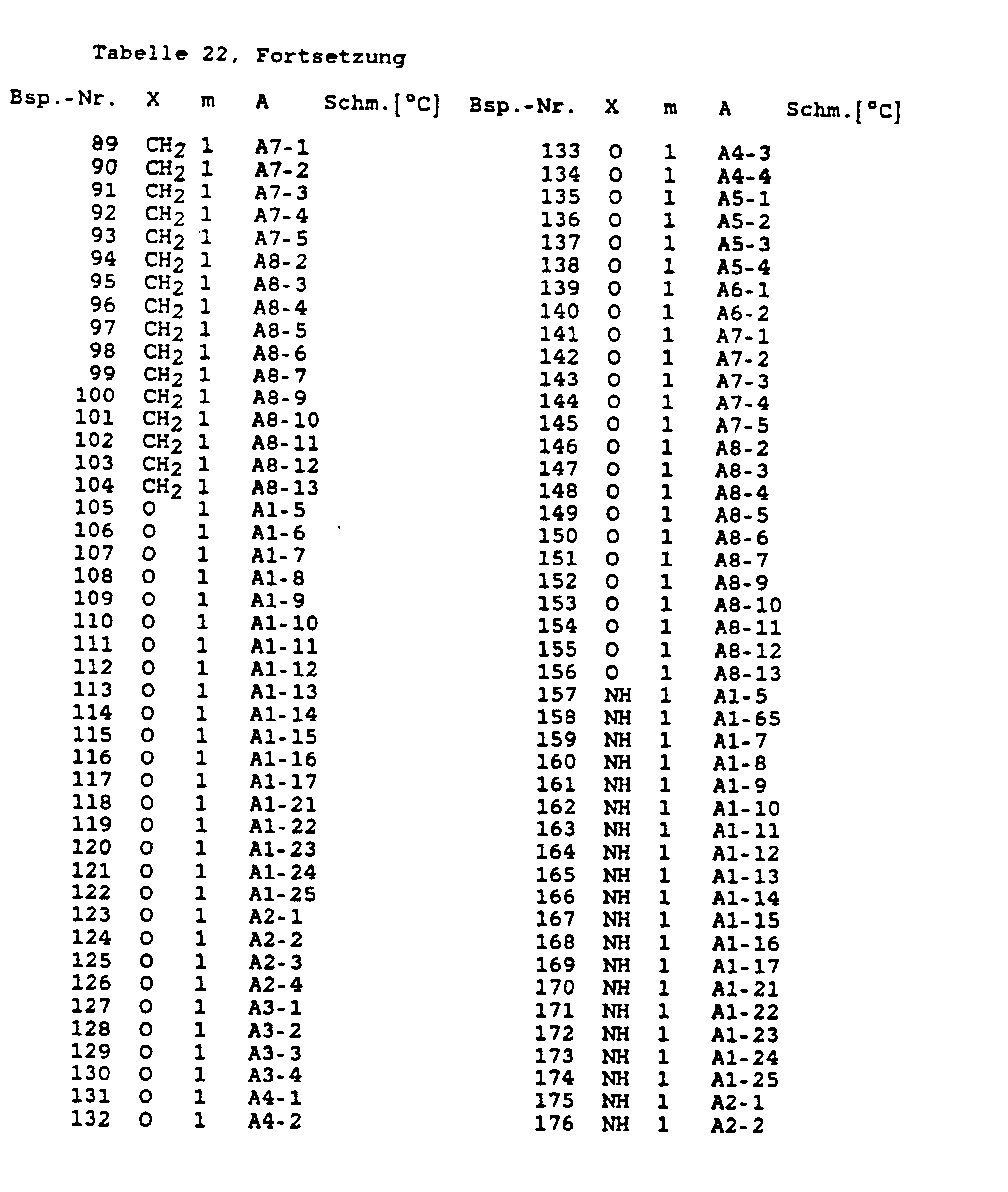

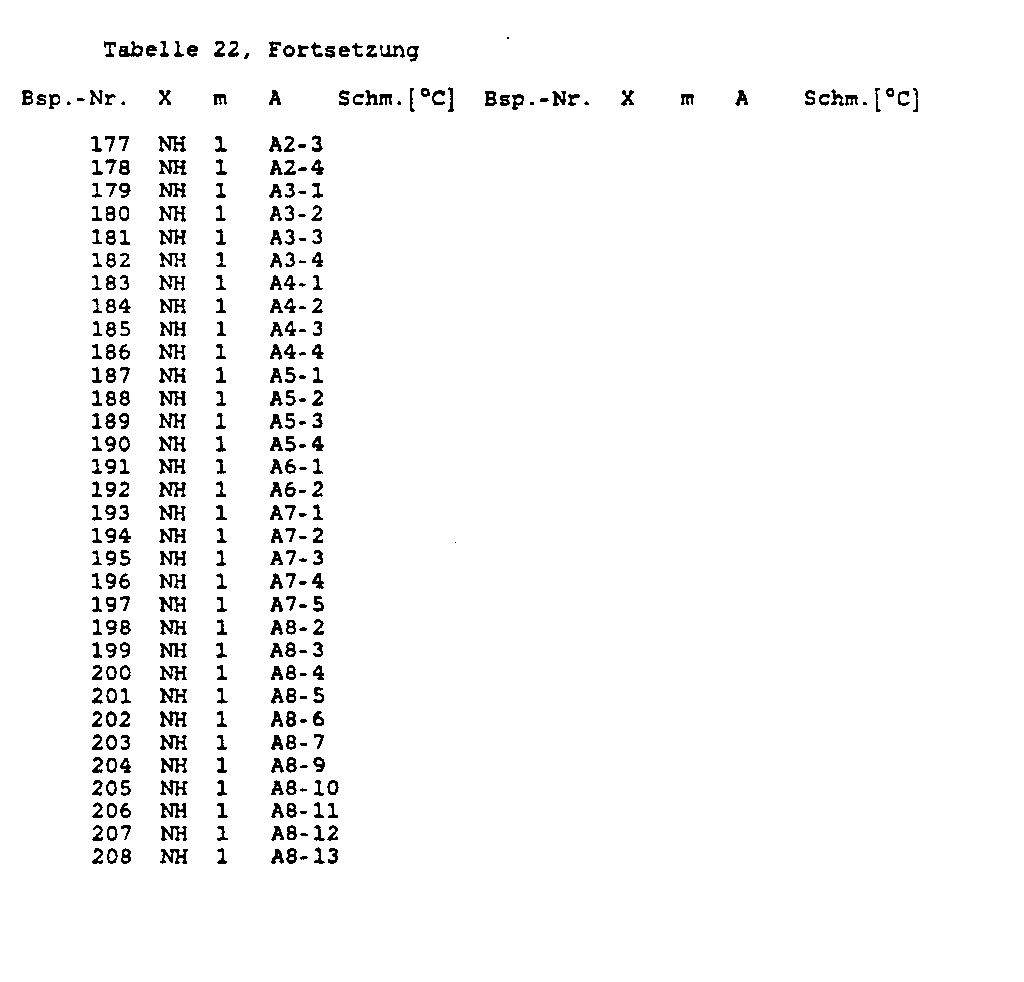

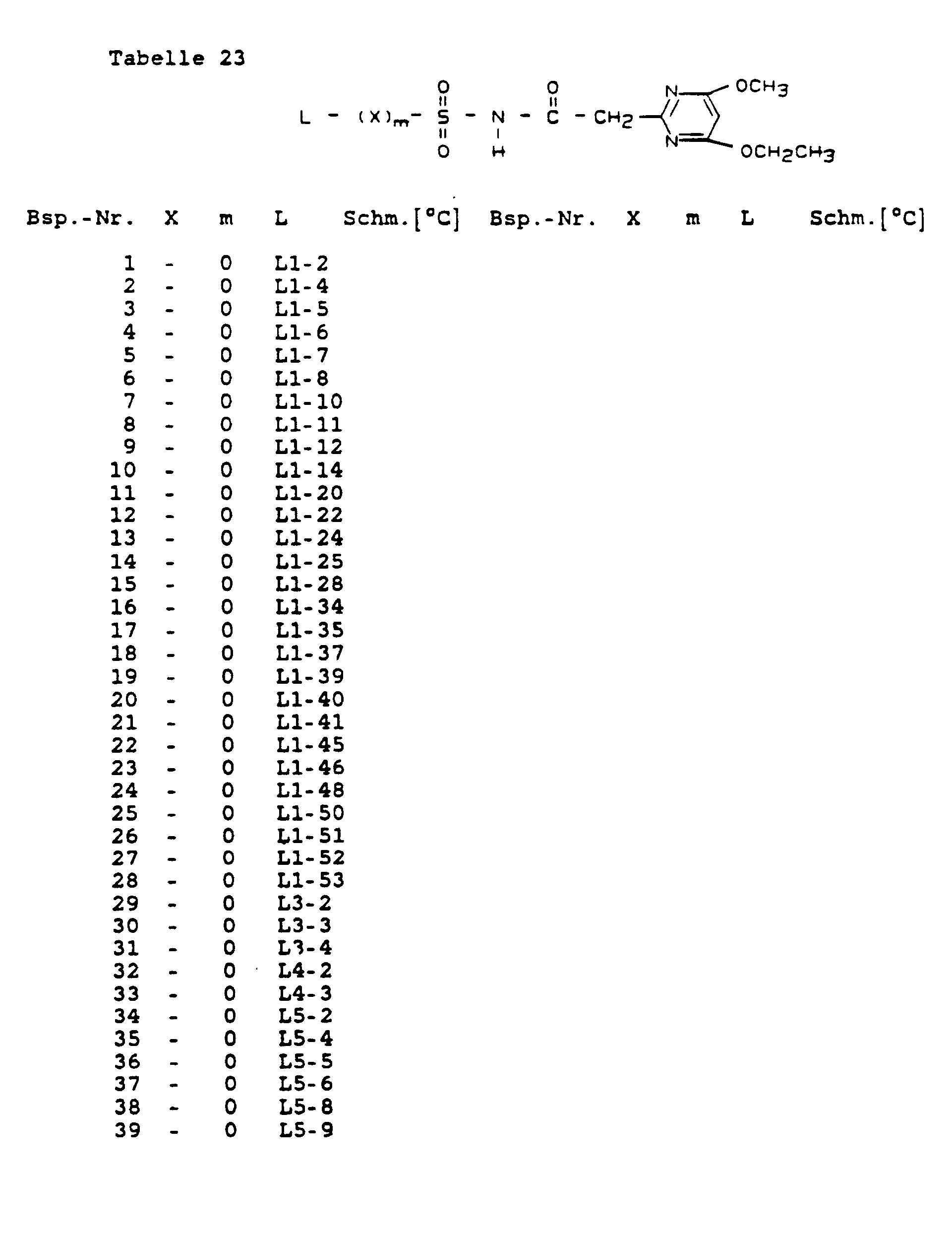

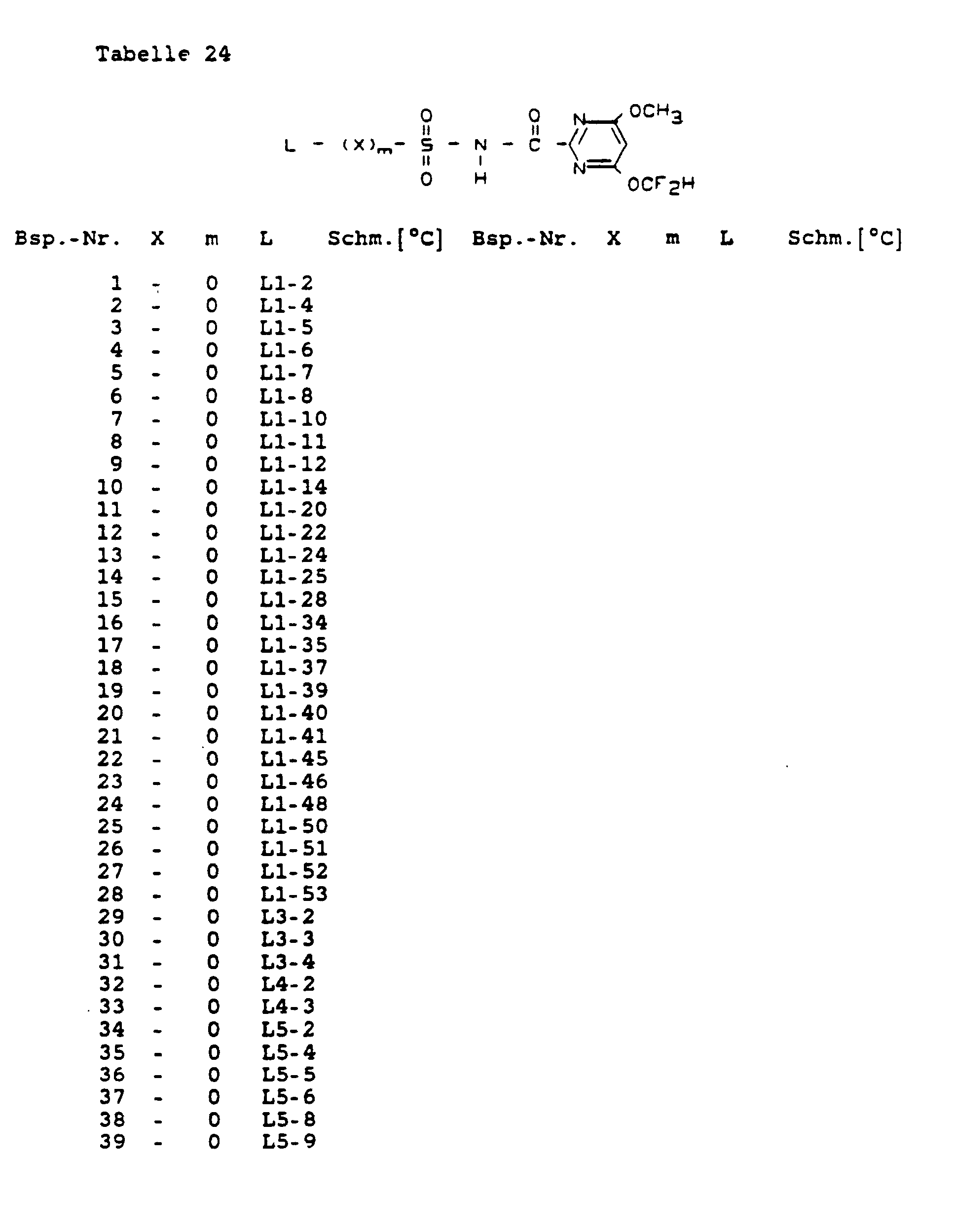

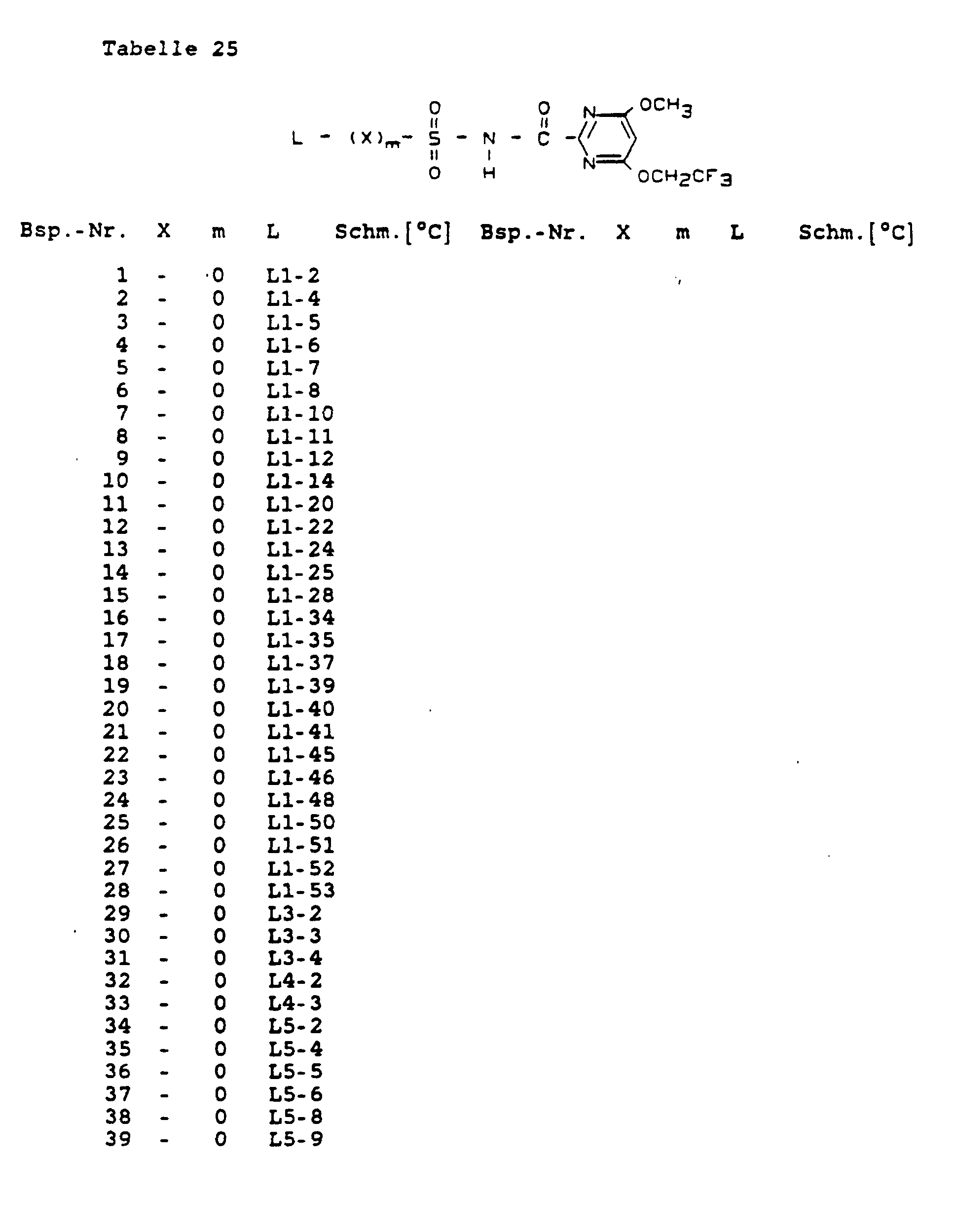

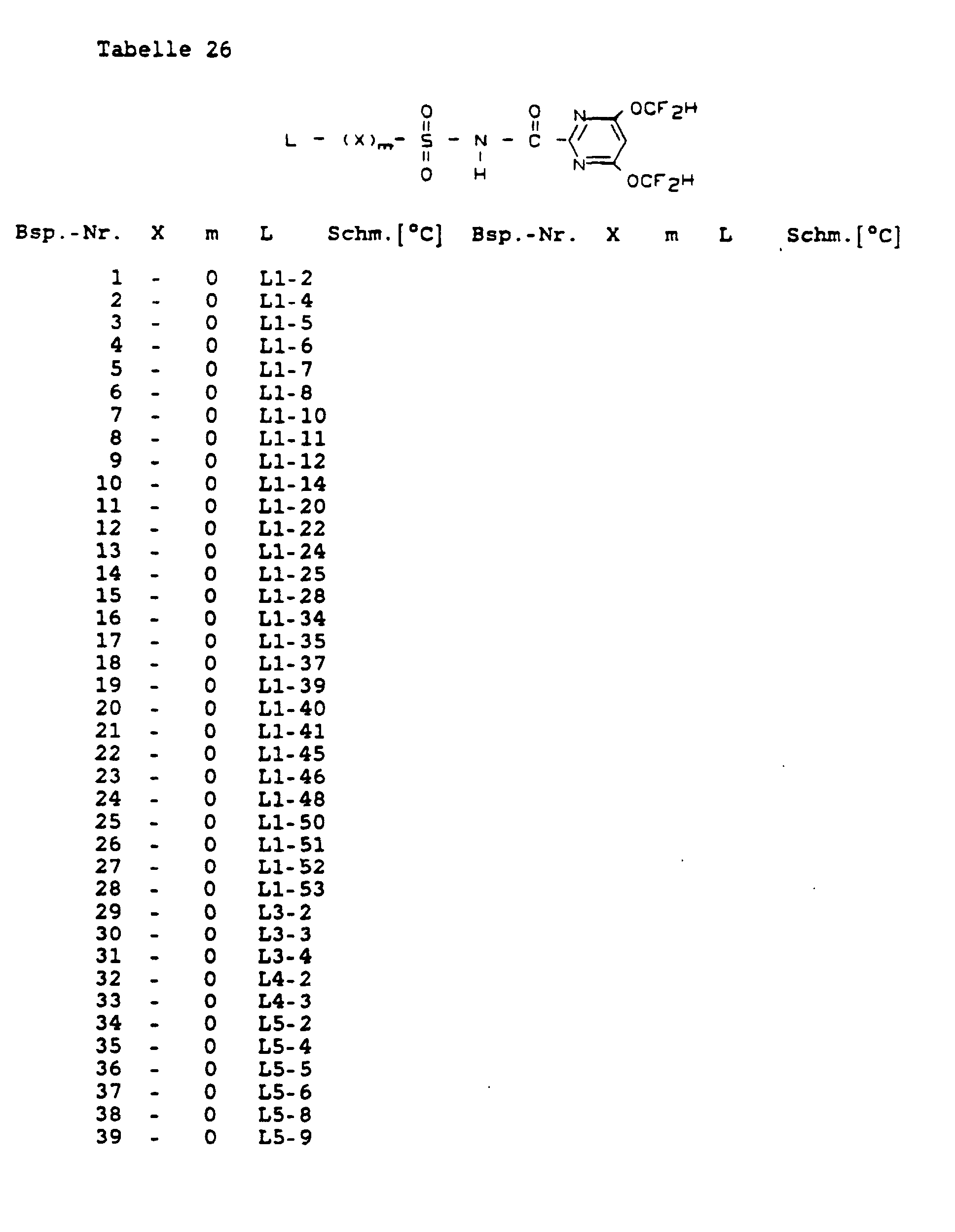

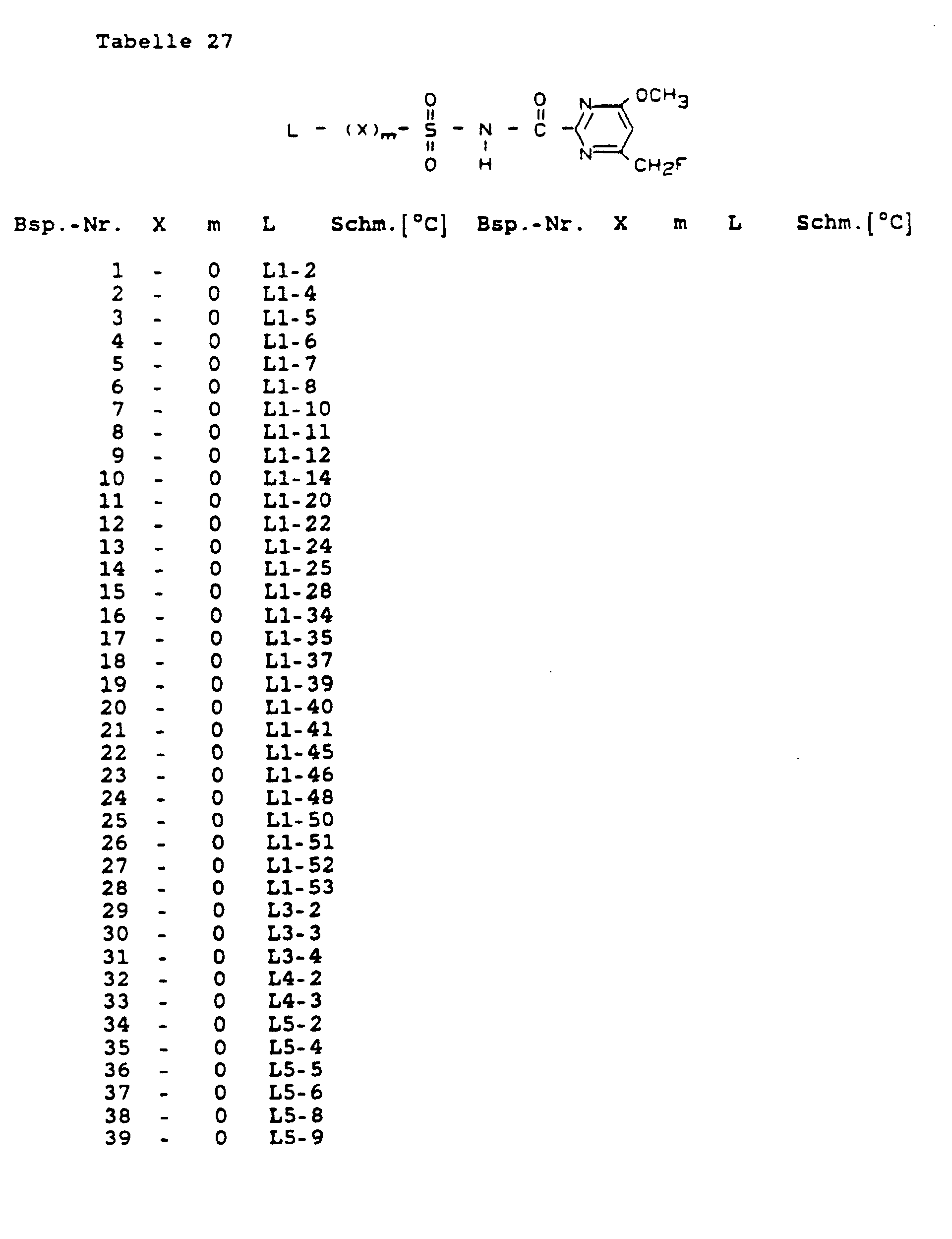

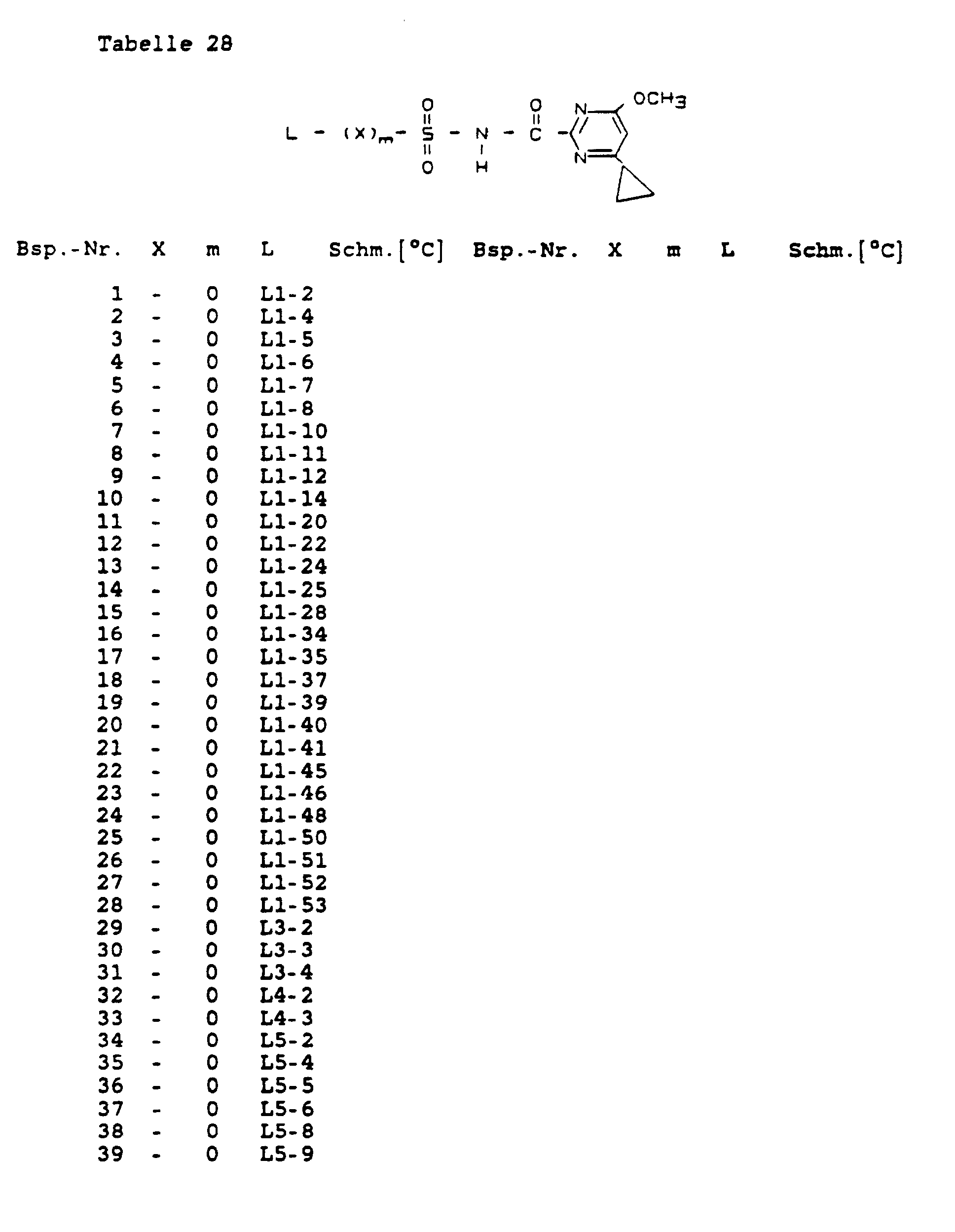

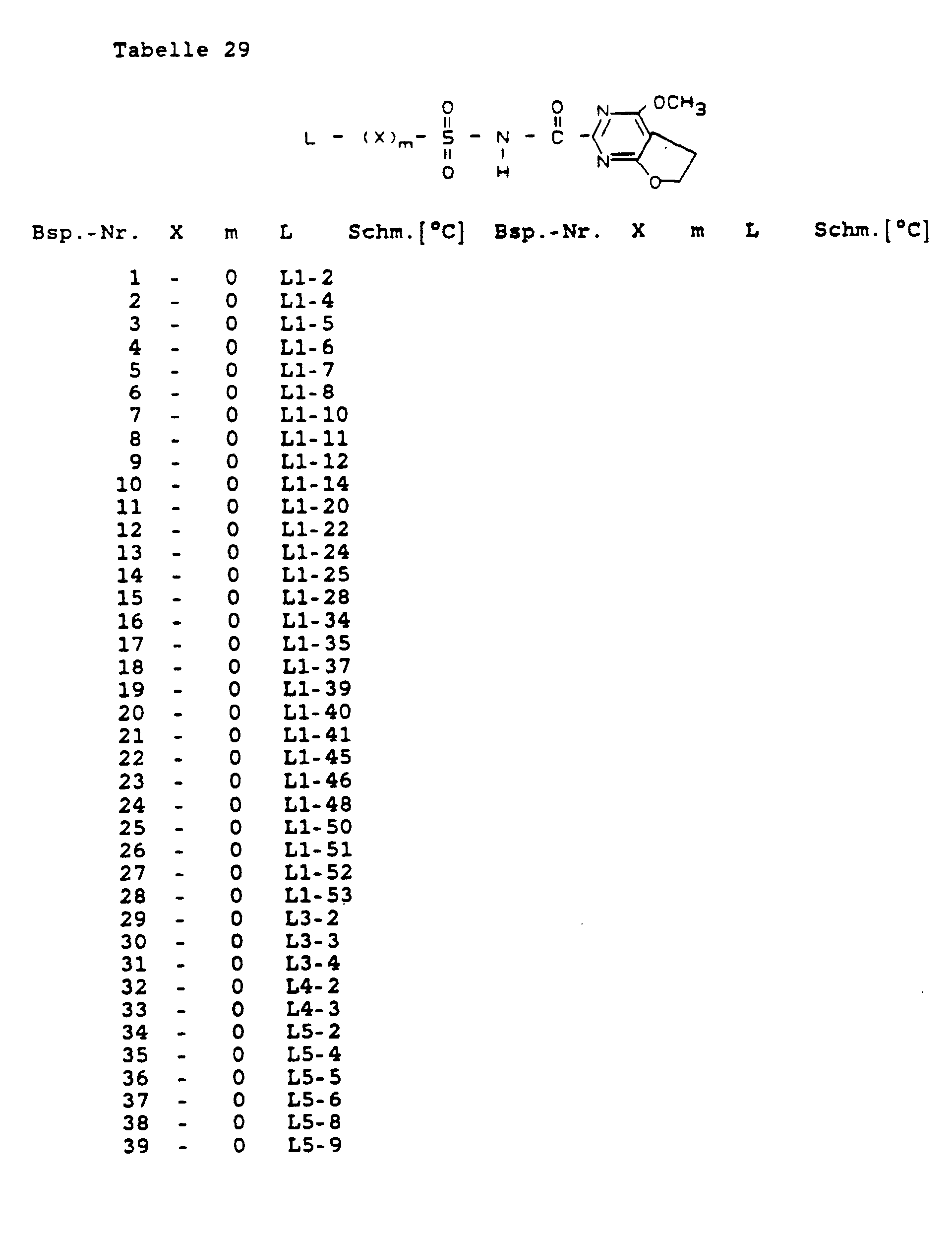

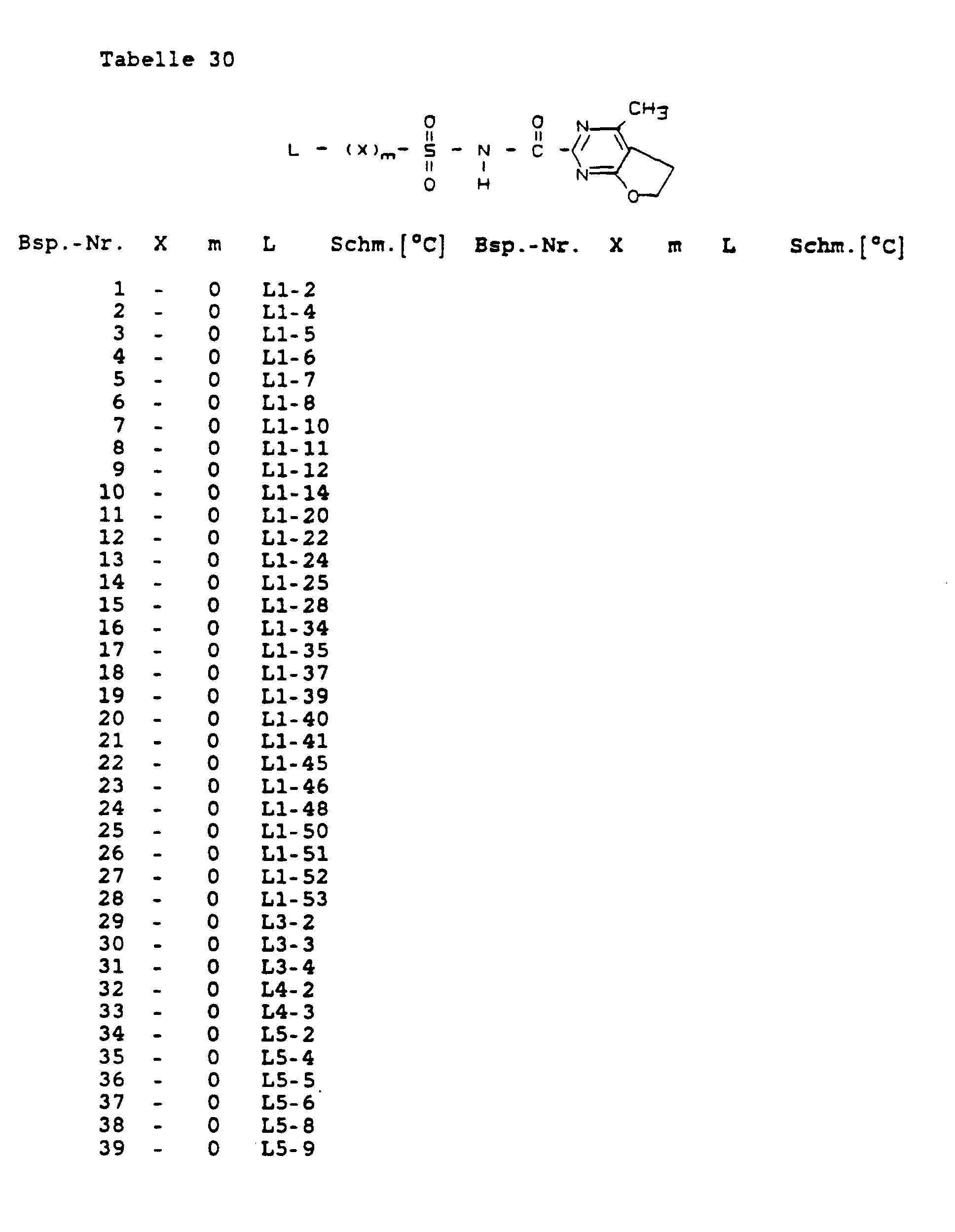

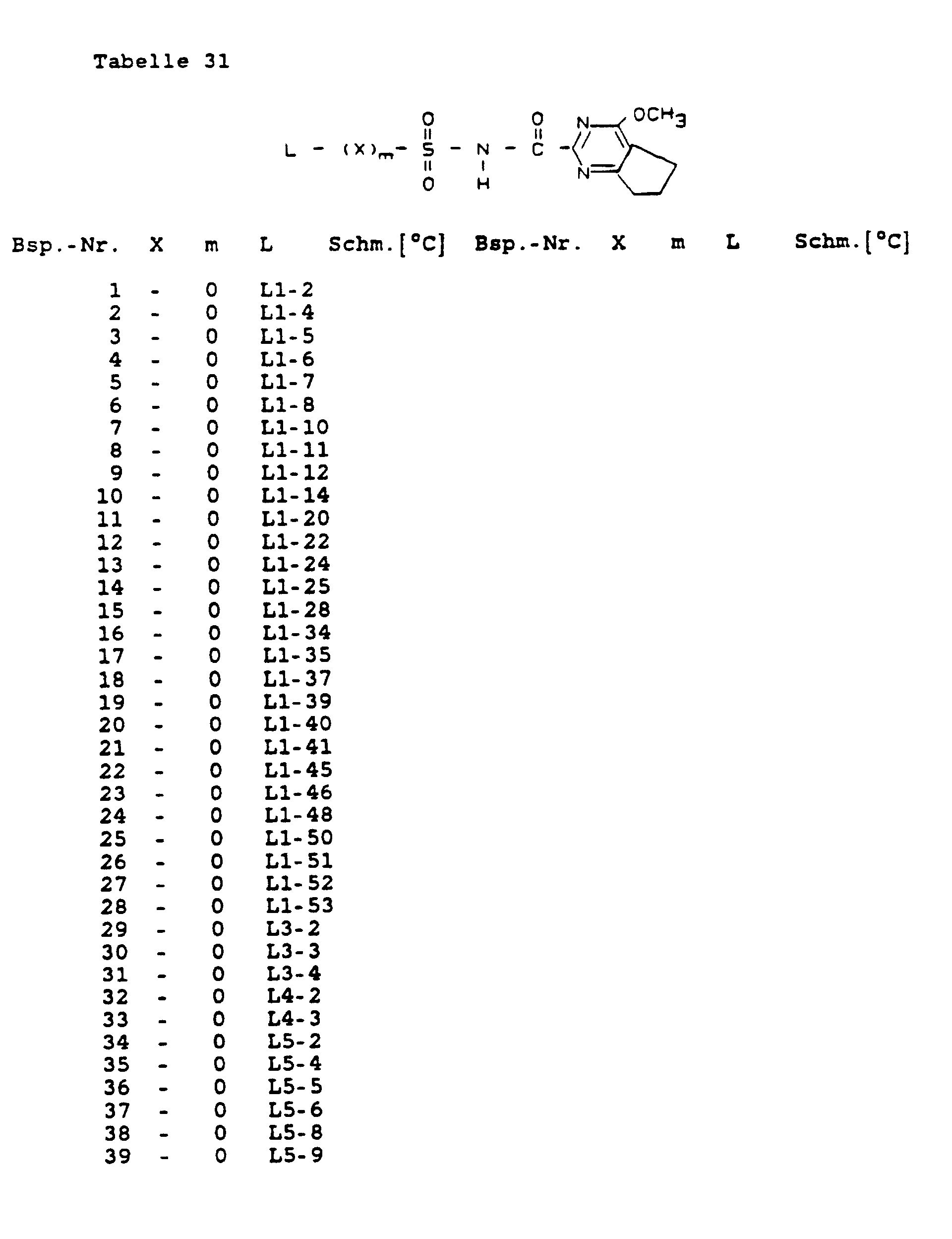

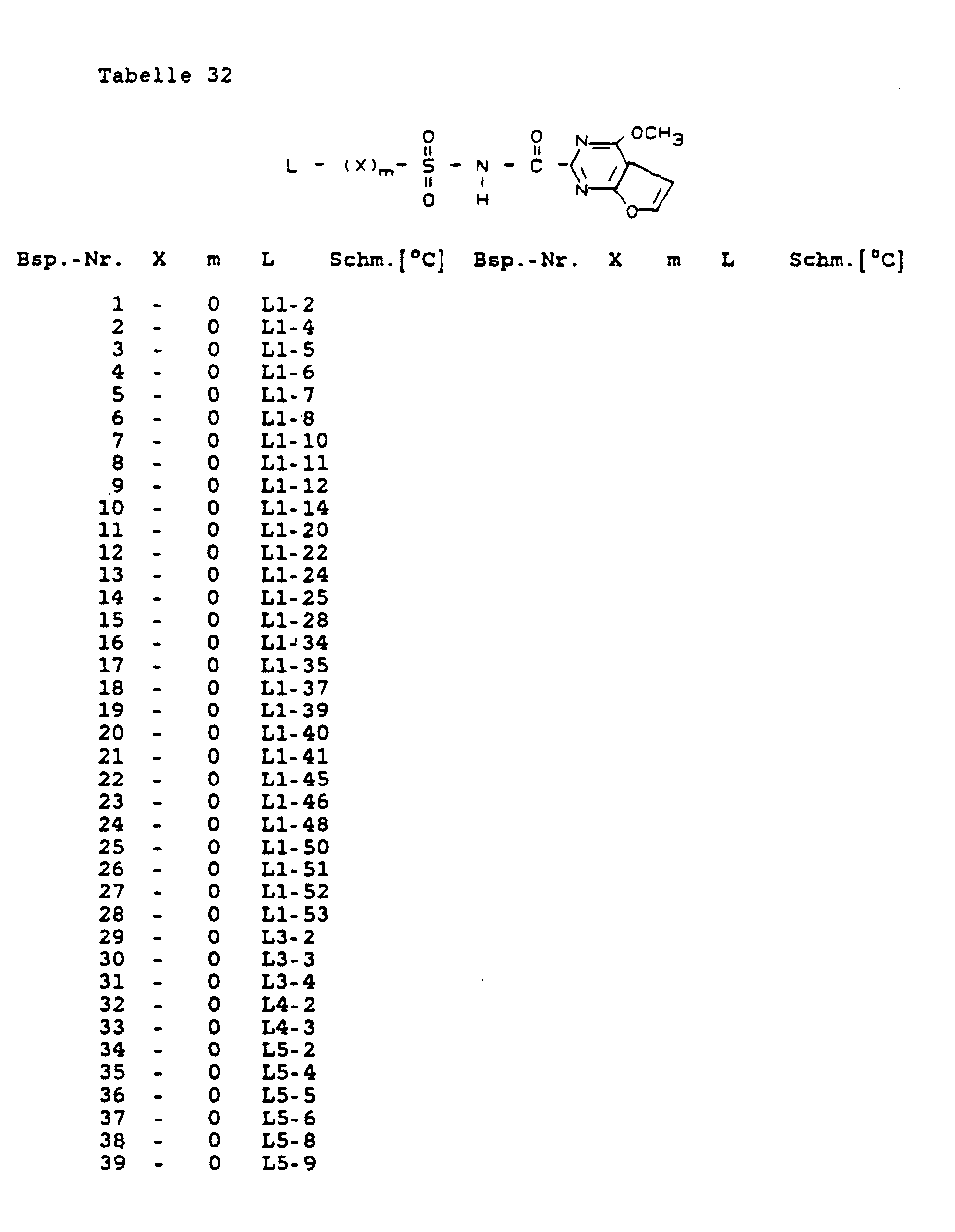

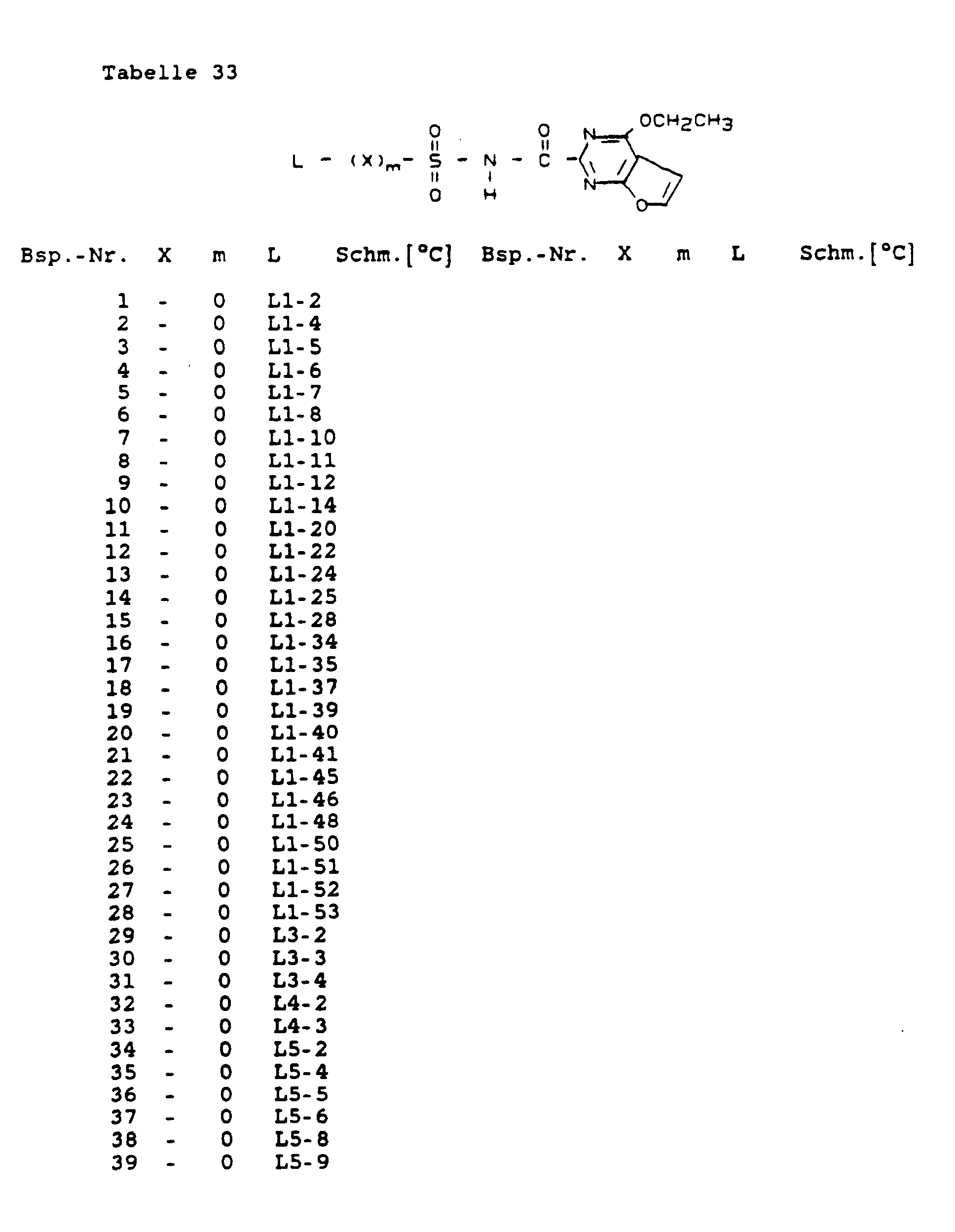

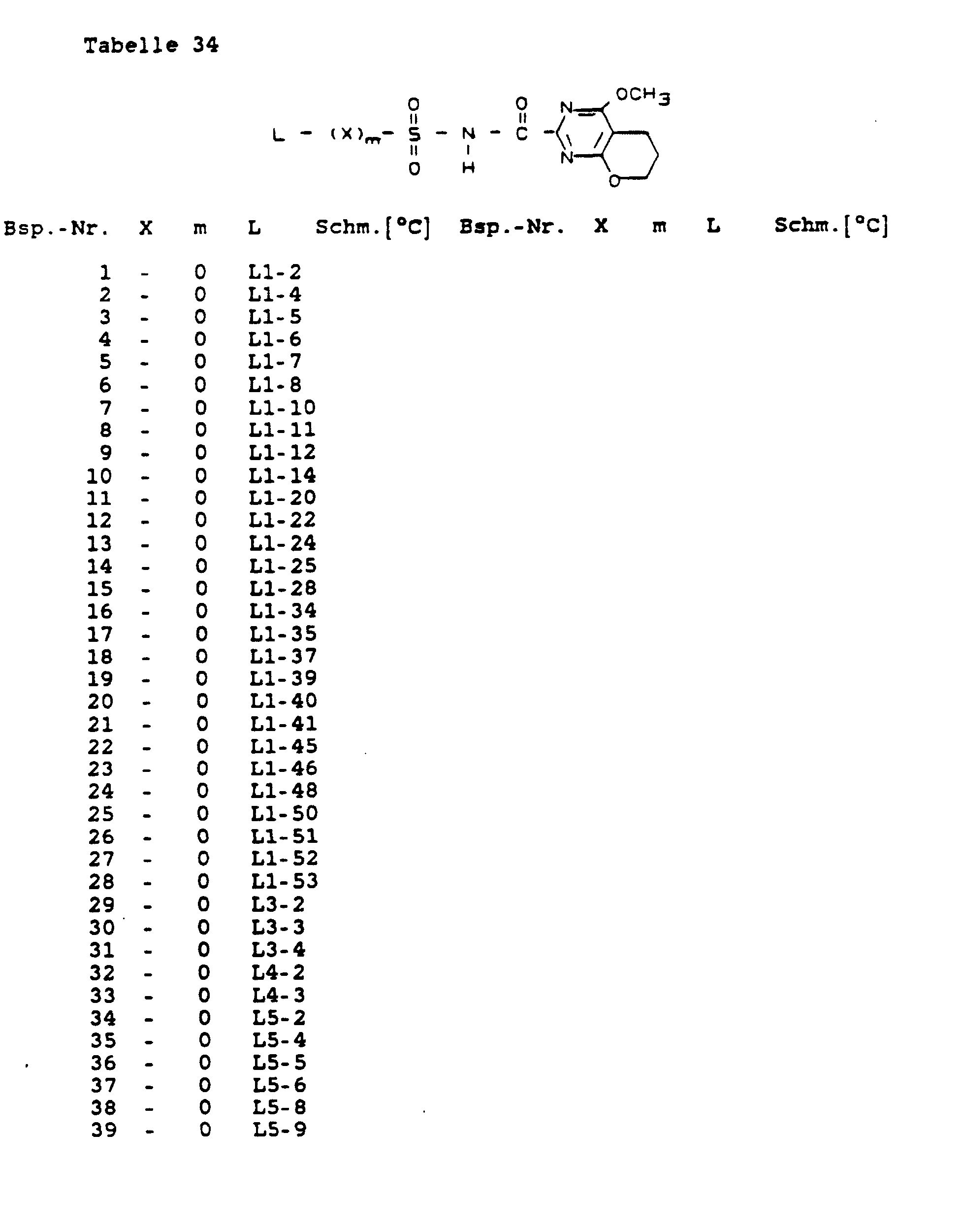

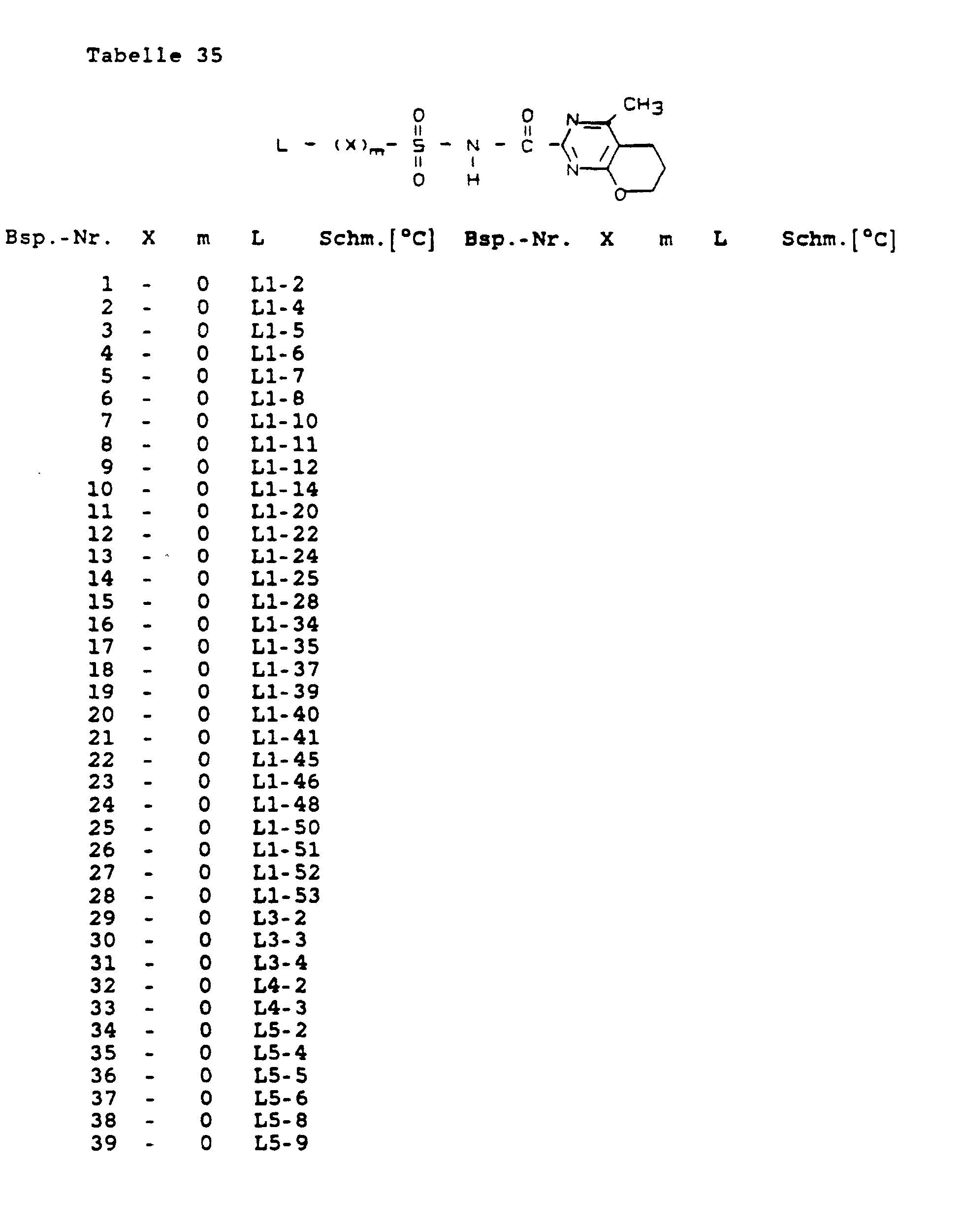

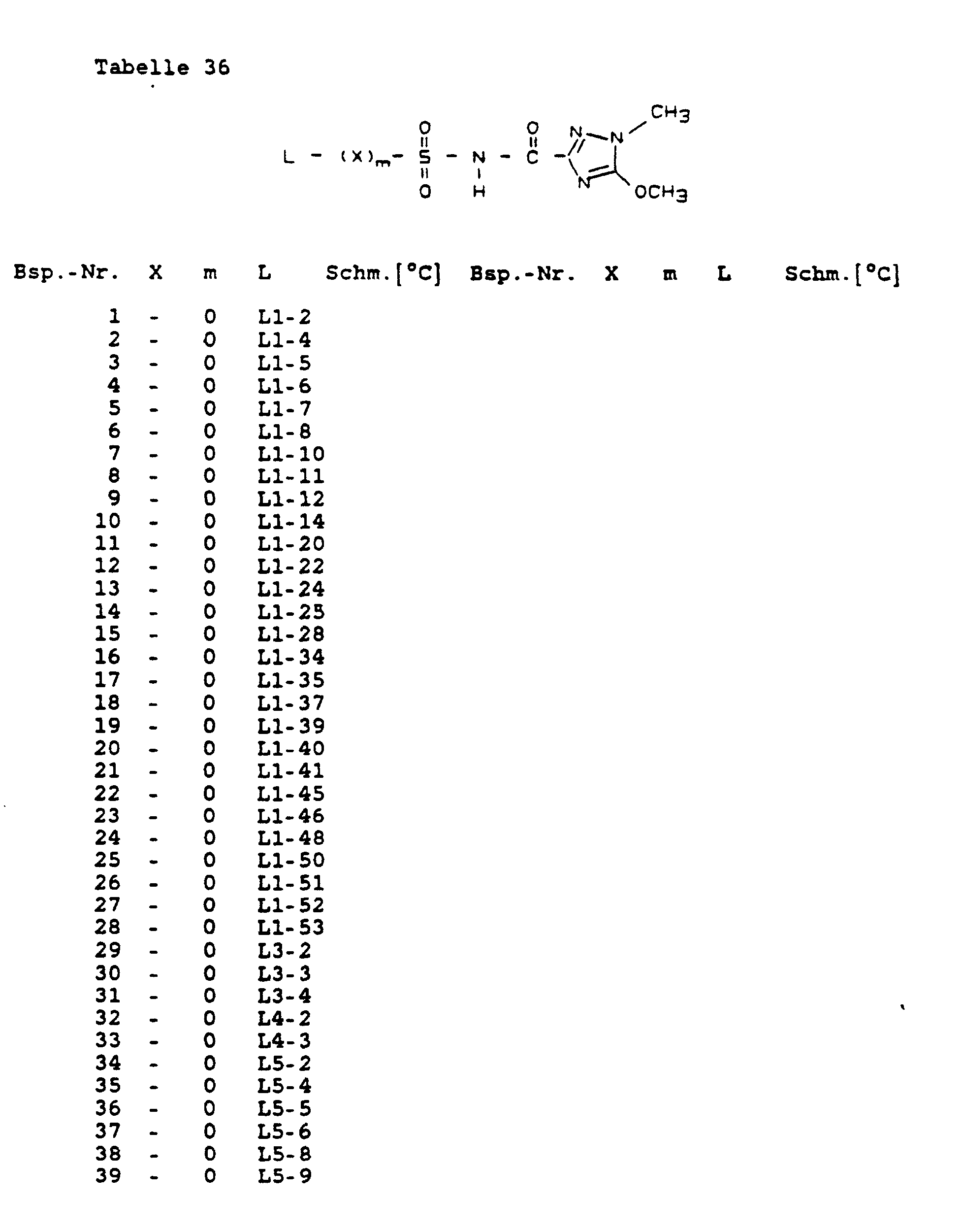

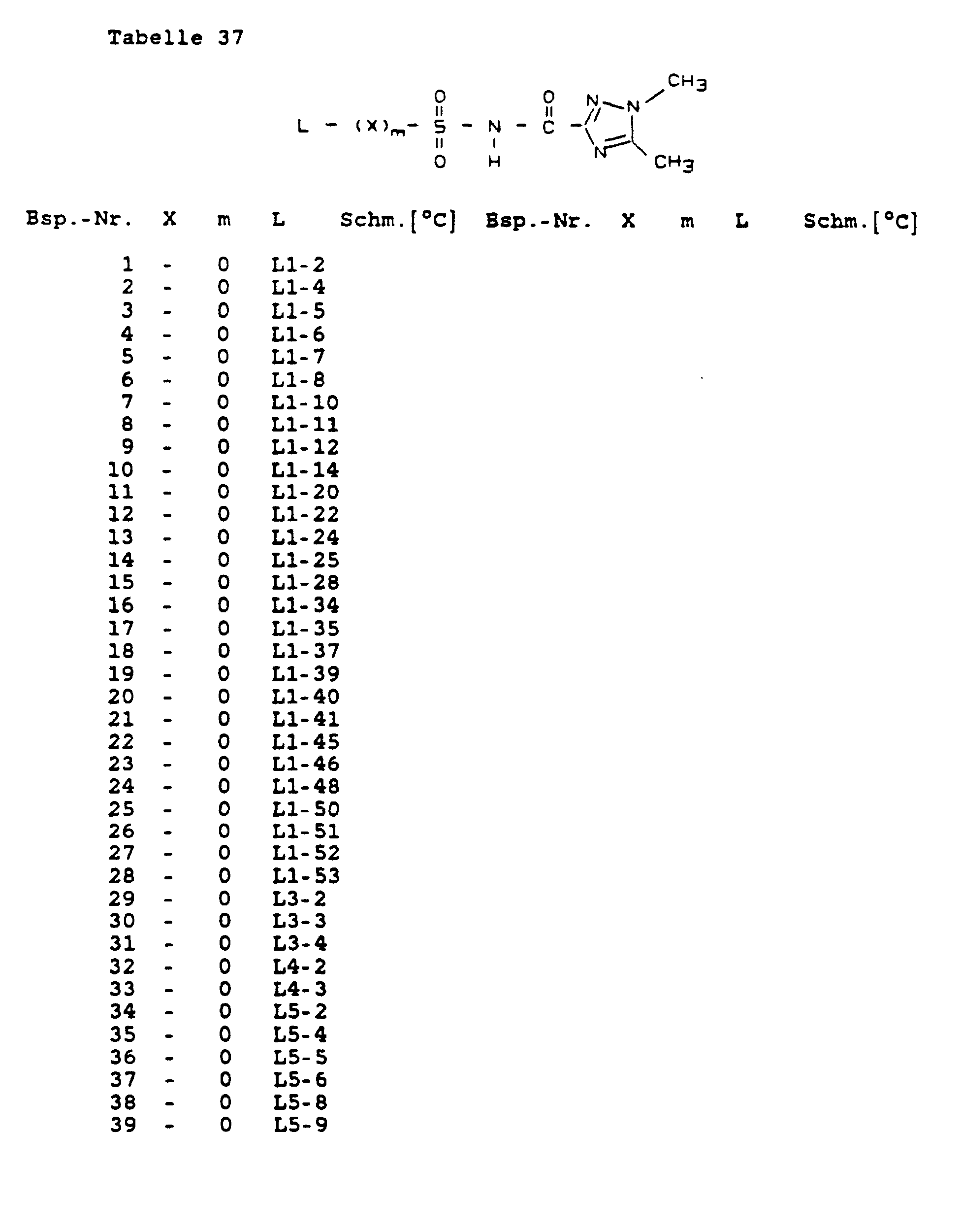

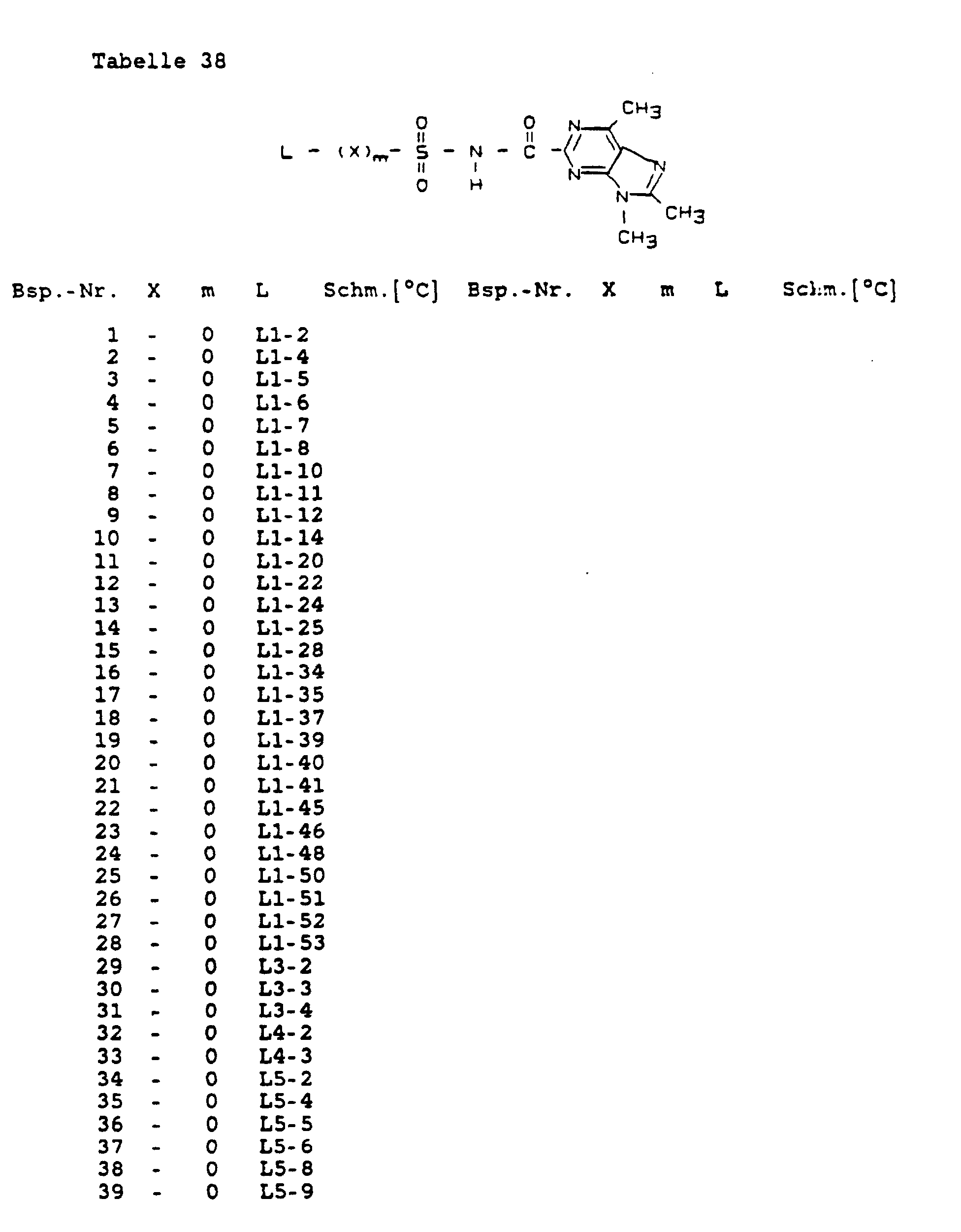

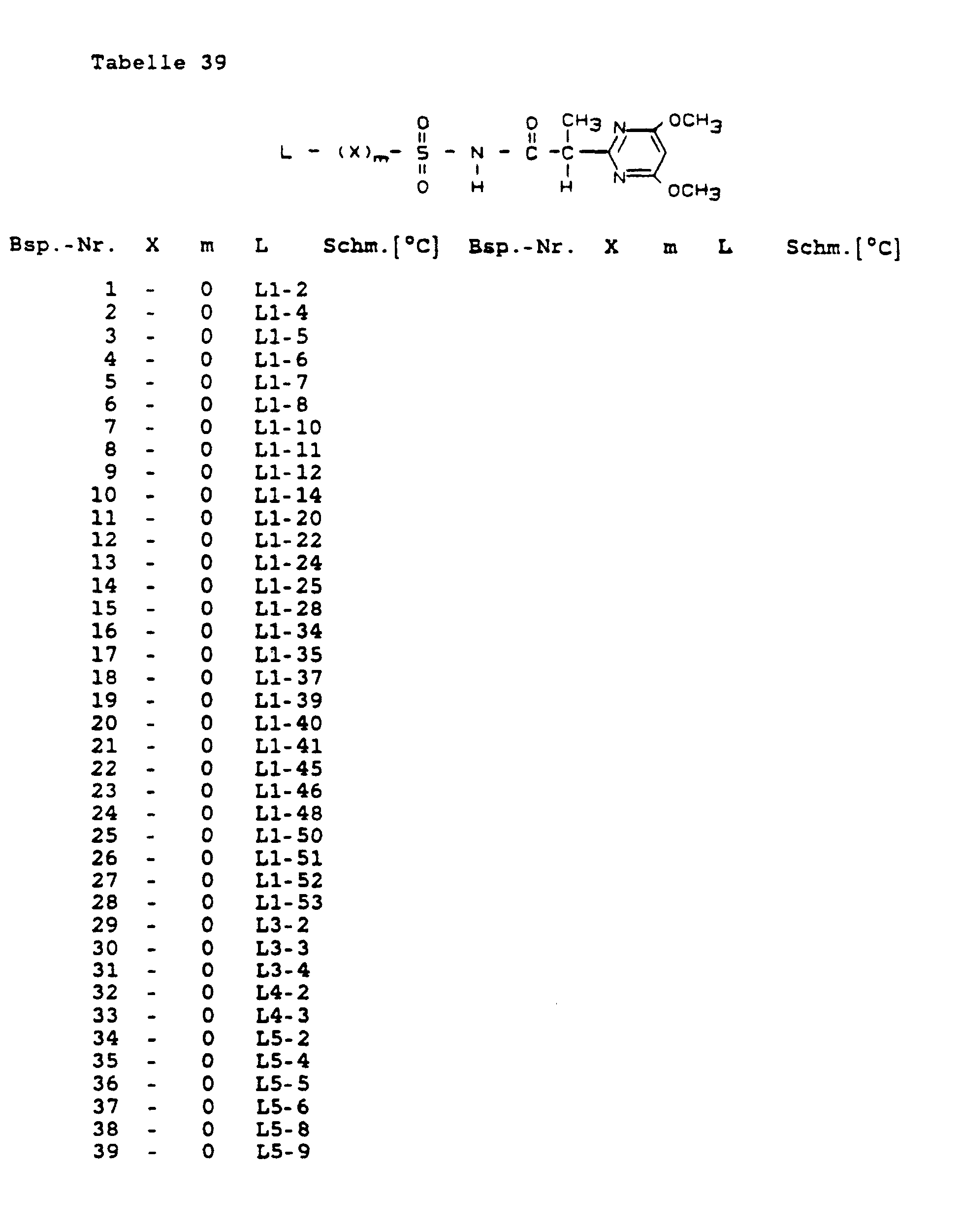

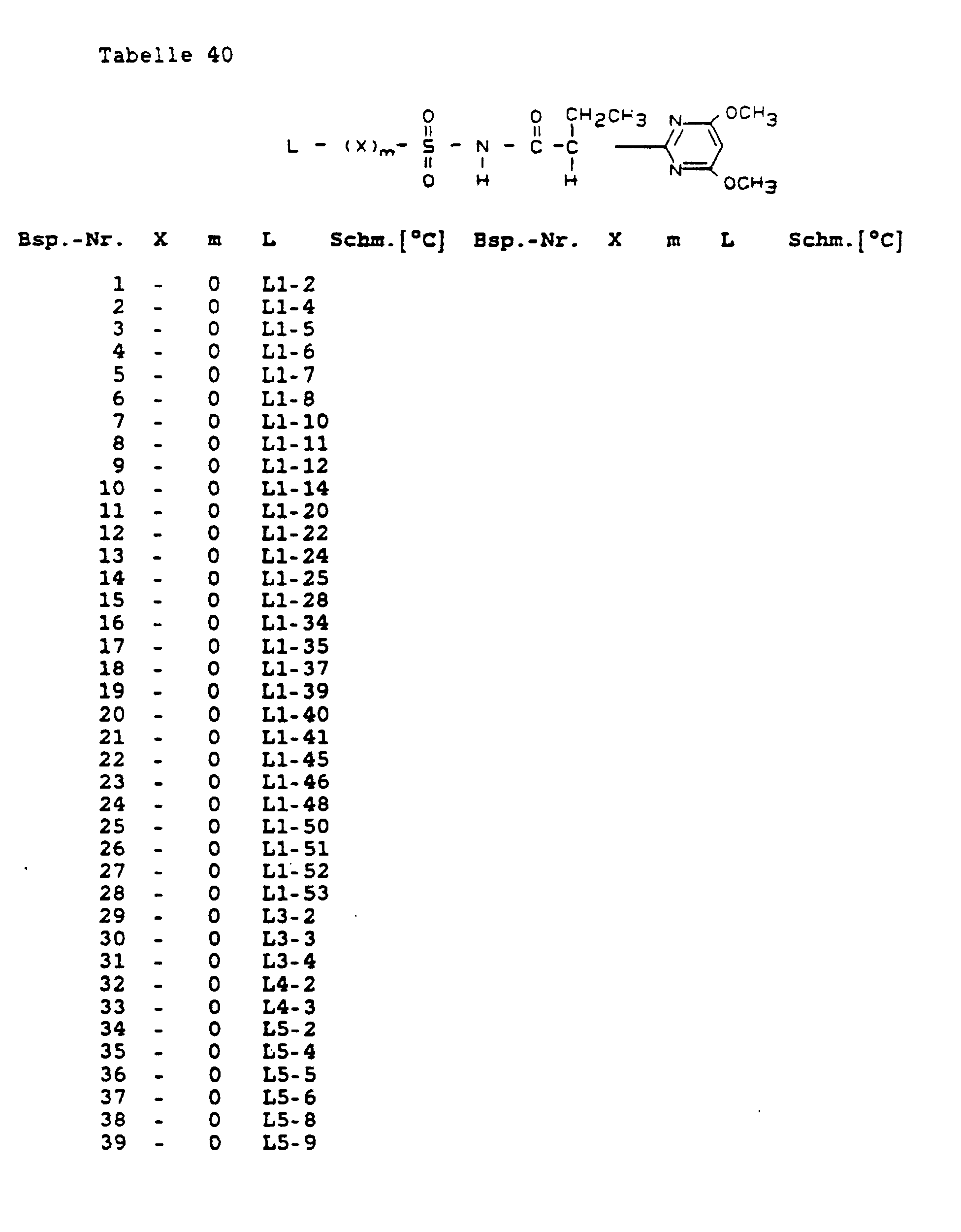

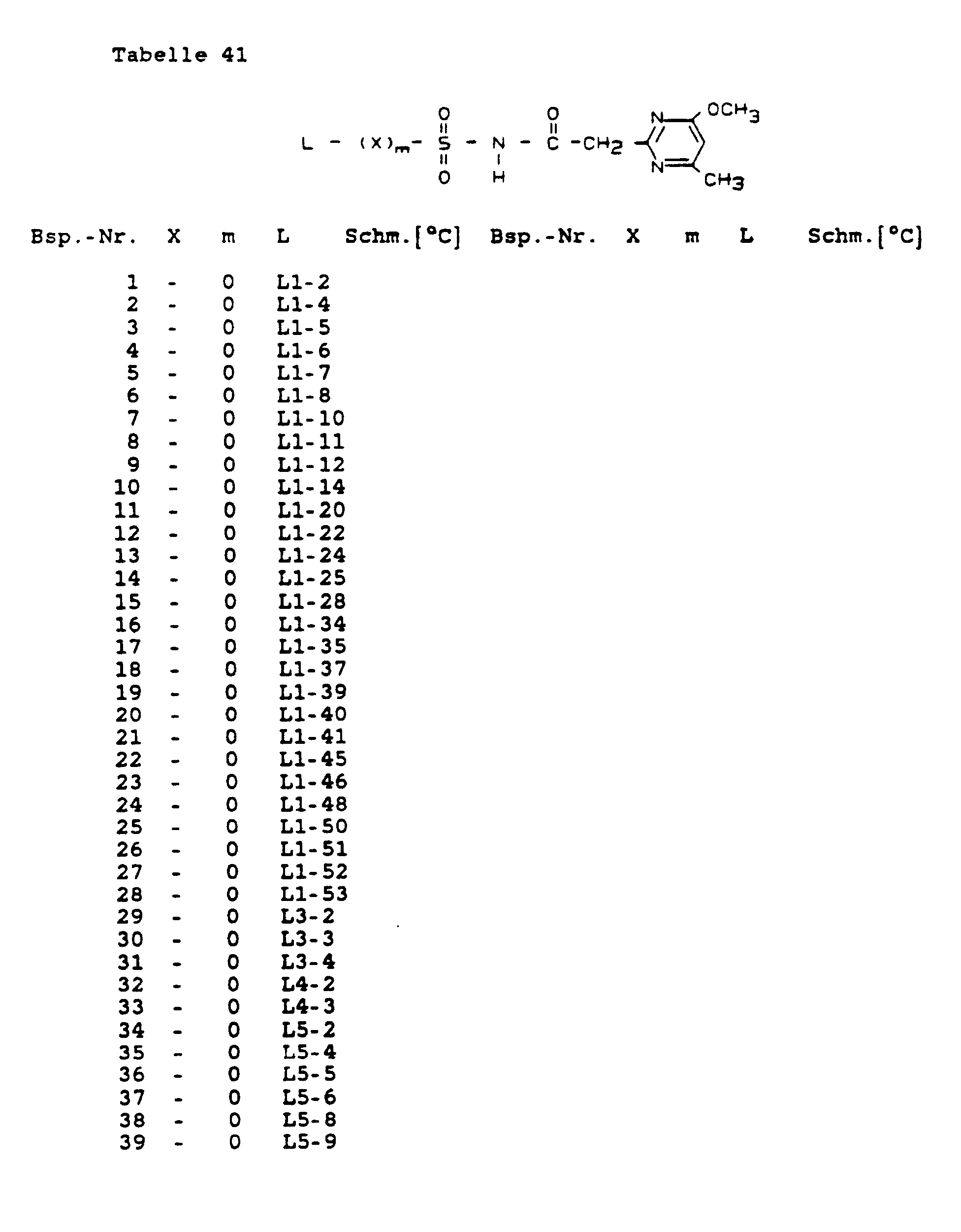

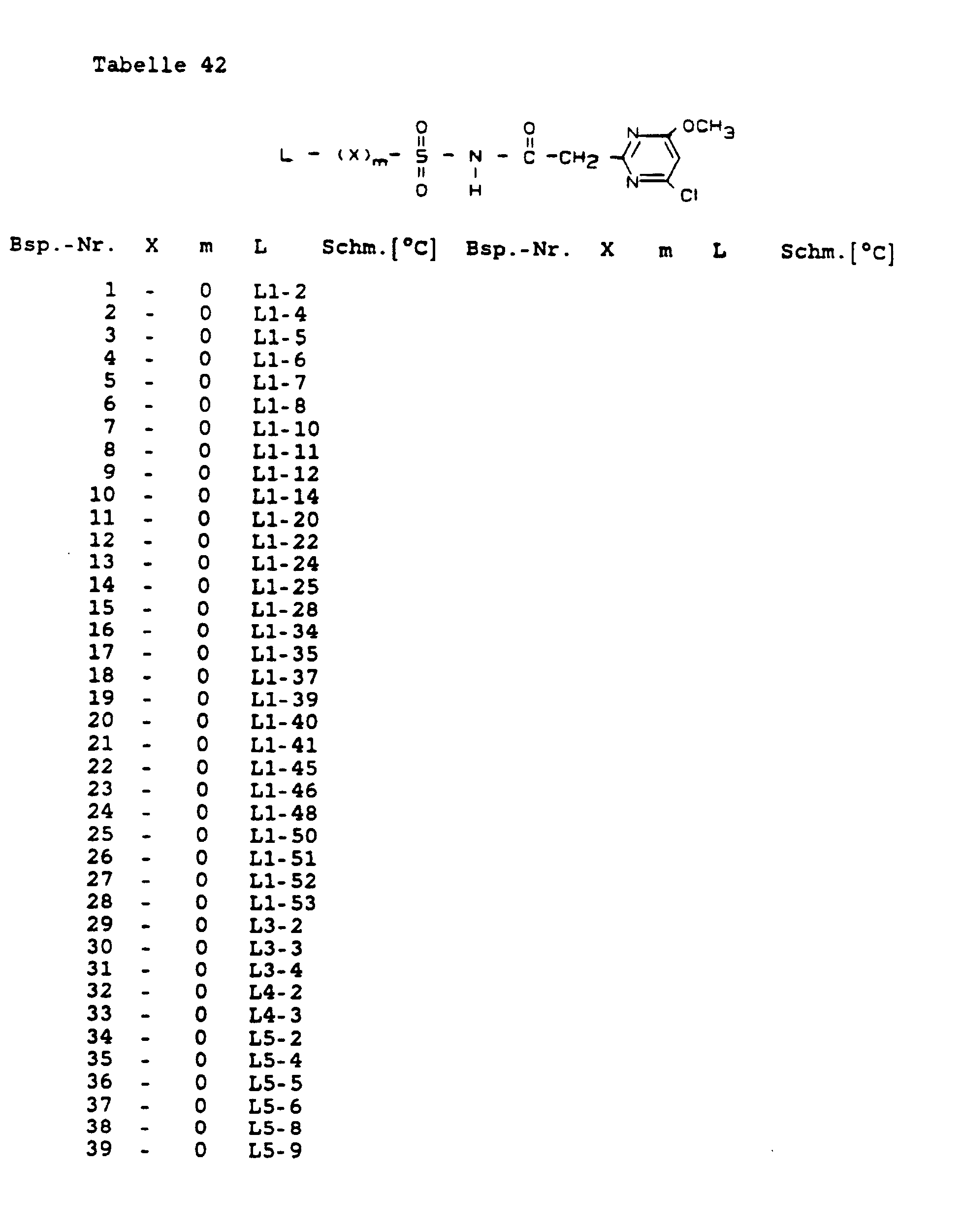

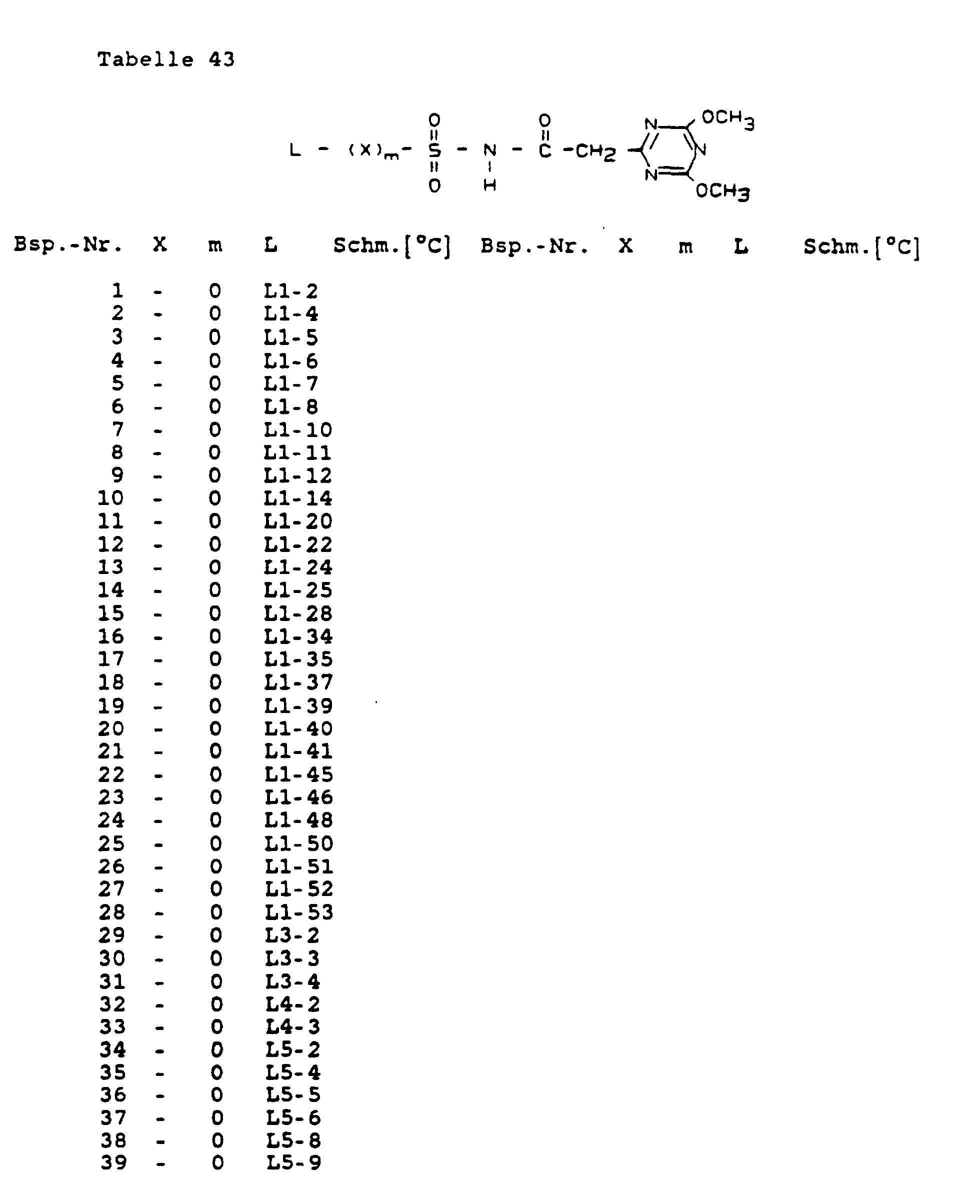

### Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:
0 = ohne Wirkung
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 - 4 Wochen im Vergleich zu unbehandelten Kontrollen. Wie die Boniturwerte in Tabelle 41 zeigen, weisen die erfindungsgemäßen Verbindungen eine gute herbizide Vorauflaufwirksamkeit gegen ein breites Spektrum von Ungräsern und Unkräutern auf.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 - 800 l/ha auf die grünen Pflanzenteile gesprüht und nach ca. 3 - 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 42).

| Tabelle | Bsp. Nr. | Dosierung (kg a.i./ha) | herbizide Wirkung | | | | | |
|---|---|---|---|---|---|---|---|---|
| | | | SIA | CRS | STM | LOM | ECG | AS |
| 1 | 26 | 0,3 | 5 | 3 | 2 | 2 | 1 | 1 |
| 1 | 38 | 0,3 | 5 | 0 | 1 | 1 | 1 | 0 |
| 1 | 1 | 0,3 | 5 | 5 | 5 | 5 | 5 | 2 |
| 1 | 2 | 0,3 | 5 | 3 | 5 | 2 | 2 | 1 |
| 1 | 55 | 0,3 | 5 | 5 | 5 | 5 | 5 | 5 |
| 1 | 19 | 0,3 | 5 | 2 | 4 | 1 | 2 | 0 |
| 1 | 124 | 0,3 | 5 | 5 | 4 | 2 | 3 | 2 |
| 1 | 109 | 0,3 | 5 | 2 | 3 | 2 | 2 | 2 |
| 1 | 28 | 0,3 | 5 | 3 | 3 | 1 | 2 | 1 |
| 1 | 238 | 0,3 | 5 | 4 | 4 | 2 | 2 | 2 |
| 8 | 26 | 0,3 | 5 | 5 | 2 | 1 | 1 | 1 |
| 8 | 221 | 0,3 | 5 | 5 | 2 | 0 | 1 | 1 |
| 8 | 55 | 0,3 | 5 | 5 | 5 | 1 | 4 | 1 |
| 8 | 38 | 0,3 | 2 | 2 | 2 | 2 | 2 | 1 |
| 8 | 28 | 0,3 | 5 | 5 | 5 | 2 | 4 | 3 |
| 1 | 35 | 0,3 | 5 | 3 | 2 | 2 | 3 | 2 |
| 1 | 194 | 0,3 | 3 | 0 | 0 | 0 | 0 | 2 |
| 1 | 82 | 0,3 | 5 | 5 | 4 | 3 | 5 | 3 |
| 1 | 15 | 0,3 | 5 | 2 | 2 | 1 | 1 | 1 |
| 1 | 44 | 0,3 | 5 | 4 | 4 | 2 | 2 | 1 |
| 1 | 16 | 0,3 | 5 | 3 | 5 | 3 | 3 | 3 |
| 1 | 170 | 0,3 | 5 | 5 | 5 | 3 | 2 | 2 |
| 1 | 178 | 0,3 | 5 | 5 | 5 | 3 | 2 | 2 |
| 1 | 45 | 0,3 | 2 | 0 | 0 | 0 | 0 | 1 |
| 1 | 80 | 0,3 | 5 | 5 | 5 | 3 | 2 | 1 |
| 1 | 89 | 0,3 | 5 | 4 | 2 | 0 | 0 | 1 |
| 1 | 79 | 0,3 | 3 | 0 | 1 | 0 | 0 | 1 |
| 1 | 32 | 0,3 | 5 | 0 | 0 | 0 | 1 | 1 |
| 1 | 257 | 0,3 | 5 | 2 | 5 | 2 | 2 | 1 |
| 1 | 62 | 0,3 | 5 | 5 | 5 | 4 | 5 | 3 |
| 1 | 206 | 0,3 | 3 | 0 | 0 | 0 | 0 | 1 |
| 1 | 25 | 0,3 | 5 | 3 | 3 | 1 | 1 | 1 |
| 1 | 237 | 0,3 | 3 | 1 | 0 | 0 | 1 | 1 |
| 1 | 37 | 0,3 | 5 | 5 | 4 | 0 | 1 | 1 |
| 1 | 218 | 0,3 | 3 | 0 | 0 | 0 | 0 | 0 |
| 1 | 306 | 0,3 | 5 | 4 | 4 | 1 | 0 | 1 |
| 1 | 88 | 0,3 | 1 | 1 | 2 | 1 | 0 | 0 |
| 2 | 26 | 0,3 | 5 | 2 | 5 | 0 | 0 | 1 |
| 2 | 38 | 0,3 | 5 | 1 | 5 | 1 | 1 | 0 |
| 3 | 62 | 0,3 | 5 | 5 | 5 | 5 | 5 | 5 |
| 1 | 233 | 0,3 | 1 | 2 | 0 | 0 | 2 | 1 |
| 2 | 1 | 0,3 | 5 | 5 | 5 | 5 | 5 | 5 |
| 2 | 55 | 0,3 | 5 | 5 | 5 | 5 | 5 | 4 |
| 1 | 47 | 0,3 | 5 | 1 | 0 | 1 | 1 | 3 |
| 3 | 8 | 0,3 | 3 | 0 | 0 | 0 | 0 | 2 |
| 2 | 62 | 0,3 | 5 | 5 | 5 | 5 | 5 | 5 |
| 2 | 8 | 0,3 | 5 | 3 | 5 | 3 | 2 | 2 |
| 1 | 116 | 0,3 | 5 | 5 | 5 | 2 | 4 | 3 |
| 1 | 78 | 0,3 | 5 | 5 | 5 | 4 | 4 | 4 |
| 1 | 70 | 0,3 | 5 | 5 | 5 | 4 | 5 | 3 |
| 1 | 297 | 0,3 | 5 | 5 | 5 | 4 | 4 | 4 |
| 1 | 69 | 0,3 | 5 | 5 | 5 | 4 | 5 | 4 |

**Tabelle 42:**

| Nachauflaufwirkung der erfindungsgemäßen Verbindungen | | | | | | | | |
|---|---|---|---|---|---|---|---|---|
| Tabelle | Bsp. Nr. | Dosierung (kg a.i./ha) | herbizide Wirkung | | | | | |
| | | | SIA | CRS | STM | LOM | ECG | AS |
| 1 | 26 | 0,3 | 5 | 4 | 3 | 2 | 2 | 1 |
| 1 | 38 | 0,3 | 4 | 2 | 2 | 2 | 2 | 2 |
| 1 | 1 | 0,3 | 5 | 3 | 5 | 2 | 2 | 2 |
| 1 | 2 | 0,3 | 5 | 2 | 5 | 2 | 2 | 2 |
| 1 | 55 | 0,3 | 5 | 5 | 5 | 4 | 5 | 4 |
| 1 | 19 | 0,3 | 5 | 2 | 4 | 3 | 2 | 2 |
| 1 | 124 | 0,3 | 5 | 4 | 2 | 1 | 2 | 2 |
| 1 | 109 | 0.3 | 4 | 2 | 3 | 2 | 2 | 2 |
| 1 | 28 | 0.3 | 5 | 2 | 5 | 2 | 3 | 2 |
| 1 | 238 | 0,3 | 5 | 2 | 2 | 2 | 1 | 1 |
| 8 | 26 | 0,3 | 5 | 5 | 4 | 2 | 2 | 1 |
| 8 | 221 | 0,3 | 4 | 2 | 3 | 2 | 2 | 1 |
| 8 | 55 | 0,3 | 5 | 5 | 3 | 2 | 3 | 1 |
| | | | | | | | | |
| 8 | 38 | 0,3 | 3 | 2 | 1 | 1 | 2 | 1 |
| 8 | 28 | 0,3 | 5 | 5 | 5 | 3 | 2 | 3 |
| 1 | 35 | 0,3 | 5 | 3 | 5 | 3 | 3 | 3 |
| 1 | 194 | 0,3 | 3 | 3 | 2 | 3 | 3 | 2 |
| 1 | 82 | 0,3 | 5 | 4 | 5 | 4 | 4 | 3 |
| 1 | 15 | 0,3 | 5 | 2 | 2 | 1 | 1 | 2 |
| 1 | 44 | 0,3 | 5 | 3 | 5 | 3 | 2 | 2 |
| 1 | 16 | 0,3 | 5 | 3 | 5 | 2 | 3 | 3 |
| 1 | 170 | 0,3 | 5 | 5 | 5 | 3 | 4 | 1 |
| 1 | 178 | 0,3 | 5 | 5 | 5 | 4 | 2 | 2 |
| 1 | 45 | 0,3 | 3 | 1 | 1 | 0 | 2 | 0 |
| 1 | 80 | 0,3 | 5 | 5 | 5 | 1 | 4 | 2 |
| 1 | 89 | 0,3 | 5 | 5 | 5 | 2 | 2 | 1 |
| 1 | 79 | 0,3 | 5 | 5 | 5 | 3 | 2 | 0 |
| 1 | 32 | 0,3 | 5 | 3 | 4 | 2 | 3 | 2 |
| 1 | 257 | 0,3 | 5 | 5 | 5 | 2 | 5 | 2 |
| 1 | 62 | 0,3 | 5 | 5 | 5 | 5 | 5 | 4 |
| 1 | 206 | 0,3 | 3 | 1 | 2 | 2 | 1 | 2 |
| 1 | 25 | 0,3 | 5 | 1 | 3 | 1 | 1 | 1 |
| 1 | 237 | 0,3 | 3 | 2 | 0 | 0 | 1 | 0 |
| 1 | 37 | 0,3 | 5 | 4 | 5 | 2 | 3 | 1 |
| 1 | 218 | 0,3 | 5 | 0 | 0 | 0 | 0 | 0 |
| 1 | 306 | 0,3 | 5 | 5 | 4 | 1 | 2 | 0 |
| 1 | 88 | 0,3 | 5 | 2 | 4 | 0 | 0 | 0 |
| 2 | 26 | 0,3 | 4 | 0 | 3 | 0 | 0 | 1 |
| 2 | 38 | 0,3 | 5 | 3 | 5 | 1 | 0 | 0 |
| 3 | 62 | 0,3 | 5 | 5 | 5 | 4 | 4 | 2 |
| 1 | 233 | 0,3 | 2 | 0 | 0 | 0 | 0 | 0 |
| 2 | 1 | 0,3 | 5 | 4 | 5 | 5 | 5 | 3 |
| 2 | 55 | 0,3 | 5 | 4 | 5 | 4 | 4 | 3 |
| 1 | 47 | 0,3 | 2 | 0 | 0 | 0 | 0 | 0 |
| | | | | | | | | |
| 2 | 62 | 0,3 | 5 | 5 | 5 | 3 | 4 | 2 |
| 2 | 8 | 0,3 | 5 | 1 | 5 | 0 | 2 | 1 |
| 1 | 116 | 0,3 | 5 | 5 | 4 | 1 | 3 | 1 |
| 1 | 78 | 0,3 | 5 | 5 | 5 | 3 | 4 | 2 |
| 1 | 70 | 0,3 | 5 | 5 | 5 | 2 | 4 | 2 |
| 1 | 297 | 0,3 | 5 | 5 | 5 | 3 | 2 | 2 |
| 1 | 69 | 0,3 | 5 | 5 | 5 | 2 | 4 | 2 |
| Abkürzungen: SIA = Sinapis alba CRS = Chrysanthemum segetum STM = Stellaria media LOM = Lolium multiflorum ECG = Echinochloa crus-galli AS = Avena sativa a.i. = Aktivsubstanz | | | | | | | | |

## Patentansprüche (Patentansprüche für folgende(n) Vertragsstaat(en): CH, DE, FR, GB, IT, LI)

1. Verbindungen der Formel I oder deren Salze 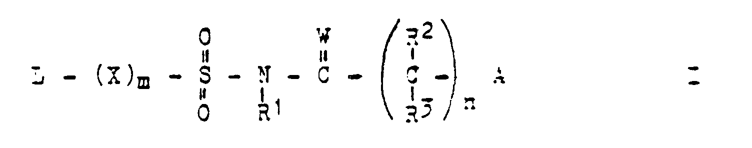 worin
R¹ Wasserstoff, (C₁-C₄)Alkyl, (C₂-C₆)-Alkenyl oder (C₂-C₆)-Alkinyl;
R²,R³ unabhängig voneinander Wasserstoff, (C₁-C₃)-Alkyl oder Phenyl;
W O, S, NR⁴ oder NOR⁴,
R⁴ Wasserstoff, (C₁-C₃)-Alkyl, (C₁-C₃)-Haloalkyl oder Phenyl;
X CHR², O, NR⁴ oder NOR⁴,
L einen hetero- oder isocyclischen Rest der Formeln (L1) - (L5), 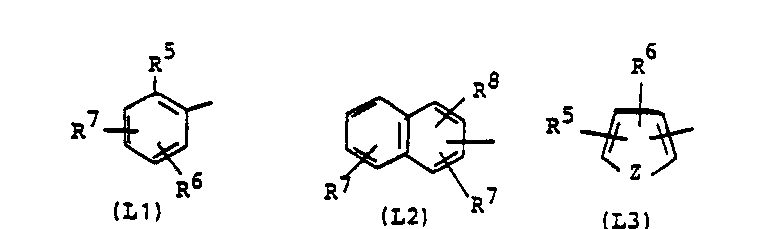 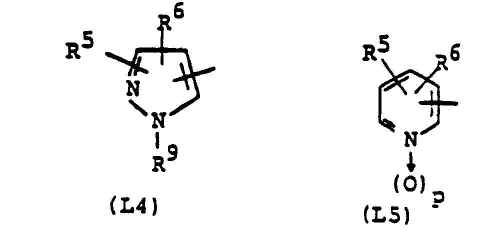
A einen heterocyclischen Rest der Formeln (A1) - (A8) 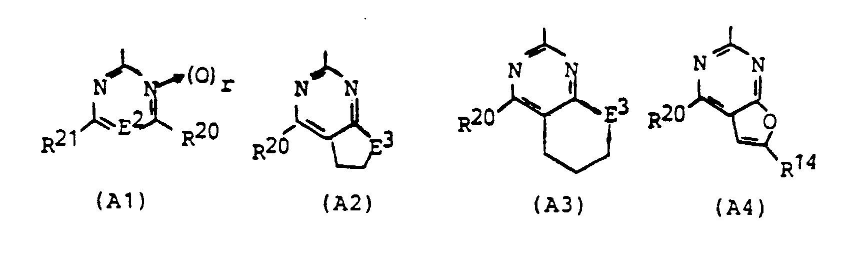 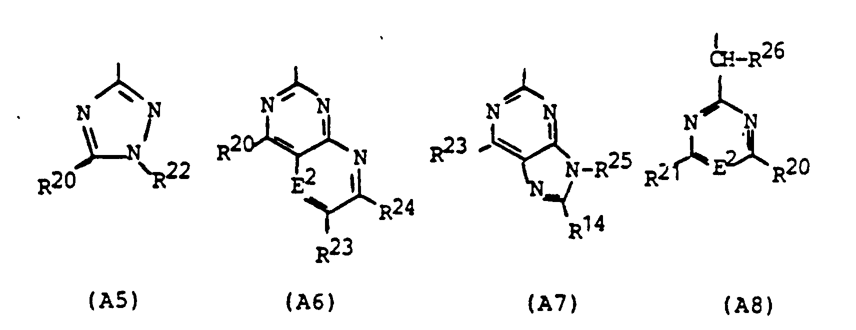
Z O oder S(O)_{q} ,
E¹ O oder S(O)_{b} ,
E² CH oder N ,
E³ O oder CH₂ ,
a;m;n;p;r;s unabhängig voneinander 0 oder 1,
b;q unabhängig voneinander 0, 1 oder 2,
R⁵ Wasserstoff, Halogen, NO₂, CN, (C₁-C₄)-Alkyl, das unsubstituiert oder ein- oder mehrfach durch F, Cl, Br, oder einfach durch CN, OCH₃ oder SCH₃ substituiert ist, (C₂-C₄)-Alkenyl, das unsubstituiert oder ein-oder mehrfach durch F, Cl, Br oder einfach durch OCH₃ substituiert ist; (C₂-C₄)-Alkinyl, das unsubstituiert oder ein- oder mehrfach durch F, Cl, Br, oder einfach durch OCH₃ oder Si(CH₃)₃ substituiert ist; (C₃-C₆)-Cycloalkyl, das unsubstituiert oder ein- oder mehrfach durch F, Cl oder CH₃ substituiert ist; -C(O)R¹⁰, -OCH₂CH₂OR¹⁰, -OH, -C(R¹⁰)(OR¹¹)(OR¹²); -CO₂R¹³, -C(O)-NR¹⁴R¹⁵, -N₃, -SO₂NR¹⁴R¹⁵, -SO₃R¹⁶, -OSO₂R¹⁷, Phenyl, das unsubstituiert oder ein- oder mehrfach durch F, Cl, Br, CH₃ oder OCH₃ substituiert ist; -E¹R¹⁸ oder -(CH₂)ₛG,
R⁶ Wasserstoff, Halogen; CN; NO₂; (C₁-C₄)-Alkyl, das unsubstituiert oder ein- oder mehrfach durch Halogen oder ein- bis zweifach durch -CO₂R¹³, -SO₂NR¹⁴R¹⁵, -NR¹⁴R¹⁵,(C₁-C₂)-Alkoxy, -E¹R¹⁹, (C₁-C₂)-Haloalkoxy, (C₁-C₂)-Alkylthio, (C₁-C₂)-Haloalkylthio, CN, OH oder SH substituiert ist; -CO₂R¹³, -SO₂NR¹⁴R¹⁵, -NR¹⁴R¹⁵ oder -E¹R¹⁹;
R⁷ unabhängig voneinander Wasserstoff, (C₁-C₄)-Alkyl, (C₁-C₄)-Haloalkyl, (C₂-C₄)-Alkenyl, Phenyl oder Phenyl, das ein- oder mehrfach durch Halogen oder -E¹R¹⁹ substituiert ist; -E¹R¹⁹ oder Halogen,
R⁸ Wasserstoff, (C₁-C₄)-Alkyl, (C₁-C₄)-Haloalkyl, Halogen, -CO₂R¹³, -SO₂NR¹⁴R¹⁵, -OSO₂R¹⁷, -S(O)_{b}R¹⁸, CN oder NO₂,
R⁹ Wasserstoff, (C₁-C₄)-Alkyl, das unsubstituiert oder ein- oder mehrfach durch Halogen oder einfach durch Phenyl substituiert ist; (C₂-C₄)-Alkenyl; Phenyl, oder Phenyl, das ein- oder mehrfach durch Halogen, (C₁-C₄)-Alkyl, NO₂, CN oder (C₁-C₄)-Alkoxy substituiert ist,
R¹⁰ Wasserstoff, (C₁-C₄)-Alkyl, (C₁-C₄)-Alkyl, das ein- oder mehrfach durch F, Cl, Br oder OCH₃ substituiert ist; (C₃-C₆)-Cycloalkyl, das unsubstituiert oder durch F, Cl, Br oder CH₃ ein- oder mehrfach substituiert ist; (C₂-C₄)-Alkenyl oder (C₂-C₄)-Alkinyl;
R¹¹,R¹² unabhängig voneinander (C₁-C₄)-Alkyl oder R¹¹ und R¹² zusammen -CH₂CH₂-, -CH₂OCH₂- oder -CH₂C(CH₃)₂CH₂- ;
R¹³ Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist; (C₃-C₄)-Alkinyl, das unsubstituiert oder durch CH₃ oder Si(CH₃)₃ substituiert ist; (C₃-C₆)-Cycloalkyl, (C₃-C₆)-Cycloalkyl-(C₁-C₄)alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂-oder -(CH₂)₂O(CH₂)₂-,
R¹⁶ (C₁-C₄)-Alkyl oder (C₁-C₄)-Haloalkyl,
R¹⁷ (C₁-C₄)-Alkyl oder NR¹⁴R¹⁵,
R¹⁸ (C₁-C₄)-Alkyl, (C₁-C₄)-Haloalkyl, (C₂-C₄)-Alkoxyalkyl, (C₂-C₄)-Alkenyl, (C₃-C₄)-Alkinyl, Phenyl oder Phenyl, das ein- oder mehrfach durch Halogen, (C₁-C₃)-Alkyl, (C₁-C₃)-Alkoxy oder (C₁-C₃)-Haloalkyl substituiert ist;
R¹⁹ (C₁-C₄)-Alkyl oder (C₁-C₄)Alkyl, das ein- oder mehrfach durch F, Cl oder einfach durch OR¹⁶ substituiert ist,
R²⁰,R²¹ unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können; -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy;
R²² (C₁-C₄)-Alkyl oder (C₁-C₄)-Haloalkyl,
R²³,R²⁴ unabhängig voneinander Wasserstoff, (C₁-C₄)-Alkyl, (C₂-C₄)-Alkenyl oder (C₂-C₄)-Alkinyl;
R²⁵ Wasserstoff, (C₁-C₄)-Alkyl oder (C₁-C₄)-Haloalkyl,
R²⁶ Wasserstoff oder (C₁-C₃)-Alkyl,
G einen heterocyclischen Rest (G 1) - (G 25), 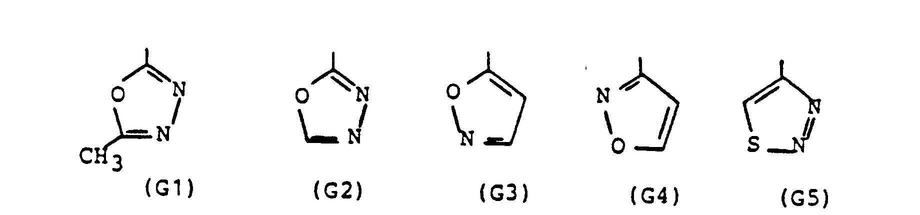 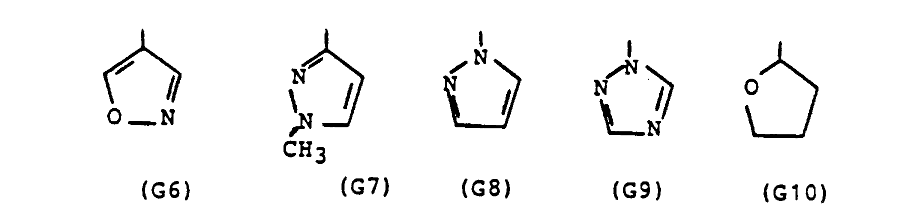 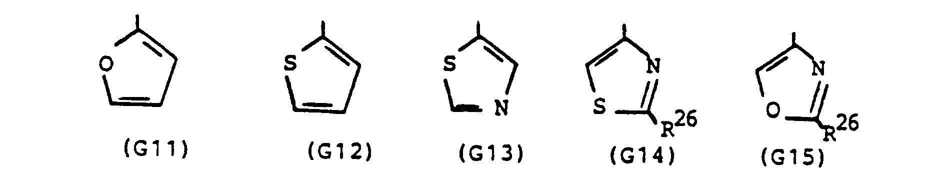 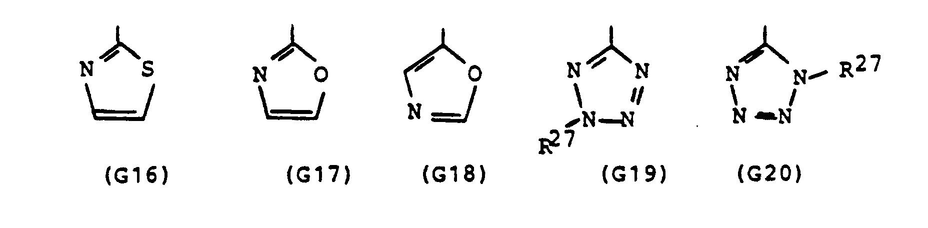 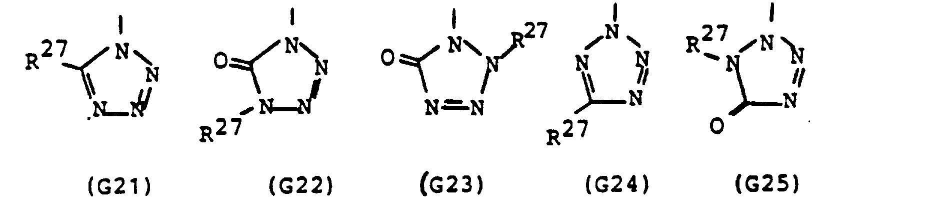
R²⁷ Wasserstoff, (C₁-C₃)-Alkyl oder (C₂-C₄)-Alkenyl; bedeuten.

2. Verbindung der Formel I von Anspruch 1 oder deren Salze, worin
A einen Rest der Formeln (A1), (A2), (A3) oder (A4), insbesondere (A1);
L einen Rest der Formeln (L1), (L3), (L4) oder (L5),
X CH₂, CH(CH₃), O, NH, NCH₃, NC₂H₅, NOCH₃, insbesondere CH₂, O oder NH,
W Sauerstoff,
R¹ Wasserstoff,
R²,R³ unabhängig voneinander Wasserstoff oder (C₁-C₃)Alkyl, insbesondere Wasserstoff,
R⁵ Halogen, NO₂, CN, (C₁-C₃)-Alkyl, das unsubstituiert oder durch F, Cl, Br, CN, OCH₃ oder SCH₃ substituiert ist; (C₃)-Alkenyl, das unsubstituiert oder durch F, Cl oder Br substituiert ist; (C₃)-Alkinyl, (C₃)-Cycloalkyl,
das unsubstituiert oder durch F, Cl oder CH₃ substituiert ist, -C(O)R¹⁰, -OCH₂CH₂OR¹⁰, OH, -C(R¹⁰)(OR¹¹) (OR¹²), -CO₂R¹³, -C(O)NR¹⁴R¹⁵, N₃, -SO₂NR¹⁴R¹⁵, -OSO₂R¹⁷, -E¹R¹⁸ oder -(CH₂)ₛG;
R⁶ Wasserstoff, Halogen, CN, NO₂, CH₃, CF₃, -E¹R¹⁹ oder (C₁-C₂)-Alkoxy-(C₁-C₂)alkyl, (C₁-C₂)-Haloalkoxy, (C₁-C₂)-Alkylthio, (C₁-C₂)-Haloalkylthio, CN, -CO₂R¹³ oder -SO₂NR¹⁴R¹⁵,
R⁷ Wasserstoff
R¹⁰ (C₁-C₃)-Alkyl, Cyclopropyl oder (C₃)-Alkenyl,
R¹¹,R¹² (C₁-C₂)-Alkyl oder R¹¹, R¹² zusammen -CH₂CH₂- ,
R¹³ (C₁-C₃)-Alkyl, (C₃)-Alkenyl, -CH₂CH₂F, -CH₂CH₂Cl, -CH₂CH₂OCH₃ oder Cyclopropylmethyl;
R¹⁴ Wasserstoff oder CH₃ oder R¹⁴ und R¹⁵ zusammen -(CH₂)₄-, -(CH₂)₅- oder -(CH₂)₂O-(CH₂)₂- ,
R¹⁵ CH₃, CH₂CH₃, OCH₃ oder R¹⁴ und R¹⁵ zusammen -(CH₂)₄- , -(CH₂)₅- oder -(CH₂)₂O-(CH₂)₂- ,
R¹⁸ (C₁-C₃)-Alkyl, (C₁-C₃)-Haloalkyl, (C₂-C₃)-Alkoxyalkyl, Allyl, Propargyl, (C₂-C₃)-Haloalkenyl,
R¹⁹ (C₁-C₂)-Alkyl, das unsubstituiert oder durch F, Cl oder OCH₃ substituiert ist,
R²⁰,R²¹ unabhängig voneinander Wasserstoff, (C₁-C₂)-Alkyl, (C₁-C₂)-Alkoxy, -CH₂OCH₃, Cl, F, Br, J, -CH₂OCH₂CH₃, -NHCH₃, -N(OCH₃)CH₃, -N(CH₃)₂, -CF₃, -SCH₃, -CH(OCH₃)₂, -OCH₂CH=CH₂; -OCH₂C≡CH, -OCH₂CH₂OCH₃, -CH₂SCH₃,-OCHF₂, -SCHF₂, Cyclopropyl, -C≡CH oder -C≡C-CH₃,
m 0 oder 1,
n 0 oder 1,
s null und
E¹ O oder S bedeuten.

3. Verfahren zur Herstellung der Verbindungen der Formel I nach Anspruch 1 oder 2 oder deren Salze, **dadurch gekennzeichnet, daß** man
a) zur Herstellung von Verbindungen mit W = O
a₁) eine Verbindung der Formel II mit 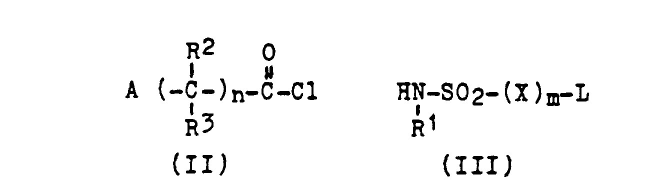 einer Verbindung der Formel III in Gegenwart einer Base umsetzt oder
a₂) eine Verbindung der Formel (IIa) 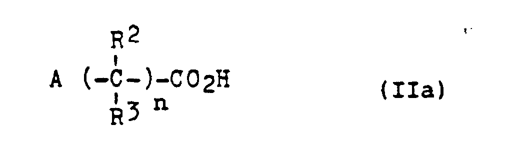 in Gegenwart von aktivierenden Reagentien wie 2-Chlor-1-methylpyridiniumchlorid(IVa), Dicyclohexylcarbodiimid (IVb) oder 1,1-Carbonyldiimidazol (IVc) und gegebenenfalls in Gegenwart einer Base mit einer Verbindung der Formel III umsetzt oder
a₃) zur Herstellung von Verbindungen mit W = O und R¹ = H eine Verbindung der Formel (V), 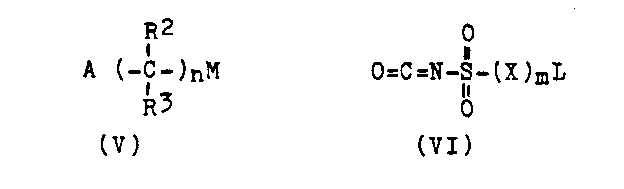 worin M Wasserstoff oder Lithium bedeutet, mit einer Verbindung der Formel (VI) umsetzt, oder
b) zur Herstellung von Verbindungen mit W = S eine unter a) erhaltene Verbindung der Formel I mit der Verbindung der Formel VII umsetzt, 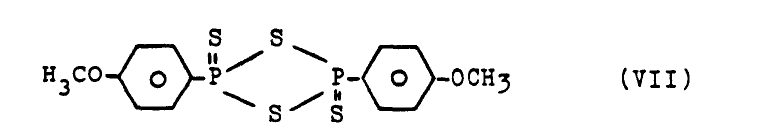
c) zur Herstellung von Verbindungen mit W= NR⁴ eine Verbindung der Formel VIII 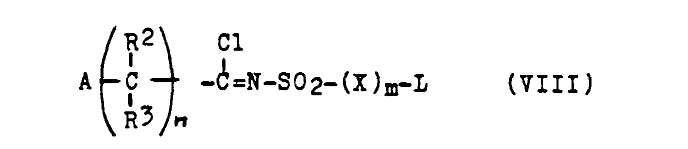 mit einem Amin der Formel H₂N-R⁴ umsetzt,
d) zur Herstellung von Verbindungen mit W = NOR⁴
d₁) eine Verbindung der Formel IX mit 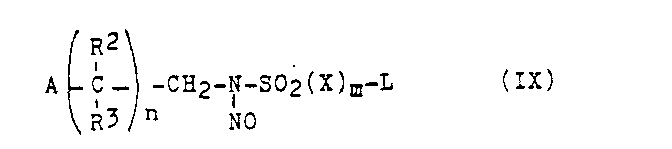 einem Alkali- oder Erdalkalihydroxid oder Ammoniumhydroxid umsetzt oder
d₂) eine Verbindung der Formel VIII in Gegenwart einer Base mit einem Hydroxylamin der Formel H₂N-OR⁴ umsetzt, oder
e) zur Herstellung von Verbindungen der Formel I mit R¹ ≠ H eine Verbindung der Formel I mit R¹ = H in Gegenwart einer Base mit einem Alkylhalogenid der Formel R^{1'}-X¹, worin R^{1'} die Bedeutung von R¹ außer Wasserstoff besitzt und X¹ Chlor, Brom oder Jod bedeutet, umsetzt und die erhaltenen Verbindungen gegebenenfalls in ihr Salze überführt.

4. Verbindung der Formel (II'), 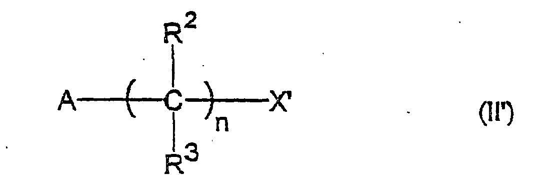 worin
X' COCl, CN, F, p-Tosyl, Tetraalkylammonium, N₃, CO₂R¹³, CO₂CH₂C₆H₅ oder E¹R¹⁸ bedeutet,
n 0 oder 1 ist,
R² und R³ unabhängig voneinander Wasserstoff, (C₁-C₃)-Alkyl oder Phenyl bedeutet,
A einen heterocyclischen Rest der Formel (A1), worin r = 1, oder (A2), (A3), (A4) oder (A5), wobei im Fall (A5) n = 1 ist, oder (A6), (A7) oder (A8) bedeutet, 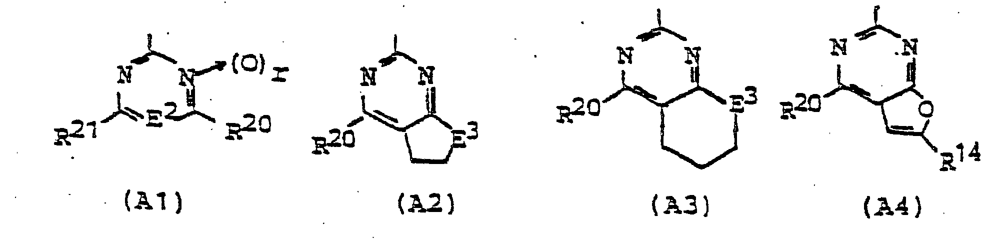 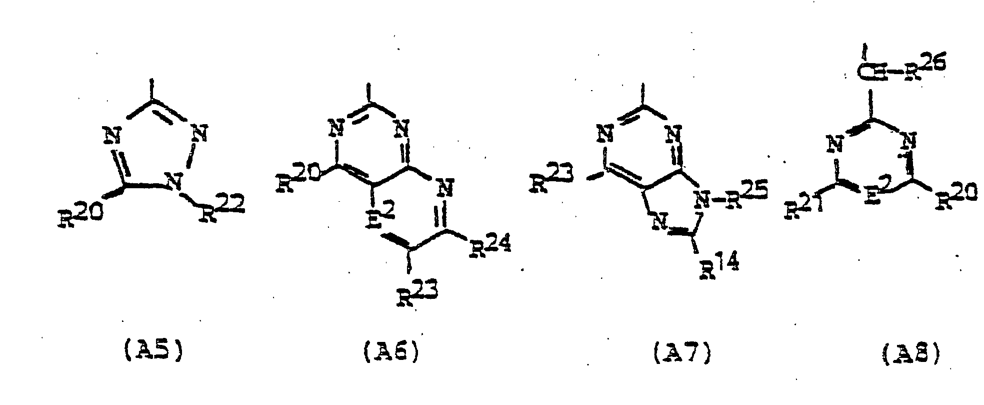 und
E¹ O oder S(O)_{b},
E² CH oder N ist,
E³ O oder CH₂,
a 0 oder 1,
b 0, 1 oder 2,
R¹⁰ Wasserstoff, (C₁-C₄)-Alkyl, (C₁-C₄)-Alkyl, das ein- oder mehrfach durch F, Cl, Br oder OCH₃ substituiert ist, (C₃-C₆)-Cycloalkyl, das unsubstituiert oder durch F, Cl, Br oder ein- oder mehrfach substituiert ist, (C₂-C₄)-Alkenyl oder (C₂-C₄)-Alkinyl,
R¹³ Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist, (C₃-C₄)-Alkinyl, das unsubstituiert oder durch CH₃ oder Si(CH₃)₃ substituiert ist, (C₃-C₆)-Cycloalkyl, (C₃-C₆)-Cycloalkyl-(C₁-C₄)-alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂- oder -(CH₂)₂O(CH₂)₂-,
R¹⁸ (C₁-C₄)-Alkyl, (C₁-C₄)-Haloalkyl, (C₂-C₄)-Alkoxyalkyl, (C₂-C₄)-Alkenyl, (C₃-C₄)-Alkinyl, Phenyl oder Phenyl, das ein- oder mehrfach durch Halogen, (C₁-C₃)-Alkyl, (C₁-C₃)-Alkoxy oder (C₁-C₃)-Haloalkyl substituiert ist,
R²⁰, R²¹ unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können, -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy,
R²² (C₁-C₄)-Alkyl oder (C₁-C₄)-Haloalkyl,
R²³, R²⁴ unabhängig voneinander Wasserstoff, (C₁-C₄)-Alkyl, (C₂-C₄)-Alkenyl oder (C₂-C₄)-Alkinyl,
R²⁵ Wasserstoff, (C₁-C₄)-Alkyl oder (C₁-C₄)-Haloalkyl und
R²⁶ Wasserstoff oder (C₁-C₃)-Alkyl
bedeuten.

5. Verbindung der Formel (II'), 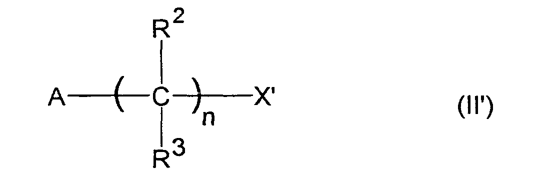 worin
X' COCl, CO₂R¹³ oder CO₂CH₂C₆H₅ bedeutet,
n 0 oder 1 ist,
R² und R³ unabhängig voneinander Wasserstoff, (C₁-C₃)-Alkyl oder Phenyl bedeutet,
A einen heterocyclischen Rest der Formel (A1), worin r = 0 und E² = CH sind, 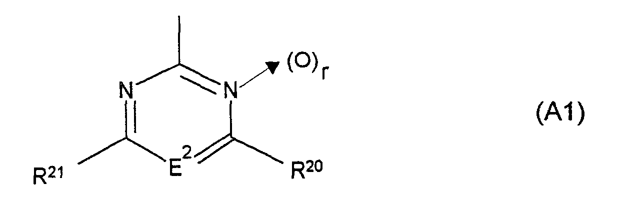 und
a 0 oder 1,
R¹⁰ Wasserstoff, (C₁-C₄)-Alkyl, (C₁-C₄)-Alkyl, das ein- oder mehrfach durch F, Cl, Br oder OCH₃ substituiert ist, (C₃-C₆)-Cycloalkyl, das unsubstituiert oder durch F, Cl, Br oder ein- oder mehrfach substituiert ist, (C₂-C₄)-Alkenyl oder (C₂-C₄)-Alkinyl,
R¹³ Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist, (C₃-C₄)-Alkinyl, das unsubstituiert oder durch CH₃ oder Si(CH₃)₃ substituiert ist, (C₃-C₆)-Cycloalkyl, (C₃-C₆)-Cycloalkyl-(C₁-C₄)-alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂- oder -(CH₂)₂O(CH₂)₂- und
R²⁰, R²¹ unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können, -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy
bedeuten, wobei
1) im Falle n = 0 und X' = COOH das Paar R²⁰/R²¹ nicht CH₃/CH₃ ist,
2) im Falle n = 0 und X' =CO₂CH₃ das Paar R²⁰/R²¹ nicht CH₃/CH₃ oder OCH₃/CH₃ ist,
3) im Falle n = 0 und X' =CO₂C₂H₅ das Paar R²⁰/R²¹ nicht OC₂H₅/OC₂H₅ oder Cl/Cl ist,
4) im Falle n = 1, R² = R³ = H und X' = COOH, COONa oder COOCH₃ das Paar R²⁰/R²¹ nicht H/F, H/Cl, H/Br, H/l, H/CH₃, H/OCH₃, H/SC₂H₅, Cl/SC₂H₅, SC₂H₅/SC₂H₅, Benzyloxy/CH₃, OCH₃/CH₃, OC₂H₅/CH₃, Cl/Cl, Cl/OCH3, OCH₃/OCH₃ oder Cl/CH₃ ist,
5) im Falle n = 1, R² = R³ = H und X' = CO₂C₂H₅ das Paar R²⁰/R²¹ nicht Cl/CH₃ oder CH₃/CH₃ ist,

6. Verbindung der Formel (II'), 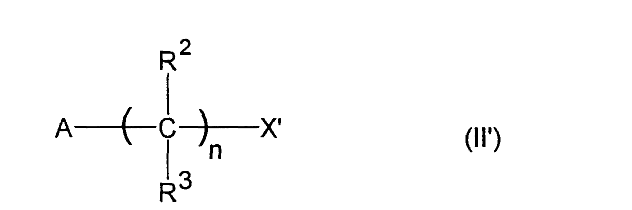 worin
X' COCl, CO₂R¹³ oder CO₂CH₂C₆H₅ bedeutet,
n 0 oder 1 ist,
R² und R³ unabhängig voneinander Wasserstoff, (C₁-C₃)-Alkyl oder Phenyl bedeutet,
A einen heterocyclischen Rest der Formel (A1), worin r = 0 und E² = N sind, 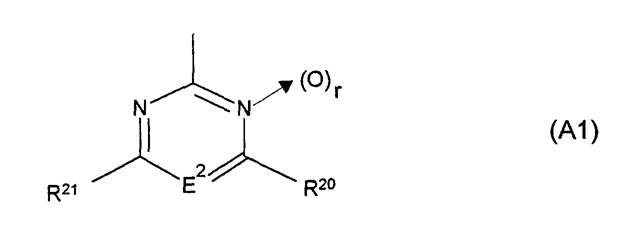 und
a 0 oder 1,
R¹⁰ Wasserstoff, (C₁-C₄)-Alkyl, (C₁-C₄)-Alkyl, das ein- oder mehrfach durch F, Cl, Br oder OCH₃ substituiert ist, (C₃-C₆)-Cycloalkyl, das unsubstituiert oder durch F, Cl, Br oder ein- oder mehrfach substituiert ist, (C₂-C₄)-Alkenyl oder (C₂-C₄)-Alkinyl,
R¹³ Wasserstoff, (C₁-C₄)-Alkyl, das unsubstituiert oder ein- oder mehrfach durch Halogen oder ein- bis zweifac/h durch CN, CO₂R¹⁰, NR¹⁴R¹⁵, OR¹⁰ oder Si(CH₃)₃ substituiert ist, (C₃-C₄)-Alkinyl, das unsubstituiert oder durch CH₃ oder Si(CH₃)₃ substituiert ist, (C₃-C₆)-Cycloalkyl, (C₃-C₆)-Cycloalkyl-(C₁-C₄)-alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂- oder -(CH₂)₂O(CH₂)₂- und
R²⁰, R²¹ unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein - oder mehrfach durch Halogen oder ein- oder zweifach durch (C₁-C₄)-Alkoxy oder (C₁-C₄)-Alkylthio substituiert sein können, -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy
bedeuten, wobei
1) im Falle n = 0 und X' =CO₂C₂H₅ das Paar R²⁰/R²¹ nicht CH₃/CH₃ oder CCl₃/NH₂ oder SCH₃/SCH₃ ist,
2) im Falle n = 1, R² = R³ = H und X' = CO₂C₂H₅ das Paar R²⁰/R²¹ nicht Cl/OCH₃ oder Cl/Cl ist,
3) im Falle n = 1, R² = R³ = H und X' = CN das Paar R²⁰/R²¹ nicht NH₂/NH₂ ist.
4) im Falle n = 0 und X' = COOH das Paar R²⁰/R²¹ nicht OCH₃/OCH₃ oder SCH₃/SCH₃ ist und
5) im Falle n = 0 und X' = CO₂CH₃ das Paar R²⁰/R²¹ nicht SCH₃/SCH₃ ist.

7. Verbindung der Formel (II'), 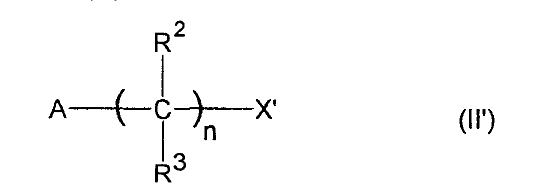 worin
X' COCI, CN, CO₂R¹³ oder CO₂CH₂C₆H₅ bedeutet,
n 0 ist,
R² und R³ unabhängig voneinander Wasserstoff, (C₁-C₃)-Alkyl oder Phenyl bedeutet,
A einen heterocyclischen Rest der Formel (A5), 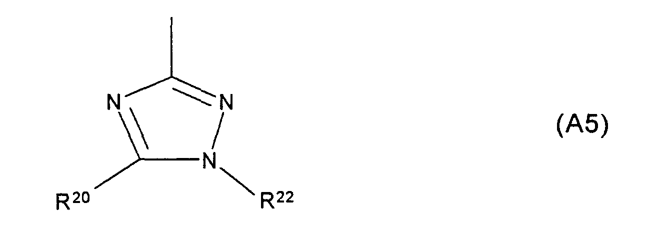 und
a 0 oder 1,
R¹⁰ Wasserstoff, (C₁-C₄)-Alkyl, (C₁-C₄)-Alkyl, das ein- oder mehrfach durch F, Cl, Br oder OCH₃ substituiert ist, (C₃-C₆)-Cycloalkyl, das unsubstituiert oder durch F, Cl, Br oder ein- oder mehrfach substituiert ist, (C₂-C₄)-Alkenyl oder (C₂-C₄)-Alkinyl,
R¹³ Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist, (C₃-C₄)-Alkinyl, das unsubstituiert oder durch CH₃ oder Si(CH₃)₃ substituiert ist, (C₃-C₆)-Cycloalkyl, (C₃-C₆)-Cycloalkyl-(C₁-C₄)-alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂- oder -(CH₂)₂O(CH₂)₂-,
R²⁰ Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können, -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy und
R²² (C₁-C₄)-Alkyl oder (C₁-C₄)-Haloalkyl
bedeuten, wobei
im Falle n = 0, X' =CO₂CH₃ oder CO₂C₂H₅ und R²⁰ = H der Rest R²² nicht gleich CH₃ ist.

8. Verfahren zur Herstellung der Verbindungen der Formel (II') gemäß Anspruch 5, **dadurch gekennzeichnet, daß** man eine Verbindung der Formel (XI) 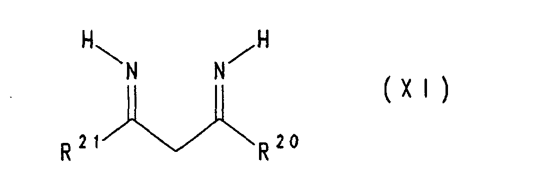 mit einer Verbindung der Formel (XII) oder (XIII) 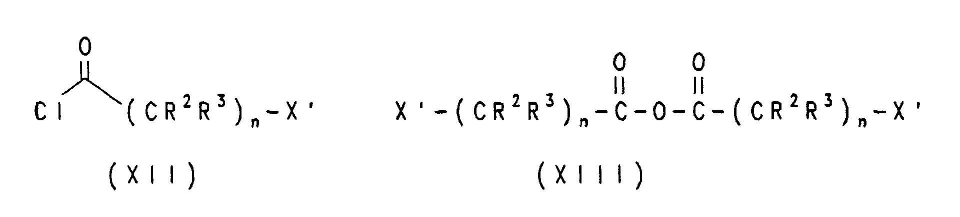 umsetzt,
wobei in den Formeln (XI), (XII) und (XIII) die Reste R², R³, R²⁰, R²¹ und X' sowie n wie in Formel (II') definiert sind.

9. Verfahren nach Anspruch 8, **dadurch gekennzeichnet, daß** man die Umsetzung in einem unter den Reaktionsbedingungen inerten Solvens in Gegenwart einer Base bei einer Temperatur von -78 °C bis 100 °C durchführt.

10. Herbizide oder pflanzenwachstumsregulierende Mittel, **dadurch gekennzeichnet, daß** sie eine Verbindung der Formel (I) von Anspruch 1 oder 2 oder deren Salz und inerte Zusatzstoffe enthalten.

11. Verwendung von Verbindungen der Formel (I) von Anspruch 1 oder 2 oder deren Salze als Herbizide oder Pflanzenwachstumsregulatoren.

12. Verfahren zur Bekämpfung von unerwünschten Pflanzen oder zur Wachstumsregulierung von Pflanzen, **dadurch gekennzeichnet, daß** man auf diese oder die Kulturböden eine wirksame Menge einer Verbindung der Formel (I) oder deren Salz von Anspruch 1 oder 2 appliziert.

## Patentansprüche (Patentansprüche für folgende(n) Vertragsstaat(en): ES)

1. Verwendung der Verbindungen der Formel I oder deren Salze 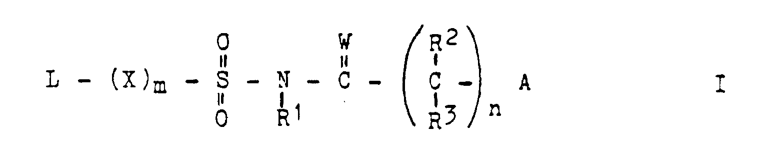 worin
R¹ Wasserstoff, (C₁-C₄)Alkyl, (C₂-C₆)-Alkenyl oder (C₂-C₆)-Alkinyl;
R²,R³ unabhängig voneinander Wasserstoff, (C₁-C₃)-Alkyl oder Phenyl;
W O, S, NR⁴ oder NOR⁴,
R⁴ Wasserstoff, (C₁-C₃)-Alkyl, (C₁-C₃)-Haloalkyl oder Phenyl;
X CHR², O, NR⁴ oder NOR⁴,
L einen hetero- oder isocyclischen Rest der Formeln (L1) - (L5), 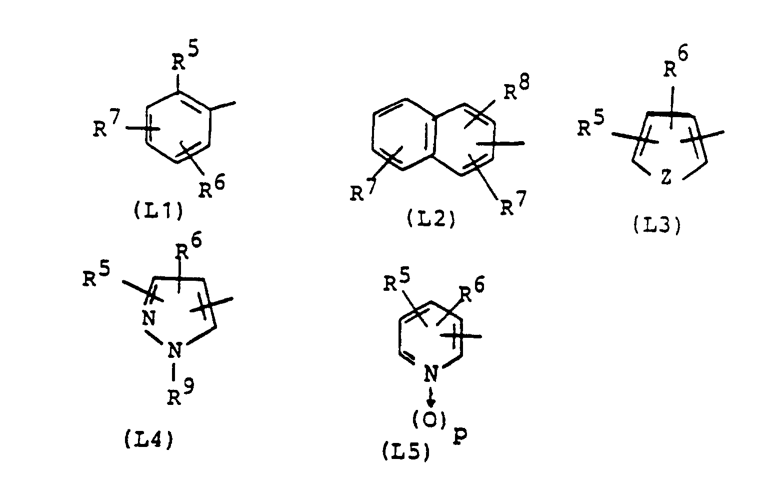
A einen heterocyclischen Rest der Formeln (A1) - (A8) 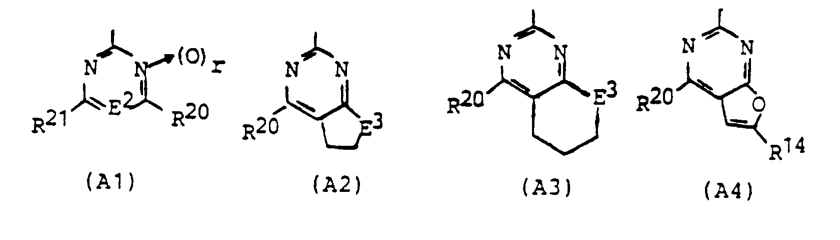 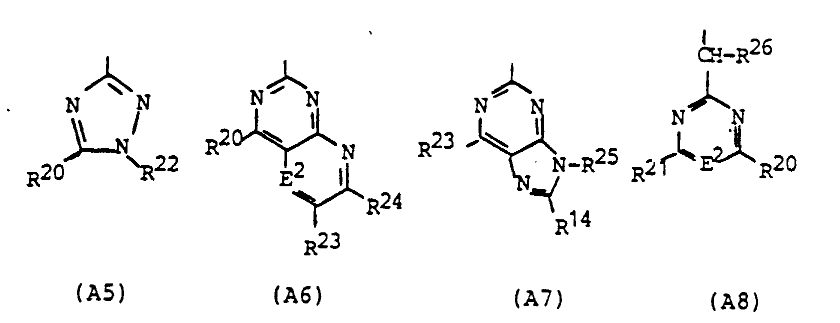
Z O oder S(O)_{q},
E¹ O oder S(O)_{b},
E² CH oder N,
E³ O oder CH₂,
a;m;n;p;r;s unabhängig voneinander 0 oder 1,
b;q unabhängig voneinander 0, 1 oder 2,
R⁵ Wasserstoff, Halogen, NO₂, CN, (C₁-C₄)-Alkyl, das unsubstituiert oder ein- oder mehrfach durch F, Cl, Br, oder einfach durch CN, OCH₃ oder SCH₃ substituiert ist, (C₂-C₄)-Alkenyl, das unsubstituiert oder ein-oder mehrfach durch F, Cl, Br oder einfach durch OCH₃ substituiert ist; (C₂-C₄)-Alkinyl, das unsubstituiert oder ein- oder mehrfach durch F, Cl, Br, oder einfach durch OCH₃ oder Si(CH₃)₃ substituiert ist; (C₃-C₆)-Cycloalkyl, das unsubstituiert oder ein- oder mehrfach durch F, Cl oder CH₃ substituiert ist; -C(O)R¹⁰, -OCH₂CH₂OR¹⁰, -OH, -C(R¹⁰)(OR¹¹)(OR¹²); -CO₂R¹³, -C(O)-NR¹⁴R¹⁵, -N₃, -SO₂NR¹⁴R¹⁵, -SO₃R¹⁵, -OSO₂R¹⁷, Phenyl, das unsubstituiert oder ein - oder mehrfach durch F, Cl, Br, CH₃ oder OCH₃ substituiert ist; -E¹R¹⁸ oder -(CH₂)ₛG,
R⁶ Wasserstoff, Halogen; CN; NO₂; (C₁-C₄)-Alkyl, das unsubstituiert oder ein- oder mehrfach durch Halogen oder ein- bis zweifach durch -CO₂R¹³, -SO₂NR¹⁴R¹⁵, -NR¹⁴R¹⁵,(C₁-C₂)-Alkoxy, -E¹R¹⁹, (C₁-C₂)-Haloalkoxy, (C₁-C₂)-Alkylthio, (C₁-C₂)-Haloalkylthio, CN, OH oder SH substituiert ist; -CO₂R¹³, -SO₂NR¹⁴R¹⁵, -NR¹⁴R¹⁵ oder -E¹R¹⁹;
R⁷ unabhängig voneinander Wasserstoff, (C₁-C₄)-Alkyl, (C₁-C₄)-Haloalkyl, (C₂-C₄)-Alkenyl, Phenyl oder Phenyl, das ein- oder mehrfach durch Halogen oder -E¹R¹⁹ substituiert ist; -E¹R¹⁹ oder Halogen,
R⁸ Wasserstoff, (C₁-C₄)-Alkyl, (C₁-C₄)-Haloalkyl, Halogen, -CO₂R¹³, -SO₂NR¹⁴R¹⁵, -OSO₂R¹⁷, -S(O)_{b}R¹⁸, CN oder NO₂,
R⁹ Wasserstoff, (C₁-C₄)-Alkyl, das unsubstituiert oder ein- oder mehrfach durch Halogen oder einfach durch Phenyl substituiert ist; (C₂-C₄)-Alkenyl; Phenyl, oder Phenyl, das ein- oder mehrfach durch Halogen, (C₁-C₄)-Alkyl, NO₂, CN oder (C₁-C₄)-Alkoxy substituiert ist,
R¹⁰ Wasserstoff, (C₁-C₄)-Alkyl, (C₁-C₄)-Alkyl, das ein- oder mehrfach durch F, Cl, Br oder OCH₃ substituiert ist; (C₃-C₆)-Cycloalkyl, das unsubstituiert oder durch F, Cl, Br oder CH₃ ein- oder mehrfach substituiert ist; (C₂-C₄)-Alkenyl oder (C₂-C₄)-Alkinyl;
R¹¹,R¹² unabhängig voneinander (C₁-C₄)-Alkyl oder R¹¹ und R¹² zusammen -CH₂CH₂-, -CH₂OCH₂- oder -CH₂C(CH₃)₂CH₂- ;
R¹³ Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist; (C₃-C₄)-Alkinyl, das unsubstituiert oder durch CH₃ oder Si(CH₃)₃ substituiert ist; (C₃-C₆)-Cycloalkyl, (C₃-C₆)-Cycloalkyl-(C₁-C₄)alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂-oder -(CH₂)₂O(CH₂)₂- ,
R¹⁶ (C₁-C₄)-Alkyl oder (C₁-C₄)-Haloalkyl,
R¹⁷ (C₁-C₄)-Alkyl oder NR¹⁴R¹⁵ ,
R¹⁸ (C₁-C₄)-Alkyl, (C₁-C₄)-Haloalkyl, (C₂-C₄)-Alkoxyalkyl, (C₂-C₄)-Alkenyl, (C₃-C₄)-Alkinyl, Phenyl oder Phenyl, das ein- oder mehrfach durch Halogen, (C₁-C₃)-Alkyl, (C₁-C₃)-Alkoxy oder (C₁-C₃)-Haloalkyl substituiert ist;
R¹⁹ (C₁-C₄)-Alkyl oder (C₁-C₄)Alkyl, das ein- oder mehrfach durch F, Cl oder einfach durch OR¹⁶ substituiert ist,
R²⁰,R²¹ unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können; -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy;
R²² (C₁-C₄)-Alkyl oder (C₁-C₄)-Haloalkyl,
R²³,R²⁴ unabhängig voneinander Wasserstoff, (C₁-C₄)-Alkyl, (C₂-C₄)-Alkenyl oder (C₂-C₄)-Alkinyl;
R²⁵ Wasserstoff, (C₁-C₄)-Alkyl oder (C₁-C₄)-Haloalkyl,
R²⁶ Wasserstoff oder (C₁-C₃)-Alkyl,
G einen heterocyclischen Rest (G 1) - (G 25), 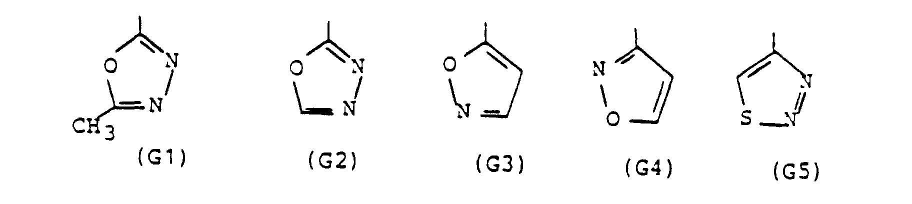 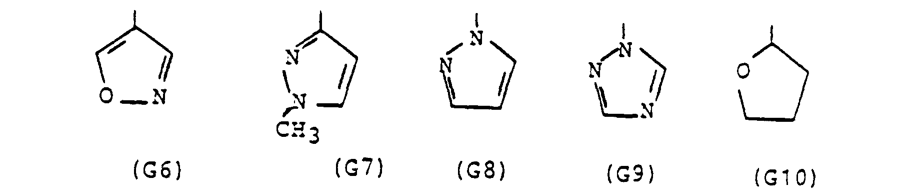 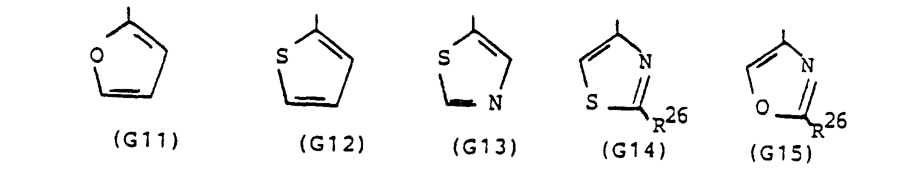 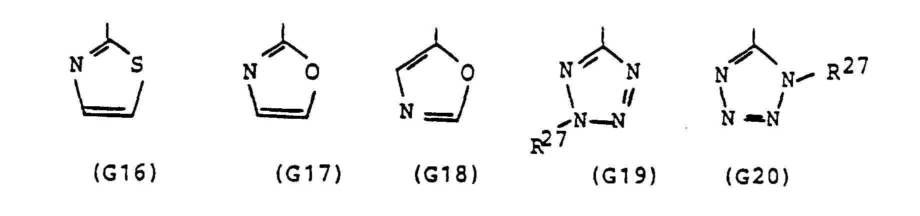 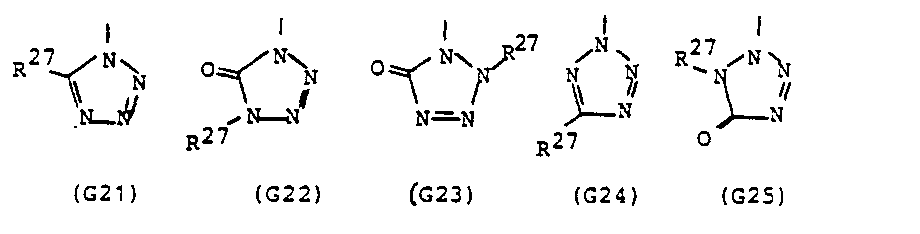
R²⁷ Wasserstoff, (C₁-C₃)-Alkyl oder (C₂-C₄)-Alkenyl;
bedeuten,
als Herbizide oder Pflanzenwachstumsregulatoren.

2. Verwendung nach Anspruch 1, **dadurch gekennzeichnet, daß** in Formel (I)
A einen Rest der Formeln (A1), (A2), (A3) oder (A4), insbesondere (A1);
L einen Rest der Formeln (L1), (L3), (L4) oder (L5),
X CH₂, CH(CH₃), O, NH, NCH₃, NC₂H₅, NOCH₃, insbesondere CH₂, O oder NH,
W Sauerstoff,
R¹ Wasserstoff
R²,R³ unabhängig voneinander Wasserstoff oder (C₁-C₃)Alkyl, insbesondere Wasserstoff,
R⁵ Halogen, NO₂, CN, (C₁-C₃)-Alkyl, das unsubstituiert oder durch F, Cl, Br, CN, OCH₃ oder SCH₃ substituiert ist; (C₃)-Alkenyl, das unsubstituiert oder durch F, Cl oder Br substituiert ist; (C₃)-Alkinyl, (C₃)-Cycloalkyl,
das unsubstituiert oder durch F, Cl oder CH₃ substituiert ist, -C(O)R¹⁰, -OCH₂CH₂OR¹⁰, OH, -C(R¹⁰)(OR¹¹)(OR¹²), -CO₂R¹³, -C(O)NR¹⁴R¹⁵, N₃, -SO₂NR¹⁴R¹⁵, -OSO₂R¹⁷, -E¹R¹⁸ oder -(CH₂)ₛG;
R⁶ Wasserstoff, Halogen, CN, NO₂, CH₃, CF₃, -E¹R¹⁹ oder (C₁-C₂)-Alkoxy-(C₁-C₂)alkyl, (C₁-C₂)-Haloalkoxy, (C₁-C₂)-Alkylthio, (C₁-C₂)-Haloalkylthio, CN, -CO₂R¹³ oder -SO₂NR¹⁴R¹⁵,
R⁷ Wasserstoff
R¹⁰ (C₁-C₃)-Alkyl, Cyclopropyl oder (C₃)-Alkenyl,
R¹¹,R¹² (C₁-C₂)-Alkyl oder R¹¹, R¹² zusammen -CH₂CH₂-,
R¹³ (C₁-C₃)-Alkyl, (C₃)-Alkenyl, -CH₂CH₂F, -CH₂CH₂Cl, -CH₂CH₂OCH₃ oder Cyclopropylmethyl;
R¹⁴ Wasserstoff oder CH₃ oder R¹⁴ und R¹⁵ zusammen -(CH₂)₄-, -(CH₂)₅- oder -(CH₂)₂O-(CH₂)₂- ,
R¹⁵ CH₃, CH₂CH₃, OCH₃ oder R¹⁴ und R¹⁵ zusammen -(CH₂)₄- , -(CH₂)₅- oder -(CH₂)₂O-(CH₂)₂- ,
R¹⁸ (C₁-C₃)-Alkyl, (C₁-C₃)-Haloalkyl, (C₂-C₃)-Alkoxyalkyl, Allyl, Propargyl, (C₂-C₃)-Haloalkenyl,
R¹⁹ (C₁-C₂)-Alkyl, das unsubstituiert oder durch F, Cl oder OCH₃ substituiert ist,
R²⁰,R²¹ unabhängig voneinander Wasserstoff, (C₁-C₂)-Alkyl, (C₁-C₂)-Alkoxy, -CH₂OCH₃, Cl, F, Br, J, -CH₂OCH₂CH₃, -NHCH₃, -N(OCH₃)CH₃, -N(CH₃)₂, -CF₃, -SCH₃, -CH(OCH₃)₂, -OCH₂CH=CH₂; -OCH₂C≡CH, -OCH₂CH₂OCH₃, -CH₂SCH₃,-OCHF₂, -SCHF₂, Cyclopropyl, -C≡CH oder -C≡C-CH₃,
m 0 oder 1
n 0 oder 1
s null und
E¹ O oder S bedeuten.

3. Verfahren zur Herstellung der Verbindungen der Formel I nach Anspruch 1 oder 2 oder deren Salze, **dadurch gekennzeichnet, daß** man
a) zur Herstellung von Verbindungen mit W = O
a₁) eine Verbindung der Formel II mit 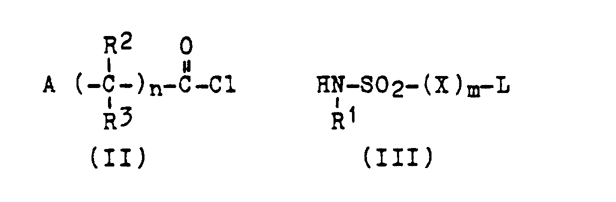 einer Verbindung der Formel III in Gegenwart einer Base umsetzt oder
a₂) eine Verbindung der Formel (IIa) 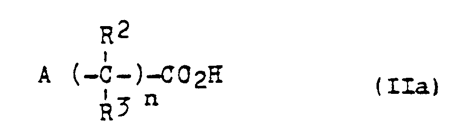 in Gegenwart von aktivierenden Reagentien wie 2-Chlor-1-methylpyridiniumchlorid(IVa), Dicyclohexylcarbodiimid (IVb) oder 1,1-Carbonyldiimidazol (IVc) und gegebenenfalls in Gegenwart einer Base mit einer Verbindung der Formel III umsetzt oder
a₃) zur Herstellung von Verbindungen mit W = O und R¹ = H eine Verbindung der Formel (V), 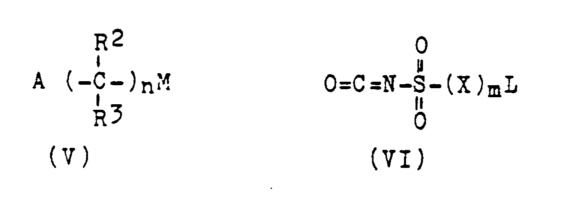 worin M Wasserstoff oder Lithium bedeutet, mit einer Verbindung der Formel (VI) umsetzt, oder
b) zur Herstellung von Verbindungen mit W = S eine unter a) erhaltene Verbindung der Formel I mit der Verbindung der Formel VII umsetzt, 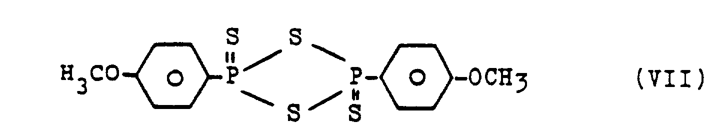
c) zur Herstellung von Verbindungen mit W= NR⁴ eine Verbindung der Formel VIII 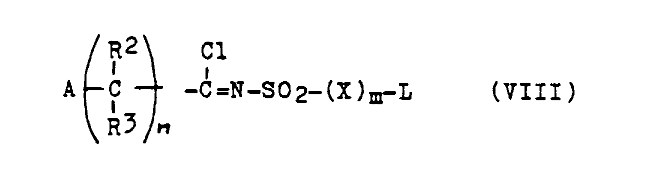 mit einem Amin der Formel H₂N-R⁴ umsetzt,
d) zur Herstellung von Verbindungen mit W= NOR⁴
d₁) eine Verbindung der Formel IX mit 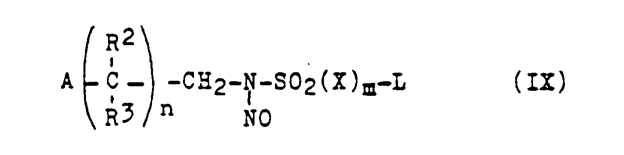 einem Alkali- oder Erdalkalihydroxid oder Ammoniumhydroxid umsetzt oder
d₂) eine Verbindung der Formel VIII in Gegenwart einer Base mit einem Hydroxylamin der Formel H₂N-OR⁴ umsetzt, oder
e) zur Herstellung von Verbindungen der Formel I mit R¹ ≠ H eine Verbindung der Formel I mit R¹ = H in Gegenwart einer Base mit einem Alkylhalogenid der Formel R^{1'}-X¹, worin R^{1'} die Bedeutung von R¹ außer Wasserstoff besitzt und X¹ Chlor, Brom oder Jod bedeutet, umsetzt und die erhaltenen Verbindungen gegebenenfalls in ihr Salz überführt.

4. Verfahren zur Herstellung der Verbindungen der Formel (II'), 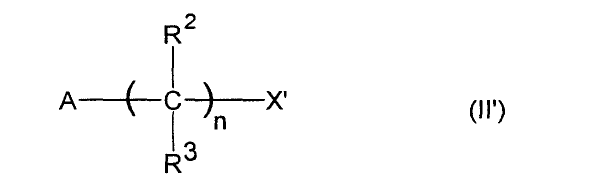 worin
X' COCI, CO₂R¹³ oder CO₂CH₂C₆H₅ bedeutet,
n 0 oder 1 ist,
R² und R³ unabhängig voneinander Wasserstoff, (C₁-C₃)-Alkyl oder Phenyl bedeutet,
A einen heterocyclischen Rest der Formel (A1), worin r = 0 und E² = CH sind, 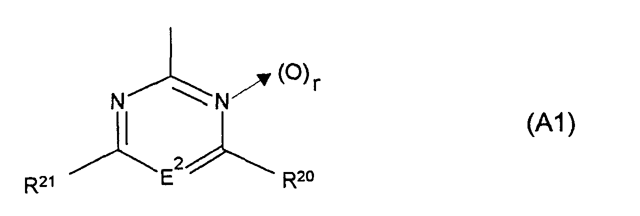 und
a 0 oder 1,
R¹⁰ Wasserstoff, (C₁-C₄)-Alkyl, (C₁-C₄)-Alkyl, das ein- oder mehrfach durch F, Cl, Br oder OCH₃ substituiert ist, (C₃-C₆)-Cycloalkyl, das unsubstituiert oder durch F, Cl, Br oder ein- oder mehrfach substituiert ist, (C₂-C₄)-Alkenyl oder (C₂-C₄)-Alkinyl,
R¹³ Wasserstoff, (C₁-C₄)-Alkyl, das unsubstituiert oder ein- oder mehrfach durch Halogen oder ein- bis zweifach durch CN, CO₂R¹⁰, NR¹⁴R¹⁵, OR¹⁰ oder Si(CH₃)₃ substituiert ist, (C₃-C₄)-Alkinyl, das unsubstituiert oder durch CH₃ oder Si(CH₃)₃ substituiert ist, (C₃-C₆)-Cycloalkyl, (C₃-C₆)-Cycloalkyl-(C₁-C₄)-alkyl, (C₁-C₄)-Alkoxy oder Si(CH₃)₃,
R¹⁴ Wasserstoff oder (C₁-C₄)-Alkyl oder R¹⁴ und R¹⁵ zusammen -(CH₂)₂(CH₂)ₐ(CH₂)₂- oder -(CH₂)₂O(CH₂)₂-,
R¹⁵ Wasserstoff, (C₁-C₄)-Alkyl, (C₁-C₄)-Alkoxy, (C₂-C₄)-Alkenyl oder R¹⁴ und R¹⁵ zusammen -(CH₂)₂(CH₂)ₐ(CH₂)₂- oder -(CH₂)₂O(CH₂)₂- und
R²⁰, R²¹ unabhängig voneinander Wasserstoff, Halogen, (C₁-C₆)-Alkyl, (C₁-C₆)-Alkoxy oder (C₁-C₆)-Alkylthio, wobei die drei vorgenannten Reste ein- oder mehrfach durch Halogen oder ein- oder zweifach durch (C₁-C₄)-Alkoxy oder (C₁-C₄)-Alkylthio substituiert sein können, -NR¹⁴R¹⁵, (C₃-C₆)-Cycloalkyl, -OCHR¹⁴CO₂R¹³, (C₂-C₅)-Alkenyl, (C₂-C₄)-Alkinyl, (C₃-C₅)-Alkenyloxy oder (C₃-C₅)-Alkinyloxy
bedeuten, wobei
1) im Falle n = 0 und X' = COOH das Paar R²⁰/R²¹ nicht CH₃/CH₃ ist,
2) im Falle n = 0 und X' =CO₂CH₃ das Paar R²⁰/R²¹ nicht CH₃/CH₃ oder OCH₃/CH₃ ist,
3) im Falle n = 0 und X' =CO₂C₂H₅ das Paar R²⁰/R²¹ nicht OC₂H₅/OC₂H₅ oder Cl/Cl ist,
4) im Falle n = 1, R² = R³ = H und X' = COOH, COONa oder COOCH₃ das Paar R²⁰/R²¹ nicht H/F, H/Cl, H/Br, H/I, H/CH₃, H/OCH₃, H/SC₂H₅, Cl/SC₂H₅, SC₂H₅/SC₂H₅, Benzyloxy/CH₃, OCH₃/CH₃, OC₂H₅/CH₃, Cl/Cl, CI/OCH₃, OCH₃/OCH₃ oder CI/CH₃ ist,
5) im Falle n = 1, R² = R³ = H und X' = CO₂C₂H₅ das Paar R²⁰/R²¹ nicht CI/CH₃ oder CH₃/CH₃ ist,
**dadurch gekennzeichnet, daß** man eine Verbindung der Formel (XI) 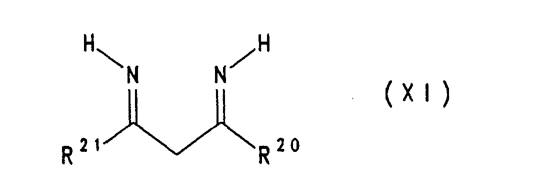 mit einer Verbindung der Formel (XII) oder (XIII) 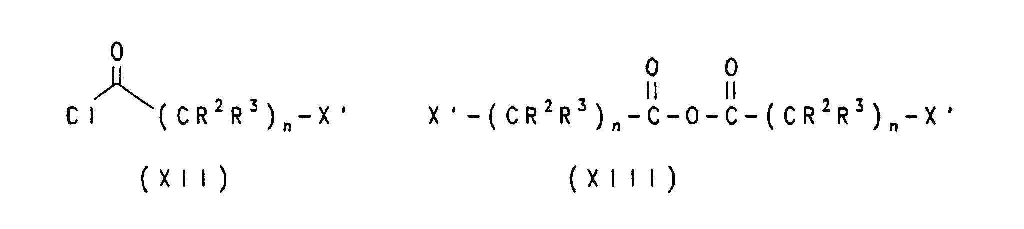 umsetzt,
wobei in den Formeln (XI), (XII) und (XIII) die Reste
R², R³, R²⁰, R²¹ und X' sowie n wie in Formel (II') definiert sind.

5. Verfahren nach Anspruch 4, **dadurch gekennzeichnet, daß** man die Umsetzung in einem unter den Reaktionsbedingungen inerten Solvens in Gegenwart einer Base bei einer Temperatur von -78 °C bis 100 °C durchführt.

6. Herbizide oder pflanzenwachstumsregulierende Mittel, **dadurch gekennzeichnet, daß** sie eine Verbindung der Formel (I) von Anspruch 1 oder 2 oder deren Salz und inerte Zusatzstoffe enthalten.

7. Verfahren zur Bekämpfung von unerwünschten Pflanzen oder zur Wachstumsregulierung von Pflanzen, **dadurch gekennzeichnet, daß** man auf diese oder die Kulturböden eine wirksame Menge einer Verbindung der Formel (I) oder deren Salz von Anspruch 1 oder 2 appliziert.

## Claims (Claims for the following Contracting State(s): CH, DE, FR, GB, IT, LI)

1. A compound of the formula I or a salt thereof 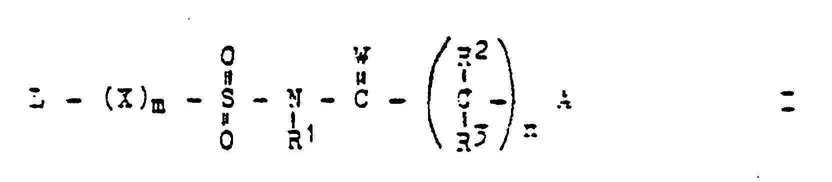 where
R¹ is hydrogen, (C₁-C₄)alkyl, (C₂-C₆)-alkenyl or (C₂-C₆)-alkynyl;
R² and R³ independently of one another are hydrogen, (C₁-C₃)-alkyl or phenyl;
W is O, S, NR⁴ or NOR⁴,
R⁴ is hydrogen, (C₁-C₃)-alkyl, (C₁-C₃)-haloalkyl or phenyl;
X is CHR², O, NR⁴ or NOR⁴,
L is a heterocyclic or isocyclic radical of the formulae (L1) - (L5), 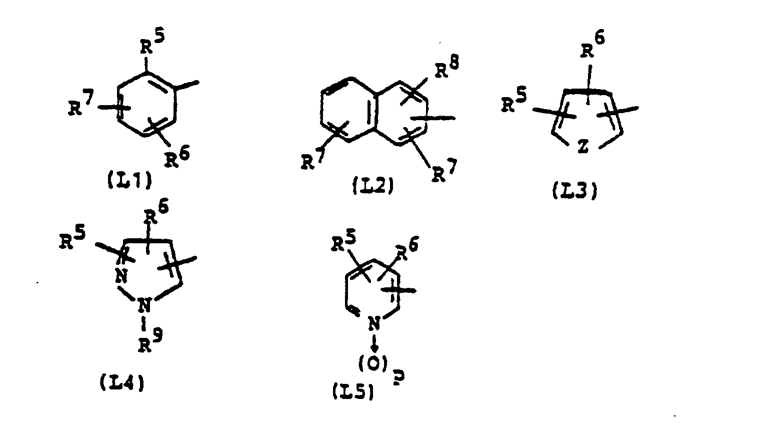
A is a heterocyclic radical of the formulae (A1) - (A8) 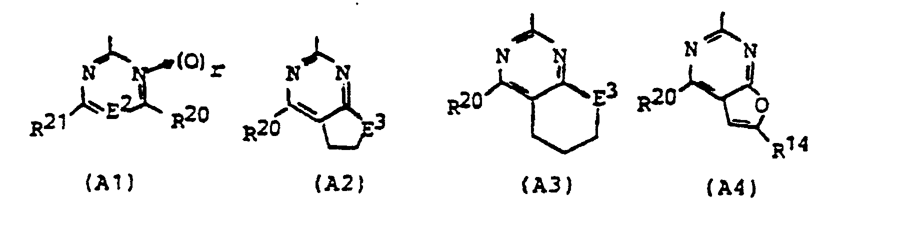 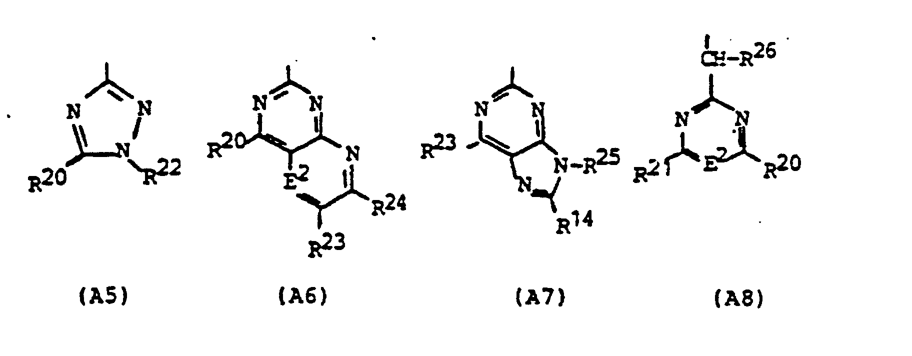
Z is O or S(O)_{q},
E¹ is O or S(O)_{b},
E² is CH or N,
E³ is O or CH₂,
a, m, n, p, r and s independently of one another are 0 or 1,
b and q independently of one another are 0, 1 or 2,
R⁵ is hydrogen, halogen, NO₂, CN, (C₁-C₄)-alkyl which is unsubstituted or monosubstituted or polysubstituted by F, Cl or Br or monosubstituted by CN, OCH₃ or SCH₃, or is (C₂-C₄)-alkenyl which is unsubstituted or monosubstituted or polysubstituted by F, Cl or Br or monosubstituted by OCH₃; or is (C₂-C₄)-alkynyl which is unsubstituted or monosubstituted or polysubstituted by F, Cl or Br or monosubstituted by OCH₃ or Si(CH₃)₃; or is (C₃-C₆)-cycloalkyl which is unsubstituted or monosubstituted or polysubstituted by F, Cl or CH₃; or is -C(O)R¹⁰, -OCH₂CH₂OR¹⁰, -OH, -C(R¹⁰)-(OR¹¹)(OR¹²); -CO₂R¹³, -C(O)NR¹⁴R¹⁵, -N₃, -SO₂NR¹⁴R¹⁵, -SO₃R¹⁶, -OSO₂R¹⁷, phenyl which is unsubstituted or monosubstituted or polysubstituted by F, Cl, Br, CH₃ or OCH₃; or is -E¹R¹⁸ or -(CH₂)ₛG,
R⁶ is hydrogen or halogen; CN; NO₂; (C₁-C₄)-alkyl which is unsubstituted or monosubstituted or polysubstituted by halogen or monosubstituted to disubstituted by -CO₂R¹³, -SO₂NR¹⁴R¹⁵, -NR¹⁴R¹⁵, (C₁-C₂)-alkoxy, -E¹R¹⁹, (C₁-C₂)-haloalkoxy, (C₁-C₂)-alkylthio, (C₁-C₂)-haloalkylthio, CN, OH or SH; or is -CO₂R¹³, -SO₂NR¹⁴R¹⁵, -NR¹⁴R¹⁵ or -E¹R¹⁹;
R⁷ radicals independently of one another are hydrogen, (C₁-C₄)-alkyl, (C₁-C₄)-haloalkyl, (C₂-C₄)-alkenyl, phenyl or phenyl which is monosubstituted or polysubstituted by halogen or -E¹R¹⁹; or is -E¹R¹⁹ or halogen,
R⁸ is hydrogen, (C₁-C₄)-alkyl, (C₁-C₄)-haloalkyl, halogen, -CO₂R¹³, -SO₂NR¹⁴R¹⁵, -OSO₂R¹⁷, -S(O)_{b}R¹⁸, CN or NO₂,
R⁹ is hydrogen, (C₁-C₄)-alkyl which is unsubstituted or monosubstituted or polysubstituted by halogen or monosubstituted by phenyl; or is (C₂-C₄)-alkenyl; or is phenyl or phenyl which is monosubstituted or polysubstituted by halogen, (C₁-C₄)-alkyl, NO₂, CN or (C₁-C₄)-alkoxy,
R¹⁰ is hydrogen, (C₁-C₄)-alkyl, (C₁-C₄)-alkyl which is monosubstituted or polysubstituted by F, Cl, Br or OCH₃; or is (C₃-C₆)-cycloalkyl which is unsubstituted or monosubstituted or poly-substituted by F, Cl, Br or CH₃; or is (C₂-C₄)-alkenyl or (C₂-C₄)-alkynyl;
R¹¹ and R¹² independently of one another are (C₁-C₄)-alkyl, or R¹¹ and R¹² together are -CH₂CH₂-, -CH₂OCH₂- or -CH₂C(CH₃)₂CH₂-;
R¹³ is hydrogen, (C₁-C₄)-alkyl which is unsubstituted or monosubstituted or polysubstituted by halogen or monosubstituted to disubstituted by CN, CO₂R¹⁰, NR¹⁴R¹⁵, OR¹⁰ or Si(CH₃)₃; or is (C₃-C₄)-alkynyl which is unsubstituted or substituted by CH₃ or Si(CH₃)₃; or is (C₃-C₆)-cycloalkyl, (C₃-C₆)-cycloalkyl- (C₁-C₄) alkyl, (C₁-C₄)-alkoxy or Si(CH₃)₃,
R¹⁴ is hydrogen or (C₁-C₄)-alkyl, or R¹⁴ and R¹⁵ together are -(CH₂)₂(CH₂)ₐ(CH₂)₂- or -(CH₂)₂O(CH₂)₂-,
R¹⁵ is hydrogen, (C₁-C₄)-alkyl, (C₁-C₄)-alkoxy, (C₂-C₄)-alkenyl, or R¹⁴ and R¹⁵ together are -(CH₂)₂(CH₂)ₐ(CH₂)₂- or -(CH₂)₂O(CH₂)₂-,
R¹⁶ is (C₁-C₄)-alkyl or (C₁-C₄)-haloalkyl,
R¹⁷ is (C₁-C₄)-alkyl or NR¹⁴R¹⁵,
R¹⁸ is (C₁-C₄)-alkyl, (C₁-C₄)-haloalkyl, (C₂-C₄)-alkoxyalkyl, (C₂-C₄)-alkenyl, (C₃-C₄)-alkynyl, phenyl or phenyl which is monosubstituted or polysubstituted by halogen, (C₁-C₃)-alkyl, (C₁-C₃)-alkoxy or (C₁-C₃)-haloalkyl;
R¹⁹ is (C₁-C₄)-alkyl or (C₁-C₄)alkyl which is monosubstituted or polysubstituted by F or Cl or monosubstituted by OR¹⁶,
R²⁰ and R²¹ independently of one another are hydrogen, halogen, (C₁-C₆)-alkyl, (C₁-C₆)-alkoxy or (C₁-C₆)-alkylthio, it being possible for the three abovementioned radicals to be monosubstituted or polysubstituted by halogen or monosubstituted or disubstituted by (C₁-C₄)-alkoxy or (C₁-C₄)-alkylthio; or are -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy;
R²² is (C₁-C₄)-alkyl or (C₁-C₄)-haloalkyl,
R²³ and R²⁴ independently of one another are hydrogen, (C₁-C₄)-alkyl, (C₂-C₄)-alkenyl or (C₂-C₄)-alkynyl;
R²⁵ is hydrogen, (C₁-C₄)-alkyl or (C₁-C₄)-haloalkyl,
R²⁶ is hydrogen or (C₁-C₃)-alkyl,
G is a heterocyclic radical (G 1) - (G 25), 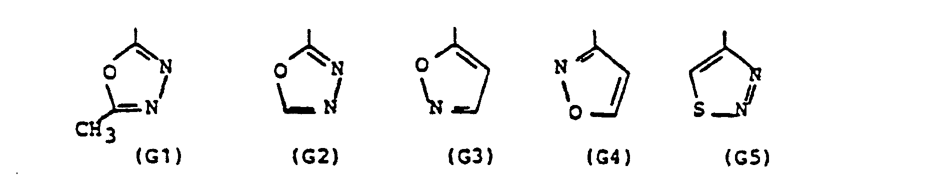 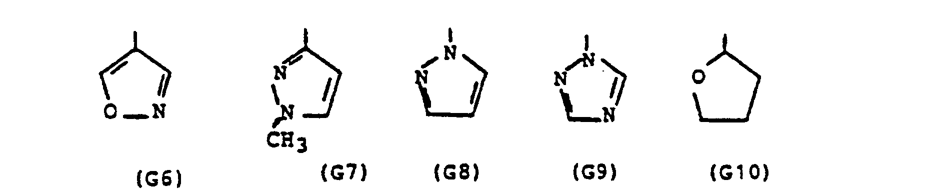 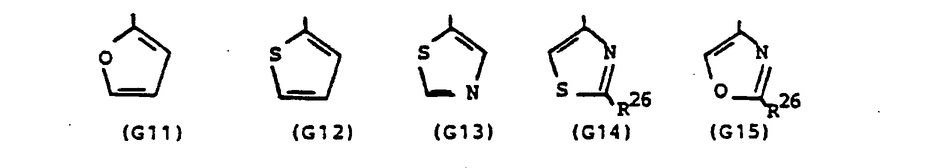 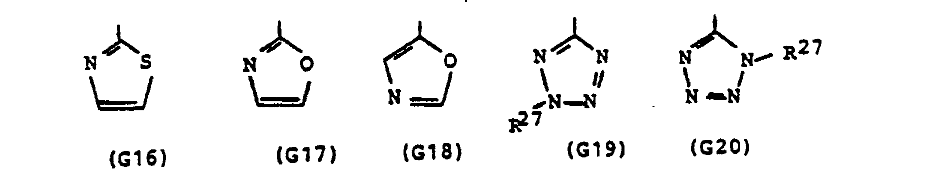 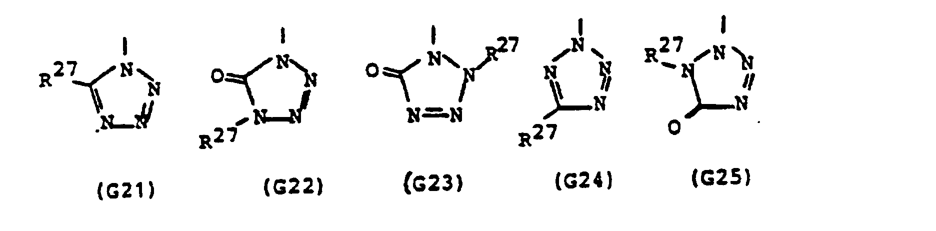
R²⁷ is hydrogen, (C₁-C₃)-alkyl or (C₂-C₄)-alkenyl.

2. A compound of the formula I of claim 1 or a salt thereof, where
A is a radical of the formulae (A1), (A2), (A3) or (A4), in particular (A1);
L is a radical of the formulae (L1), (L3), (L4) or (L5),
X is CH₂, CH(CH₃), O, NH, NCH₃, NC₂H₅ or NOCH₃, in particular CH₂, O or NH,
W is oxygen,
R¹ is hydrogen,
R² and R³ independently of one another are hydrogen or (C₁-C₃)alkyl, in particular hydrogen,
R⁵ is halogen, NO₂, CN, (C₁-C₃)-alkyl which is unsubstituted or substituted by F, Cl, Br, CN, OCH₃ or SCH₃; or is (C₃)-alkenyl which is unsubstituted or substituted by F, Cl or Br; or is (C₃)-alkynyl, (C₃)-cycloalkyl, which is unsubstituted or substituted by F, Cl or CH₃, or is -C(O)R¹⁰, -OCH₂CH₂OR¹⁰, OH, -C(R¹⁰)(OR¹¹)(OR¹²), -CO₂R¹³, -C(O)NR¹⁴R¹⁵, N₃, -SO₂NR¹⁴R¹⁵, -OSO₂R¹⁷, -E¹R¹⁸ or -(CH₂)ₛG;
R⁶ is hydrogen, halogen, CN, NO₂, CH₃, CF₃, -E¹R¹⁹ or (C₁-C₂)-alkoxy-(C₁-C₂)-alkyl, (C₁-C₂)-haloalkoxy, (C₁-C₂)-alkylthio, (C₁-C₂)-haloalkylthio, CN, -CO₂R¹³ or -SO₂NR¹⁴R¹⁵,
R⁷ is hydrogen,
R¹⁰ is (C₁-C₃)-alkyl, cyclopropyl or (C₃)-alkenyl,
R¹¹ and R¹² are (C₁-C₂)-alkyl, or R¹¹ and R¹² together are -CH₂CH₂-,
R¹³ is (C₁-C₃)-alkyl, (C₃)-alkenyl, -CH₂CH₂F, -CH₂CH₂Cl, -CH₂CH₂OCH₃ or cyclopropylmethyl;
R¹⁴ is hydrogen or CH₃, or R¹⁴ and R¹⁵ together are -(CH₂)₄-, -(CH₂)₅- or -(CH₂)₂O(CH₂)₂-,
R¹⁵ is CH₃, CH₂CH₃ or OCH₃, or R¹⁴ and R¹⁵ together are -(CH₂)₄-, -(CH₂)₅- or -(CH₂)₂O(CH₂)₂-,
R¹⁸ is (C₁-C₃)-alkyl, (C₁-C₃)-haloalkyl, (C₂-C₃)-alkoxyalkyl, allyl, propargyl or (C₂-C₃)-haloalkenyl.
R¹⁹ is (C₁-C₂)-alkyl which is unsubstituted or substituted by F, Cl or OCH₃,
R²⁰ and R²¹ independently of one another are hydrogen, (C₁-C₂)-alkyl, (C₁-C₂)-alkoxy, -CH₂OCH₃, Cl, F, Br, I, -CH₂OCH₂CH₃, -NHCH₃, -N(OCH₃)CH₃, -N(CH₃)₂, -CF₃, -SCH₃, -CH(OCH₃)₂, -OCH₂CH=CH₂; -OCH₂C≡CH, -OCH₂CH₂OCH₃, -CH₂SCH₃, -OCHF₂, -SCHF₂, cyclopropyl, -C≡CH or -C≡C-CH₃,
m is 0 or 1,
n is 0 or 1,
s is zero and
E¹ is O or S.

3. A process for the preparation of the compounds of the formula I as claimed in claim 1 or 2 or of a salt thereof, which comprises
a) for the preparation of a compound with W = O,
a₁) reacting a compound of the formula II with 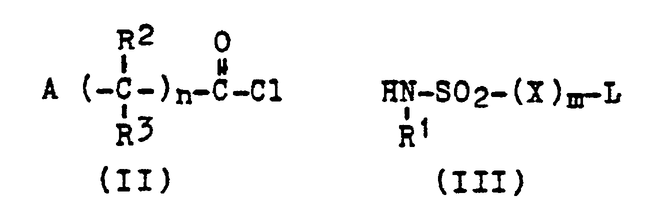 a compound of the formula III in the presence of a base, or
a₂) reacting a compound of the formula (IIa) 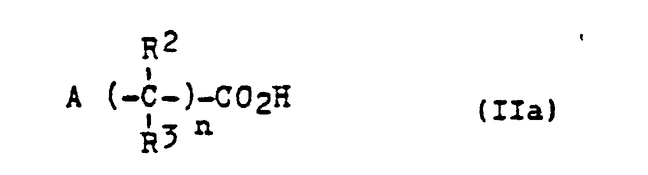 with a compound of the formula III in the presence of activating reagents, such as 2-chloro-1-methylpyridinium chloride (IVa), dicyclohexylcarbodiimide (IVb) or 1,1-carbonyldiimidazole (IVc) and if appropriate in the presence of a base, or
a₃) for the preparation of a compound with W = O and R¹ = H, reacting a compound of the formula (V), 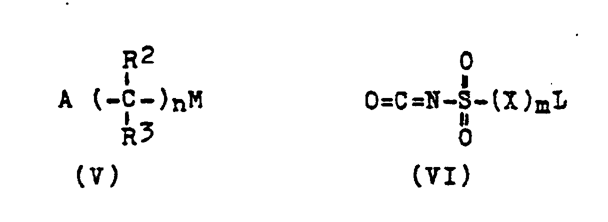 where M is hydrogen or lithium, with a compound of the formula (VI), or
b) for the preparation of a compound with W = S, reacting a compound of the formula I obtained in a) with the compound of the formula VII, 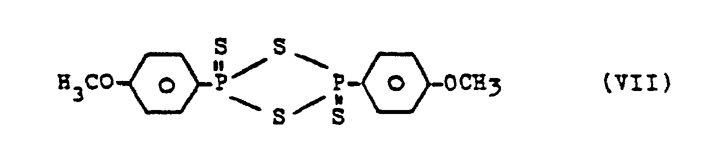
c) for the preparation of a compound with W = NR⁴, reacting a compound of the formula VIII 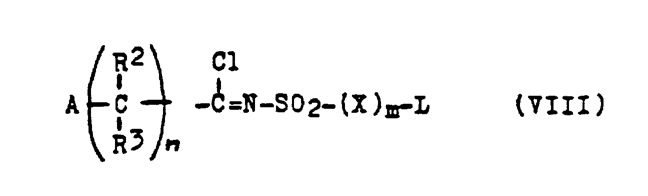 with an amine of the formula H₂N-R⁴,
d) for the preparation of a compound with W = NOR⁴,
d₁) reacting a compound of the formula IX with 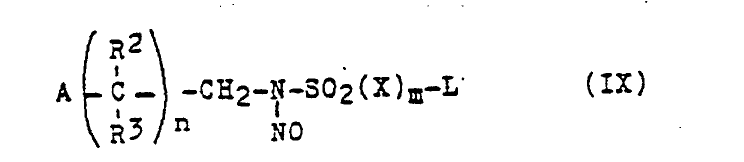 an alkali metal hydroxide or alkaline earth metal hydroxide or ammonium hydroxide, or
d₂) reacting a compound of the formula VIII with a hydroxylamine of the formula H₂N-OR⁴ in the presence of a base, or
e) for the preparation of a compound of the formula I with R¹ ≠ H, reacting a compound of the formula I with R¹ = H with an alkyl halide of the formula R^{1'}-X¹ in the presence of a base, where R^{1'} has the meaning indicated for R¹ with the exception of hydrogen and X¹ is chlorine, bromine or iodine, and, if appropriate, converting the compound obtained into its salt.

4. A compound of the formula (II') 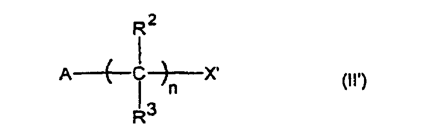 where
X' is COCl, CN, F, p-tosyl, tetraalkylammonium, N₃, CO₂R¹³, CO₂CH₂C₆H₅ or E¹R¹⁸,
n is 0 or 1,
R² and R³ independently of one another are hydrogen, (C₁-C₃)-alkyl or phenyl,
A is a heterocyclic radical of the formula (A1) where r = 1 or (A2), (A3), (A4) or (A5), where in the case of (A5) n = 1, or (A6), (A7) or (A8), 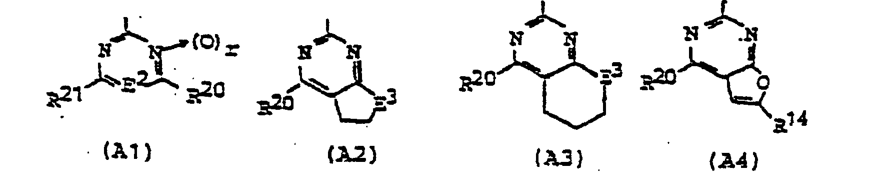 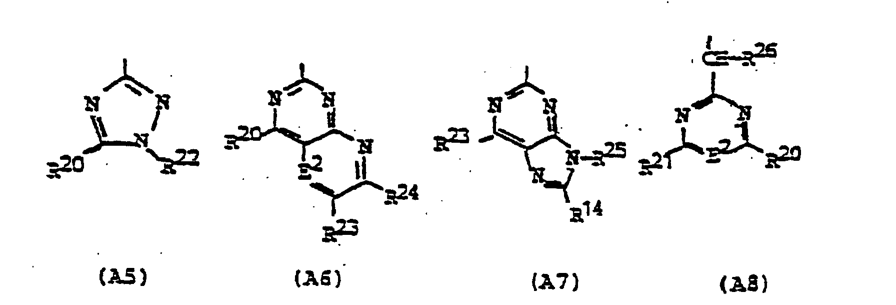 and
E¹ is O or S(O)_{b},
E² is CH or N,
E³ is O or CH₂,
a is 0 or 1,
b is 0, 1 or 2,
R¹⁰ is hydrogen, (C₁-C₄)-alkyl, (C₁-C₄)-alkyl which is monosubstituted or polysubstituted by F, Cl, Br or OCH₃, or is (C₃-C₆)-cycloalkyl which is unsubstituted or monosubstituted or polysubstituted by F, Cl, Br, or is (C₂-C₄)-alkenyl or (C₂-C₄)-alkynyl,
R¹³ is hydrogen, (C₁-C₄)-alkyl which is unsubstituted or monosubstituted or polysubstituted by halogen or monosubstituted or disubstituted by CN, CO₂R¹⁰, NR¹⁴R¹⁵, OR¹⁰ or Si(CH₃)₃, or is (C₃-C₄)-alkynyl which is unsubstituted or substituted by CH₃ or Si(CH₃)₃, or is (C₃-C₆)-cycloalkyl, (C₃-C₆)-cycloalkyl-(C₁-C₄)-alkyl, (C₁-C₄)-alkoxy or Si(CH₃)₃,
R¹⁴ is hydrogen or (C₁-C₄)-alkyl or R¹⁴ and R¹⁵ together are -(CH₂)₂(CH₂)ₐ(CH₂)₂- or -(CH₂)₂O(CH₂)₂-,
R¹⁵ is hydrogen, (C₁-C₄)-alkyl, (C₁-C₄)-alkoxy, (C₂-C₄)-alkenyl or R¹⁴ and R¹⁵ together are -(CH₂)₂(CH₂)ₐ(CH₂)₂- or -(CH₂)₂O(CH₂)₂-,
R¹⁸ is (C₁-C₄)-alkyl, (C₁-C₄)-haloalkyl, (C₂-C₄)-alkoxyalkyl, (C₂-C₄)-alkenyl, (C₃-C₄)-alkynyl, phenyl or phenyl which is monosubstituted or polysubstituted by halogen, or is (C₁-C₃)-alkyl, (C₁-C₃)-alkoxy or (C₁-C₃)-haloalkyl,
R²⁰, R²¹ independently of one another are hydrogen, halogen, (C₁-C₆)-alkyl, (C₁-C₆)-alkoxy or (C₁-C₆)-alkylthio, where the three above-mentioned radicals can be monosubstituted or polysubstituted by halogen or monosubstituted or disubstituted by (C₁-C₄)-alkoxy or (C₁-C₄)-alkylthio, or are -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy,
R²² is (C₁-C₄)-alkyl or (C₁-C₄)-haloalkyl,
R²³, R²⁴ independently of one another are hydrogen, (C₁-C₄)-alkyl, (C₂-C₄)-alkenyl or (C₂-C₄)-alkynyl,
R²⁵ is hydrogen, (C₁-C₄)-alkyl or (C₁-C₄)-haloalkyl and
R²⁶ is hydrogen or (C₁-C₃)-alkyl.

5. A compound of the formula (II') 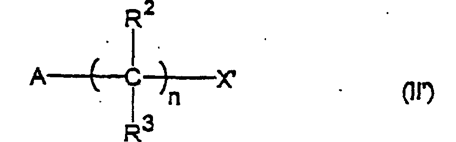 where
X' is COCl, CO₂R¹³ or CO₂CH₂C₆H₅,
n is 0 or 1,
R² and R³ independently of one another are hydrogen, (C₁-C₃)-alkyl or phenyl,
A is a heterocyclic radical of the formula (A1) where r = 0 and E² = CH, 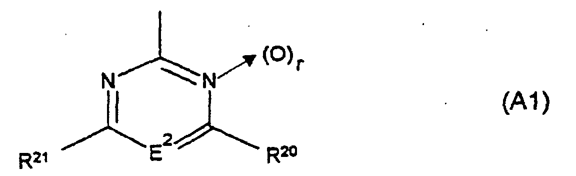 and
a is 0 or 1,
R¹⁰ is hydrogen, (C₁-C₄)-alkyl, (C₁-C₄)-alkyl which is monosubstituted or polysubstituted by F, Cl, Br or OCH₃, or is (C₃-C₆)-cycloalkyl which is unsubstituted or monosubstituted or polysubstituted by F, Cl, Br or, or is (C₂-C₄)-alkenyl or (C₂-C₄)-alkynyl,
R¹³ is hydrogen, (C₁-C₄)-alkyl which is unsubstituted or monosubstituted or polysubstituted by halogen or monosubstituted or disubstituted by CN, CO₂R¹⁰, NR¹⁴R¹⁵, OR¹⁰ or Si(CH₃)₃, or is (C₃-C₄)-alkynyl which is unsubstituted or substituted by CH₃ or Si(CH₃)₃, or is (C₃-C₆)-cycloalkyl, (C₃-C₆)-cycloalkyl-(C₁-C₄)-alkyl, (C₁-C₄)-alkoxy or Si(CH₃)₃,
R¹⁴ is hydrogen or (C₁-C₄)-alkyl or R¹⁴ and R¹⁵ together are -(CH₂)₂(CH₂)ₐ(CH₂)₂- or -(CH₂)₂O(CH₂)₂-,
R¹⁵ is hydrogen, (C₁-C₄)-alkyl, (C₁-C₄)-alkoxy, (C₂-C₄)-alkenyl or R¹⁴ and R¹⁵ together are -(CH₂)₂(CH₂)ₐ(CH₂)₂- or -(CH₂)₂O(CH₂)₂- and
R²⁰, R²¹ independently of one another are hydrogen, halogen, (C₁-C₆)-alkyl, (C₁-C₆)-alkoxy or (C₁-C₆)-alkylthio where the three abovementioned radicals can be monosubstituted or polysubstituted by halogen or monosubstituted or disubstituted by (C₁-C₄)-alkoxy or (C₁-C₄)-alkylthio, or are -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy,
where
1) in the event that n = 0 and X' = COOH the pair R²⁰/R²¹ is other than CH₃/CH₃,
2) in the event that n = 0 and X' = CO₂CH₃ the pair R²⁰/R²¹ is other than CH₃/CH₃ or OCH₃/CH₃,
3) in the event that n = 0 and X' = CO₂C₂H₅ the pair R²⁰/R²¹ is other than OC₂H₅/OC₂H₅ or Cl/Cl,
4) in the event that n = 1, R² = R³ = H and X' = COOH, COONa or COOCH₃ the pair R²⁰/R²¹ is other than H/F, H/Cl, H/Br, H/I, H/CH₃, H/OCH₃, H/SC₂H₅, Cl/SC₂H₅, SC₂H₅/SC₂H₅, benzyloxy/CH₃, OCH₃CH₃, OC₂H₅/CH₃, Cl/Cl, Cl/OCH₃, OCH₃/OCH₃ or Cl/CH₃,
5) in the event that n = 1, R² = R³ = H and X' = CO₂C₂H₅ the pair R²⁰/R²¹ is other than Cl/CH₃ or CH₃/CH₃.

6. A compound of the formula (II') 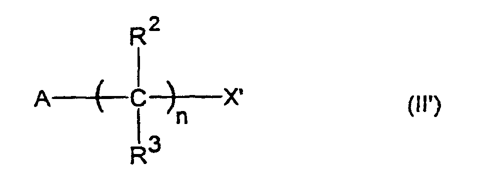 where
X' is COCl, CO₂R¹³ or CO₂CH₂C₆H₅,
n is 0 or 1,
R² and R³ independently of one another are hydrogen, (C₁-C₃)-alkyl or phenyl,
A is a heterocyclic radical of the formula (A1) where r = 0 and E² = N, 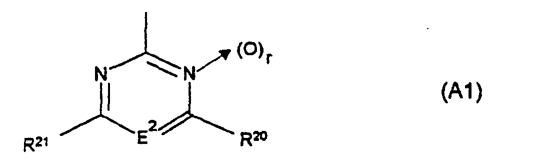 and
a is 0 or 1,
R¹⁰ is hydrogen, (C₁-C₄)-alkyl, (C₁-C₄)-alkyl which is monosubstituted or polysubstituted by F, Cl, Br or OCH₃, or is (C₃-C₆)-cycloalkyl which is unsubstituted or monosubstituted or polysubstituted by F, Cl, Br or, or is (C₂-C₄)-alkenyl or (C₂-C₄)-alkynyl,
R¹³ is hydrogen, (C₁-C₄)-alkyl which is unsubstituted or monosubstituted or polysubstituted by halogen or monosubstituted or disubstituted by CN, CO₂R¹⁰, NR¹⁴R¹⁵, OR¹⁰ or Si(CH₃)₃, or is (C₃-C₄)-alkynyl which is unsubstituted or substituted by CH₃ or Si(CH₃)₃, or is (C₃-C₆)cycloalkyl, (C₃-C₆)-cycloalkyl-(C₁-C₄)-alkyl, (C₁-C₄)-alkoxy or Si(CH₃)₃,
R¹⁴ is hydrogen or (C₁-C₄)-alkyl or R¹⁴ and R¹⁵ together are -(CH₂)₂(CH₂)ₐ(CH₂)₂- or -(CH₂)₂O(CH₂)₂-,
R¹⁵ is hydrogen, (C₁-C₄)-alkyl, (C₁-C₄)-alkoxy, (C₂-C₄)-alkenyl or R¹⁴ and R¹⁵ together are -(CH₂)₂(CH₂)ₐ(CH₂)₂- or -(CH₂)₂O(CH₂)₂)₂- and
R²⁰, R²¹ independently of one another are hydrogen, halogen, (C₁-C₆)-alkyl, (C₁-C₆)-alkoxy or (C₁-C₆)-alkylthio, where the three above-mentioned radicals can be monosubstituted or polysubstituted by halogen or monosubstituted or disubstituted by (C₁-C₄)-alkoxy or (C₁-C₄)-alkylthio, or are -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy,
where
1) in the event that n = 0 and X' = CO₂C₂H₅ the pair R²⁰/R²¹ is other than CH₃/CH₃ or CCl₃/NH₂ or SCH₃/SCH₃,
2) in the event that n = 1, R² = R³ = H and X' = CO₂C₂H₅ the pair R²⁰/R²¹ is other than Cl/OCH₃ or Cl/Cl,
3) in the event that n = 1, R² = R³ = H and X' = CN the pair R²⁰/R²¹ is other than NH₂/NH₂,
4) in the event that n = 0 and X' = COOH the pair R²⁰/R²¹ is other than OCH₃/OCH₃ or SCH₃/SCH₃ and
5) in the event that n = 0 and X' = CO₂CH₃ the pair R²⁰/R²¹ is other than SCH₃/SCH₃.

7. A compound of the formula (II') 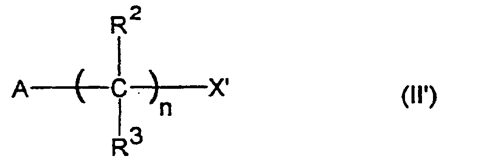 where
X' is COCl, CN, CO₂R¹³ or CO₂CH₂C₆H₅,
n is 0,
R² and R³ independently of one another are hydrogen, (C₁-C₃)-alkyl or phenyl,
A is a heterocyclic radical of the formula (A5), 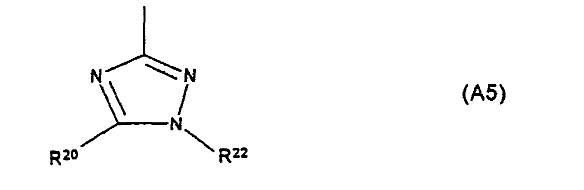 and
a is 0 or 1,
R¹⁰ is hydrogen, (C₁-C₄)-alkyl, (C₁-C₄)-alkyl which is monosubstituted or polysubstituted by F, Cl, Br or OCH₃, or is (C₃-C₆)-cycloalkyl which is unsubstituted or monosubstituted or polysubstituted by F, Cl, Br or, or is (C₂-C₄)-alkenyl or (C₂-C₄)-alkynyl,
R¹³ is hydrogen, (C₁-C₄)-alkyl which is unsubstituted or monosubstituted or polysubstituted by halogen or monosubstituted or disubstituted by CN, CO₂R¹⁰, NR¹⁴R¹⁵, OR¹⁰ or Si(CH₃)₃, or is (C₃-C₄)-alkynyl which is unsubstituted or substituted by CH₃ or Si(CH₃)₃, or is (C₃-C₆)-cycloalkyl, (C₃-C₆)-cycloalkyl-(C₁-C₄)-alkyl, (C₁-C₄)-alkoxy or Si(CH₃)₃,
R¹⁴ is hydrogen or (C₁-C₄)-alkyl or R¹⁴ and R¹⁵ together are -(CH₂)₂(CH₂)ₐ(CH₂)₂- or - (CH₂)₂O(CH₂)₂-,
R¹⁵ is hydrogen, (C₁-C₄)-alkyl, (C₁-C₄)-alkoxy, (C₂-C₄)-alkenyl or R¹⁴ and R¹⁵ together are -(CH₂)₂(CH₂)ₐ(CH₂)₂- or -(CH₂)₂O(CH₂)₂-,
R²⁰ is hydrogen, halogen, (C₁-C₆)-alkyl, (C₁-C₆)-alkoxy or (C₁-C₆)-alkylthio, where the three abovementioned radicals can be monosubstituted or polysubstituted by halogen or monosubstituted or disubstituted by (C₁-C₄)-alkoxy or (C₁-C₄)-alkylthio, or is -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy and
R²² is (C₁-C₄)-alkyl or (C₁-C₄)-haloalkyl,
where
in the event that n = 0, X' = CO₂CH₃ or CO₂C₂H₅ and R²⁰ = H the radical R²² is other than CH₃.

8. A process for the preparation of the compounds of the formula (II') as claimed in claim 5, wherein a compound of the formula (XI) 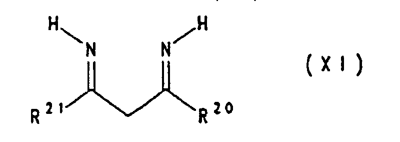 is reacted with a compound of the formula (XII) or (XIII) 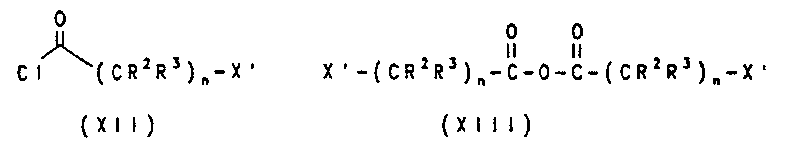 where, in formulae (XI), (XII) and (XIII), the radicals R², R³, R²⁰, R²¹ and X' and n are as defined in formula (II').

9. A process as claimed in claim 8, wherein the reaction is carried out at a temperature of from -78°C to 100°C in a solvent which is inert under the reaction conditions in the presence of a base.

10. A herbicidal or plant growth-regulating agent, containing a compound of the formula (I) of claim 1 or 2 or a salt thereof and inert additives.

11. Use of a compound of the formula (I) of claim 1 or 2 or a salt thereof as a herbicide or plant growth-regulator.

12. A method of controlling undesired plants or for regulating the growth of plants, which comprises applying an effective amount of a compound of the formula (I) or a salt thereof, of claim 1 or 2, to these plants or the cropping areas.

## Claims (Claims for the following Contracting State(s): ES)

1. The use of a compound of the formula I or a salt thereof 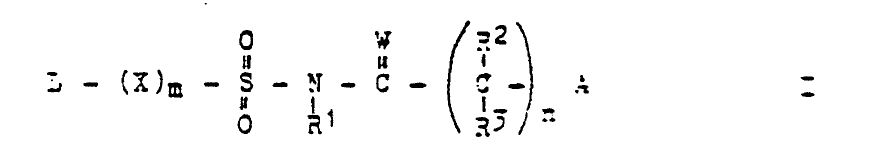 where
R¹ is hydrogen, (C₁-C₄)alkyl, (C₂-C₆)-alkenyl or (C₂-C₆)-alkynyl;
R² and R³ independently of one another are hydrogen, (C₁-C₃)-alkyl or phenyl;
W is O, S, NR⁴ or NOR⁴,
R⁴ is hydrogen, (C₁-C₃)-alkyl, (C₁-C₃)-haloalkyl or phenyl;
X is CHR², O, NR⁴ or NOR⁴,
L is a heterocyclic or isocyclic radical of the formulae (L1) - (L5), 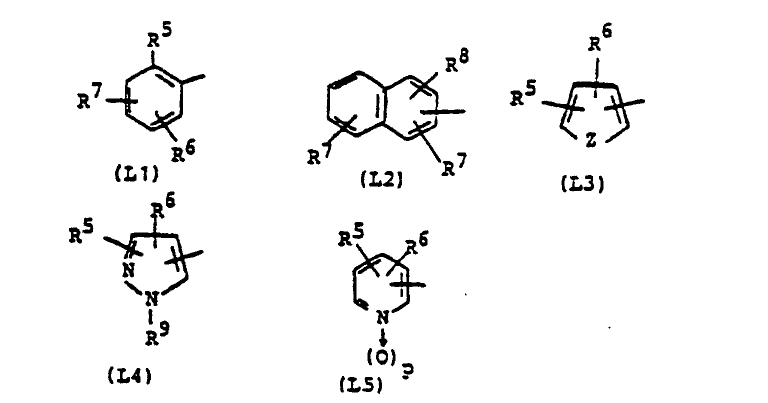
A is a heterocyclic radical of the formulae (A1) - (A8) 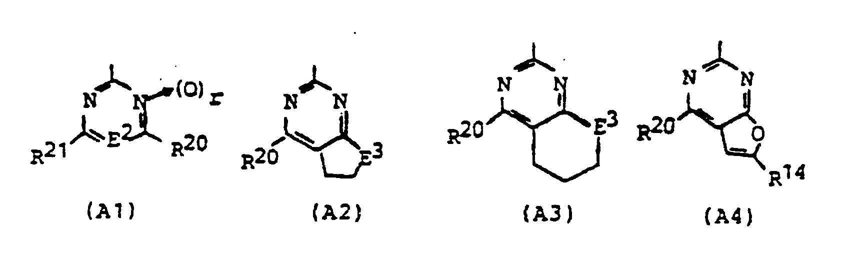 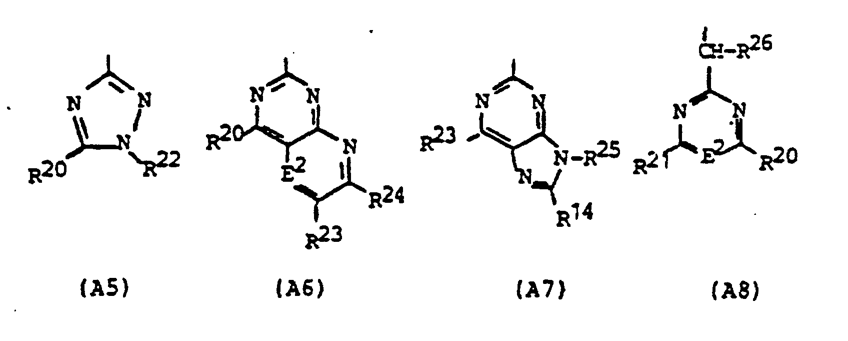
Z is O or S(O)_{q},
E¹ is O or S(O)_{b},
E² is CH or N,
E³ is O or CH₂,
a, m, n, p, r and s independently of one another are 0 or 1,
b and q independently of one another are 0, 1 or 2,
R⁵ is hydrogen, halogen, NO₂, CN, (C₁-C₄)-alkyl which is unsubstituted or monosubstituted or polysubstituted by F, Cl or Br or monosubstituted by CN, OCH₃ or SCH₃, or is (C₂-C₄)-alkenyl which is unsubstituted or monosubstituted or polysubstituted by F, Cl or Br or monosubstituted by OCH₃; or is (C₂-C₄)-alkynyl which is unsubstituted or monosubstituted or polysubstituted by F, Cl or Br or monosubstituted by OCH₃ or Si(CH₃)₃; or is (C₃-C₆)-cycloalkyl which is unsubstituted or monosubstituted or polysubstituted by F, Cl or CH₃; or is -C(O)R¹⁰, -OCH₂CH₂OR¹⁰, -OH, -C(R¹⁰)-(OR¹¹)(OR¹²); -CO₂R¹³, -C(O)NR¹⁴R¹⁵, -N₃, -SO₂NR¹⁴R¹⁵, -SO₃R¹⁶, -OSO₂R¹⁷, phenyl which is unsubstituted or monosubstituted or polysubstituted by F, Cl, Br, CH₃ or OCH₃; or is -E¹R¹⁸ or -(CH₂)ₛG,
R⁶ is hydrogen or halogen; CN; NO₂; (C₁-C₄)-alkyl which is unsubstituted or monosubstituted or polysubstituted by halogen or monosubstituted to disubstituted by -CO₂R¹³, -SO₂NR¹⁴R¹⁵, -NR¹⁴R¹⁵, (C₁-C₂)-alkoxy, -E¹R¹⁹, (C₁-C₂)-haloalkoxy, (C₁-C₂)-alkylthio, (C₁-C₂)-haloalkylthio, CN, OH or SH; or is -CO₂R¹³, -SO₂NR¹⁴R¹⁵, -NR¹⁴R¹⁵ or -E¹R¹⁹;
R⁷ radicals independently of one another are hydrogen, (C₁-C₄)-alkyl, (C₁-C₄)-haloalkyl, (C₂-C₄)-alkenyl, phenyl or phenyl which is monosubstituted or polysubstituted by halogen or -E¹R¹⁹; or is -E¹R¹⁹ or halogen,
R⁸ is hydrogen, (C₁-C₄)-alkyl, (C₁-C₄)-haloalkyl, halogen, -CO₂R¹³, -SO₂NR¹⁴R¹⁵, -OSO₂R¹⁷, -S(O)_{b}R¹⁸, CN or NO₂,
R⁹ is hydrogen, (C₁-C₄)-alkyl which is unsubstituted or monosubstituted or polysubstituted by halogen or monosubstituted by phenyl; or is (C₂-C₄)-alkenyl; or is phenyl or phenyl which is monosubstituted or polysubstituted by halogen, (C₁-C₄)-alkyl, NO₂, CN or (C₁-C₄)-alkoxy,
R¹⁰ is hydrogen, (C₁-C₄)-alkyl, (C₁-C₄)-alkyl which is monosubstituted or polysubstituted by F, Cl, Br or OCH₃; or is (C₃-C₆)-cycloalkyl which is unsubstituted or monosubstituted or polysubstituted by F, Cl, Br or CH₃; or is (C₂-C₄)-alkenyl or (C₂-C₄)-alkynyl;
R¹¹ and R¹² independently of one another are (C₁-C₄)-alkyl, or R¹¹ and R¹² together are -CH₂CH₂-, -CH₂OCH₂- or -CH₂C (CH₃)₂CH₂-;
R¹³ is hydrogen, (C₁-C₄)-alkyl which is unsubstituted or monosubstituted or polysubstituted by halogen or monosubstituted to disubstituted by ON, CO₂R¹⁰, NR¹⁴R¹⁵, OR¹⁰ or Si(CH₃)₃; or is (C₃-C₄)-alkynyl which is unsubstituted or substituted by CH₃ or Si(CH₃)₃; or is (C₃-C₆)-cycloalkyl, (C₃-C₆)-cycloalkyl-(C₁-C₄)alkyl, (C₁-C₄)-alkoxy or Si(CH₃)₃,
R¹⁴ is hydrogen or (C₁-C₄)-alkyl, or R¹⁴ and R¹⁵ together are -(CH₂)₂(CH₂)ₐ(CH₂)₂- or - (CH₂)₂O(CH₂)₂-,
R¹⁵ is hydrogen, (C₁-C₄)-alkyl, (C₁-C₄)-alkoxy, (C₂-C₄)-alkenyl, or R¹⁴ and R¹⁵ together are -(CH₂)₂(CH₂)ₐ(CH₂)₂- or -(CH₂)₂O(CH₂)₂-,
R¹⁶ is (C₁-C₄)-alkyl or (C₁-C₄)-haloalkyl,
R¹⁷ is (C₁-C₄)-alkyl or NR¹⁴R¹⁵,
R¹⁸ is (C₁-C₄)-alkyl, (C₁-C₄)-haloalkyl, (C₂-C₄)-alkoxyalkyl, (C₂-C₄)-alkenyl, (C₃-C₄)-alkynyl, phenyl or phenyl which is monosubstituted or polysubstituted by halogen, (C₁-C₃)-alkyl, (C₁-C₃)-alkoxy or (C₁-C₃)-haloalkyl;
R¹⁹ is (C₁-C₄)-alkyl or (C₁-C₄)alkyl which is monosubstituted or polysubstituted by F or Cl or monosubstituted by OR¹⁶,
R²⁰ and R²¹ independently of one another are hydrogen, halogen, (C₁-C₆)-alkyl, (C₁-C₆)-alkoxy or (C₁-C₆)-alkylthio, it being possible for the three abovementioned radicals to be monosubstituted or polysubstituted by halogen or monosubstituted or disubstituted by (C₁-C₄)-alkoxy or (C₁-C₄)-alkylthio; or are -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy;
R²² is (C₁-C₄)-alkyl or (C₁-C₄)-haloalkyl,
R²³ and R²⁴ independently of one another are hydrogen, (C₁-C₄)-alkyl, (C₂-C₄)-alkenyl or (C₂-C₄)-alkynyl;
R²⁵ is hydrogen, (C₁-C₄)-alkyl or (C₁-C₄)-haloalkyl,
R²⁶ is hydrogen or (C₁-C₃)-alkyl,
G is a heterocyclic radical (G 1) - (G 25), 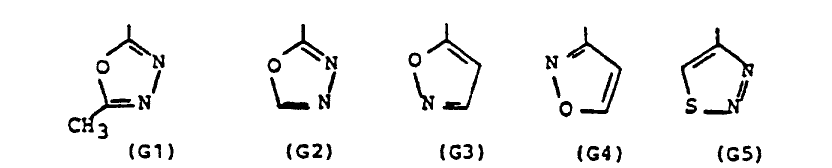 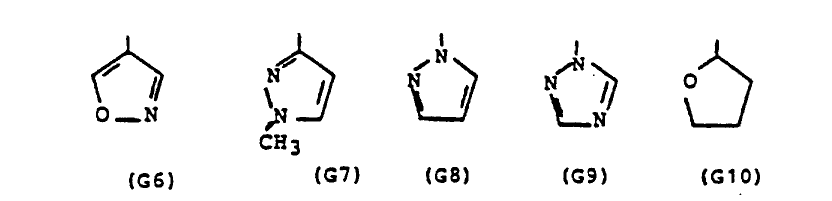 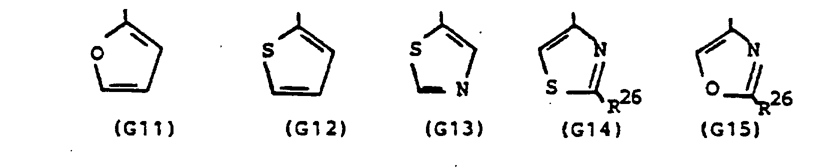 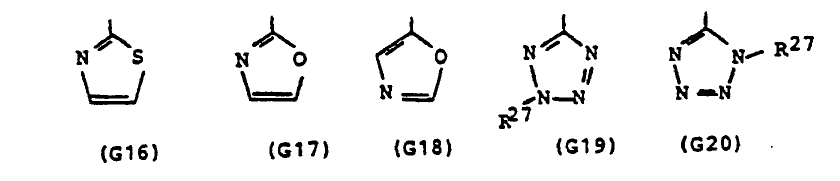 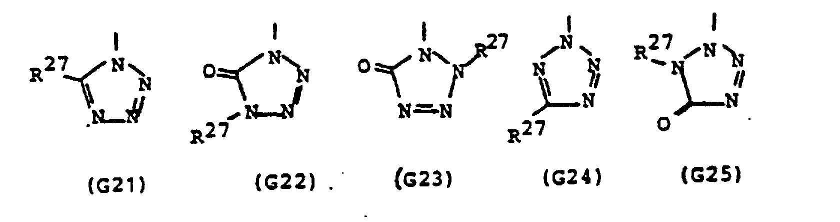
R²⁷ is hydrogen, (C₁-C₃)-alkyl or (C₂-C₄)-alkenyl; as herbicides or plant-growth regulators.

2. The use as claimed in claim 1, wherein in formula (I)
A is a radical of the formulae (A1), (A2), (A3) or (A4), in particular (A1);
L is a radical of the formulae (L1), (L3), (L4) or (L5),
X is CH₂, CH(CH₃), O, NH, NCH₃, NC₂H₅ or NOCH₃, in particular CH₂, O or NH,
W is oxygen,
R¹ is hydrogen,
R² and R³ independently of one another are hydrogen or (C₁-C₃)alkyl, in particular hydrogen,
R⁵ is halogen, NO₂, CN, (C₁-C₃)-alkyl which is unsubstituted or substituted by F, Cl, Br, CN, OCH₃ or SCH₃; or is (C₃)-alkenyl which is unsubstituted or substituted by F, Cl or Br; or is (C₃)-alkynyl, (C₃)-cycloalkyl, which is unsubstituted or substituted by F, Cl or CH₃, or is -C(O)R¹⁰, -OCH₂CH₂OR¹⁰, OH, -C(R¹⁰)(OR¹¹)(OR¹²), -CO₂R¹³, -C(O)NR¹⁴R¹⁵, N₃, -SO₂NR¹⁴R¹⁵, -OSO₂R¹⁷, -E¹R¹⁸ or -(CH₂)ₛG;
R⁶ is hydrogen, halogen, CN, NO₂, CH₃, CF₃, -E¹R¹⁹ or (C₁-C₂)-alkoxy-(C₁-C₂)-alkyl, (C₁-C₂)-haloalkoxy, (C₁-C₂)-alkylthio, (C₁-C₂)-haloalkylthio, CN, -CO₂R¹³ or -SO₂NR¹⁴R¹⁵,
R⁷ is hydrogen,
R¹⁰ is (C₁-C₃)-alkyl, cyclopropyl or (C₃)-alkenyl,
R¹¹ and R¹² are (C₁-C₂)-alkyl, or R¹¹ and R¹² together are -CH₂CH₂-,
R¹³ is (C₁-C₃)-alkyl, (C₃)-alkenyl, -CH₂CH₂F, -CH₂CH₂Cl, -CH₂CH₂OCH₃ or cyclopropylmethyl;
R¹⁴ is hydrogen or CH₃, or R¹⁴ and R¹⁵ together are -(CH₂)₄-, -(CH₂)₅- or -(CH₂)₂O(CH₂)₂-,
R¹⁵ is CH₃, CH₂CH₃ or OCH₃, or R¹⁴ and R¹⁵ together are-(CH₂)₄-, -(CH₂)₅- or -(CH₂)₂O(CH₂)₂-,
R¹⁸ is (C₁-C₃)-alkyl, (C₁-C₃)-haloalkyl, (C₂-C₃)-alkoxyalkyl, allyl, propargyl or (C₂-C₃)-haloalkenyl,
R¹⁹ is (C₁-C₂)-alkyl which is unsubstituted or substituted by F, Cl or OCH₃,
R²⁰ and R²¹ independently of one another are hydrogen, (C₁-C₂)-alkyl, (C₁-C₂)-alkoxy, -CH₂OCH₃, Cl, F, Br, I, -CH₂OCH₂CH₃, -NHCH₃, -N(OCH₃)CH₃, -N(CH₃)₂, -CF₃, -SCH₃, -CH(OCH₃)₂, -OCH₂CH=CH₂; -OCH₂C≡CH, -OCH₂CH₂OCH₃, -CH₂SCH₃, -OCHF₂, -SCHF₂, cyclopropyl, -C≡CH or -C≡C-CH₃,
m is 0 or 1,
n is 0 or 1,
s is zero and
E¹ is O or S.

3. A process for the preparation of the compounds of the formula I as claimed in claim 1 or 2 or of a salt thereof, which comprises
a) for the preparation of a compound with W = O,
a₁) reacting a compound of the formula II with 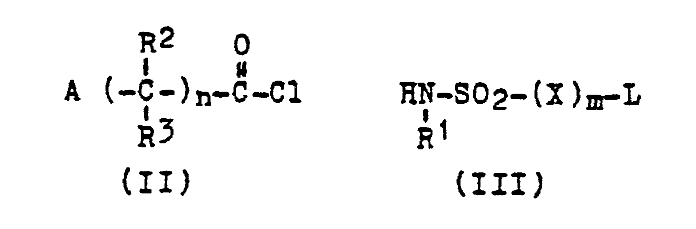 a compound of the formula III in the presence of a base, or
a₂) reacting a compound of the formula (IIa) 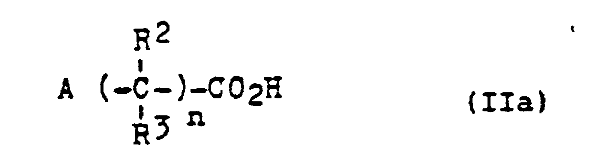 with a compound of the formula III in the presence of activating reagents, such as 2-chloro-1-methylpyridinium chloride (IVa), dicyclohexylcarbodiimide (IVb) or 1,1-carbonyldiimidazole (IVc) and if appropriate in the presence of a base, or
a₃) for the preparation of a compound with W = O and R¹ = H, reacting a compound of the formula (V), 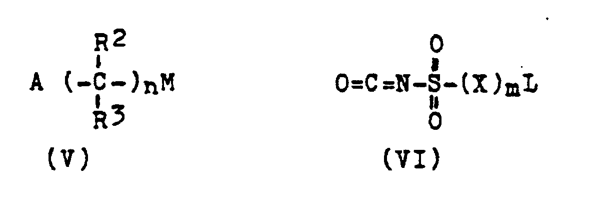 where M is hydrogen or lithium, with a compound of the formula (VI), or
b) for the preparation of a compound with W = S, reacting a compound of the formula I obtained in a) with the compound of the formula VII, 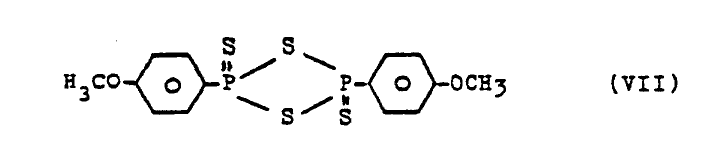
c) for the preparation of a compound with W = NR⁴, reacting a compound of the formula VIII 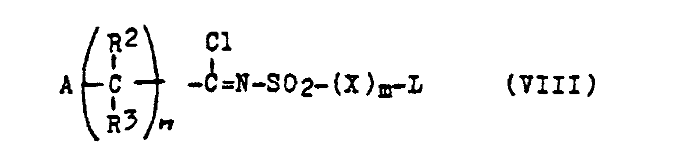 with an amine of the formula H₂N-R⁴,
d) for the preparation of a compound with W = NOR⁴,
d₁) reacting a compound of the formula IX with 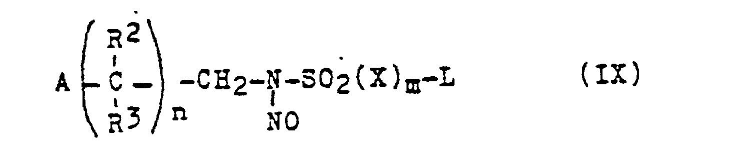 an alkali metal hydroxide or alkaline earth metal hydroxide or ammonium hydroxide, or
d₂) reacting a compound of the formula VIII with a hydroxylamine of the formula H₂N-OR⁴ in the presence of a base, or
e) for the preparation of a compound of the formula I with R¹ ≠ H, reacting a compound of the formula I with R¹ = H with an alkyl halide of the formula R^{1'}-X¹ in the presence of a base, where R^{1'} has the meaning indicated for R¹ with the exception of hydrogen and X¹ is chlorine, bromine or iodine, and, if appropriate, converting the compound obtained into its salt.

4. A process for preparing a compound of the formula (II') 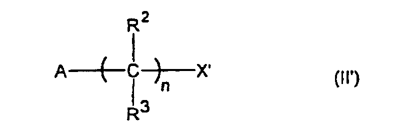 where
X' is COCl, CO₂R¹³ or CO₂CH₂C₆H₅,
n is 0 or 1,
R² and R³ independently of one another are hydrogen, (C₁-C₃)-alkyl or phenyl,
A is a heterocyclic radical of the formula (A1) where r = 0 and E² = CH, 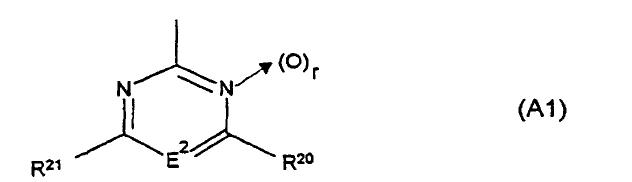 and
a is 0 or 1,
R¹⁰ is hydrogen, (C₁-C₄)-alkyl, (C₁-C₄)-alkyl which is monosubstituted or polysubstituted by F, Cl, Br or OCH₃, or is (C₃-C₆)-cycloalkyl which is unsubstituted or monosubstituted or polysubstituted by F, Cl, Br or, or is (C₂-C₄)-alkenyl or (C₂-C₄)-alkynyl,
R¹³ is hydrogen, (C₁-C₄)-alkyl which is unsubstituted or monosubstituted or polysubstituted by halogen or monosubstituted or disubstituted by CN, CO₂R¹⁰, NR¹⁴R¹⁵, OR¹⁰ or Si(CH₃)₃, or is (C₃-C₄)-alkynyl which is unsubstituted or substituted by CH₃ or Si(CH₃)₃, or is (C₃-C₆)-cycloalkyl, (C₃-C₆)-cycloalkyl-(C₁-C₄)-alkyl, (C₁-C₄)-alkoxy or Si(CH₃)₃,
R¹⁴ is hydrogen or (C₁-C₄)-alkyl or R¹⁴ and R¹⁵ together are -(CH₂)₂(CH₂)ₐ(CH₂)₂- or -(CH₂)₂O(CH₂)₂-,
R¹⁵ is hydrogen, (C₁-C₄)-alkyl, (C₁-C₄)-alkoxy, (C₂-C₄)-alkenyl or R¹⁴ and R¹⁵ together are -(CH₂)₂(CH₂)ₐ(CH₂)₂- or -(CH₂)₂O(CH₂)₂- and
R²⁰, R²¹ independently of one another are hydrogen, halogen, (C₁-C₆)-alkyl, (C₁-C₆)-alkoxy or (C₁-C₆)-alkylthio where the three abovementioned radicals can be monosubstituted or polysubstituted by halogen or monosubstituted or disubstituted by (C₁-C₄)-alkoxy or (C₁-C₄)-alkylthio, or are -NR¹⁴R¹⁵, (C₃-C₆)-cycloalkyl, -OCHR¹⁴CO₂R¹³, (C₂-C₅)-alkenyl, (C₂-C₄)-alkynyl, (C₃-C₅)-alkenyloxy or (C₃-C₅)-alkynyloxy,
where
1) in the event that n = 0 and X' = COOH the pair R²⁰/R²¹ is other than CH₃/CH₃,
2) in the event that n = 0 and X' = CO₂CH₃ the pair R²⁰/R²¹ is other than CH₃/CH₃ or OCH₃/CH₃,
3) in the event that n = 0 and X' = CO₂C₂H₅ the pair R²⁰/R²¹ is other than OC₂H₅/OC₂H₅ or Cl/Cl,
4) in the event that n = 1, R² = R³ = H and X' = COOH, COONa or COOCH₃ the pair R²⁰/R²¹ is other than H/F, H/Cl, H/Br, H/I, H/CH₃, H/OCH₃, H/SC₂H₅, Cl/SC₂H₅, SC₂H₅/SC₂H₅, benzyloxy/CH₃, OCH₃/CH₃, OC₂H₅/CH₃, Cl/Cl, Cl/OCH₃, OCH₃/OCH₃ or Cl/CH₃,
5) in the event that n = 1, R² = R³ = H and X' = CO₂C₂H₅ the pair R²⁰/R²¹ is other than Cl/CH₃ or CH₃/CH₃,
wherein a compound of the formula (XI) 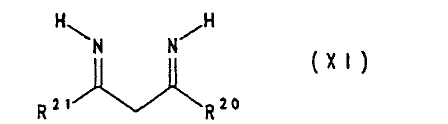 is reacted with a compound of the formula (XII) or (XIII) 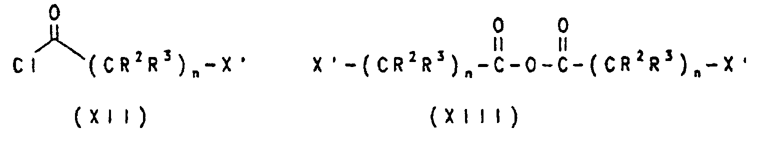 where, in formulae (XI), (XII) and (XIII), the radicals R², R³, R²⁰, R²¹ and X' and n are as defined in formula (II').

5. A process as claimed in claim 4, wherein the reaction is carried out at a temperature of from -78°C to 100°C in a solvent which is inert under the reaction conditions in the presence of a base.

6. A herbicidal or plant growth-regulating agent, containing a compound of the formula (I) of claim 1 or 2 or a salt thereof and inert additives.

7. A method of controlling undesired plants or for regulating the growth of plants, which comprises applying an effective amount of a compound of the formula (I) or a salt thereof, of claim 1 or 2, to these plants or the cropping areas.

## Revendications (Revendications pour l'(les) Etat(s) contractant(s) suivant(s): CH, DE, FR, GB, IT, LI)

1. Composés de formule I ou leurs sels 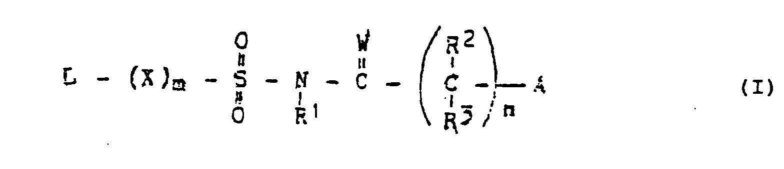 dans laquelle
R¹ représente hydrogène, alkyle en (C₁-C₄), alcényle en (C₂-C₆) ou alcynyle en (C₂-C₆) ;
R², R³ indépendamment l'un de l'autre, représentent hydrogène, alkyle en (C₁-C₃) ou phényle ;
W représente O, S, NR⁴ ou NOR⁴ ;
R⁴ représente hydrogène, alkyle en (C₁-C₃), halogénoalkyle en (C₁-C₃) ou phényle ;
X représente CHR², O, NR⁴ ou NOR⁴ ;
L représente un radical hétéro- ou isocyclique de formules (L1) à (L5) 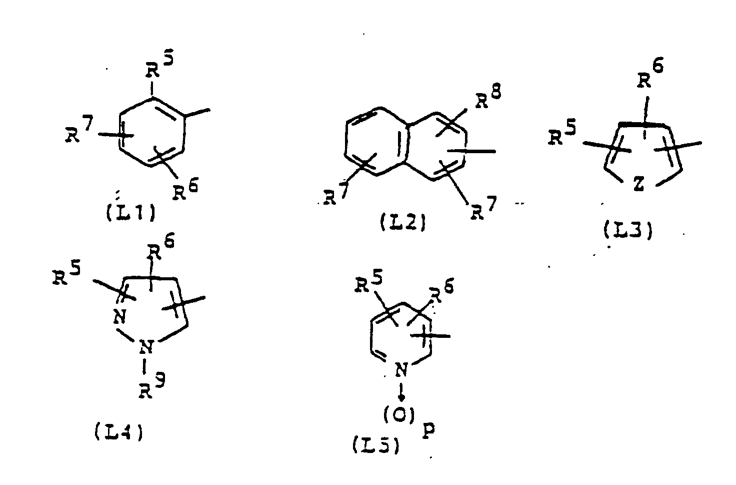
A représente un radical hétérocyclique de formules (A1) à (A8) 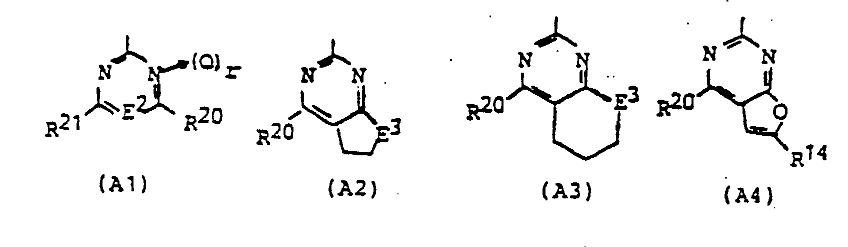 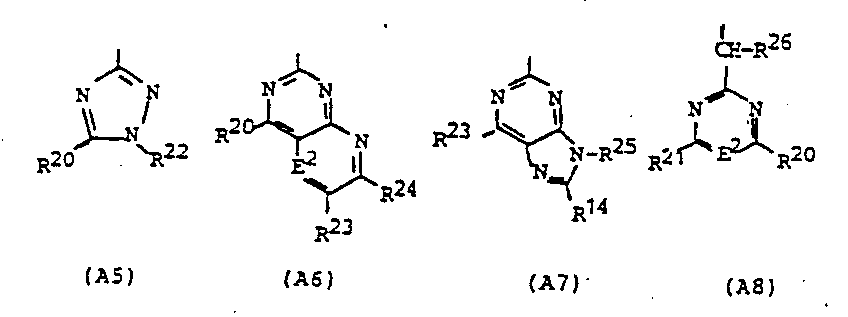
Z représente O ou S(O)_{q} ;
E¹ représente O ou S(O)_{b} ;
E² représente CH ou N ;
E³ représente O ou CH₂ ;
a ; m ; n ; p ; r ; s, indépendamment l'un de l'autre, valent 0 ou 1 ;
b ; q, indépendamment l'un de l'autre, valent 0, 1 ou 2 ;
R⁵ représente hydrogène, halogène, NO₂, CN, alkyle en (C₁-C₄) qui est non substitué ou substitué une ou plusieurs fois par F, Cl, Br, ou une fois par CN, OCH₃ ou SCH₃, alcényle en (C₂-C₄) qui est non substitué ou substitué une ou plusieurs fois par. F, Cl, Br ou une fois par OCH₃ ; alcynyle en (C₂-C₄) qui est non substitué ou substitué une ou plusieurs fois par F, Cl, Br, ou une fois par OCH₃ ou Si(CH₃)₃ ; cycloalkyle en (C₃-C₆) qui est non substitué ou substitué une ou plusieurs fois par F, Cl, ou CH₃ ; -C(O)R¹⁰, -OCH₂CH₂OR¹⁰, -OH, -C(R¹⁰)(OR¹¹)(OR¹²) ; -CO₂R¹³, -C(O)NR¹⁴R¹⁵, -N₃, -SO₂NR¹⁴R¹⁵, -SO₃R¹⁶, -OSO₂R¹⁷, phényle qui est non substitué ou substitué une ou plusieurs fois par F, Cl, Br, CH₃ ou OCH₃ ; -E¹R¹⁸ ou -(CH₂)ₛG ;
R⁶ représente hydrogène, halogène ; CN ; NO₂; alkyle en (C₁-C₄) qui est non substitué ou substitué une ou plusieurs fois par halogène, une à deux fois par -CO₂R¹³, -SO₂NR¹⁴R¹⁵, -NR¹⁴R¹⁵, alcoxy en (C₁-C₂), -E¹R¹⁹, halogénoalcoxy en (C₁-C₂), alkylthio en (C₁-C₂), halogénoalkylthio en (C₁-C₂), CN, OH ou SH ; -CO₂R¹³, -SO₂NR¹⁴R¹⁵, -NR¹⁴R¹⁵ ou -E¹R¹⁹ ;
les radicaux R⁷, indépendamment l'un de l'autre, représentent hydrogène, alkyle en (C₁-C₄) , halogénoalkyle en (C₁-C₄), alcényle en (C₂-C₄), phényle ou phényle qui est substitué une ou plusieurs fois par halogène ou -E¹R¹⁹ ; -E¹R¹⁹ ou halogène,
R⁸ représente hydrogène, alkyle en (C₁-C₄), halogénoalkyle en (C₁-C₄), halogène, -CO₂R¹³, -SO₂NR¹⁴R¹⁵, -OSO₂ R¹⁷, -S(O)_{b}R¹⁸, CN ou NO₂,
R⁹ représente hydrogène, alkyle en (C₁-C₄) qui est non substitué ou substitué une ou plusieurs fois par halogène ou une fois par phényle ; alcényle en (C₂-C₄) ; phényle ou phényle qui est substitué une ou plusieurs fois par halogène, alkyle en (C₁-C₄), NO₂, CN ou alcoxy en (C₁-C₄),
R¹⁰ représente hydrogène, alkyle en (C₁-C₄), alkyle en (C₁-C₄) qui est substitué une ou plusieurs fois par F, Cl, Br ou OCH₃ ; cycloalkyle en (C₃-C₆) qui est non substitué ou substitué une ou plusieurs fois par F, Cl, Br ou CH₃ ; alcényle en (C₂-C₄) ou alcynyle en (C₂-C₄) ;
R¹¹, R¹², indépendamment l'un de l'autre, représentent alkyle en (C₁-C₄) ou R¹¹ et R¹² ensemble représentent -CH₂CH₂-, -CH₂OCH₂- ou -CH₂C(CH₃)₂CH₂- ;
R¹³ représente hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃ ; alcynyle en (C₃-C₄) qui est non substitué ou substitué par CH₃ ou Si(CH₃)₃ ; cycloalkyle en (C₃-C₆), cycloalkyl en (C₃-C₆)-alkyle en (C₁-C₄), alcoxy en (C₁-C₄) ou Si(CH₃)₃ ;
R¹⁴ représente hydrogène ou alkyle en (C₁-C₄) ou R¹⁴ et R¹⁵ ensemble représentent -(CH₂)₂(CH₂)ₐ(CH₂)₂- ou -(CH₂)₂O(CH₂)₂-,
R¹⁵ représente hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂- ou -(CH₂)₂O(CH₂)₂-,
R¹⁶ représente alkyle en (C₁-C₄) ou halogénoalkyle en (C₁-C₄) ;
R¹⁷ représente alkyle en (C₁-C₄) ou NR¹⁴R¹⁵,
R¹⁸ représente alkyle en (C₁-C₄), halogénoalkyle en (C₁-C₄), alcoxyalkyle en (C₂-C₄), alcényle en (C₂-C₄), alcynyle en (C₃-C₄), phényle ou phényle qui est substitué une ou plusieurs fois par halogène, alkyle en (C₁-C₃), alcoxy en (C₁-C₃) ou halogénoalkyle en (C₁-C₃) ;
R¹⁹ représente alkyle en (C₁-C₄) ou alkyle en (C₁-C₄) qui est substitué une ou plusieurs fois par F, Cl ou une fois par OR¹⁶ ;
R²⁰, R²¹, indépendamment l'un de l'autre représentent hydrogène, halogène, alkyle en (C₁-C₆), alcoxy en (C₁-C₆) ou alkylthio en (C₁-C₆), les trois radicaux précités pouvant être substitués une ou plusieurs fois par halogène ou une ou deux fois par alcoxy en (C₁-C₄) ou alkylthio en (C₁-C₄) ; -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅) ;
R²² représente alkyle en (C₁-C₄) ou halogénoalkyle en (C₁-C₄) ;
R²³, R²⁴, indépendamment l'un de l'autre, représentent hydrogène, alkyle en (C₁-C₄), alcényle en (C₂-C₄) ou alcynyle en (C₂-C₄) ;
R²⁵ représente hydrogène, alkyle en (C₁-C₄) ou halogénoalkyle en (C₁-C₄) ;
R²⁶ représente hydrogène ou alkyle en (C₁-C₃) ;
G représente un radical hétérocyclique (G 1) à (G 25), 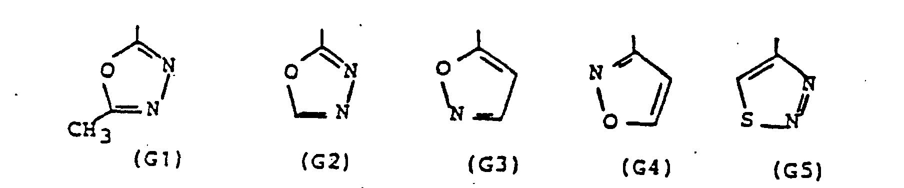 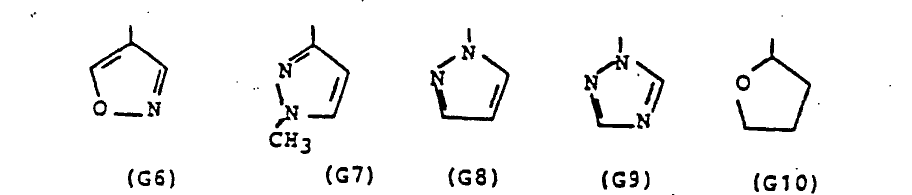 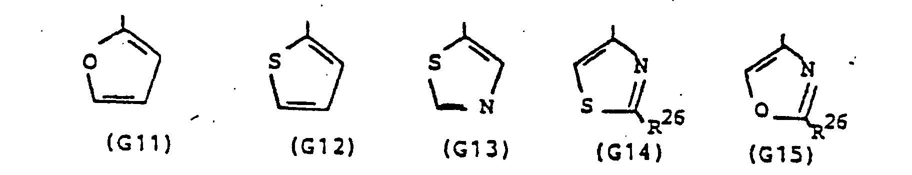 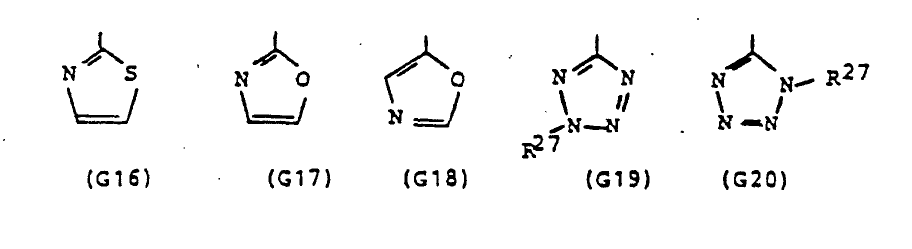 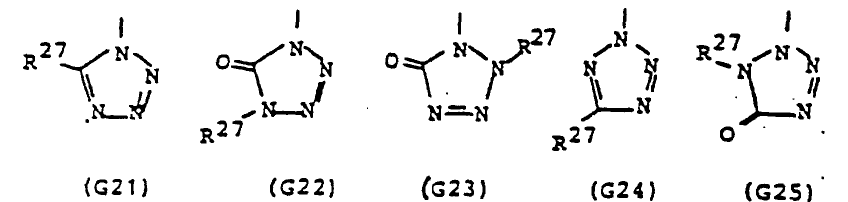
R²⁷ représente hydrogène, alkyle en (C₁-C₃) ou alcényle en (C₂-C₄).

2. Composés de formule (I) selon la revendication 1 ou leurs sels, dans lesquels
A représente un radical de formules (A1), (A2), (A3) ou (A4), notamment (A1) ;
L représente un radical de formules (L1), (L3), (L4) ou (L5) ;
X représente CH₂, CH(CH₃), O, NH, NCH₃, NC₂H₅, NOCH₃ notamment CH₂,O ou NH ;
W représente oxygène ;
R¹ représente hydrogène ;
R², R³, indépendamment l'un de l'autre, représentent hydrogène ou alkyle en (C₁-C₃), plus particulièrement hydrogène ;
R⁵ représente halogène, NO₂, CN, alkyle en (C₁-C₃) qui est non substitué ou substitué par F, Cl, Br, CN, OCH₃ ou SCH₃ ; alcényle (en C₃) qui est non substitué ou substitué par F, Cl, ou Br ; alcynyle (en C₃), cycloalkyle (en C₃) qui est non substitué ou substitué par F, Cl ou CH₃, -C(O)R¹⁰, -OCH₂CH₂OR¹⁰, OH, -C(R¹⁰)(OR¹¹)(OR¹²), -CO₂R¹³, -C(O)NR¹⁴R¹⁵, N₃, -SO₂NR¹⁴R¹⁵, -OSO₂R¹⁷, -E¹R¹⁸ ou -(CH₂)ₛG ;
R⁶ représente hydrogène, halogène, CN, NO₂, CH₃, CF₃, -E¹R¹⁹ ou alcoxy en (C₁-C₂)-alkyle en (C₁-C₂), halogénoalcoxy en (C₁-C₂), alkylthio en (C₁-C₂), halogénoalkylthio en (C₁-C₂), CN, -CO₂R¹³ ou -SO₂NR¹⁴R¹⁵,
R⁷ représente hydrogène,
R¹⁰ représente alkyle en (C₁-C₃), cyclopropyle ou alcényle en (C₃),
R¹¹,R¹² représentent alkyle en (C₁-C₂) ou R¹¹, R¹² ensemble représentent -CH₂CH₂- ;
R¹³ représente alkyle en (C₁-C₃), alcényle (en C₃), -CH₂CH₂F, -CH₂CH₂Cl, -CH₂CH₂OCH₃ ou cyclopropylméthyle ;
R¹⁴ représente hydrogène ou CH₃ ou R¹⁴ et R¹⁵ ensemble représentent -(CH₂)₄-, (CH₂)₅- ou -(CH₂)₂O(CH₂)₂- ;
R¹⁵ représente CH₃, CH₂CH₃, OCH₃ ou R¹⁴ et R¹⁵ ensemble représentent -(CH₂)₄-, -(CH₂)₅- ou -(CH₂)₂O(CH₂)₂- ;
R¹⁸ représente alkyle en (C₁-C₃), halogénoalkyle en (C₁-C₃), alcoxyalkyle en (C₂-C₃), allyle, propargyle, halogénoalkyle en (C₂-C₃) ;
R¹⁹ représente alkyle en (C₁-C₂) qui est non substitué ou substitué par F, Cl ou OCH₃ ;
R²⁰, R²¹, indépendamment l'un de l'autre, représentent hydrogène, alkyle en (C₁-C₂), alcoxy en (C₁-C₂), -CH₂OCH₃, Cl, F, Br, I, -CH₂OCH₂CH₃, -NHCH₃, -N(OCH₃)CH₃, -N(CH₃)₂, -CF₃, -SCH₃, -CH(OCH₃)₂, -OCH₂CH=CH₂ ; -OCH₂C≡CH, -OCH₂CH₂OCH₃, -CH₂SCH₃, -OCHF₂, -SCHF₂, cyclopropyle, -C≡CH ou -C≡C-CH₃ ;
m vaut 0 ou 1 ;
n vaut 0 ou 1 ;
s vaut zéro et
E¹ représente O ou S.

3. Procédé pour la préparation des composés de formule I selon la revendication 1 ou 2 ou de leurs sels, **caractérisé en ce que**
a) pour la préparation de composés avec W = 0,
on fait réagir
a₁) un composé de formule II avec 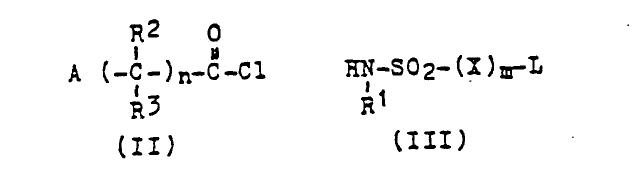 un composé de formule III en présence d'une base ou
on fait réagir
a2) un composé de formule (IIa) 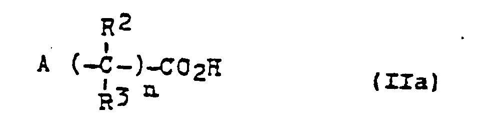 en présence de réactifs activants tels que le chlorure de 2-chloro-1-méthylpyridinium (IVa), le dicyclohexylcarbodiimide (IVb) ou le 1,1-carbonyldiimidazole (IVc) et éventuellement en présence d'une base avec un composé de formule III ou
a₃) pour la préparation de composés avec W = 0 et R¹ = H, on fait réagir un composé de formule (V). 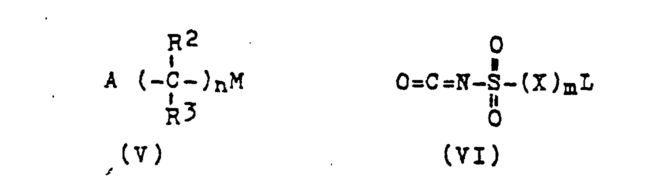 où M représente l'hydrogène ou le lithium, avec un composé de formule (VI), ou
b) pour la préparation de composés avec W = S, on fait réagir un composé de formule I obtenu dans a) avec le composé de formule VII 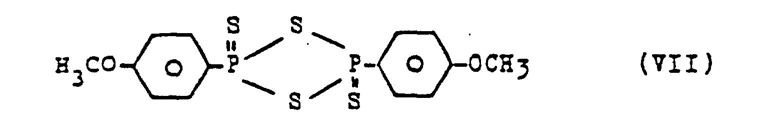
c) pour la préparation de composés avec W = NR⁴, on fait réagir un composé de formule VIII 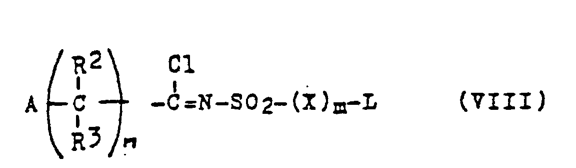 avec une amine de formule H₂N-R⁴,
d) pour la préparation de composés avec W = NOR⁴, on fait réagir
d₁) un composé de formule IX avec 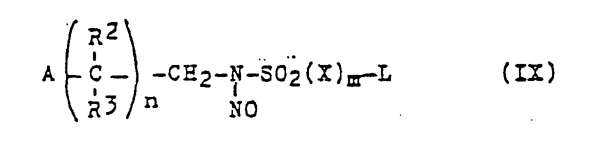 un hydroxyde alcalin ou alcalino-terreux ou l'hydroxyde d'ammonium ou
d₂) on fait réagir un composé de formule VIII en présence d'une base avec une hydroxylamine de formule H₂N-OR⁴, ou
e) pour la préparation de composés de formule I avec R¹ ≠ H, on fait réagir un composé de formule I avec R¹ = H en présence d'une base avec un halogénoalkyle de formule R^{1'}-X¹ où R^{1'} a la signification de R¹ sauf celle de l'hydrogène et X¹ représente le chlore, le brome ou l'iode et on transforme les composés de obtenus éventuellement en leurs sels.

4. Composé de formule (II'), 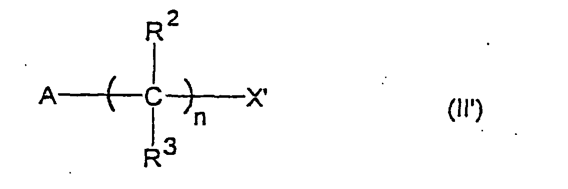 dans laquelle
X' représente COCl, CN, F, p-tosyle, tétraalkylammonium, N₃, CO₂R¹³, CO₂CH₂C₆H₅ ou E¹R¹⁸,
n vaut 0 ou 1,
R² et R³, l'un indépendamment de l'autre, représentent l'hydrogène, alkyle en (C₁-C₃) ou phényle,
A représente un radical hétérocyclique de formule (A1), où r=1, ou (A2), (A3), (A4) ou (A5), et où dans le cas de (A5) n=1, ou (A6), (A7) ou (A8),
et 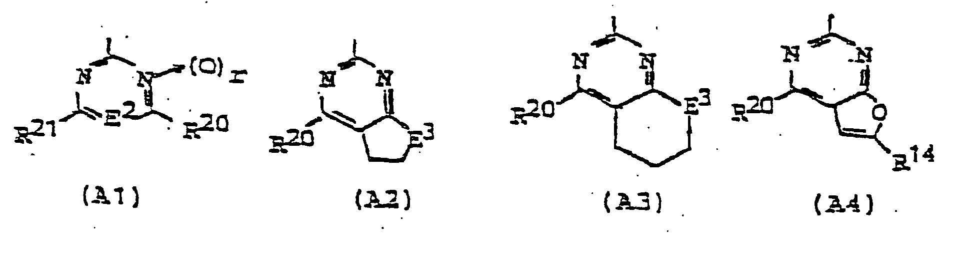 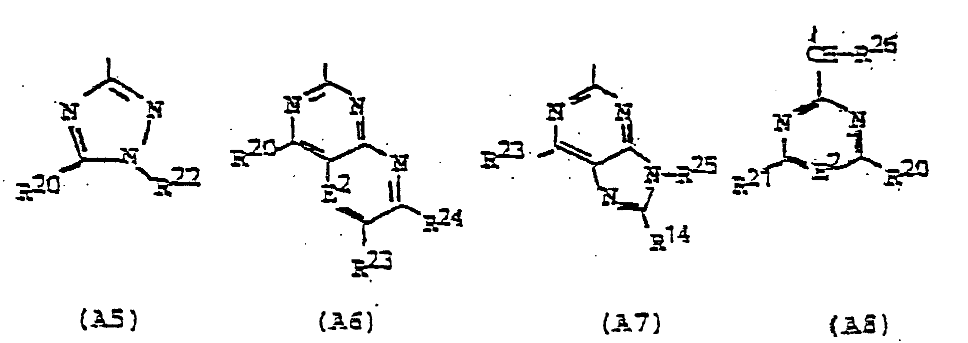
E¹ représente O ou S(O)_{b},
E² représente CH ou N,
E³ représente O ou CH₂,
a vaut 0 ou 1,
b vaut 0, 1 ou 2
R¹⁰ représente l'hydrogène, alkyle en (C₁-C₄), alkyle en (C₁-C₄) qui est substitué une ou plusieurs fois par F, Cl, Br ou OCH₃ ; cycloalkyle en (C₃-C₆) qui est non substitué. ou substitué une ou plusieurs fois par F, Cl, Br ; alcényle en (C₂-C₄) ou alcynyle en (C₂-C₄),
R¹³ représente l'hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃ ; alcynyle en (C₃-C₄) qui est non substitué ou substitué par CH₃ ou Si(CH₃)₃ ; cycloalkyle en (C₃-C₆), cycloalkyle en (C₃-C₆)-alkyle en (C₁-C₄), alcoxy en (C₁-C₄) ou Si(CH₃)₃,
R¹⁴ représente l'hydrogène ou alkyle en (C₁-C₄) ou R¹⁴ et R¹⁵ ensemble représentent -(CH₂)₂(CH₂)ₐ(CH₂)₂- ou -(CH₂)₂O(CH₂)₂-,
R¹⁵ représente l'hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂- ou -(CH₂)₂O(CH₂)₂-,
R¹⁸ représente alkyle en (C₁-C₄), halogénoalkyle en (C₁-C₄), alcoxyalkyle en (C₂-C₄), alcényle en (C₂-C₄), alcynyle en (C₃-C₄), phényle ou phényle qui est substitué une ou plusieurs fois par halogène, alkyle en (C₁-C₃), alcoxy en (C₁-C₃) ou halogénoalkyle en (C₁-C₃),
R²⁰, R²¹, indépendamment l'un de l'autre, représentent l'hydrogène, halogène, alkyle en (C₁-C₆), alcoxy en (C₁-C₆) ou thioalkyle en (C₁-C₆), les trois radicaux précités pouvant être substitués une ou plusieurs fois par halogène ou une ou deux fois par alcoxy en (C₁-C₄) ou thioalkyle en (C₁-C₄); -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅),
R²² représente alkyle en (C₁-C₄) ou halogénoalkyle en (C₁-C₄),
R²³, R²⁴, indépendamment l'un de l'autre, représentent l'hydrogène, alkyle en (C₁-C₄), alcényle en (C₂-C₄) ou alcynyle en (C₂-C₄),
R²⁵ représente l'hydrogène, alkyle en (C₁-C₄) ou halogénoalkyle en (C₁-C₄), et
R²⁶ représente l'hydrogène ou alkyle en (C₁-C₃).

5. Composé de formule (II') 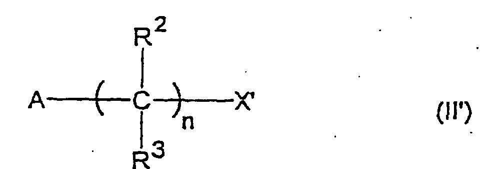 dans laquelle
X' représente COCl, CO₂R¹³ ou CO₂CH₂C₆H₅,
n vaut 0 ou 1,
R² et R³, l'un indépendamment de l'autre, représentent l'hydrogène, alkyle en C₁-C₃ ou phényle,
A représente un radical hétérocyclique de formule (A1), où r=0 et E² = CH, 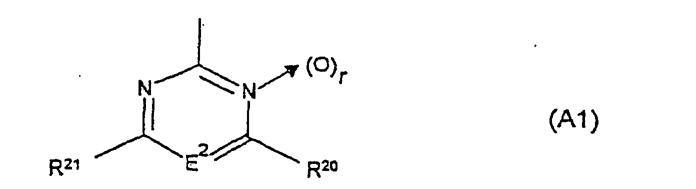
et
a vaut 0 ou 1
R¹⁰ représente l'hydrogène, alkyle en (C₁-C₄), alkyle en (C₁-C₄) qui est substitué une ou plusieurs fois par F, Cl, Br ou OCH₃; cycloalkyle en (C₃-C₆) qui est non substitué ou substitué une ou plusieurs fois par F, Cl, Br ; alcényle en (C₂-C₄) ou alcynyle en (C₂-C₄),
R¹³ représente l'hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃ ; alcynyle en (C₃-C₄) qui est non substitué ou substitué par CH₃ ou Si(CH₃)₃ ; cycloalkyle en (C₃-C₆), cycloalkyle en (C₃-C₆)-alkyle en (C₁-C₄), alcoxy en (C₁-C₄) ou Si(CH₃)₃,
R¹⁴ représente l'hydrogène ou alkyle en (C₁-C₄) ou R¹⁴ et R¹⁵ ensemble représentent -(CH₂)₂(CH₂)ₐ(CH₂)₂- ou -(CH₂)₂O(CH₂)₂-,
R¹⁵ représente l'hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂ - ou -(CH₂)₂O(CH₂)₂-, et
R²⁰, R²¹, indépendamment l'un de l'autre, représentent l'hydrogène, halogène, alkyle en (C₁-C₆), alcoxy en (C₁-C₆) ou thioalkyle en (C₁-C₆), les trois radicaux précités pouvant être substitués une ou plusieurs fois par halogène ou une ou deux fois par alcoxy en (C₁-C₄) ou thioalkyle en (C₁-C₄); -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅),
où
1) dans le cas où n=0 et X'=COOH, la paire R²⁰/R²¹ ne représente pas CH₃/CH₃,
2) dans le cas où n=0 et X'=CO₂CH₃, la paire R²⁰/R²¹ ne représente pas CH₃/CH3 ou OCH₃/CH₃,
3) dans le cas où n=0 et X'=CO₂C₂H₅, la paire R²⁰/R²¹ ne représente pas OC₂H₅/OC₂H₅ ou Cl/Cl,
4) dans le cas où n=1, R² = R³ = H et X'=COOH, COONa ou COOCH₃, la paire R²⁰/R²¹ ne représente pas H/F, H/Cl, H/Br, H/I, H/CH₃, H/OCH₃, H/SC₂H₅, Cl/SC₂H₅, SC₂H₅/SC₂H₅, benzyloxy/CH₃, OCH₃/CH₃, OC₂H₅/CH₃, Cl/Cl, Cl/OCH₃, OCH₃/OCH₃ ou Cl/CH₃,
5) dans le cas où n=1, R² = R³ = H et X'= COOC₂H₅, la paire R²⁰/R²¹ ne représente pas Cl/CH₃ ou CH₃/CH₃.

6. Composé de formule (II') 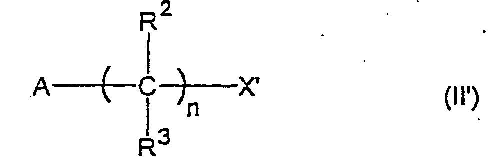 dans laquelle
X' représente COCl, CO₂R¹³ ou CO₂CH₂C₆H₅,
n vaut 0 ou 1,
R² et R³, l'un indépendamment de l'autre, représentent l'hydrogène, alkyle en C₁-C₃ ou phényle,
A représente un radical hétérocyclique de formule (A1), où r=0 et E² = N, 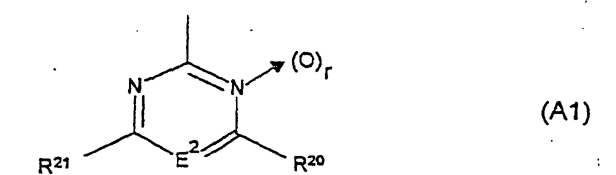 et
a vaut 0 ou 1
R¹⁰ représente l'hydrogène, alkyle en (C₁-C₄), alkyle en (C₁-C₄) qui est substitué une ou plusieurs fois par F, Cl, Br ou OCH₃; cycloalkyle en (C₃-C₆) qui est non substitué ou substitué une ou plusieurs fois par F, Cl, Br ; alcényle en (C₂-C₄) ou alcynyle en (C₂-C₄),
R¹³ représente l'hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃ ; alcynyle en (C₃-C₄) qui est non substitué ou substitué par CH₃ ou Si(CH₃)₃ ; cycloalkyle en (C₃-C₆), cycloalkyle en (C₃-C₆)-alkyle en (C₁-C₄), alcoxy en (C₁-C₄) ou Si(CH₃)₃,
R¹⁴ représente l'hydrogène ou alkyle en (C₁-C₄) ou R¹⁴ et R¹⁵ ensemble représentent -(CH₂)₂(CH₂)ₐ(CH₂)₂- ou - (CH₂)₂O(CH₂)₂-,
R¹⁵ représente l'hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂- ou -(CH₂)₂O(CH₂)₂-, et
R²⁰, R²¹, indépendamment l'un de l'autre, représentent l'hydrogène, halogène, alkyle en (C₁-C₆), alcoxy en (C₁-C₆) ou thioalkyle en (C₁-C₆), les trois radicaux précités pouvant être substitués une ou plusieurs fois par halogène ou une ou deux fois par alcoxy en (C₁-C₄) ou thioalkyle en (C₁-C₄); -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅),
où
1) dans le cas où n=0 et X'=CO₂C₂H₅, la paire R²⁰/R²¹ ne représente pas CH₃/CH₃, CCl₃/NH₂ ou SCH₃/SCH₃,
2) dans le cas où n=1, R² = R³ = H et et X'=CO₂C₂H₅, la paire R²⁰/R²¹ ne représente pas Cl/OCH₃ ou Cl/Cl,
3) dans le cas où n=1, R² = R³ = H et X' = CN, la paire R²⁰/R²¹ ne représente pas NH₂/NH₂,
4) dans le cas où n=0 et X'=COOH, la paire R²⁰/R²¹ ne représente pas OCH₃/OCH₃ ou SCH₃/SCH₃,
5) dans le cas où n=0 et X'= CO₂CH₃, la paire R²⁰/R²¹ ne représente pas SCH₃/SCH₃.

7. Composé de formule (II') 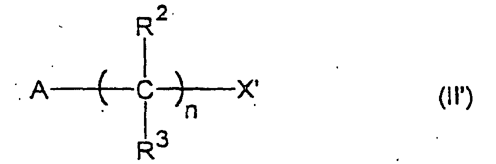 dans laquelle
X' représente COCl, CN, CO₂R¹³ ou CO₂CH₂C₆H₅,
n vaut 0,
R² et R³, l'un indépendamment de l'autre, représentent l'hydrogène, alkyle en C₁-C₃ ou phényle,
A représente un radical hétérocyclique de formule (A5), 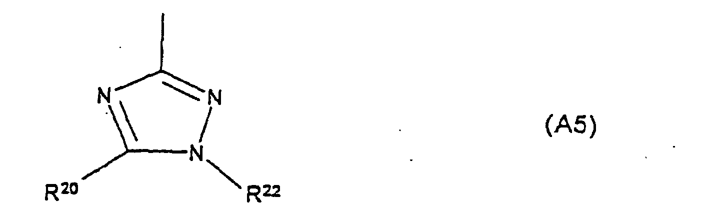 et
a vaut 0 ou 1
R¹⁰ représente l'hydrogène, alkyle en (C₁-C₄), alkyle en (C₁-C₄) qui est substitué une ou plusieurs fois par F, Cl, Brou OCH₃ ; cycloalkyle en (C3-C6) qui est non substitué ou substitué une ou plusieurs fois par F, Cl, Br ; alcényle en (C₂-C₄) ou alcynyle en (C₂-C₄),
R¹³ représente l'hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃; alcynyle en (C₃-C₄) qui est non substitué ou substitué par CH₃ ou Si(CH₃)₃ ; cycloalkyle en (C₃-C₆), cycloalkyle en (C₃-C₆)-alkyle en (C₁-C₄), alcoxy en (C₁-C₄) ou Si(CH₃)₃,
R¹⁴ représente l'hydrogène ou alkyle en (C₁-C₄) ou R¹⁴ et R¹⁵ ensemble représentent-(CH₂)₂(CH₂)ₐ(CH₂)₂- ou - (CH₂)₂O(CH₂)₂-,
R¹⁵ représente l'hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂- ou -(CH₂)₂O(CH₂)₂-,
R²⁰ représente l'hydrogène, halogène, alkyle en (C₁-C₆), alcoxy en (C₁-C₆) ou thioalkyle en (C₁-C₆), les trois radicaux précités pouvant être substitués une ou plusieurs fois par halogène ou une ou deux fois par alcoxy en (C₁-C₄) ou thioalkyle en (C₁-C₄); -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅),
R²² représente alkyle en (C₁-C₄) ou halogénoalkyle en (C₁-C₄), et
où
dans le cas où n=0, X' = CO₂CH₃ ou CO₂C₂H₅ et R²⁰ = H, le radical R²² n'est pas identique à CH₃.

8. Procédé de préparation des composés de formule (II') selon la revendication 5, **caractérisé en ce qu'**on fait réagir un composé de formule (XI) 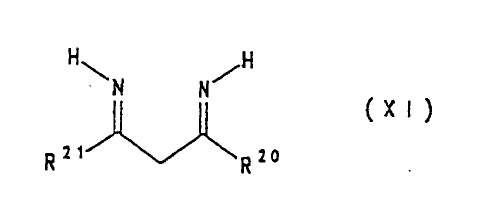 avec un composé de formule (XII) ou (XIII) 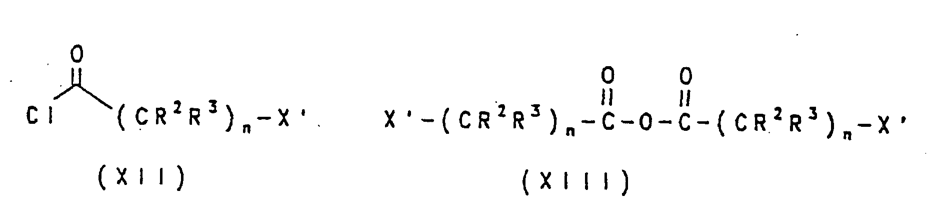 dans les formules (XI), (XII) et (XIII), les radicaux R², R³, R²⁰, R²¹ et X' ainsi que n étant définis comme dans la formule (II').

9. Procédé selon la revendication 8, **caractérisé en ce qu'**on met en oeuvre la réaction dans un solvant inerte dans les conditions de la réaction en présence d'une base à une température de -78°C à 100°C.

10. Agents herbicides ou régulateurs de croissance de plantes, **caractérisés en ce qu'**ils contiennent un composé de formule (I) de la revendication 1 ou 2 ou son sel et des adjuvants inertes.

11. Utilisation de composés de formule (I) de la revendication 1 ou 2 ou de leurs sels en tant qu'herbicides ou agents régulateurs de croissance de plantes.

12. Procédé pour la lutte contre la végétation non désirée ou pour la régulation de la croissance de plantes, **caractérisé en ce qu'**on applique sur les plantes ou sur les sols cultivables une quantité efficace d'un composé de formule (I) ou de son sel de la revendication 1 ou 2.

## Revendications (Revendications pour l'(les) Etat(s) contractant(s) suivant(s): ES)

1. Utilisation des composés de formule I ou leurs sels 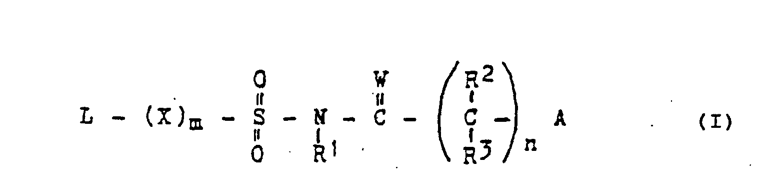 dans laquelle
R¹ représente hydrogène, alkyle en (C₁-C₄), alcényle en (C₂-C₆) ou alcynyle en (C₂-C₆) ;
R², R³, indépendamment l'un de l'autre, représentent hydrogène, alkyle en (C₁-C₃) ou phényle ;
W représente O, S, NR⁴ ou NOR⁴ ;
R⁴ représente hydrogène, alkyle en (C₁-C₃), halogénoalkyle en (C₁-C₃) ou phényle ;
X représente CHR², O, NR⁴ ou NOR⁴ ;
L représente un radical hétéro- ou isocyclique de formules (L1) à (L5) 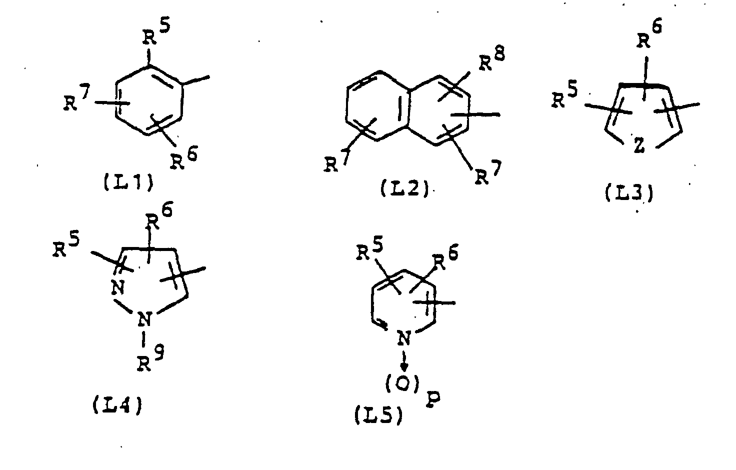
A représente un radical hétérocyclique de formules (A1) à (A8) 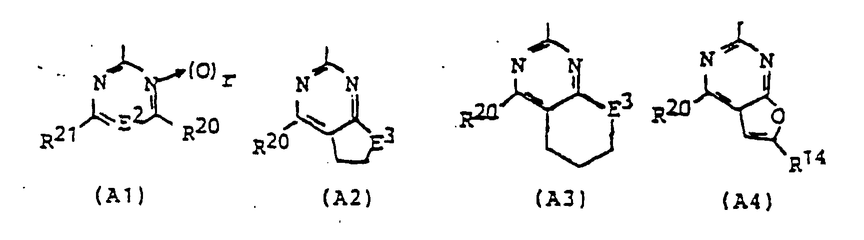 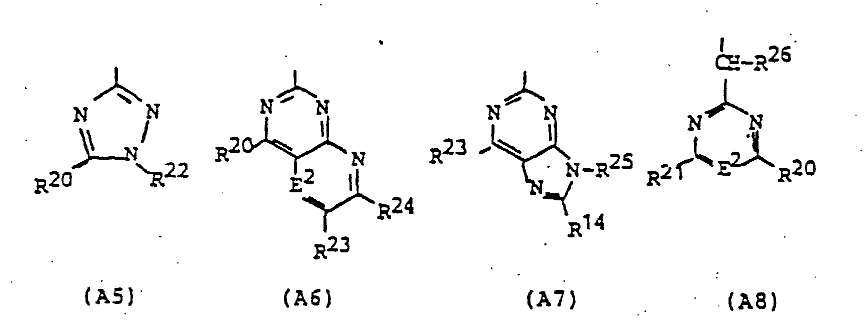
Z représente O ou S(O)_{q} ;
E¹ représente O ou S(O)_{b} ;
E² représente CH ou N ;
E³ représente O ou CH₂ ;
a ; m ; n ; p ; r ; s, indépendamment l'un de l'autre, valent 0 ou 1 ;
b ;q, indépendamment l'un de l'autre, valent 0, 1 ou 2 ;
R⁵ représente hydrogène, halogène, NO₂, CN, alkyle en (C₁-C₄) qui est non substitué ou substitué une ou plusieurs fois par F, Cl, Br, ou une fois par CN, OCH₃ ou SCH₃, alcényle en (C₂-C₄) qui est non substitué ou substitué une ou plusieurs fois par F, Cl, Br ou une fois par OCH₃ ; alcynyle en (C₂-C₄) qui est non substitué ou substitué une ou plusieurs fois par F, Cl, Br, ou une fois par OCH₃ ou Si(CH₃)₃ ; cycloalkyle en (C₃-C₆) qui est non substitué ou substitué une ou plusieurs fois par F, Cl, ou CH₃ ; -C(O)R¹⁰, -OCH₂CH₂OR¹⁰, -OH, -C(R¹⁰)(OR¹¹)(OR¹²) ; -CO₂R¹³, -C(O)NR¹⁴R¹⁵, -N₃, -SO₂NR¹⁴R¹⁵, -SO₃R¹⁶, -OSO₂R¹⁷, phényle qui est non substitué ou substitué une ou plusieurs fois par F, Cl, Br, CH₃ ou OCH₃ ; -E¹R¹⁸ ou -(CH₂)ₛG ;
R⁶ représente hydrogène, halogène ; CN ; NO₂ ; alkyle en (C₁-C₄) qui est non substitué ou substitué une ou plusieurs fois par halogène ou une à deux fois par -CO₂R¹³, -SO₂NR¹⁴R¹⁵, -NR¹⁴R¹⁵, alcoxy en (C₁-C₂), -E₁R₁₉, halogénoalcoxy en (C₁-C₂), alkylthio en (C₁-C₂), halogénoalkylthio en (C₁-C₂), CN, OH ou SH ; -CO₂R¹³, -SO₂NR¹⁴R¹⁵, -NR¹⁴R¹⁵ ou -E¹R¹⁹ ;
les radicaux R⁷, indépendamment l'un de l'autre, représentent hydrogène, alkyle en (C₁-C₄), halogénoalkyle en (C₁-C₄), alcényle en (C₂-C₄), phényle ou phényle qui est substitué une ou plusieurs fois par halogène ou -E¹R¹⁹ ; -E¹R¹⁹ ou halogène,
R⁸ représente hydrogène, alkyle en (C₁-C₄), halogénoalkyle en (C₁-C₄), halogène, -CO₂R¹³, -SO₂NR¹⁴R¹⁵, -OSO₂ R¹⁷, -S(O)_{b}R¹⁸, CN ou NO₂,
R⁹ représente hydrogène, alkyle en (C₁-C₄) qui est non substitué ou substitué une ou plusieurs fois par halogène ou une fois par phényle ; alcényle en (C₂-C₄) ; phényle ou phényle qui est substitué une ou plusieurs fois par halogène, alkyle en (C₁-C₄), NO₂, CN ou alcoxy en (C₁-C₄),
R¹⁰ représente hydrogène, alkyle en (C₁-C₄), alkyle en (C₁-C₄) qui est substitué une ou plusieurs fois par F, Cl, Br ou OCH₃ ; cycloalkyle en (C₃-C₆) qui est non substitué ou substitué une ou plusieurs fois par F, Cl, Br ou CH₃ ; alcényle en (C₂-C₄) ou alcynyle en (C₂-C₄) ;
R¹¹, R¹², indépendamment l'un de l'autre, représentent alkyle en (C₁-C₄) ou R¹¹ et R¹² ensemble représentent -CH₂CH₂-, -CH₂OCH₂- ou -CH₂C(CH₃)₂CH₂- ;
R¹³ représente hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃ ; alcynyle en (C₃-C₄) qui est non substitué ou substitué par CH₃ ou Si(CH₃)₃ ; cycloalkyle en (C₃-C₆), cycloalkyl en (C₃-C₆)-alkyle en (C₁-C₄), alcoxy en (C₁-C₄) ou Si(CH₃)₃,
R¹⁴ représente hydrogène ou alkyle en (C₁-C₄) ou R¹⁴ et R¹⁵ ensemble représentent -(CH₂)₂(CH₂)ₐ(CH₂)₂- ou -(CH₂)₂O(CH₂)₂-,
R¹⁵ représente hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂- ou -(CH₂)₂O(CH₂)₂- ;
R¹⁶ représente alkyle en (C₁-C₄) ou halogénoalkyle en (C₁-C₄) ;
R¹⁷ représente alkyle en (C₁-C₄) ou NR¹⁴R¹⁵ ;
R¹⁸ représente alkyle en (C₁-C₄), halogénoalkyle en (C₁-C₄), alcoxyalkyle en (C₂-C₄), alcényle en (C₂-C₄), alcynyle en (C₃-C₄), phényle ou phényle qui est substitué une ou plusieurs fois par halogène, alkyle en (C₁-C₃), alcoxy en (C₁-C₃) ou halogénoalkyle en (C₁-C₃) ;
R¹⁹ représente alkyle en (C₁-C₄) ou alkyle en (C₁-C₄) qui est substitué une ou plusieurs fois par F, Cl ou une fois par OR¹⁶ ;
R²⁰, R²¹, indépendamment l'un de l'autre, représentent hydrogène, halogène, alkyle en (C₁-C₆), alcoxy en (C₁-C₆) ou alkylthio en (C₁-C₆), les trois radicaux précités pouvant être substitués une ou plusieurs fois par halogène ou une ou deux fois par alcoxy en (C₁-C₄) ou alkylthio en (C₁-C₄) ; -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅),
R²² représente alkyle en (C₁-C₄) ou halogénoalkyle en (C₁-C₄) ;
R²³, R²⁴, indépendamment l'un de l'autre, représentent hydrogène, alkyle en (C₁-C₄), alcényle en (C₂-C₄) ou alcynyle en (C₂-C₄) ;
R²⁵ représente hydrogène, alkyle en (C₁-C₄) ou halogénoalkyle en (C₁-C₄) ;
R²⁶ représente hydrogène ou alkyle en (C₁-C₃) ;
G représente un radical hétérocyclique (G 1) à (G 25) 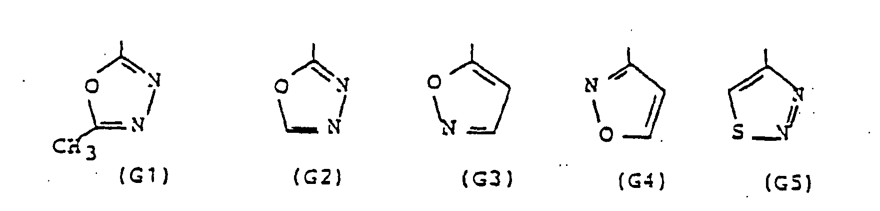 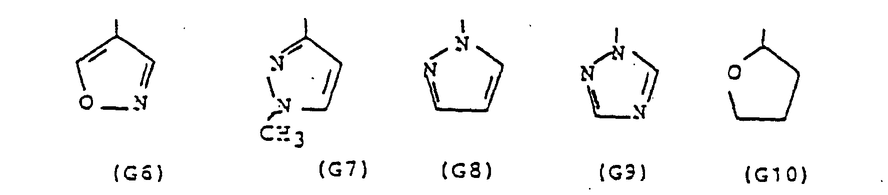 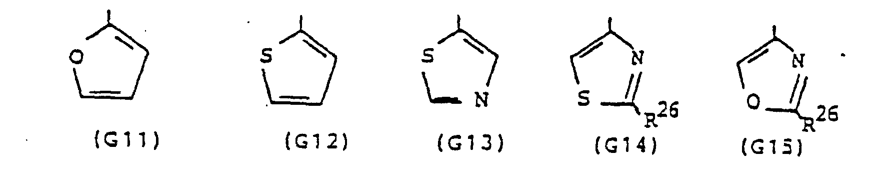 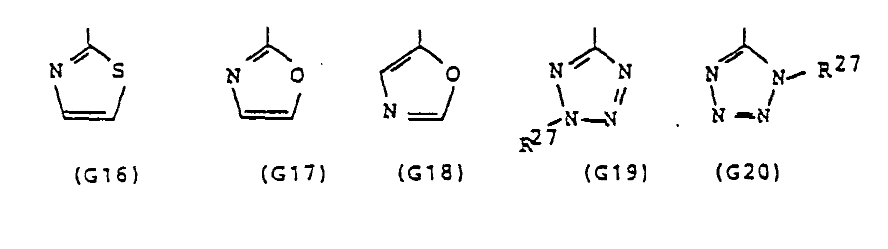 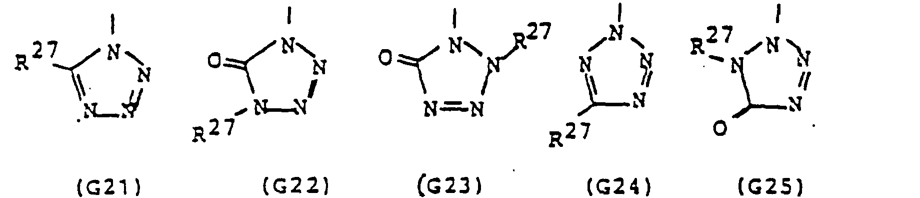
R²⁷ représente hydrogène, alkyle en (C₁-C₃) ou alcényle en (C₂-C₄) ;
en tant qu'herbicides ou agents régulateurs de croissance de plantes.

2. Utilisation selon la revendication 1, **caractérisée en ce que** dans la formule (I)
A représente un radical de formules (A1), (A2), (A3) ou (A4), notamment (A1) ;
L représente un radical de formules (L1), (L3), (L4) ou (L5) ;
X représente CH₂, CH(CH₃), O, NH, NCH₃, NC₂H₅, NOCH₃, notamment CH₂, O ou NH ;
W représente oxygène ;
R¹ représente hydrogène ;
R², R³, indépendamment l'un de l'autre, représentent hydrogène ou alkyle en (C₁-C₃), plus particulièrement hydrogène ;
R⁵ représente halogène, NO₂, CN, alkyle en (C₁-C₃) qui est non substitué ou substitué par F, Cl, Br, CN, OCH₃ ou SCH₃ ; alcényle (en C₃) qui est non substitué ou substitué par F, Cl, ou Br ; alcynyle (en C₃), cycloalkyle (en C₃) qui est non substitué ou substitué par F, Cl ou CH₃, -C(O)R¹⁰, -OCH₂CH₂OR¹⁰, OH, -C(R¹⁰) (OR¹¹) (OR¹²), -CO₂R¹³, -C(O)NR¹⁴R¹⁵, N₃, -SO₂NR¹⁴R¹⁵, -OSO₂R¹⁷, -E¹R¹⁸ ou -(CH₂)ₛG ;
R⁶ représente hydrogène, halogène, CN, NO₂, CH₃, CF₃, -E¹R¹⁹ ou alcoxy en (C₁-C₂)-alkyle en (C₁-C₂), halogénoalcoxy en (C₁-C₂), alkylthio en (C₁-C₂), halogénoalkylthio en (C₁-C₂), CN, -CO₂R¹³ ou -SO₂NR¹⁴R¹⁵,
R⁷ représente hydrogène ;
R¹⁰ représente alkyle en (C₁-C₃), cyclopropyle ou alcényle en (C₃),
R¹¹, R¹² représentent alkyle en (C₁-C₂) ou R¹¹, R¹² ensemble représentent -CH₂CH₂- ;
R¹³ représente alkyle en (C₁-C₃), alcényle (en C₃), -CH₂CH₂F, -CH₂CH₂Cl, -CH₂CH₂OCH₃ ou cyclopropylméthyle ;
R¹⁴ représente hydrogène ou CH₃ ou R¹⁴ et R¹⁵ ensemble représentent -(CH₂)₄-, (CH₂)₅- ou -(CH₂)₂O(CH₂)₂- ;
R¹⁵ représente CH₃, CH₂CH₃, OCH₃ ou R¹⁴ et R¹⁵ ensemble représentent -(CH₂)₄-, -(CH₂)₅- ou -(CH₂)₂O(CH₂)₂- ;
R¹⁸ représente alkyle en (C₁-C₃), halogénoalkyle en (C₁-C₃), alcoxyalkyle en (C₂-C₃), allyle, propargyle, halogénoalkyle en (C₂-C₃) ;
R¹⁹ représente alkyle en (C₁-C₂) qui est non substitué ou substitué par F, Cl ou OCH₃ ;
R²⁰, R²¹, indépendamment l'un de l'autre, représentent hydrogène, alkyle en (C₁-C₂), alcoxy en (C₁-C₂), -CH₂OCH₃, Cl, F, Br, I, -CH₂OCH₂CH₃, -NHCH₃, -N(OCH₃)CH₃, -N(CH₃)₂, -CF₃, -SCH₃, -CH(OCH₃)₂, -OCH₂CH=CH₂ ; -OCH₂C≡CH, -OCH₂CH₂OCH₃, -CH₂SCH₃, -OCHF₂, -SCHF₂, cyclopropyle, -C≡CH ou -C≡C-CH₃ ;
m vaut 0 ou 1 ;
n vaut 0 ou 1 ;
s vaut zéro et
E¹ représente O ou S.

3. Procédé pour la préparation des composés de formule I selon la revendication 1 ou 2 ou de leurs sels, **caractérisé en ce que**
a) pour la préparation de composés avec W = 0, on fait réagir
a₁) un composé de formule II avec 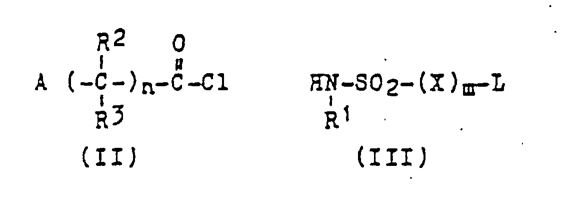 un composé de formule III en présence d'une base ou
on fait réagir
a₂) un composé de formule (IIa) 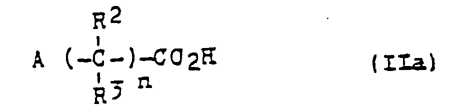 en présence de réactifs activants tels que le chlorure de 2-chloro-1-méthylpyridinium (IVa), le dicyclohexylcarbodiimide (IVb) ou le 1,1-carbonyldiimidazole (IVc) et éventuellement en présence d'une base avec un composé de formule III ou
a₃) pour la préparation de composés avec W = 0 et R¹ = H, on fait réagir un composé de formule (V), 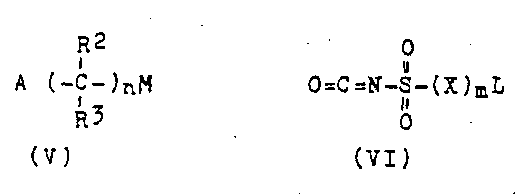 où M représente l'hydrogène ou le lithium, avec un composé de formule (VI), ou
b) pour la préparation de composés avec W = S, on fait réagir un composé de formule I obtenu dans a) avec le composé de formule VII 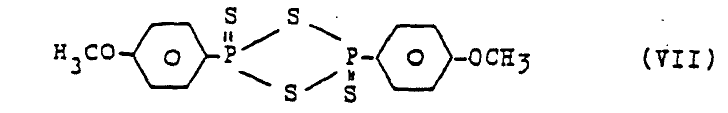
c) pour la préparation de composés avec W = NR⁴, on fait réagir un composé de formule VIII 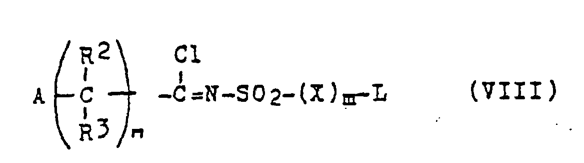 avec une amine de formule H₂N-R⁴,
d) pour la préparation de composés avec W = NOR⁴, on fait réagir
d₁) un composé de formule IX avec 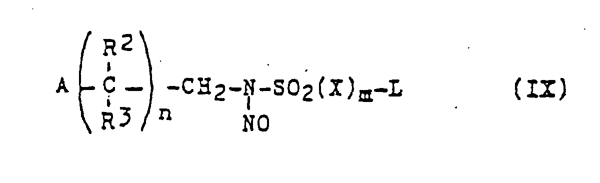 un hydroxyde alcalin ou alcalino-terreux ou l'hydroxyde d'ammonium ou
d₂) on fait réagir un composé de formule VIII en présence d'une base avec une hydroxylamine de formule H₂N-OR⁴, ou
e) pour la préparation de composés de formule I avec R¹ ≠ H, on fait réagir un composé de formule I avec R¹ = H en présence d'une base avec un halogénoalkyle de formule R^{1'}-X¹ où R^{1'} a la signification de R¹ sauf celle de l'hydrogène et X¹ représente le chlore, le brome ou l'iode et on transforme les composés obtenus éventuellement en leurs sels.

4. Procédé de préparation des composés de formule (II') 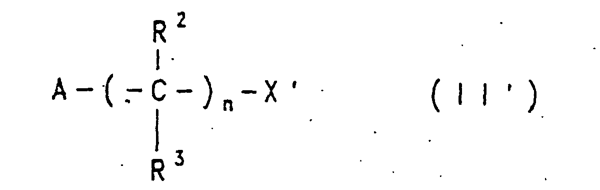 dans laquelle
X' représente COCl, CO₂R¹³ ou CO₂CH₂C₆H₅,
n vaut 0 ou 1,
R² et R³, l'un indépendamment de l'autre, représentent l'hydrogène, alkyle en C₁-C₃ ou phényle,
A représente un radical hétérocyclique de formule (A1), où r=0 et E² = CH, 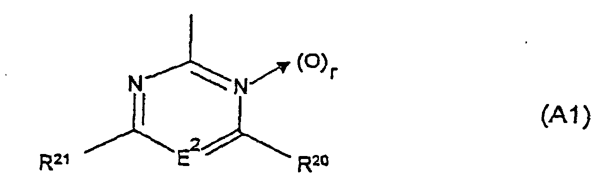 et
a vaut 0 ou 1
R¹⁰ représente l'hydrogène, alkyle en (C₁-C₄), alkyle en (C₁-C₄) qui est substitué une ou plusieurs fois par F, Cl, Br ou OCH₃ ; cycloalkyle en (C₃-C₆) qui est non substitué ou substitué une ou plusieurs fois par F, Cl, Br ; alcényle en (C₂-C₄) ou alcynyle en (C₂-C₄),
R¹³ représente l'hydrogène, alkyle en (C₁-C₄) qui est non substitué ou substitué une ou plusieurs fois par halogène ou une à deux fois par CN, CO₂R¹⁰, NR¹⁴R¹⁵, OR¹⁰ ou Si(CH₃)₃ ; alcynyle en (C₃-C₄) qui est non substitué ou substitué par CH₃ ou Si(CH₃)₃ ; cycloalkyle en (C₃-C₆), cycloalkyle en (C₃-C₆)-alkyle en (C₁-C₄), alcoxy en (C₁-C₄) ou Si(CH₃)₃,
R¹⁴ représente l'hydrogène ou alkyle en (C₁-C₄) ou R¹⁴ et R¹⁵ ensemble représentent -(CH₂)₂(CH₂)ₐ(CH₂)₂- ou - (CH₂)₂O(CH₂)₂-,
R¹⁵ représente l'hydrogène, alkyle en (C₁-C₄), alcoxy en (C₁-C₄), alcényle en (C₂-C₄) ou R¹⁴ et R¹⁵ ensemble représentent -(CH₂)₂(CH₂)ₐ(CH₂)₂- ou -(CH₂)₂O(CH₂)₂-, et
R²⁰, R²¹, indépendamment l'un de l'autre, représentent l'hydrogène, halogène, alkyle en (C₁-C₆), alcoxy en (C₁-C₆) ou thioalkyle en (C₁-C₆), les trois radicaux précités pouvant être substitués une ou plusieurs fois par halogène ou une ou deux fois par alcoxy en (C₁-C₄) ou thioalkyle en (C₁-C₄); -NR¹⁴R¹⁵, cycloalkyle en (C₃-C₆), -OCHR¹⁴CO₂R¹³, alcényle en (C₂-C₅), alcynyle en (C₂-C₄), alcényloxy en (C₃-C₅) ou alcynyloxy en (C₃-C₅),
où
1) dans le cas où n=0 et X'=COOH, la paire R²⁰/R²¹ ne représente pas CH₃/CH₃,
2) dans le cas où n=0 et X'=CO₂CH₃, la paire R²⁰/R²¹ ne représente pas CH₃/CH3 ou OCH₃/CH₃,
3) dans le cas où n=0 et X'=CO₂C₂H₅, la paire R²⁰/R²¹ ne représente pas OC₂H₅/OC₂H₅ ou Cl/Cl,
4) dans le cas où n=1, R² = R³ = H et X'=COOH, COONa ou COOCH₃, la paire R²⁰/R²¹ ne représente pas H/F, H/Cl, H/Br, H/I, H/CH₃, H/OCH₃, H/SC₂H₅, Cl/SC₂H₅, SC₂H₅/SC₂H₅, benzyloxy/CH₃, OCH₃/CH₃, OC₂H₅/CH₃, Cl/Cl, Cl/OCH₃, OCH₃/OCH3 ou Cl/CH₃,
5) dans le cas où n=1, R² = R³ = H et X'= COOC₂H₅, la paire R²⁰/R²¹ ne représente pas Cl/CH₃ ou CH₃/CH₃,
**caractérisé en ce qu'**on fait réagir un composé de formule (XI) 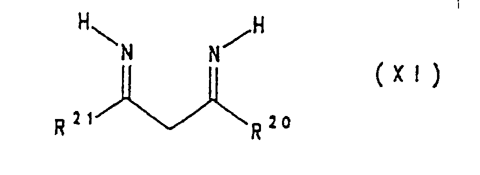 avec un composé de formule (XII) ou (XIII) 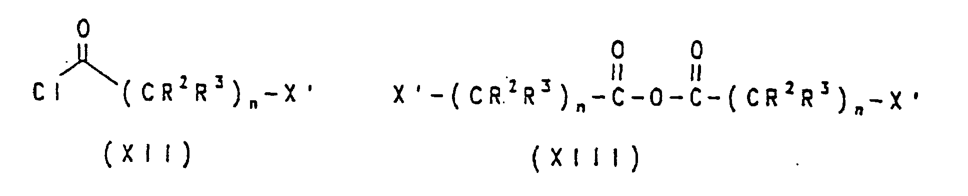 dans les formules (XI), (XII) et (XIII), les radicaux R², R³, R²⁰, R²¹ et X' ainsi que n étant définis comme dans la formule (II').

5. Procédé selon la revendication 4, **caractérisé en ce qu'**on met en oeuvre la réaction dans un solvant inerte dans les conditions de réaction en présence d'une base à une température de -78°C à 100°C.

6. Agents herbicides ou régulateurs de croissance de plantes, **caractérisés en ce qu'**ils contiennent un composé de formule I selon la revendication 1 ou 2 ou leur sel et des adjuvants inertes.

7. Procédé pour la lutte contre la végétation non désirée ou pour la régulation de la croissance de plantes, **caractérisé en ce qu'**on applique sur les plantes ou sur les sols cultivables une quantité efficace d'un composé de formule I ou de son sel selon la revendication 1 ou 2.
